(19) Europäisches Patentamt
European Patent Office
Office européen des brevets

(11) EP 2 327 704 A1

(12) EUROPEAN PATENT APPLICATION
published in accordance with Art. 153(4) EPC

(43) Date of publication:
**01.06.2011 Bulletin 2011/22**

(21) Application number: **09809909.6**

(22) Date of filing: **25.08.2009**

(51) Int Cl.:
***C07D 487/04*** *(2006.01)* ***A61K 31/4188*** *(2006.01)*
***A61K 31/422*** *(2006.01)* ***A61K 31/427*** *(2006.01)*
***A61K 31/428*** *(2006.01)* ***A61K 31/429*** *(2006.01)*
***A61K 31/437*** *(2006.01)* ***A61K 31/4439*** *(2006.01)*
***A61K 31/454*** *(2006.01)* ***A61K 31/497*** *(2006.01)*
***A61K 31/4985*** *(2006.01)* ***A61K 31/506*** *(2006.01)*
***A61K 31/53*** *(2006.01)* ***A61K 31/5377*** *(2006.01)*
***A61P 1/00*** *(2006.01)* ***A61P 1/02*** *(2006.01)*
***A61P 1/04*** *(2006.01)* ***A61P 1/06*** *(2006.01)*
***A61P 1/16*** *(2006.01)* ***A61P 1/18*** *(2006.01)*

(86) International application number:
**PCT/JP2009/064808**

(87) International publication number:
**WO 2010/024258 (04.03.2010 Gazette 2010/09)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **29.08.2008 JP 2008221935**

(71) Applicant: **Shionogi&Co., Ltd.**
**Osaka 541-0045 (JP)**

(72) Inventors:
- **MITSUOKA Yasunori**
  **Osaka-shi**
  **Osaka 553-0002 (JP)**
- **MASUDA Manami**
  **Osaka-shi**
  **Osaka 553-0002 (JP)**
- **TANIYAMA Daisuke**
  **Osaka-shi**
  **Osaka 553-0002 (JP)**

(74) Representative: **UEXKÜLL & STOLBERG**
**Patentanwälte**
**Beselerstrasse 4**
**22607 Hamburg (DE)**

# (54) RING-FUSED AZOLE DERIVATIVE HAVING PI3K-INHIBITING ACTIVITY

(57) It is an object of the present invention to provide a compound or a pharmaceutically acceptable salt thereof which inhibits the activity of PI3K to regulate many biological processes including the growth, differentiation, survival, proliferation, migration, metabolism, and the like of cells and is therefore useful for the prophylaxis/therapy of diseases including inflammatory diseases, arteriosclerosis, vascular/circulatory diseases, cancer/tumors, immune system diseases, cell proliferative diseases, infectious diseases, and the like. The above problem was solved by providing a ring-fused azole compound shown in the present specification, or a pharmaceutically acceptable salt thereof.

EP 2 327 704 A1

## Description

TECHNICAL FIELD

The present invention is related to: a compound that has phosphatidylinositol-3-kinase (hereinafter also referred to as "PI3K") inhibitory activity and is useful in the therapy/prophylaxis of a variety of phosphatidylinositol-3-kinase dependent diseases including cancers, inflammatory diseases, circulatory diseases, and the like; a salt thereof; or the like.

BACKGROUND ART

Phosphatidylinositol-3-kinase is an enzyme that catalyzes not only the production of a specific phospholipase, but also an intracellular mediatorfrom phosphatidylinositol (hereinafter also referred to as "PI") of a membrane lipid. The 3'-OH group of phosphatidylinositol is phosphorylated, and thus, when phosphatidylinositol, phosphatidylinositol 4-phosphate, and phosphatidylinositol 4,5-bisphosphate are used as substrates, phosphatidylinositol 3-phosphate, phosphatidylinositol 3,4-bisphosphate, and phosphatidylinositol 3,4,5-triphosphate (PIP3) are produced respectively.

A phospholipid (PIP3) in which the hydroxyl group at the 3-position of the inositol ring is phosphorylated by this PI3K works as a second messenger that activates a serine/threonine kinase such as PDK1, Akt/PKB, and the like in a signal transduction route mediated by receptor stimulation. This second messenger is said to regulate many biological processes including growth, differentiation, survival, proliferation, migration and metabolism, and the like of cells.

PI3Ks are classified into three groups (i.e. Classes I to III) by primary structure, regulatory mechanism of activity, and specificity to a substrate. Among these, Class I is important in signaling.

Depending on the differences in the heterodimer, Class I is classified into IA (α, β, and δ), containing a subunit of 85 kDa, and IB (γ), containing a subunit of 101 kDa.

Class IA is associated with a variety of cell surface receptors such as hormones/growth factors and the like. For a signal transduction route, it is said to be a protein/kinase receptor type. Class IB is associated with a G protein receptor (GCPR), which is a receptor for chemokine and the like. Furthermore, it is said that when a specific tyrosine residue of a receptor is phosphorylated by stimulation of an activator such as a chemokine and the like, a regulatory subunit is bound to a catalytic subunit via the SH2 domain, and thereby the inhibitory activity of the regulatory subunit is reduced to exhibit enzyme activity.

PIP3 works as a messenger in intracellular signaling. In the immediate downstream of PIP3, AKT (also known as protein kinase B (PKB)) and the like are known. It is said that in the downstream route thereof, a functional protein having the PH domain is activated and thereby, a signal is transmitted.

PI3Kα and PI3Kβ are widely distributed in a variety of cells and related to cell growth/glycometabolism. Based on these actions, inhibitors of PI3Kα and PI3Kβ are utilized as anticancer agents and the like. PI3Kδ and PI3Kγ exist mainly in blood and cells of the immune (lymphatic) system. PI3Kγ is also known to be widely distributed in inflammatory cells.

Regarding PI3Kγ, on the basis of studies of knock-out mice thereof and the like, it was found that respiratory burst of a neutrophil by a chemotactic factor and the migration of a macrophage/neutrophil to an infection focus were blocked, functions of T cells/dendritic cells were thereby decreased, the degranulation of mast cells was thereby blocked, and anaphylaxis was thereby decreased. Accordingly, an inhibitor of PI3Kγ is considered useful as a therapeutic agent for these diseases. Furthermore, on the basis of studies of arthritis, it is considered useful as an inhibitor of the inflammatory-cell infiltration in a part of a joint (Non-patent Documents 1 and 2). Furthermore, studies using a PI3Kγ inhibitor report the inhibition of the activation of a mast cell (Non-patent Document 3), the inhibition of the activation/migration of a leukocyte (Non-patent Documents 4 and 5), the inhibition of lymphocyte activation (Non-patent Document 6), and the like.

On the basis of these studies, a PI3Kγ inhibitor is believed to be useful in the therapy of the following diseases/disorders: thrombus; allergy/anaphylaxis (allergic diseases include, for example, asthma, atopic dermatitis, allergic rhinitis, and the like); inflammation such as pancreatitis (Non-patent Document 7), pneumonia, airway inflammation, chronic obstructive pulmonary disease (COPD) (Non-patent Document 8 and Non-patent Document 9), arthritis (e.g., articular rheumatism (Non-patent Document 8 and 9), glomerulonephritis, and the like; systemic lupus erythematosus (SLE) (Non-patent Document 8 and Non-patent Document 9); autoimmune diseases; pulmonary disorder; circulatory diseases such as heart failure (systolic), cardiac ischemia (systolic), high blood pressure, and the like (Non-patent Document 10); wound healing; infectious diseases (Non-patent Document 11); cancer/tumors such as neoplasm (Patent Document 1) ; suppression of the immune reaction in organ transplantation and autoimmune diseases (Patent Document 2); and the like.

Regarding PI3Kδ, on the basis of studies of knock-out mice thereof and the like, the B cell differentiation disorder of bone marrow is induced and in immunomodulation, the role thereof is expected.

PI3K is reported to be deeply involved in various stages of articular rheumatism, such as: the T cell/B cell

activation by presenting an antigen; the inflammatory cell infiltration such as neutrophil, macrophage, or the like; the synovial cell proliferation; the mast cell activation; and the like (Non-Patent Document 12).

As examples of compounds that have PI3 kinase inhibitory activity, classically, wortmannin (Non-Patent Document 13), 2-(4-morpholinyl)-8-phenyl-4H-1-benzopyran-4-one (Patent Document 2), 17β-hydroxywortmannin and a derivative thereof (Patent Document 1), and the like are known.

Patent Documents 3, 4, 5, 6, 7, 13, and 14 disclose thiazole derivatives having PI3K inhibitory activity. However, each of them is a tricyclic derivative of which the thiazole ring is fused with a carbocycle and a heterocycle. They do not disclose a bicyclic derivative of which the thiazole ring is fused with a heterocycle, such as a compound of the present invention.

Patent Documents 8, 9, 10, 11, and 12 disclose bicyclic derivatives of which the thiazole ring is fused with a heterocycle. However, none of them discloses PI3K inhibitory activity thereof.

PRIOR ART REFERENCES

[Patent Document]

[Patent Document 1] Japanese Laid-Open Publication No. 7-145051
[Patent Document 2] International Publication No. 95/29673 pamphlet
[Patent Document 3] International Publication No. 2006/040279 pamphlet
[Patent Document 4] International Publication No. 2007/115929 pamphlet
[Patent Document 5] International Publication No. 2007/115933 pamphlet
[Patent Document 6] International Publication No. 2007/115930 pamphlet
[Patent Document 7] International Publication No. 2007/115931 pamphlet
[Patent Document 8] International Publication No. 2006/053323 pamphlet
[Patent Document 9] International Publication No. 2005/066145 pamphlet
[Patent Document 10] International Publication No. 96/04271 pamphlet
[Patent Document 11] International Publication No. 92/07849 pamphlet
[Patent Document 12] United States Patent No. 4289524 Specification
[Patent Document 13] International Publication No. 2007/115932 pamphlet
[Patent Document 14] International Publication No. 2006/040281 pamphlet


[Non-patent document]

[Non-patent document 1] M. P. Wymann, et al., Biochemical Society Transactions 2003, 31, pp.275-280
[Non-patent document 2] Rueckle T. et al., NATURE REVIEWS DRUG DISCOVERY 2006, 5, pp.903-918
[Non-patent document 3] Laffargue M. et al., Immunity 2002 16: pp.441-451
[Non-patent document 4] Hirsch E. et al., Science 2000 287: pp.1049-1053
[Non-patent document 5] Li Z. et al., Science 2000 287; pp.982-983
[Non-patent document 6] Sasaki T. et al., Science 2000 287; pp.1040-1046
[Non-patent document 7] Lupia E. et al., Am J Pathol. 2004; 165, pp.2003-2011
[Non-patent document 8] Barber DF et al., Nat Med 2005 11: pp.933-935
[Non-patent document 9] Camps, Nat Med 2005 11: pp.936-943
[Non-patent document 10] Campbell et al., Circ Res. 2005, 96, pp.197-206
[Non-patent document 11] Yadav M. et al., J Immunol. 2006, 176, pp.5494-503
[Non-patent document 12] Japanese Journal of Clinical Immunology, Vol.30, 2007, 5, pp.369-374
[Non-patent document 13] Ui MT et al., Trends Biochem. Sci., 1995, 20, pp.303-307


SUMMARY OF THE INVENTION

PROBLEMS TO BE SOLVED BY THE INVENTION

It is an object of the present invention to provide ring-fused azole derivatives or a pharmaceutically acceptable salt thereof which inhibit the activity of PI3K to regulate many biological processes including the growth, differentiation, survival, proliferation, migration, metabolism, and the like of cells, and are therefore useful for the prophylaxis/therapy

of diseases including inflammatory diseases (allergic diseases (allergic dermatitis/allergic rhinitis, and the like), rheumatism arthritisarticular rheumatism, anaphylaxis, and the like), arteriosclerosis, vascular/circulatory diseases, cancer/tumors, immune system diseases, cell-proliferative diseases, infectious diseases, and the like.

[Means for Solving Problem]

Accordingly, for example, the present invention provides the following items:
(1) A compound represented by formula (I):
[Formula 1]

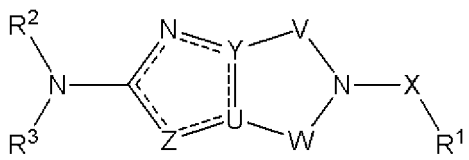

(I)

wherein
V is $-(CR^4R^5)_m-$ or $-CR^6=CR^7-$;
W is a single bond, $-(CR^8R9)_n-$, or -C(=O)-;
X is a single bond, -C(=O)-, $-(CR^{10}R^{11})_p-$, $-(CR^{12}R^{13})_p-C(=O)-$, $-SO_2-$, or -SO-;
a group represented by formula (G):
[Formula 2]

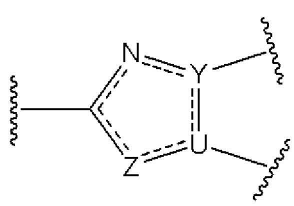

(G)

is selected from the following:
[Formula 3]

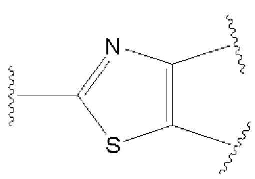

(G1) ,

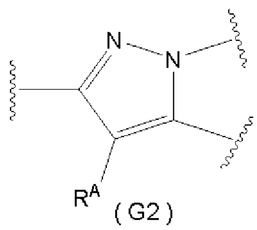

(G2) ,

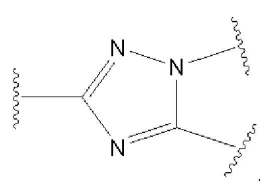

(G3) ,

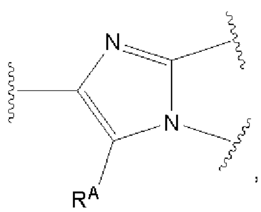

(G4) ,

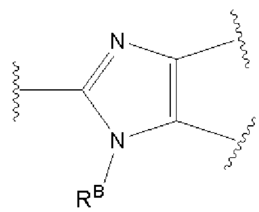

(G5) or

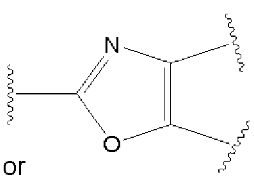

(G6) ,

;

$R^A$ is hydrogen, halogen, cyano, hydroxy, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted alkoxy, substituted or unsubstituted aryloxy, substituted or unsubstituted cycloalkyloxy, substituted or unsubstituted heteroaryloxy, substituted or unsubstituted heterocyclyloxy, substituted or unsubstituted acyl, substituted or unsubstituted carbamoyl, substituted or unsubstituted amino, a group represented by the formula: $-SO-R^c$, a group represented by the formula: $-SO_2R^C$, or a group represented by the formula: $-SR^C$;

$R^B$ is hydrogen, substituted or unsubstituted alkyl, substituted or unsubstituted carbamoyl, or substituted or unsubstituted acyl;

$R^C$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^1$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl;

$R^2$ is hydrogen, substituted or unsubstituted alkyl, or substituted or unsubstituted acyl;

$R^3$ is hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-C(=O)-R^{14}$, or a group represented by the formula: $-C(=O)-NR^{15}R^{16}$; or

$R^2$ and $R^3$ may be taken together with the adjacent nitrogen atom to form a substituted or unsubstituted nitrogenated heterocycle;

$R^4$ to $R^{13}$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;

each $R^{14}$ is independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted acyl, or substituted or unsubstituted carbamoyl;

$R^{15}$ and $R^{16}$ are each independently hydrogen, substituted or unsubstituted alkyl, substituted or unsubstituted alkenyl,

substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl, or $R^{15}$ and $R^{16}$ may be taken together with the adjacent nitrogen atom to form a substituted or unsubstituted nitrogenated heterocycle;

m, n, and p are each independently an integer from 1 to 3; provided that when X is a single bond, $R^1$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, or substituted or unsubstituted heterocyclyl, and

when a group represented by formula (G) is the group represented by formula (G1),

(i) $R^3$ is a group representedby the formula: -C(=O)-$R^{14}$ wherein $R^{14}$ is defined as above; X is a single bond; $R^1$ is substituted or unsubstituted aryl, substituted or unsubstituted cycloalkyl, substituted or unsubstituted cycloalkenyl, substituted or unsubstituted heteroaryl, or substituted or unsubstituted heterocyclyl; or

(ii) $R^3$ is a group represented by the formula: -C(=O)-$NR^{15}R^{16}$ wherein $R^{15}$ and $R^{16}$ are defined as above; and provided that the compounds represented by the following formulae are excluded:

[Formula 4]

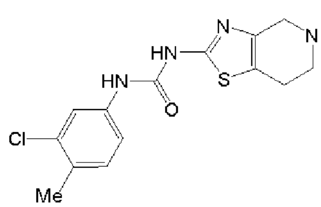
,
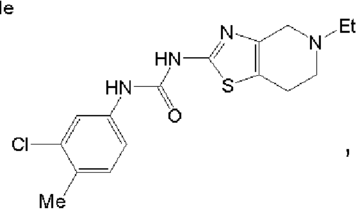
,
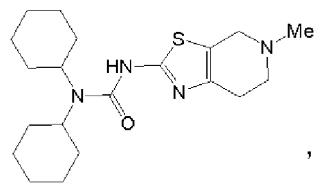
,
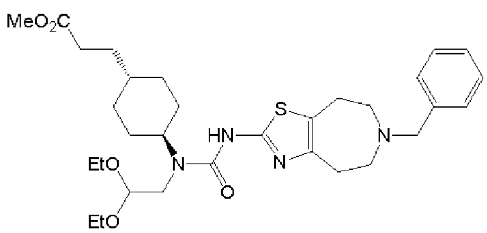
,
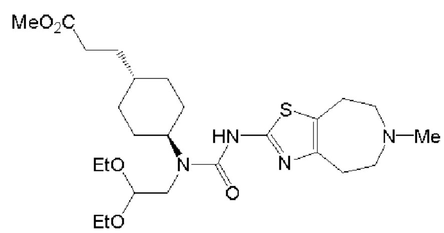
,
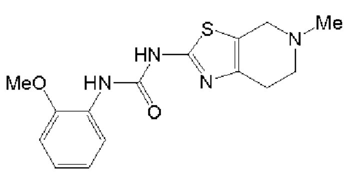
and
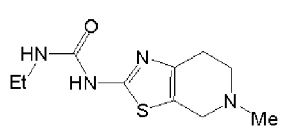
,

or a pharmaceutically acceptable salt thereof, or a solvate thereof.

(2) The compound according to the preceding item (1), wherein a group represented by formula (G) is the group represented by formula (G1) or (G2); $R^3$ is substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: -C(=O)-$R^{14}$ wherein $R^{14}$ is defined as in item (1), or a group represented by the formula: -C(=O)-$NR^{15}R^{16}$ wherein $R^{15}$ and $R^{16}$ are defined as in item (1), or a pharmaceutically acceptable salt thereof, or a solvate thereof.

(3) The compound according to the preceding item (1) or (2), wherein a group represented by formula (G) is the group represented by formula (G1),

or a pharmaceutically acceptable salt thereof, or a solvate thereof.

(4) The compound according to the preceding item (1) or (2), wherein a group represented by formula (G) is the group represented by formula (G2),
or a pharmaceutically acceptable salt thereof, or a solvate thereof.

(5) The compound according to the preceding item (1) or (4), wherein $R^A$ is hydrogen,
or a pharmaceutically acceptable salt thereof, or a solvate thereof.

(6) The compound according to any of the preceding items (1) to (5), wherein V is - $(CR^4R^5)_m$- wherein $R^4$, $R^5$, and m are defined as in item (1) ; and W is -$(CR^8R^9)_n$- wherein $R^8$, $R^9$, and n are defined as in item (1),
or a pharmaceutically acceptable salt thereof, or a solvate thereof.

(7) The compound according to any of the preceding items (1) to (5), wherein V is -$CR^6$=$CR^7$- wherein $R^6$ and $R^7$ are defined as in item (1) ; and W is a single bond,
or a pharmaceutically acceptable salt thereof, or a solvate thereof.

(8) The compound according to any of the preceding items (1) to (5), wherein V is - $(CR^4R^5)_m$- wherein $R^4$, $R^5$, and m are defined as in item (1) ; and W is a single bond or -$(CR^8R^9)_n$-wherein $R^8$, $R^9$, and n are defined as in item (1),
or a pharmaceutically acceptable salt thereof, or a solvate thereof.

(9) The compound according to any of the preceding items (1) to (6) and (8), wherein $R^4$, $R^5$, $R^8$, and $R^9$ are each hydrogen; m is 2; and n is 1,
or a pharmaceutically acceptable salt thereof, or a solvate thereof.

(10) The compound according to any of the preceding items (1) to (5) and (7), wherein $R^6$ and $R^7$ are each hydrogen, or a pharmaceutically acceptable salt thereof, or a solvate thereof.

(11) The compound according to any of the preceding items (1) to (10), wherein X is a single bond or -C(=O)-,
or a pharmaceutically acceptable salt thereof, or a solvate thereof.

(12) The compound according to any of the preceding items (1) to (11), wherein $R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino, or a pharmaceutically acceptable salt thereof, or a solvate thereof.

(13) The compound according to any of the preceding items (1) to (12), wherein $R^2$ is hydrogen,
or a pharmaceutically acceptable salt thereof, or a solvate thereof.

(14) The compound according to any of the preceding items (1) to (13), wherein $R^3$ is a group represented by the formula: -C(=O)-$NR^{15}R^{16}$ wherein $R^{15}$ and $R^{16}$ are defined as in item (1), or a pharmaceutically acceptable salt thereof, or a solvate thereof.

(15) The compound according to any of the preceding items (1) to (14), wherein $R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl; or $R^{15}$ and $R^{16}$ are taken together with the adjacent nitrogen atom to form a substituted or unsubstituted nitrogenated heterocycle,
or a pharmaceutically acceptable salt thereof, or a solvate thereof.

(16) The compound according to any of the preceding items (1) to (13), wherein $R^3$ is a group represented by the formula: -C(=O)-$R^{14}$ wherein $R^{14}$ is defined as in item (1),
or a pharmaceutically acceptable salt thereof, or a solvate thereof.

(17) The compound according to any of the preceding items (1) to (13) and (16), wherein $R^{14}$ is substituted or unsubstituted alkyl, substituted or unsubstituted aryl, substituted or unsubstituted heteroaryl, substituted or unsubstituted heterocyclyl, substituted or unsubstituted cycloalkyl, substituted or unsubstituted alkoxy, or substituted or unsubstituted acyl,
or a pharmaceutically acceptable salt thereof, or a solvate thereof.

(18) A pharmaceutical composition containing the compound according to any of the preceding items (1) to (17), or a pharmaceutically acceptable salt thereof, or a solvate thereof.

(19) The pharmaceutical composition according to the preceding item (18), which is a phosphatidylinositol-3-kinase inhibitor.

(20) The pharmaceutical composition according to any of the preceding items (18) and (19), which is a therapeutic agent and/or a prophylactic agent for inflammation.

(21) Use of the compound, or a pharmaceutically acceptable salt thereof, or a solvate thereof according to any of the preceding items (1) to (17) for producing a therapeutic agent and/or a prophylactic agent for inflammation.

(22) The compound, or a pharmaceutically acceptable salt thereof, or a solvate thereof according to any of the preceding items (1) to (17) for the therapy and/or prophylaxis of inflammation.

(23) A method for the therapy and/or prophylaxis of inflammation, characterized by administering the compound, or a pharmaceutically acceptable salt thereof, or a solvate thereof according to any of the preceding items (1) to (17).

(24) A method for the therapy and/or prophylaxis of inflammation, characterized by administering the pharmaceutical composition having PI3K inhibitory activity according to any of the preceding items (18) and (19).

(25) A phosphatidylinositol-3-kinase inhibitor containing the compound, or a pharmaceutically acceptable salt

thereof, or a solvate thereof according to any of the preceding items (1) to (17) as an active ingredient.

(26) The inhibitor according to the preceding item (25), which may be specific to one or more types of α, β, γ, and δ phosphatidylinositol-3-kinase inhibitors.

(27) The pharmaceutical composition according to the preceding item (18) or (19) for the treatment of the following phosphatidylinositol-3-kinase dependent diseases: encephalitis, myelitis and encephalomyelitis, meningitis, inflammatory polyneuropathy, neuritis, dacryoadenitis, orbital inflammation, conjunctivitis (allergic conjunctivitis, vernalkeratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or burn, traumatic inflammation, and the like.

(28) A protein kinase B (AKT) inhibitor containing the compound, or a pharmaceutically acceptable salt thereof, or a solvate thereof according to any of the preceding items (1) to (17).

(29) An anticancer agent containing the compound, or a pharmaceutically acceptable salt thereof, or a solvate thereof according to any of the preceding items (1) to (17).

(30) An anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound, or a pharmaceutically acceptable salt thereof, or a solvate thereof according to any of the preceding items (1) to (17).

(31) An antiallergic agent (asthma, atopic dermatitis, allergic rhinitis, and the like) containing the compound, or a pharmaceutically acceptable salt thereof, or a solvate thereof according to any of the preceding items (1) to (17).

(32) A therapeutic agent for immune system diseases wherein the therapeutic agent contains the compound, or a pharmaceutically acceptable salt thereof, or a solvate thereof according to any of the preceding items (1) to (17).

(33) An immunosuppressant containing the compound, or a pharmaceutically acceptable salt thereof, or a solvate thereof according to any of the preceding items (1) to (17).

(34) A therapeutic agent for autoimmune diseases wherein the therapeutic agent contains the compound, or a pharmaceutically acceptable salt thereof, or a solvate thereof according to any of the preceding items (1) to (17).

(35) An anti-circulatory-disease agent (such as antihypertensive agent and the like) wherein the agent contains the compound, or a pharmaceutically acceptable salt thereof, or a solvate thereof according to any of the preceding items (1) to (17).

(36) An antiinfectant containing the compound, or a pharmaceutically acceptable salt thereof, or a solvate thereof according to any of the preceding items (1) to (17).

(37) A wound-healing agent containing the compound, or a pharmaceutically acceptable salt thereof, or a solvate thereof according to any of the preceding items (1) to (17).

(38) A method, a system, an apparatus, a kit, and the like for producing the compound, or a pharmaceutically acceptable salt thereof, or a solvate thereof according to any of the preceding items (1) to (17).

(39) A method, a system, an apparatus, a kit, and the like for preparing a pharmaceutical composition containing the compound, or a pharmaceutically acceptable salt thereof, or a solvate thereof according to any of the preceding items (1) to (17).

(40) A method, a system, an apparatus, a kit, and the like using the compound, or a pharmaceutically acceptable salt thereof, or a solvate thereof according to any of the preceding items (1) to (17).

In one aspect, the present invention relates to a compound represented by formula (I):

[Formula 5]

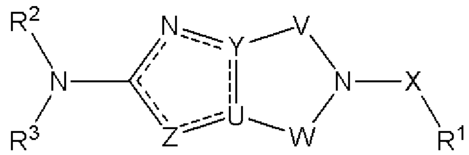

(I)

wherein

V is $-(CR^4R^5)_m-$ or $-CR^6=CR^7-$;

W is a single bond, $-(CR^8R_9)_n-$, or -C(=O)-;

X is a single bond, -C(=O)-, $-(CR^{10}R^{11})_p-$, $-(CR^{12}R^{13})_p-C(=O)-$, $-SO_2-$, or -SO-;

a group represented by formula (G):

[Formula 6]

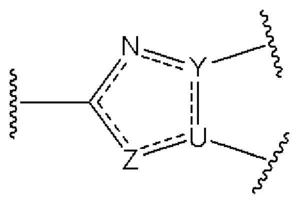

(G)

is selected from the following:

[Formula 7]

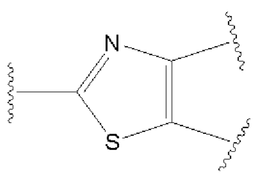

,

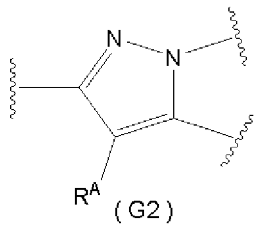

(G1) (G2)

,

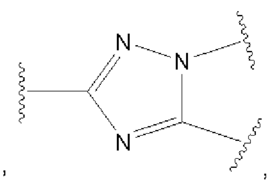

,

(G3)

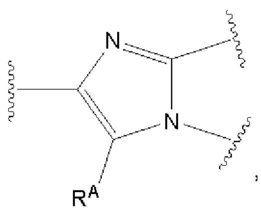

,

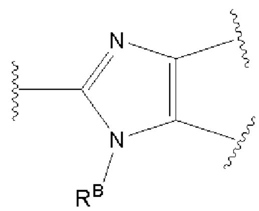

or

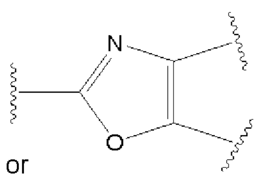

,

(G4) (G5) (G6) ;

$R^A$ is hydrogen, halogen, cyano, hydroxy, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted alkoxy, substituted or unsubstituted aryloxy, substituted or unsubstituted cycloalkyloxy, substituted or unsubstituted heteroaryloxy, substituted or unsubstituted heterocyclyloxy, substituted or unsubstituted acyl, substituted or unsubstituted carbamoyl, substituted or unsubstituted amino, a group represented by the formula: $-SO-R^C$, a group represented by the formula: $-SO_2R^C$, or a group represented by the formula: $-SR^C$;

$R^B$ is hydrogen, substituted or unsubstituted alkyl, substituted or unsubstituted carbamoyl, or substituted or unsubstituted acyl;

$R^C$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^1$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl;

$R^2$ is hydrogen, substituted or unsubstituted alkyl, or substituted or unsubstituted acyl;

$R^3$ is hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-C(=O)-R^{14}$, a group represented by the formula: $-C(=O)-NR^{15}R^{16}$; or $R^2$ and $R^3$ may be taken together with the adjacent nitrogen atom to form a substituted or unsubstituted nitrogenated heterocycle;

$R^4$ to $R^{13}$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;

each $R^{14}$ is independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl;

$R^{15}$ and $R^{16}$ are each independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted

or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl, or $R^{15}$ and $R^{16}$ may be taken together with the adjacent nitrogen atom to form a substituted or unsubstituted nitrogenated heterocycle;

m, n, and p are each independently an integer from 1 to 3; provided that when X is a single bond, $R^1$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, or substituted or unsubstituted heterocyclyl,

when a group represented by formula (G) is the group represented by formula (G1), (i) $R^3$ is a group representedby the formula: -C(=O)-$R^{14}$ wherein $R^{14}$ is defined as in item (1) ; X is a single bond; $R^1$ is substituted or unsubstituted aryl, substituted or unsubstituted cycloalkyl, substituted or unsubstituted cycloalkenyl, substituted or unsubstituted heteroaryl, or substituted or unsubstituted heterocyclyl, or (ii) $R^3$ is a group represented by the formula: -C(=O)-$NR^{15}R^{16}$ wherein $R^{15}$ and $R^{16}$ are defined as in item (1) ; and provided that the compounds represented by the following formulae are excluded:

[Formula 8]

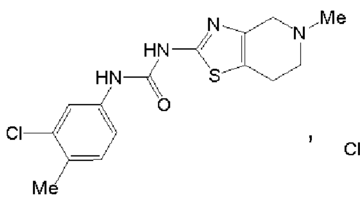
,
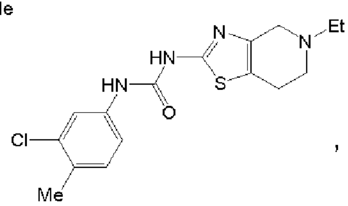
,
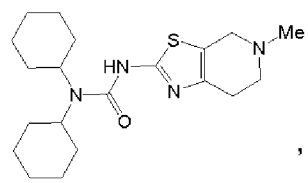
,
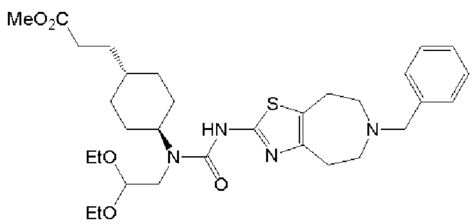
,
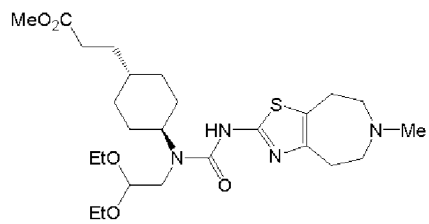
,
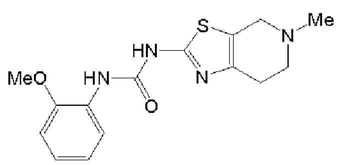
and
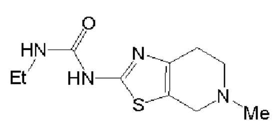
,

or a pharmaceutically acceptable salt thereof, or a solvate thereof.

In one embodiment, a group represented by formula (G) is the formula represented by formula (G1) or (G2), and $R^3$ is substituted or unsubstituted aryl, substituted or unsubstituted cycloalkyl, substituted or unsubstituted cycloalkenyl, substituted or unsubstituted heteroaryl, substituted or unsubstituted heterocyclyl, a group of the formula: -C(=O)-$R^{14}$ wherein $R^{14}$ is defined as in item (1), or a group represented by the formula: -C(=O)-$NR^{15}R^{16}$ wherein $R^{15}$ and $R^{16}$ are defined as in item (1).

In one embodiment, a group represented by formula (G) is the group represented by formula (G1).

In one embodiment, a group represented by formula (G) is the group represented by formula (G2).

In one embodiment, $R^A$ is hydrogen.

In one embodiment, V is - $(CR^4R^5)_m$- wherein $R^4$, $R^5$, and m are defined as in item (1), and W is -$(CR^8R^9)_n$- wherein $R^8$, $R^9$, and n are defined as in item (1).

In one embodiment, V is -$CR^6$=$CR^7$- wherein $R^6$ and $R^7$ are defined as in item (1), and W is a single bond.

In one embodiment, V is -$(CR^4R^5)_m$- wherein $R^4$, $R^5$, and m are defined as in item (1) ; and W is a single bond or -$(CR^8R^9)_n$-wherein $R^8$, $R^9$, and n are defined as in item (1).

In one embodiment, $R^4$, $R^5$, $R^8$, and $R^9$ are each hydrogen; m is 2; and n is 1.

In one embodiment, $R^6$ and $R^7$ are each hydrogen.

In one embodiment, X is a single bond or -C(=O)-.

In one embodiment, $R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino.

In one embodiment, $R^2$ is hydrogen.

In one embodiment, $R^3$ is a group represented by the formula: $-C(=O)-NR^{15}R^{16}$ wherein $R^{15}$ and $R^{16}$ are defined as in item (1).

In one embodiment, $R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl; or $R^{15}$ and $R^{16}$ are taken together with the adjacent nitrogen atom to form a substituted or unsubstituted nitrogenated heterocycle.

The present invention includes compounds shown by combining a part or all of the above-described embodiments.

In embodiments of the present invention, Y includes a carbon atom and a nitrogen atom. U includes a carbon atom and a nitrogen atom. Z includes a sulfur atom, a carbon atom, a nitrogen atom, and an oxygen atom. 5-membered rings containing Y, U, and Z include (G1) to (G6) described above.

In an embodiment of the present invention, when pluralities of $R^4$, $R^5$, $R^8$, $R^9$, $R^{10}$, $R^{11}$, $R^{12}$, and $R^{13}$ are present, they may be the same or different.

In one embodiment, the present invention relates to a pharmaceutical composition containing the compound, or a pharmaceutically acceptable salt thereof, or a solvate thereof.

In one embodiment, the pharmaceutical composition is a phosphatidylinositol-3-kinase inhibitor.

In one embodiment, the pharmaceutical composition is a therapeutic agent and/or a prophylactic agent for inflammation.

In another aspect, the present invention relates to use of the above-described compound, or a pharmaceutically acceptable salt thereof, or a solvate thereof for producing a therapeutic agent and/or a prophylactic agent for inflammation.

In one embodiment, the present invention relates to the above-described compound, or a pharmaceutically acceptable salt thereof, or a solvate thereof for the therapy and/or prophylaxis of inflammation.

In another aspect, the present invention relates to a method for the therapy and/or prophylaxis of inflammation, the method being characterized by administering the above-described compound, or a pharmaceutically acceptable salt thereof, or a solvate thereof.

In another aspect, the present invention relates to a phosphatidylinositol-3-kinase inhibitor containing the compound of the present invention, or a pharmaceutically acceptable salt thereof, or a solvate thereof as an active ingredient.

In one embodiment, the inhibitor of the present invention may be specific to one or more types of $\alpha$, $\beta$, $\gamma$, and $\delta$ phosphatidylinositol-3-kinase inhibitors.

In pharmaceutical aspect, in a preferred embodiment, the pharmaceutical composition of the present invention may be a composition for the treatment of phosphatidylinositol-3-kinase dependent diseases. Such phosphatidylinositol-3-kinase dependent diseases can include: encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary disease, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis,

iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or burn, traumatic inflammation, and the like.

In an embodiment, the present invention relates to a phosphatidylinositol-3-kinase inhibitor containing the compound of the invention, or a pharmaceutically acceptable salt thereof, or a solvate thereof.

In an embodiment, the present invention relates to a protein kinase B (AKT) inhibitor containing the compound of the invention, or a pharmaceutically acceptable salt thereof, or a solvate thereof.

In an embodiment, the present invention relates to an anticancer agent containing the compound of the invention, or a pharmaceutically acceptable salt thereof, or a solvate thereof.

In an embodiment, the present invention relates to an anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound of the invention, or a pharmaceutically acceptable salt thereof, or a solvate thereof.

In an embodiment, the present invention relates to an antiallergic agent (asthma, atopic dermatitis, allergic rhinitis, and the like) containing the compound of the invention, or a pharmaceutically acceptable salt thereof, or a solvate thereof.

In an embodiment, the present invention relates to a therapeutic agent for immune system diseases wherein the therapeutic agent contains the compound of the invention, or a pharmaceutically acceptable salt thereof, or a solvate thereof.

In an embodiment, the present invention relates to an immunosuppressant containing the compound of the invention, or a pharmaceutically acceptable salt thereof, or a solvate thereof.

In an embodiment, the present invention relates to a therapeutic agent for autoimmune diseases wherein the therapeutic agent contains the compound of the invention, or a pharmaceutically acceptable salt thereof, or a solvate thereof.

In an embodiment, the present invention relates to an anti-circulatory-disease agent (such as antihypertensive agent and the like) wherein the agent contains the compound of the invention, or a pharmaceutically acceptable salt thereof, or a solvate thereof.

In an embodiment, the present invention relates to an antiinfectant containing the compound of the invention, or a pharmaceutically acceptable salt thereof, or a solvate thereof.

In an embodiment, the present invention relates to a wound-healing agent containing the compound of the invention, or a pharmaceutically acceptable salt thereof, or a solvate thereof.

The present invention also relates to a method, a system, an apparatus, a kit, and the like for producing the compound of the invention, or a pharmaceutically acceptable salt thereof, or a solvate thereof.

The present invention also relates to a method, a system, an apparatus, a kit, and the like for preparing a pharmaceutical composition containing the compound of the invention, or a pharmaceutically acceptable salt thereof, or a solvate thereof.

The present invention also relates to a method, a system, an apparatus, a kit, and the like using the compound of the invention, or a pharmaceutically acceptable salt thereof, or a solvate thereof.

Thus, these and other advantages of the present invention are apparent when the following detailed description is read.

## EFFECT OF THE INVENTION

The present invention provides a medicament for the treatment of phosphatidylinositol-3-kinase dependent diseases; a compound used therefor; or a pharmaceutically acceptable salt thereof; or a solvate thereof. The compound of the present invention exhibits excellent PI3K inhibitory activity as described in Examples below. Furthermore, the compound of the present invention encompasses compounds exhibiting PI3K$\alpha$ and $\gamma$ inhibitory activity. Accordingly, the pharmaceutical composition of the present invention may be used for the prophylaxis and/or as a therapeutic agent for diseases such as encephalitis, myelitis and encephalomyelitis, meningitis, inflammatorypolyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The compound of the present invention is a compound having utility as a medicament. Here, utility as a medicament includes the following points: the compound has good metabolic stability; the induction of a drug-metabolizing enzyme is low; the inhibition of a drug-metabolizing enzyme which metabolizes another drug is also low; the compound has high oral absorbency; the clearance is low; the half-life is sufficiently long to express the efficacy; or the like.

## MODE FOR CARRYING OUT THE INVENTION

Hereinafter, the present invention is described with reference to embodiments. It should be understood that, throughout the present specification, the expression of a singular form includes the concept of its plural form unless specified otherwise. Accordingly, it should be understood that an article in singular form (for example, in the English language, "a, " "an, " "the, " and the like) includes the concept of its plural form unless specified otherwise. Furthermore, it should be understood that the terms used herein are used in a meaning normally used in the art unless specified otherwise. Thus, unless defined otherwise, all technical and scientific terms used herein have the same meaning as those generally understood by those skilled in the art in the field to which the present invention pertains. If there is a contradiction, the present specification (including definitions) precedes.

Each meaning of terms used herein is described below. In the present specification, each term is used in an unequivocal meaning. Both when used alone and in combination with another word, each term is used in the same meaning.

As used herein, the term "halogen" means fluorine, chlorine, bromine, and iodine. Examples thereof include fluorine, chlorine, and bromine.

As used herein, the term "alkyl" encompasses a linear or branched monovalent hydrocarbon group having 1 to 8 carbon atoms. Examples thereof include methyl, ethyl, *n*-propyl, isopropyl, *n*-butyl, isobutyl, *sec*-butyl, *tert*-butyl, *n*-pentyl, isopentyl, *neo*-pentyl, *n*-hexyl, isohexyl, *n*-heptyl, *n*-octyl, and the like. An example is C1-C6 alkyl. Another example is C1-C4 alkyl. When the carbon number is specified in particular, an "alkyl" having carbon in a range of the number is meant.

As used herein, the term "alkenyl" encompasses a linear or branched monovalent hydrocarbon group having 2 to 8 carbon atoms and one or more double bonds. Examples thereof include vinyl, allyl, 1-propenyl, 2-butenyl, 2-pentenyl, 2-hexenyl, 2-heptenyl, 2-octenyl, and the like. An example is C2-C6 alkenyl. Another example is C2-C4 alkenyl.

As used herein, the term "alkynyl" encompasses a linear or branched monovalent hydrocarbon group having 2 to 8 carbon atoms and one or more triple bonds. Examples thereof include ethynyl, 1-propynyl, 2-propynyl, 2-butynyl, 2-pentynyl, 2-hexynyl, 2-heptynyl, 2-octynyl, and the like. An example is C2-C6 alkynyl. Another example is C2-C4 alkynyl.

As used herein, the term "cycloalkyl" encompasses cycloalkyl having 3 to 8 carbon atoms. Examples thereof include cyclopropyl, cyclobutyl, cyclopentyl, cyclohexyl, cycloheptyl, and cyclooctyl. An example is C3-C6 cycloalkyl.

As used herein, the term "cycloalkenyl" encompasses cycloalkenyl having 3 to 8 carbon atoms. Examples thereof include cyclopropenyl, cyclobutenyl, cyclopentenyl, cyclohexenyl, and cycloheptenyl. An example is C3-C6 cycloalkenyl.

As used herein, the term "alkoxy" includes methoxy, ethoxy, *n*-propoxy, isopropoxy, *n*-butoxy, isobutoxy, *sec*-butoxy, *tert*-butoxy, *n*-pentyloxy, isopentyloxy, 2-pentyloxy, 3-pentyloxy, *n*-hexyloxy, isohexyloxy, 2-hexyloxy, 3-hexyloxy, *n*-heptyloxy, *n*-octyloxy, and the like. An example is C1-C6 alkoxy. Another example is C1-C4 alkoxy. When the carbon

number is specified in particular, an "alkoxy" having carbon in a range thereof is meant.

As used herein, the term "alkylthio" includes methylthio, ethylthio, *n*-propylthio, isopropylthio, *n*-butylthio, isobutylthio,sec-butylthio, *tert*-butylthio,*n*-pentylthio, isopentylthio, 2-pentylthio, 3-pentylthio, *n*-hexylthio, isohexylthio, 2-hexylthio, 3-hexylthio, *n*-heptylthio, *n*-octylthio, and the like. An example is C1-C6 alkylthio. Another example is C1-C4 alkylthio. When the carbon number is specified in particular, an "alkylthio" having carbon in a range of the number is meant.

As used herein, the term "alkylsulfonyl" includes methylsulfonyl, ethylsulfonyl, *n*-propylsulfonyl, isopropylsulfonyl, *n*-butylsulfonyl, isobutylsulfonyl, sec-butylsulfonyl, *tert*-butylsulfonyl, *n*-pentylsulfonyl, isopentylsulfonyl, 2-pentylsulfonyl, 3-pentylsulfonyl, *n*-hexylsulfonyl, isohexylsulfonyl, 2-hexylsulfonyl, 3-hexylsulfonyl, *n*-heptylsulfonyl, *n*-octylsulfonyl, and the like. An example is C1-C6 alkylsulfonyl. Another example is C1-C4 alkylsulfonyl.

As used herein, the term "alkoxycarbonyl" includes methoxycarbonyl, ethoxycarbonyl, *n*-propoxycarbonyl, isopropoxycarbonyl, *n*-butoxycarbonyl, isobutoxycarbonyl, sec-butoxycarbonyl, *tert*-butoxycarbonyl, *n*-pentyloxycarbonyl, and the like. An example is C1-C4 alkyloxycarbonyl. Another example is C1-C2 alkyloxycarbonyl.

As used herein, the term "acyl" encompasses formyl, alkylcarbonyl, alkenylcarbonyl, alkynylcarbonyl, cycloalkylcarbonyl, cycloalkenylcarbonyl, arylcarbonyl, heteroarylcarbonyl, heterocyclylcarbonyl. Examples thereof include acetyl, propionyl, butyloyl, benzoyl, and the like.

As used herein, the term "substituted or unsubstituted amino" encompasses amino that may be substituted with the aforementioned "alkyl," the below-mentioned "aryl," the below-mentioned "heteroaryl," the below-mentioned "heterocyclyl," the aforementioned "acyl," the aforementioned "alkoxycarbonyl," the aforementioned "alkylsulfonyl," the below-mentioned "arylsulfonyl," the below-mentioned "heteroarylsulfonyl," and/or the below-mentioned "heterocyclylsulfonyl" at 1 or 2 positions. Examples thereof include amino, methylamino, dimethylamino, ethylamino, diethylamino, ethylmethylamino, benzylamino, acetylamino, benzoylamino, methyloxycarbonylamino, methylsulfonylamino, and the like. Examples thereof include amino, methylamino, dimethylamino, ethylmethylamino, diethylamino, acetylamino, methylsulfonylamino, and the like.

As used herein, the term "substituted or unsubstituted carbamoyl" encompasses substituted or unsubstituted aminocarbonyl in which the substituted or unsubstituted amino portion is the aforementioned "substituted or unsubstituted amino." Examples thereof include carbamoyl, N-methylcarbamoyl, N,N-dimethylcarbamoyl, N-ethyl-N-methylcarbamoyl, N,N-diethylcarbamoyl, N-benzylcarbamoyl, N-acetylcarbamoyl, N-methylsulfonylcarbamoyl, and the like. Examples include carbamoyl, N-methylcarbamoyl, N,N-dimethylcarbamoyl, N-methylsulfonylcarbamoyl, and the like.

As used herein, the term "aryl" encompasses monocyclic or fused-cyclic aromatic hydrocarbon, which may be fused with the aforementioned "cycloalkyl" at any possible position. Both in the cases where aryl is monocyclic and fused-cyclic, it may be bound at any possible position. Examples thereof include phenyl, 1-naphthyl, 2-naphthyl, anthryl, tetrahydronaphthyl, and the like. Examples include phenyl, 1-naphthyl, and 2-naphthyl. An example is phenyl.

As used herein, the term "heteroaryl" encompasses a 5 to 6-membered aromatic ring containing one or more optionally-selected oxygen atoms, sulfur atoms, or nitrogen atoms in the ring. This may be fused with the aforementioned "cycloalkyl," the aforementioned "aryl," the below-mentioned "heterocyclyl", or another heteroaryl at any possible position. Both in the cases that heteroaryl is monocyclic and fused-cyclic, it maybe bound at any possible position. Examples thereof include pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), imidazolyl (e.g., 2-imidazolyl, 4-imidazolyl), pyrazolyl (e.g., 1-pyrazolyl, 3-pyrazolyl, 4-pyrazolyl), isothiazolyl (e.g., 3-isothiazolyl), isoxazolyl (e.g., 3-isoxazolyl), oxazolyl (e.g., 2-oxazolyl, 4-oxazolyl, 5-oxazolyl), thiazolyl (e.g., 2-thiazolyl, 4-thiazolyl, 5-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4-thiadiazolyl), indolizinyl (e.g., 2-indolizinyl, 6-indolizinyl), isoindolyl (e.g., 2-isoindolyl), indolyl (e.g., 1-indolyl, 2-indolyl, 3-indolyl), indazolyl (e.g., 3-indazolyl),purinyl (e.g., 8-purinyl), quinolizinyl (e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 4-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g.,3-benzisoxazolyl), benzoxazolyl (e.g., 2-benzoxazolyl), benzoxadiazolyl (e.g., 4-benzoxadiazolyl), benzisothiazolyl (e.g., 3-benzisothiazolyl), benzothiazolyl (e.g., 2-benzothiazolyl), benzofuryl (e.g., 3-benzofuryl), benzothienyl (e.g., 2-benzothienyl), dibenzothienyl (e.g., 2-dibenzothienyl), benzodioxolyl (e.g.,1,3-benzodioxolyl), and the like.

As used herein, the term "heterocyclyl" encompasses a non-aromatic heterocyclic group that may have 1 to 4 oxygen, sulfur, and/or nitrogen atoms in the ring and may be substituted at any possible position. Additionally, such a non-aromatic heterocyclic group may be further crosslinked via C1-C4 alkyl chain, or may be fused with cycloalkane (a 5- or 6-membered ring is preferable) or a benzene ring. The heterocyclic group may be saturated or unsaturated as long as it is non-aromatic. Preferred is a 5- to 8- membered ring. Examples thereof include pyrrolinyl (e.g., 1-pyrrolinyl, 2-pyrrolinyl, 3-pyrrolinyl), pyrrolidinyl (e.g., 1-pyrrolidinyl, 2-pyrrolidinyl, 3-pyrrolidinyl), pyrrolidinone, imidazolinyl (e.g., 1-imidazolinyl, 2-imidazolinyl, 4-imidazolinyl), imidazolidinyl (e.g., 1-imidazolidinyl, 2-imidazolidinyl, 4-imidazolidinyl), imidazolidinone, pyrazolinyl (e.g., 1-pyrazolinyl, 3-pyrazolinyl, 4-pyrazolinyl), pyrazolidinyl (e.g., 1-pyrazolidinyl, 3-pyrazolidinyl, 4-pyrazolidinyl), piperidinone, piperidino, piperidinyl (e.g., 2-piperidinyl, 3-piperidinyl, 4-piperidinyl), piperazinyl (e.g., 1-piperazinyl, 2-piperazinyl), piperazinone, morpholinyl (e.g., 2-morpholinyl, 3-morpholinyl), morpholino, tetrahydropyranyl, tetrahydrofuranyl, and the like.

In the present specification, a nitrogen atom of "heteroaryl" and "heterocyclyl" may form an N-oxide.

In the present specification, a "substituted or unsubstituted nitrogenated heterocycle formed after $R^2$ and $R^3$ are taken together with the adjacent nitrogen atom" and a "substituted or unsubstituted nitrogenated heterocycle formed after $R^{15}$ and $R^{16}$ are taken together with the adjacent nitrogen atom" encompass a ring that has at least one N in the ring and may further have O, S, and/or N. The ring encompasses amonocycle and a fused ring, and may be an aromatic heterocycle or non-aromatic heterocycle. Examples thereof include:

[Formula 9]

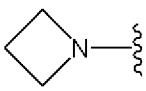 , 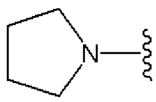 , 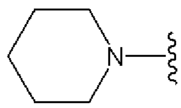 , 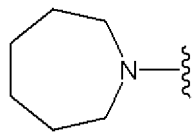 ,

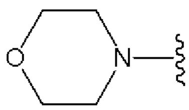 , 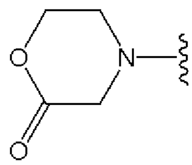 , 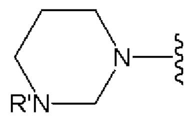 , 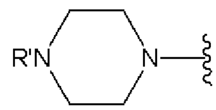 ,

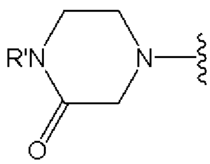 , 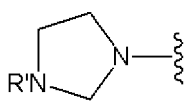 , 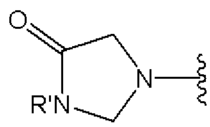 , 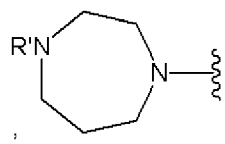 ,

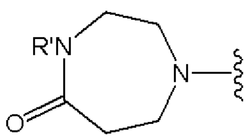

wherein R' is, for example, hydrogen, substituted or unsubstituted alkyl, substituted or unsubstituted acyl, substituted or unsubstituted amino, or hydroxy.

Examples of "substituted or unsubstituted nitrogenated heterocycle formed after $R^2$ and $R^3$ are taken together with the adjacent nitrogen atom" and "substituted or unsubstituted nitrogenated heterocycle formed after $R^{15}$ snd $R^{16}$ are taken together with the adjacent nitrogen atom" include the following rings:

[Formula 10]

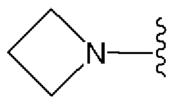, 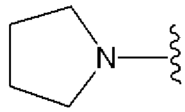, 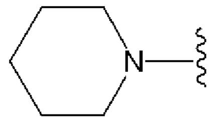, 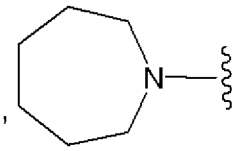,

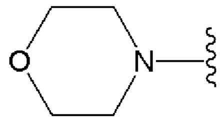, 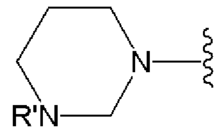, 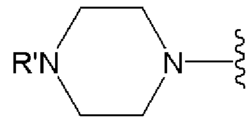,

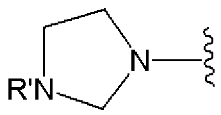, 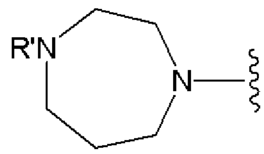

wherein R' is, for example, hydrogen, substituted or unsubstituted alkyl, substituted or unsubstituted acyl, substituted or unsubstituted amino, or hydroxy.

As used herein, the alkyl portion of "alkoxy," "alkylsulfonyl," "alkoxycarbonyl," and "alkylcarbonyl" means the aforementioned "alkyl."

As used herein, the alkenyl portion of "alkenyloxy" and "alkenylcarbonyl" means the aforementioned "alkenyl."

As used herein, the alkynyl portion of "alkynyloxy" and "alkynylcarbonyl" means the aforementioned "alkynyl."

As used herein, the cycloalkyl portion of "cycloalkyloxy," "cycloalkylsulfonyl," and "cycloalkylcarbonyl" means the aforementioned "cycloalkyl."

As used herein, the cycloalkenyl portion of "cycloalkenyloxy" and "cycloalkenylcarbonyl" means the aforementioned "cycloalkenyl."

As used herein, the aryl portion of "aryloxy," "arylsulfonyl," "arylcarbonyl," and "aryloxycarbonyl" means the aforementioned "aryl."

As used herein, the heteroaryl portion of "heteroaryloxy," "heteroarylsulfonyl," "heteroarylcarbonyl," and "heteroaryloxycarbonyl" means the aforementioned "heteroaryl."

As used herein, the heterocyclyl portion of "heterocyclyloxy," "heterocyclylsulfonyl," "heterocyclylcarbonyl," and "heterocyclyloxycarbonyl" means the aforementioned "heterocyclyl."

As used herein, substituents of "substituted or unsubstituted alkyl," "substituted or unsubstituted alkenyl," "substituted or unsubstituted alkynyl," "substituted or unsubstituted aryl," "substituted or unsubstituted cycloalkyl," "substituted or unsubstituted cycloalkenyl," "substituted or unsubstituted heteroaryl," "substituted or unsubstituted heterocyclyl," "substituted or unsubstituted acyl," "substituted or unsubstituted alkoxy," "substituted or unsubstituted aryloxy," "substituted or unsubstituted alkenyloxy," "substituted or unsubstituted alkynyloxy," "substituted or unsubstituted cycloalkyloxy," "substituted or unsubstituted cycloalkenyloxy," "substituted or unsubstituted heteroaryloxy," "substituted or unsubstituted heterocyclyloxy," "substituted or unsubstituted nitrogenated heterocycle formed after $R^2$ and $R^3$ are taken together with the adjacent nitrogen atom," "substituted or unsubstituted nitrogenated heterocycle formed after $R^{15}$ and $R^{16}$ are taken together with the adjacent nitrogen atom," and "substituted sulfonyl" are selected from the group consisting of, for example, hydroxy, carboxy, halogen, halogenated alkyl (e.g., $CF_3$, $CH_2CF_3$, $CH_2CCl_3$), nitro, nitroso, cyano, alkyl (e.g., methyl, ethyl, isopropyl, tert-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), aryl (e.g., phenyl, naphthyl), arylalkyl (e.g., benzyl, phenethyl), heteroaryl (e.g., pyridyl, furyl), heteroarylalkyl (e.g., pyridylmethyl), heterocyclyl (e.g., piperidyl), heterocyclylalkyl (e.g., morpholylmethyl), alkoxy(e.g., methoxy, ethoxy, propoxy, butoxy), halogenated alkyloxy (e. g. , $OCF_3$), alkenyloxy (e. g. , vinyloxy, allyloxy), aryloxy(e.g., phenyloxy), alkoxycarbonyl (e.g., methoxycarbonyl, ethoxycarbonyl, tert-butoxycarbonyl), arylalkyloxy (e.g., benzyloxy), unsubstituted amino, substituted amino [(e.g., alkylamino (e.g., methylamino, ethylamino, dimethylamino), acylamino (e.g., acetylamino, benzoylamino), arylalkylamino (e.g., benzylamino, tritylamino), hydroxyamino, alkoxycarbonylamino (e.g., tert-butoxycarbonylamino)], alkylaminoalkyl (e.g., diethylaminomethyl), sulfamoyl, carbamoyl, acyl (e.g., acetyl), alkylthio (e.g., methylthio), oxo, sulfonyl (e.g., alkylsulfonyl, aminosulfonyl), and the like. Substitution may occur with 1 to 4 of such substituents.

As used herein, substituents of "substituted or unsubstituted amino" and "substituted or unsubstituted carbamoyl" include alkyl, alkenyl, aryl, heteroaryl, alkylcarbonyl, arylcarbonyl, heteroarylcarbonyl, heterocyclylcarbonyl, alkoxycarbonyl, aryloxycarbonyl, heteroaryloxycarbonyl, heterocyclyloxycarbonyl, sulfamoyl, alkylsulfonyl, carbamoyl, cycloalkylsulfonyl, arylsulfonyl, heteroarylsulfonyl, heterocyclylsulfonyl, hydroxy, sulfonyl, sulfinyl, amino, and the like.

Pharmaceutically acceptable salts of the compound of the present invention include the following salts.

Examples of basic salts thereof include: alkali metal salts such as sodium salts, potassium salts, and the like; alkaline-earth metal salts such as calcium salts, magnesium salts, and the like; ammonium salts; aliphatic amine salts such as trimethylamine salts, triethylamine salts, dicyclohexylamine salts, ethanolamine salts, diethanolamine salts, triethanolamine salts, procaine salts, meglumine salts, diethanolamine salts, ethylenediamine salts, and the like; aralkylamine salts such as N,N-dibenzylethylenediamine salts, benethamine salts, and the like; heterocyclic aromatic amine salts such as pyridine salts, picoline salts, quinoline salts, isoquinoline salts, and the like; quaternary ammonium salts such as tetramethylammonium salts, tetraethylammonium salts, benzyltrimethylammonium salts, benzyltriethylammonium salts, benzyltributylammonium salts, methyltrioctylammonium salts, tetrabutylammonium salts, and the like; basic amino acid salts such as arginine salts, lysine salts; and the like.

Examples of acidic salts include: inorganic acid salts such as hydrochloride, sulfate, nitrate, phosphate, carbonate, bicarbonate, perchlorate, and the like; organic acid salts such as acetate, propionate, lactate, maleate, fumarate, tartrate, malate, citrate, ascorbate, and the like; sulfonate such as methanesulfonate, isethionate, benzenesulfonate, p-toluenesulfonate; acidic amino acids salts such as aspartate, glutamate, and the like.

As a pharmaceutically acceptable prodrug of the present invention, any form known in the art can be adopted. Aprodrug refers to a compound that, taking advantage of a metabolic machinery *in vivo*, does not exhibit a pharmaceutical effect or merely exhibits very low activity in its original form, but is modified so as to, when metabolized *in vivo*, thereby exhibit or increase pharmacological activity for the first time. Examples of prodrugs can include not only salts, solvates, and the like, but also esters, amides, and the like.

The term "solvate" means a solvate of the compound of the present invention, or a pharmaceutically acceptable salt thereof. Examples thereof include solvates formed with alcohol (e.g., ethanol), hydrates, and the like. Examples of hydrates can include monohydrate, dihydrate, and the like.

Moreover, one or more hydrogen, carbon, or other atoms in the compound of formula (I) may be replaced with isotopes of hydrogen, carbon, or other atoms respectively. The compound of formula (I) encompasses all of radiolabeled compounds of the compound of formula (I). Such "radiolabeling," "a radiolabeled compound," and the like of the compound of formula (I) are each encompassed by the present invention, and are useful for studies on metabolized drug pharmacokinetics and studies on binding assay, and/or a diagnostic tool. Furthermore, they are also useful as medicines.

Examples of isotopes that may be incorporated in the compound of formula (I) include hydrogen, carbon, nitrogen, oxygen, phosphorus, sulfur, fluorine, and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$ respectively. A radiolabeled compound of the present invention can be prepared using a well-known method in the relevant technical field. For example, a tritium-labeled compound of formula (I) can be prepared by introducing a tritium to a certain compound of formula (I), for example, through a catalytic dehalogenation reaction using a tritium. This method may comprise reacting with an appropriately-halogenated precursor of the compound of formula (I) with tritium gas in the presence of an appropriate catalyst, such as Pd/C, and in the presence or absent of a base. For another appropriate method of preparing a tritium-labeled compound, the document: Isotopes in the Physical and Biomedical Sciences, Vol. 1, Labeled Compounds (Part A), Chapter 6 (1987) can be referred to. A $^{14}C$-labeled compound can be prepared by using a raw material having $^{14}C$.

In the compound of the present invention represented by formula (I), the following embodiments of compounds are included.

(A)

Each substituent is defined as in item (1) above unless specified otherwise.

In general formula (I),

V is $-(CH_2)_{1-2}-$ or -CH=CH-;

W is a single bond or $-(CH_2)_{1-2}-$;

X is a single bond or -C(=O)-;

a group represented by formula (G) is the group represented by formula (G1) ;

$R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino;

$R^2$ is hydrogen;

$R^3$ is $-C(=O)-NR^{15}R^{16}$; and

$R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl, or $R^{15}$ and $R^{16}$ are taken together with the adjacent nitrogen atom to form a substituted or unsubstituted nitrogenated heterocycle.

(B)

Each substituent is defined as in item (1) above unless specified otherwise.

In general formula (I),

V is $-(CH_2)_{1-2}-$;

W is a single bond or $-(CH_2)_{1-2}-$;

X is a single bond or -C(=O)-;

a group represented by formula (G) is the group represented by formula (G1) ;

$R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino;

$R^2$ is hydrogen;

$R^3$ is $-C(=O)-NR^{15}R^{16}$; and

$R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl, or $R^{15}$ and $R^{16}$ are taken together with the adj acent nitrogen atom to form a substituted or unsubstituted nitrogenated heterocycle.

(C)

Each substituent is defined as in item (1) above unless specified otherwise.

In general formula (I),

V is $-(CH_2)_{1-2}-$ or -CH=CH-;

W is a single bond or $-(CH_2)_{1-2}-$;

X is a single bond or -C(=O)-;

a group represented by formula (G) is the group represented by formula (G2);

$R^A$ is hydrogen;

$R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substitutedor unsubstituted aryloxy, or substituted or unsubstituted amino;

$R^2$ is hydrogen;

$R^3$ is $-C(=O)-R^{14}$ or $-C(=O)-NR^{15}R^{16}$;

$R^{14}$ is substituted or unsubstituted alkyl; and

$R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl, or $R^{15}$ and $R^{16}$ are taken together with the adj acent nitrogen atom to form a substituted or unsubstituted nitrogenated heterocycle.

(D)

Each substituent is defined as in item (1) above unless specified otherwise.

In general formula (I),

V is $-(CH_2)_{1-2}-$ or -CH=CH-;

W is a single bond or $-(CH_2)_{1-2}-$;

X is a single bond or -C(=O)-;

a group represented by formula (G) is the group represented by formula (G2);

$R^A$ is hydrogen;

$R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino;

$R^2$ is hydrogen;

$R^3$ is $-C(=O)-NR^{15}R^{16}$; and

$R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl, or $R^{15}$ and $R^{16}$ are taken together with the adj acent nitrogen atom to form a substituted or unsubstituted nitrogenated heterocycle.

(E)

Each substituent is defined as in item (1) above unless specified otherwise.

In general formula (I),

V is $-(CH_2)_{1-2}-$;

W is a single bond or $-(CH_2)_{1-2}-$;

X is a single bond or -C(=O)-;

a group represented by formula (G) is the group represented by formula (G2);

$R^A$ is hydrogen;
$R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino;
$R^2$ is hydrogen;
$R^3$ is a -C(=O)-$R^{14}$ or -C(=O)-N$R^{15}R^{16}$;
$R^{14}$ is substituted or unsubstituted alkyl; and
$R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl, or $R^{15}$ and $R^{16}$ are taken together with the adj acent nitrogen atom to form a substituted or unsubstituted nitrogenated heterocycle.

(F)

Each substituent is defined as in item (1) above unless specified otherwise.
In general formula (I),
V is -CH=CH-;
W is a single bond or $-(CH_2)_{1-2}-$;
X is a single bond or -C(=O)-;
a group represented by formula (G) is the group represented by formula (G2);
$R^A$ is hydrogen;
$R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy, substituted or unsubstituted aryloxy, or substituted or unsubstituted amino;
$R^2$ is hydrogen;
$R^3$ is -C(=O)-$R^{14}$ or -C(=O)-N$R^{15}R^{16}$;
$R^{14}$ is substituted or unsubstituted alkyl; and
$R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl, or $R^{15}$ and $R^{16}$ are taken together with the adj acent nitrogen atom to form a substituted or unsubstituted nitrogenated heterocycle.

(H)

Each substituent is defined as in item (1) above unless specified otherwise.
In general formula (I):
A group represented by formula (G) is the group represented by formula (G1) or (G2).

In the group represented by formula (G2), $R^A$ includes hydrogen.

V includes $-(CR^4R^5)_m-$ or $-CR^6=CR^7-$.

V includes $-(CR^4R^5)-$ or - $(CR^4R^5)_2-$.

V includes $-(CH_2)-$ or $-(CH_2)_2-$.

V includes -CH=CH-.

W includes a single bond or $-(CR^8R^9)_n-$.

W includes a single bond or $-(CH_2)-$.

X includes a single bond or -C(=O)-.

$R^1$ includes substituted or unsubstituted aryl, or substituted or unsubstituted heteroaryl.

$R^1$ includes unsubstituted aryl, or aryl substituted with: C1-C6 alkoxy; halogen; C1-C6 alkyl; hydroxy; acyl; substituted sulfonyl; or amino.

$R^1$ includes unsubstituted heteroaryl, or heteroaryl substituted with: halogen; C1-C6 alkyl, C1-C6 alkoxy; aryl; C1-C6 alkoxycarbonyl; carboxy; carbamoyl; amino; C1-C6 alkylthio; heteroaryl; aryloxy; substituted sulfonyl; or hydroxy.

$R^1$ includes unsubstituted aryl, or aryl substituted with: C1-C6 alkoxy; halogen; C1-C6 alkyl; hydroxy; acyl; substituted sulfonyl; or amino.

$R^1$ includes unsubstituted heteroaryl, or heteroaryl substituted with: halogen; C1-C6 alkyl; C1-C6 alkoxy; aryl; C1-C6 alkoxycarbonyl; carboxy; carbamoyl; amino; C1-C6 alkylthio; heteroaryl; aryloxy; substituted sulfonyl; or hydroxy.

$R^1$ includes substituted or unsubstituted pyridyl, or substituted or unsubstituted pyrimidinyl.

$R^1$ includes unsubstituted pyridyl, or pyridyl substituted with: halogen; C1-C6 alkyl; C1-C6 alkoxy; aryl; C1-C6 alkoxycarbonyl; carboxy; carbamoyl; amino; C1-C6 alkylthio; heteroaryl; aryloxy; substituted sulfonyl; or hydroxy.

$R^1$ includes unsubstituted pyrimidinyl, or pyrimidinyl substituted with: halogen; C1-C6 alkyl; C1-C6 alkoxy; aryl; C1-C6 alkoxycarbonyl; carboxy; carbamoyl; amino; C1-C6 alkylthio; heteroaryl; aryloxy; substituted sulfonyl; or hydroxy.

$R^1$ includes substituted or unsubstituted aryl, substituted or unsubstituted alkoxy, substituted or unsubstituted alkyl, substituted or unsubstituted heteroaryl, substituted or unsubstituted heterocyclyl, substituted or unsubstituted cycloalkyl, substituted or unsubstituted aryloxy, or substituted or unsubstituted amino.

$R^1$ includes substituted or unsubstituted pyridyl, substituted or unsubstituted pyrimidinyl, substituted or unsubstituted thiazolyl, substituted or unsubstituted benzthiazolyl, substituted or unsubstituted thiophenyl, substituted or unsubstituted isoquinolinyl, or substituted or unsubstituted tetrahydroisoquinolinyl.

$R^1$ includes unsubstituted aryl, or aryl substituted with: halogen; C1-C6 alkyl; C2-C6 alkenyl; C2-C6 alkynyl; C1-C6 alkoxy; cyano; acyl; carboxy; hydroxy; heteroaryl; amino; C1-C6 alkoxycarbonyl; substituted sulfonyl; carbamoyl; aryl; aryloxy; C3-C8 cycloalkyl; or C3-C8 cycloalkenyl.

$R^1$ includes unsubstituted heteroaryl, or heteroaryl substituted with: halogen; C1-C6 alkyl; C1-C6 alkylthio; C1-C6 alkoxy; C1-C6 alkoxycarbonyl; carboxy; carbamoyl; substituted sulfonyl; or oxo.

$R^2$ includes hydrogen.

$R^3$ includes a group of the formula: $-C(=O)-R^{14}$, or a group of the formula: $-C(=O)-NR^{15}R^{16}$.

$R^{14}$ includes substituted or unsubstituted aryl, substituted or unsubstituted alkyl, substituted or unsubstituted alkenyl, substituted or unsubstituted heteroaryl, substituted or unsubstituted acyl, substituted or unsubstituted cycloalkyl, or substituted or unsubstituted heterocyclyl.

$R^{14}$ includes substituted or unsubstituted alkyl, or substituted or unsubstituted alkenyl.

$R^{14}$ includes substituted or unsubstituted oxazolyl, substituted or unsubstituted pyrazolyl, substituted or unsubstituted imidazolyl, substituted or unsubstituted isoxazolyl, substituted or unsubstituted pyridyl, substituted or unsubstituted pyrazinyl, substituted or unsubstituted furanyl, or substituted or unsubstituted thiophenyl.

$R^{15}$ includes hydrogen.

$R^{16}$ includes substituted or unsubstituted aryl, substituted or unsubstituted alkyl, substituted or unsubstituted cycloalkyl, substituted or unsubstituted heteroaryl, or substituted or unsubstituted alkenyl.

$R^{16}$ includes substituted or unsubstituted alkyl, substituted or unsubstituted aryl, or substituted or unsubstituted heteroaryl.

$R^{16}$ includes substituted or unsubstituted aryl, or substituted or unsubstituted heteroaryl.

$R^{16}$ includes substituted or unsubstituted aryl.

$R^{16}$ includes unsubstituted aryl, or aryl substituted with: halogen; C1-C6 alkyl; C1-C6 alkoxy; C1-C6 alkoxycarbonyl; carbamoyl; amino; or cyano.

$R^{16}$ includes unsubstituted aryl, or aryl substituted with: C1-C6 alkyl; C1-C6 alkoxy; amino; or carbamoyl.

$R^{16}$ includes substituted or unsubstituted heteroaryl.

$R^{16}$ includes substituted or unsubstituted thiophenyl, substituted or unsubstituted pyridyl, or substituted or unsubstituted isoxazolyl.

(J)

In the compound of the present invention represented by formula (I), the following embodiments of compounds are also included.

[Formula 11]

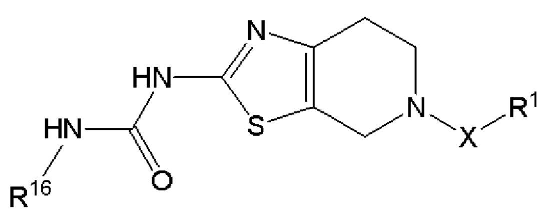

(I-a)

In formula (I-a) above:

X is

(a1) a single bond or -C(=O)-; or
(a2) a single bond.

$R^1$ is

(b1) substituted or unsubstituted aryl or substituted or unsubstituted heteroaryl;
(b2) substituted or unsubstituted aryl;
(b3) substituted or unsubstituted heteroaryl;

(b4) unsubstituted aryl, or aryl substituted with: C1-C6 alkoxy; halogen; C1-C6 alkyl; hydroxy; acyl; substituted sulfonyl; or amino, or unsubstituted heteroaryl, or heteroaryl substituted with: halogen; C1-C6 alkyl; C1-C6 alkoxy; aryl; C1-C6 alkoxycarbonyl; carboxy; carbamoyl; amino; C1-C6 alkylthio; heteroaryl; aryloxy; substituted sulfonyl; or hydroxy;
(b5) unsubstituted aryl, or aryl substituted with: C1-C6 alkoxy; halogen; C1-C6 alkyl; hydroxy; acyl; substituted sulfonyl; or amino;
(b6) unsubstituted heteroaryl, or heteroaryl substituted with: halogen; C1-C6 alkyl; C1-C6 alkoxy; aryl; C1-C6 alkoxycarbonyl; carboxy; carbamoyl; amino; C1-C6alkylthio; heteroaryl; aryloxy; substituted sulfonyl; or hydroxy;
(b7) substituted or unsubstituted pyridyl, or substituted or unsubstituted pyrimidinyl;
(b8) substituted or unsubstituted pyrimidinyl; or (b9) unsubstituted pyridyl, or pyridyl substituted with: halogen; C1-C6 alkyl; C1-C6 alkoxy; aryl; C1-C6 alkoxycarbonyl; carboxy; carbamoyl; amino; C1-C6alkylthio; heteroaryl; aryloxy; substituted sulfonyl; or hydroxy, or unsubstituted pyrimidinyl, or pyrimidinyl substituted with: halogen; C1-C6 alkyl; C1-C6 alkoxy; aryl; C1-C6 alkoxycarbonyl; carboxy; carbamoyl; amino; C1-C6alkylthio; heteroaryl; aryloxy; substituted sulfonyl; or hydroxy.

$R^{16}$ is

(c1) substituted or unsubstituted aryl, substituted or unsubstituted alkyl, substituted or unsubstituted cycloalkyl, substituted or unsubstituted heteroaryl, or substituted or unsubstituted alkenyl;
(c2) substituted or unsubstituted alkyl, substituted or unsubstituted aryl, or substituted or unsubstituted heteroaryl;
(c3) substituted or unsubstituted aryl, or substituted or unsubstituted heteroaryl;
(c4) substituted or unsubstituted aryl;
(c5) unsubstituted aryl, or aryl substituted with: halogen; C1-C6 alkyl; C1-C6 alkoxy; C1-C6 alkoxycarbonyl; carbamoyl; amino; or cyano;
(c6) unsubstituted aryl, or aryl substituted with: C1-C6 alkyl; C1-C6 alkoxy; amino; or carbamoyl;
(c7) substituted or unsubstituted heteroaryl; or
(c8) substituted or unsubstituted thiophenyl, substituted or unsubstituted pyridyl, or substituted or unsubstituted isoxazolyl.

A group of compounds represented by general formula ( I-a) include the following combinations:

(X, $R^1$, $R^{16}$) = (a1, b1, c1), (a1, b1, c2), (a1, b1, c3), (a1, b1, c4), (a1, b1, c5), (a1, b1, c6), (a1, b1, c7), (a1, b1, c8), (a1, b2, c1), (a1, b2, c2), (a1, b2, c3), (a1, b2, c4), (a1, b2, c5), (a1, b2, c6), (a1, b2, c7), (a1, b2, c8), (a1, b3, c1), (a1, b3, c2), (a1, b3, c3), (a1, b3, c4), (a1, b3, c5), (a1, b3, c6), (a1, b3, c7), (a1, b3, c8), (a1, b4, c1), (a1, b4, c2), (a1, b4, c3), (a1, b4, c4), (a1, b4, c5), (a1, b4, c6), (a1, b4, c7), (a1, b4, c8), (a1, b5, c1), (a1, b5, c2), (a1, b5, c3), (a1, b5, c4), (a1, b5, c5), (a1, b5, c6), (a1, b5, c7), (a1, b5, c8), (a1, b6, c1), (a1, b6, c2), (a1, b6, c3), (a1, b6, c4), (a1, b6, c5), (a1, b6, c6), (a1, b6, c7), (a1, b6, c8), (a1, b7, c1), (a1, b7, c2), (a1, b7, c3), (a1, b7, c4), (a1, b7, c5), (a1, b7, c6), (a1, b7, c7), (a1, b7, c8), (a1, b8, c1), (a1, b8, c2), (a1, b8, c3), (a1, b8, c4), (a1, b8, c5), (a1, b8, c6), (a1, b8, c7), (a1, b8, c8), (a1, b9, c1), (a1, b9, c2), (a1, b9, c3), (a1, b9, c4), (a1, b9, c5), (a1, b9, c6), (a1, b9, c7), (a1, b9, c8), (a2, b1, c1), (a2, b1, c2), (a2, b1, c3), (a2, b1, c4), (a2, b1, c5), (a2, b1, c6), (a2, b1, c7), (a2, b1, c8), (a2, b2, c1), (a2, b2, c2), (a2, b2, c3), (a2, b2, c4), (a2, b2, c5), (a2, b2, c6), (a2, b2, c7), (a2, b2, c8), (a2, b3, c1), (a2, b3, c2), (a2, b3, c3), (a2, b3, c4), (a2, b3, c5), (a2, b3, c6), (a2, b3, c7), (a2, b3, c8), (a2, b4, c1), (a2, b4, c2), (a2, b4, c3), (a2, b4, c4), (a2, b4, c5), (a2, b4, c6), (a2, b4, c7), (a2, b4, c8), (a2, b5, c1), (a2, b5, c2), (a2, b5, c3), (a2, b5, c4), (a2, b5, c5), (a2, b5, c6), (a2, b5, c7), (a2, b5, c8), (a2, b6, c1), (a2, b6, c2), (a2, b6, c3), (a2, b6, c4), (a2, b6, c5), (a2, b6, c6), (a2, b6, c7), (a2, b6, c8), (a2, b7, c1), (a2, b7, c2), (a2, b7, c3), (a2, b7, c4), (a2, b7, c5), (a2, b7, c6), (a2, b7, c7), (a2, b7, c8), (a2, b8, c1), (a2, b8, c2), (a2, b8, c3), (a2, b8, c4), (a2, b8, c5), (a2, b8, c6), (a2, b8, c7), (a2, b8, c8), (a2, b9, c1), (a2, b9, c2), (a2, b9, c3), (a2, b9, c4), (a2, b9, c5), (a2, b9, c6), (a2, b9, c7), (a2, b9, c8).

(K)
In the compound of the present invention represented by formula (I), the following embodiments of compounds are also included.
[Formula 12]

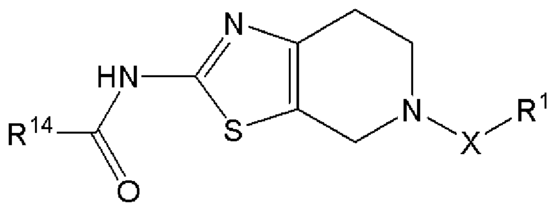

( I-b)

In formula (I-b) above:
X is

(d1) a single bond or -C(=O)-; or
(d2) a single bond.

$R^1$ is

(e1) substituted or unsubstituted aryl, or substituted or unsubstituted heteroaryl;
(e2) substituted or unsubstituted aryl;
(e3) substituted or unsubstituted heteroaryl;
(e4) unsubstituted aryl, or aryl substituted with: C1-C6 alkoxy; halogen; C1-C6 alkyl; hydroxy; acyl; substituted sulfonyl; or amino, or unsubstituted heteroaryl, or heteroaryl substituted with: halogen; C1-C6 alkyl; C1-C6 alkoxy; aryl; C1-C6 alkoxycarbonyl; carboxy; carbamoyl; amino; C1-C6 alkylthio; heteroaryl; aryloxy; substituted sulfonyl; or hydroxy;
(e5) unsubstituted aryl, or aryl substituted with: C1-C6 alkoxy; halogen; C1-C6 alkyl; hydroxy; acyl; substituted sulfonyl; or amino;
(e6) unsubstituted heteroaryl, or heteroaryl substituted with: halogen; C1-C6 alkyl; C1-C6 alkoxy; aryl; C1-C6 alkoxycarbonyl; carboxy; carbamoyl; amino; C1-C6alkylthio; heteroaryl; aryloxy; substituted sulfonyl; or hydroxy;
(e7) substituted or unsubstituted pyridyl, or substituted or unsubstituted pyrimidinyl;
(e8) substituted or unsubstituted pyrimidinyl; or
(e9) unsubstituted pyridyl, or pyridyl substituted with: halogen; C1-C6 alkyl; C1-C6 alkoxy; aryl; C1-C6 alkoxycarbonyl; carboxy; carbamoyl; amino; C1-C6alkylthio; heteroaryl; aryloxy; substituted sulfonyl; or hydroxy, or unsubstituted pyrimidinyl, or pyrimidinyl substituted with: halogen; C1-C6 alkyl; C1-C6 alkoxy; aryl; C1-C6 alkoxycarbonyl; carboxy; carbamoyl; amino; C1-C6alkylthio; heteroaryl; aryloxy; substituted sulfonyl; or hydroxy.

$R^{14}$ is

(f1) substituted or unsubstituted aryl, substituted or unsubstituted alkyl, substituted or unsubstituted alkenyl, substituted or unsubstituted heteroaryl, substituted or unsubstituted acyl, substituted or unsubstituted cycloalkyl, or substituted or unsubstituted heterocyclyl;
(f2) substituted or unsubstituted alkyl, or substituted or unsubstituted alkenyl; or
(f3) substituted or unsubstituted oxazolyl, substituted or unsubstituted pyrazolyl, substituted or unsubstituted imidazolyl, substituted or unsubstituted isoxazolyl, substituted or unsubstituted pyridyl, substituted or unsubstituted pyrazinyl, substituted or unsubstituted furanyl, or substituted or unsubstituted thiophenyl.

A group of compounds represented by general formula (I-b) include the following combinations:

(X, $R^1$, $R^{14}$) = (d1, e1, f1), (d1, e1, f2), (d1, e1, f3), (d1, e2, f1), (d1, e2, f2), (d1, e2, f3), (d1, e3, f1), (d1, e3, f2), (d1, e3, f3), (d1, e4, f1), (d1, e4, f2), (d1, e4, f3), (d1, e5, f1), (d1, e5, f2), (d1, e5, f3), (d1, e6, f1), (d1, e6, f2), (d1, e6, f3), (d1, e7, f1), (d1, e7, f2), (d1, e7, f3), (d1, e8, f1), (d1, e8, f2), (d1, e8, f3), (d1, e9, f1), (d1, e9, f2), (d1, e9, f3), (d2, e1, f1), (d2, e1, f2), (d2, e1, f3), (d2, e2, f1), (d2, e2, f2), (d2, e2, f3), (d2, e3, f1), (d2, e3, f2), (d2, e3, f3), (d2, e4, f1), (d2, e4, f2), (d2, e4, f3), (d2, e5, f1), (d2, e5, f2), (d2, e5, f3), (d2, e6, f1), (d2, e6, f2), (d2, e6, f3), (d2, e7, f1), (d2, e7, f2), (d2, e7, f3), (d2, e8, f1), (d2, e8, f2), (d2, e8, f3), (d2, e9, f1), (d2, e9, f2), (d2, e9, f3).

(L)

In the compound of the present invention represented by formula (I), the following embodiments of compounds are also included.

[Formula 13]

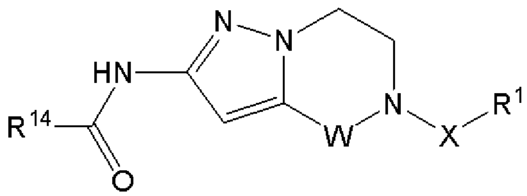

( I-c)

In formula (I-c) above:

W is

(g1) a single bond or -($CH_2$)-; or
(g2) a single bond.

X is

(h1) a single bond or -C(=O)-; or
(h2) a single bond;

$R^1$ is

(i1) substituted or unsubstituted aryl, substituted or unsubstituted alkoxy, substituted or unsubstituted alkyl, substituted or unsubstituted heteroaryl, substituted or unsubstituted heterocyclyl, substituted or unsubstituted cycloalkyl, substituted or unsubstituted aryloxy, or substituted or unsubstituted amino;
(i2) substituted or unsubstituted aryl, or substituted or unsubstituted heteroaryl;
(i3) substituted or unsubstituted aryl;
(i4) substituted or unsubstituted heteroaryl;
(i5) substituted or unsubstituted pyridyl, substituted or unsubstituted pyrimidinyl, substituted or unsubstituted thiazolyl, substituted or unsubstituted benzthiazolyl, substituted or unsubstituted thiophenyl, substituted or unsubstituted isoquinolinyl, or substituted or unsubstituted tetrahydroisoquinolinyl;
(i6) unsubstituted aryl, or aryl substituted with: halogen; C1-C6 alkyl; C2-C6 alkenyl; C2-C6 alkynyl; C1-C6 alkoxy; cyano; acyl; carboxy; hydroxy; heteroaryl; amino; C1-C6 alkoxycarbonyl; substituted sulfonyl; carbamoyl; aryl; aryloxy; C3-C8 cycloalkyl; or C3-C8 cycloalkenyl; or
(i7) unsubstituted heteroaryl, or heteroaryl substituted with: halogen; C1-C6 alkyl; C1-C6 alkylthio; C1-C6 alkoxy; C1-C6 alkoxycarbonyl; carboxy; carbamoyl; substituted sulfonyl; or oxo.

$R^{14}$ is

(j1) substituted or unsubstituted aryl, substituted or unsubstituted alkyl, substituted or unsubstituted alkenyl, substituted or unsubstituted heteroaryl, substituted or unsubstituted acyl, substituted or unsubstituted cycloalkyl, or substituted or unsubstituted heterocyclyl;
(j2) substituted or unsubstituted alkyl, or substituted or unsubstituted alkenyl; or
(j3) substituted or unsubstituted oxazolyl, substituted or unsubstituted pyrazolyl, substituted or unsubstituted imidazolyl, substituted or unsubstituted isoxazolyl, substituted or unsubstituted pyridyl, substituted or unsubstituted pyrazinyl, substituted or unsubstituted furanyl, or substituted or unsubstituted thiophenyl.

A group of compounds represented by general formula (I-c) include the following combinations:

(W, X, $R^1$, $R^{14}$) = (g1, h1, i1, j1), (g1, h1, i1, j2), (g1, h1, i1, j3), (g1, h1, i2, j1), (g1, h1, i2, j2), (g1, h1, i2, j3), (g1,

h1, i3, j1), (g1, h1, i3, j2), (g1, h1, i3, j3), (g1, h1, i4, j1), (g1, h1, i4, j2), (g1, h1, i4, j3), (g1, h1, i5, j1), (g1, h1, i5, j2), (g1, h1, i5, j3), (g1, h1, i6, j1), (g1, h1, i6, j2), (g1, h1, i6, j3), (g1, h1, i7, j1), (g1, h1, i7, j2), (g1, h1, i7, j3), (g1, h2, i1, j1), (g1, h2, i1, j2), (g1, h2, i1, j3), (g1, h2, i2, j1), (g1, h2, i2, j2), (g1, h2, i2, j3), (g1, h2, i3, j1), (g1, h2, i3, j2), (g1, h2, i3, j3), (g1, h2, i4, j1), (g1, h2, i4, j2), (g1, h2, i4, j3), (g1, h2, i5, j1), (g1, h2, i5, j2), (g1, h2, i5, j3), (g1, h2, i6, j1), (g1, h2, i6, j2), (g1, h2, i6, j3), (g1, h2, i7, j1), (g1, h2, i7, j2), (g1, h2, i7, j3), (g2, h1, i1, j1), (g2, h1, i1, j2), (g2, h1, i1, j3), (g2, h1, i2, j1), (g2, h1, i2, j2), (g2, h1, i2, j3), (g2, h1, i3, j1), (g2, h1, i3, j2), (g2, h1, i3, j3), (g2, h1, i4, j1), (g2, h1, i4, j2), (g2, h1, i4, j3), (g2, h1, i5, j1), (g2, h1, i5, j2), (g2, h1, i5, j3), (g2, h1, i6, j1), (g2, h1, i6, j2), (g2, h1, i6, j3), (g2, h1, i7, j1), (g2, h1, i7, j2), (g2, h1, i7, j3), (g2, h2, i1, j1), (g2, h2, i1, j2), (g2, h2, i1, j3), (g2, h2, i2, j1), (g2, h2, i2, j2), (g2, h2, i2, j3), (g2, h2, i3, j1), (g2, h2, i3, j2), (g2, h2, i3, j3), (g2, h2, i4, j1), (g2, h2, i4, j2), (g2, h2, i4, j3), (g2, h2, i5, j1), (g2, h2, i5, j2), (g2, h2, i5, j3), (g2, h2, i6, j1), (g2, h2, i6, j2), (g2, h2, i6, j3), (g2, h2, i7, j1), (g2, h2, i7, j2), (g2, h2, i7, j3).

(M)
In the compound of the present invention represented by formula (I), the following embodiments of compounds are also included.
[Formula 14]

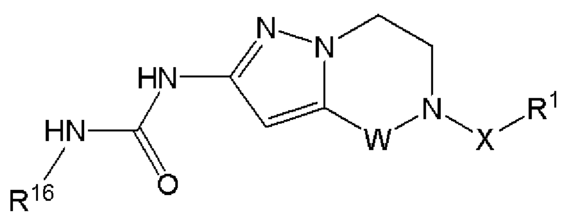

(I-d)

In formula (I-d) above:
W is

(k1) a single bond or $-(CH_2)-$; or
(k2) a single bond.

X is

(m1) a single bond or -C(=O)-; or
(m2) a single bond.

$R^1$ is

(n1) substituted or unsubstituted aryl, substituted or unsubstituted alkoxy, substituted or unsubstituted alkyl, substituted or unsubstituted heteroaryl, substituted or unsubstituted heterocyclyl, substituted or unsubstituted cycloalkyl, substituted or unsubstituted aryloxy, or substituted or unsubstituted amino;
(n2) substituted or unsubstituted aryl, or substituted or unsubstituted heteroaryl;
(n3) substituted or unsubstituted aryl;
(n4) substituted or unsubstituted heteroaryl;
(n5) substituted or unsubstituted pyridyl, substituted or unsubstituted pyrimidinyl, substituted or unsubstituted thiazolyl, substituted or unsubstituted benzthiazolyl, substituted or unsubstituted thiophenyl, substituted or unsubstituted isoquinolinyl, or substituted or unsubstituted tetrahydroisoquinolinyl;
(n6) unsubstituted aryl, or aryl substituted with: halogen; C1-C6 alkyl; C2-C6 alkenyl; C2-C6 alkynyl; C1-C6 alkoxy; cyano; acyl; carboxy; hydroxy; heteroaryl; amino; C1-C6 alkoxycarbonyl; substituted sulfonyl; carbamoyl; aryl; aryloxy; C3-C8 cycloalkyl; or C3-C8 cycloalkenyl; or
(n7) unsubstituted heteroaryl, or heteroaryl substituted with: halogen; C1-C6 alkyl; C1-C6 alkylthio; C1-C6 alkoxy; C1-C6 alkoxycarbonyl; carboxy; carbamoyl; substituted sulfonyl; or oxo.

$R^{16}$ is

(o1) substituted or unsubstituted aryl, substituted or unsubstituted alkyl, substituted or unsubstituted cycloalkyl, substituted or unsubstituted heteroaryl, or substituted or unsubstituted alkenyl;
(o2) substituted or unsubstituted alkyl, substituted or unsubstituted aryl, or substituted or unsubstituted heteroaryl;
(o3) substituted or unsubstituted aryl, or substituted or unsubstituted heteroaryl;
(o4) substituted or unsubstituted aryl;
(o5) unsubstituted aryl, or aryl substituted with: halogen; C1-C6 alkyl; C1-C6 alkoxy; C1-C6 alkoxycarbonyl; carbamoyl; amino; or cyano;
(o6) unsubstituted aryl, or aryl substituted with: C1-C6 alkyl; C1-C6 alkoxy; amino; or carbamoyl;
(o7) substituted or unsubstituted heteroaryl; or (o8) substituted or unsubstituted thiophenyl, substituted or unsubstituted pyridyl, or substituted or unsubstituted isoxazolyl.

A group of compounds represented by general formula (I-d) include the following combinations:

(W, X, $R^1$, $R^{16}$) = (k1, m1, n1, o1), (k1, m1, n1, o2), (k1, m1, n1, o3), (k1, m1, n1, o4), (k1, m1, n1, o5), (k1, m1, n1, o6), (k1, m1, n1, o7), (k1, m1, n1, o8), (k1, m1, n2, o1), (k1, m1, n2, o2), (k1, m1, n2, o3), (k1, m1, n2, o4), (k1, m1, n2, o5), (k1, m1, n2, o6), (k1, m1, n2, o7), (k1, m1, n2, o8), (k1, m1, n3, o1), (k1, m1, n3, o2), (k1, m1, n3, o3), (k1, m1, n3, o4), (k1, m1, n3, o5), (k1, m1, n3, o6), (k1, m1, n3, o7), (k1, m1, n3, o8), (k1, m1, n4, o1), (k1, m1, n4, o2), (k1, m1, n4, o3), (k1, m1, n4, o4), (k1, m1, n4, o5), (k1, m1, n4, o6), (k1, m1, n4, o7), (k1, m1, n4, o8), (k1, m1, n5, o1), (k1, m1, n5, o2), (k1, m1, n5, o3), (k1, m1, n5, o4), (k1, m1, n5, o5), (k1, m1, n5, o6), (k1, m1, n5, o7), (k1, m1, n5, o8), (k1, m1, n6, o1), (k1, m1, n6, o2), (k1, m1, n6, o3), (k1, m1, n6, o4), (k1, m1, n6, o5), (k1, m1, n6, o6), (k1, m1, n6, o7), (k1, m1, n6, o8), (k1, m1, n7, o1), (k1, m1, n7, o2), (k1, m1, n7, o3), (k1, m1, n7, o4), (k1, m1, n7, o5), (k1, m1, n7, o6), (k1,
m1, n7, o7), (k1, m1, n7, o8), (k1, m2, n1, o1), (k1, m2, n1, o2), (k1, m2, n1, o3), (k1, m2, n1, o4), (k1, m2, n1, o5), (k1, m2, n1, o6), (k1, m2, n1, o7), (k1, m2, n1, o8), (k1, m2, n2, o1), (k1, m2, n2, o2), (k1, m2, n2, o3), (k1, m2, n2, o4), (k1, m2, n2, o5), (k1, m2, n2, o6), (k1, m2, n2, o7), (k1, m2, n2, o8), (k1, m2, n3, o1), (k1, m2, n3, o2), (k1, m2, n3, o3), (k1, m2, n3, o4), (k1, m2, n3, o5), (k1, m2, n3, o6), (k1, m2, n3, o7), (k1, m2, n3, o8), (k1, m2, n4, o1), (k1, m2, n4, o2), (k1, m2, n4, o3), (k1, m2, n4, o4), (k1, m2, n4, o5), (k1, m2, n4, o6), (k1, m2, n4, o7), (k1, m2, n4, o8), (k1, m2, n5, o1), (k1, m2, n5, o2), (k1, m2, n5, o3), (k1, m2, n5, o4), (k1, m2, n5, o5), (k1, m2, n5, o6), (k1, m2, n5, o7), (k1, m2, n5, o8), (k1, m2, n6, o1), (k1, m2, n6, o2), (k1, m2, n6, o3), (k1, m2, n6, o4), (k1, m2, n6, o5), (k1, m2, n6, o6), (k1, m2, n6, o7), (k1, m2, n6, o8), (k1, m2, n7, o1), (k1, m2, n7, o2), (k1, m2, n7, o3), (k1, m2, n7, o4), (k1, m2, n7, o5), (k1, m2, n7, o6), (k1, m2, n7, o7), (k1, m2, n7, o8), (k2, m1, n1, o1), (k2, m1, n1, o2), (k2, m1, n1, o3), (k2, m1, n1, o4), (k2, m1, n1, o5), (k2, m1, n1, o6), (k2, m1, n1, o7), (k2, m1, n1, o8), (k2, m1, n2, o1), (k2, m1, n2, o2), (k2, m1, n2, o3), (k2, m1, n2, o4), (k2, m1, n2, o5), (k2, m1, n2, o6), (k2, m1, n2, o7), (k2, m1, n2, o8), (k2, m1, n3, o1), (k2, m1, n3, o2), (k2, m1, n3, o3), (k2, m1, n3, o4), (k2, m1, n3, o5), (k2, m1, n3, o6), (k2, m1, n3, o7), (k2, m1, n3, o8), (k2, m1, n4, o1), (k2, m1, n4, o2), (k2, m1, n4, o3), (k2, m1, n4, o4), (k2, m1, n4, o5), (k2, m1, n4, o6), (k2, m1, n4, o7), (k2, m1, n4, o8), (k2, m1, n5, o1), (k2, m1, n5, o2), (k2, m1, n5, o3), (k2, m1, n5, o4), (k2, m1, n5, o5), (k2, m1, n5, o6), (k2, m1, n5, o7), (k2, m1, n5, o8), (k2, m1, n6, o1), (k2, m1, n6, o2), (k2, m1, n6, o3), (k2, m1, n6, o4), (k2, m1, n6, o5), (k2, m1, n6, o6), (k2, m1, n6, o7), (k2, m1, n6, o8), (k2, m1, n7, o1), (k2, m1, n7, o2), (k2, m1, n7, o3), (k2, m1, n7, o4), (k2, m1, n7, o5), (k2, m1, n7, o6), (k2, m1, n7, o7), (k2, m1, n7, o8), (k2, m2, n1, o1), (k2, m2, n1, o2), (k2, m2, n1, o3), (k2, m2, n1, o4), (k2, m2, n1, o5), (k2, m2, n1, o6), (k2, m2, n1, o7), (k2, m2, n1, o8), (k2, m2, n2, o1), (k2, m2, n2, o2), (k2, m2, n2, o3), (k2, m2, n2, o4), (k2, m2, n2, o5), (k2, m2, n2, o6), (k2, m2, n2, o7), (k2, m2, n2, o8), (k2, m2, n3, o1), (k2, m2, n3, o2), (k2, m2, n3, o3), (k2, m2, n3, o4), (k2, m2, n3, o5), (k2, m2, n3, o6), (k2, m2, n3, o7), (k2, m2, n3, o8), (k2, m2, n4, o1), (k2, m2, n4, o2), (k2, m2, n4, o3), (k2, m2, n4, o4), (k2, m2, n4, o5), (k2, m2, n4, o6), (k2, m2, n4, o7), (k2, m2, n4, o8), (k2, m2, n5, o1), (k2, m2, n5, o2), (k2, m2, n5, o3), (k2, m2, n5, o4), (k2, m2, n5, o5), (k2, m2, n5, o6), (k2, m2, n5, o7), (k2, m2, n5, o8), (k2, m2, n6, o1), (k2, m2, n6, o2), (k2, m2, n6, o3), (k2, m2, n6, o4), (k2, m2, n6, o5), (k2, m2, n6, o6), (k2, m2, n6, o7), (k2, m2, n6, o8), (k2, m2, n7, o1), (k2, m2, n7, o2), (k2, m2, n7, o3), (k2, m2, n7, o4), (k2, m2, n7, o5), (k2, m2, n7, o6), (k2, m2, n7, o7), (k2, m2, n7, o8).

(Production Method)

General production methods of the compound of the present invention are illustrated hereinbelow. Furthermore, with regard to extraction, purification, and the like, treatments as in usual experiments of organic chemistry may be carried out.
Hereinafter, production methods of the compound of the present invention are described.
Synthesis of the compound of the present invention can be performed by reference to a known method in the relevant field.
As a raw material compounds, the following is available: commercially available compounds; those described

in Patent Document 3, 4, 5, 6, 7, 8, 9, 10, 11, 12, 13, or 14; those described in International Publication No. WO 2006/066174 pamphlet, Synthetic Communications, 29 (2), 311-341 (1999), Heterocycles, 63(7), 1555 (2004), or the present specification; those described in another document cited herein; and other known compounds.

Regarding some of the compound of the present invention, a tautomer, regioisomer, or optical isomer thereof may exist. The present invention encompasses all possible isomers including these, and mixtures thereof.

When it is desired to obtain a salt of the compound of the present invention, in the case that the compound of the present invention is obtained in salt form, it may be purified as it is. Furthermore, in the case that it is obtained in free form, it may be dissolved or suspended in an appropriate organic solvent and then an acid or base may be added thereto to form a salt thereof using a general method.

Furthermore, the compound of the present invention and a pharmaceutically acceptable salt thereof may exist in form of adduct with water or any kind of solvent (hydrate or solvate). These adducts are also encompassed by the present invention.

Derivatives thereof are converted in the body and consequently activated, which are named "prodrug" herein. It is understood that examples of prodrugs include not only the aforementioned salts and solvates, but also esters (e.g., alkyl ester and the like), amides, and the like.

Various examples of the compound of the present invention are listed in Examples. By reference to these, those skilled in the art can produce and use compounds that are not illustrated in the present invention.

The present invention is also related to a system, an apparatus, and a kit for producing the compound of the present invention. It is understood that, as elements of such a system, an apparatus, and a kit, matters known in the relevant field are available, and those skilled in the art can appropriately design them.

(General Synthesis Method)

In the case where a group represented by formula (G) is the group represented by formula (G2) or (G3):

[Formula 15]

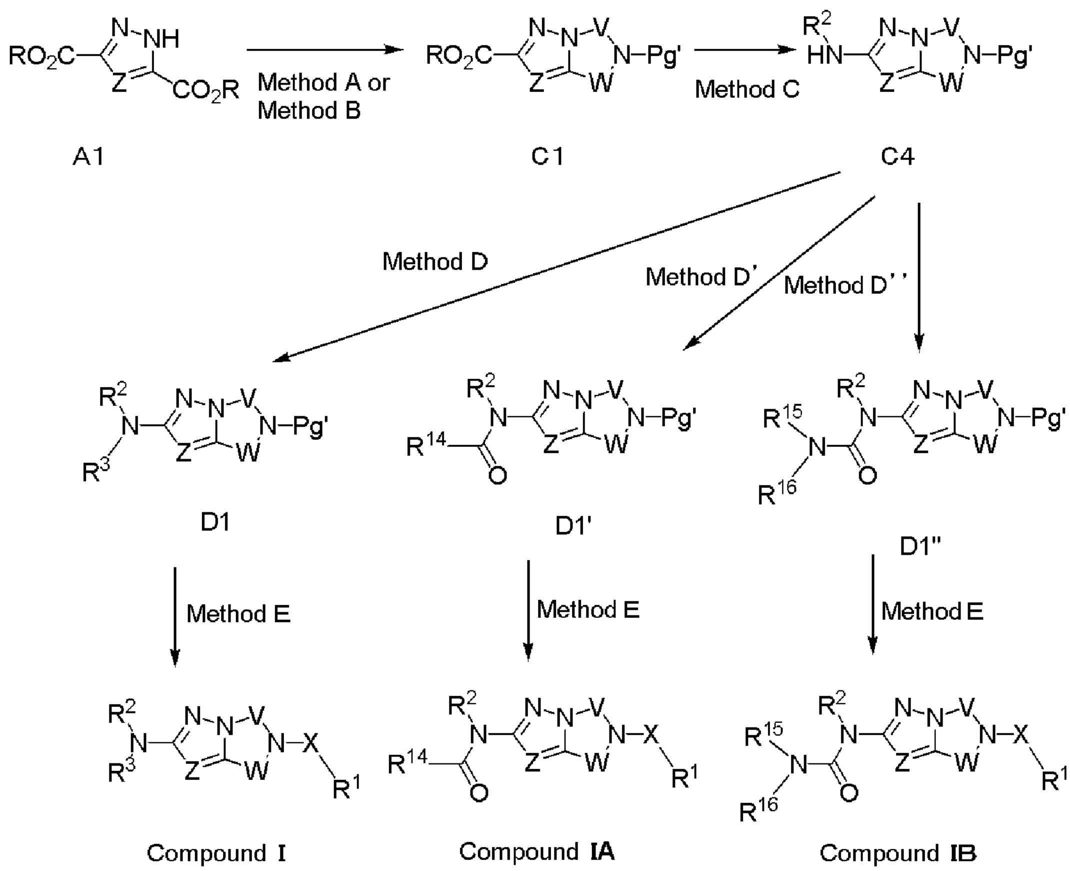


A1 C1 C4

D1 D1' D1"

Compound I Compound IA Compound IB

wherein each symbol is defined the same as above, a known compound may be used as the compound represented by formula (A1), and a compound derived from a known compound using a usual method may be also used. The above-described method generally describes methods for synthesizing Compound I of the present invention from the compound represented by formula (A1). The compound represented by formula (C1) is synthesized using Method A or B from formula (A1). Subsequently, the compound represented by formula (C4) is synthesized using Method C. Furthermore, the compound represented by formula (D1), (D1'), or (D1") is synthesized using Method D, D', or D" respectively, and then Compounds I, IA, and IB of the present invention are synthesized using Method E.

Methods A to E are described in detail below.

1) Method A: Synthesis method of the compound represented by formula (C1) wherein W is a single bond

1)-1

[Formula 16]

1)－1

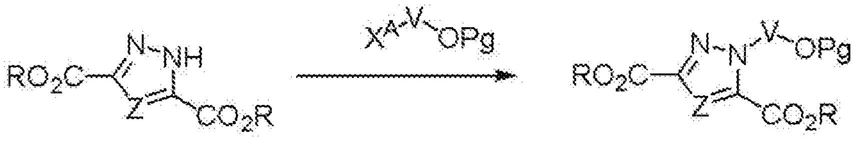


A1 A2

wherein each symbol is defined the same as above; R includes C1-C6 alkyl; and $X^A$ includes a leaving group such as halogen (e.g., Cl, Br, I, and the like), -OMs, -OTs, -OTf, -ONs, and the like. Here, "Ms" refers to a methanesulfonyl group, "Ts" refers to a para-toluenesulfonyl group, "Tf" refers to a trifluoromethanesulfonyl group, and "Ns" refers to an ortho-nitrobenzenesulfonyl group. Pg refers to a hydroxy-protecting group (e.g., benzyl group, p-methoxybenzyl group, acetyl group, and the like). Known compounds maybe used as the compounds represented by formulae (A1) and the formula:

[Formula 17]

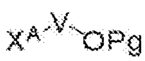

A compound derived from a known compound using a usual method may be also used.

The above step is of reacting the compound represented by formula (A1) with the compound represented by the formula:

[Formula 18]

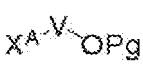

in the presence of a base to synthesize the compound represented by formula (A2).

Reaction solvents include N,N-dimethylformamide (DMF), N-methyl-2-pyrrolidone (NMP),N,N-dimethylacetamide (DMA), dimethylsulfoxide, aromatic hydrocarbons (e.g., toluene, benzene, xylene, and the like), saturatedhydrocarbons (e.g., cyclohexane, hexane, and the like), halogenated hydrocarbons (e.g., dichloromethane, chloroform, 1,2-dichloroethane, and the like), ethers (e.g., tetrahydrofuran, diethyl ether, dioxane, 1,2-dimethoxyethane, and the like), esters (e.g., methyl acetate, ethyl acetate, and the like), ketones (e.g., acetone, methylethylketone, and the like), nitriles (e.g., acetonitrile, and the like), alcohols (e.g., methanol, ethanol, t-butanol, and the like), pyridines (pyridine, 2, 6-lutidine, and the like), water and mixed solvents thereof, and the like.

Examples of bases include metal hydrides (e.g., sodium hydride, and the like), metal hydroxides (e.g., sodium hydroxide, potassium hydroxide, lithium hydroxide, barium hydroxide, and the like), metal carbonate salts (e.g., sodium carbonate, potassium carbonate, calcium carbonate, cesium carbonate, and the like), metal alkoxides (e.g., sodium methoxide, sodium ethoxide, potassium t-butoxide, and the like), sodium bicarbonate, metallic sodium, organic amines (e.g., triethylamine, diisopropylethylamine, diazabicycloundecene (DBU), pyridine, 2,6-lutidine, and the like), alkyl lithium (*n*-butyl lithium (*n*-BuLi), sec-butyl lithium (sec-BuLi), *tert*-butyl lithium (*tert*-BuLi)), and the like.

Preferably, an ether (e.g., tetrahydrofuran, diethyl ether, dioxane, and the like), N,N-dimethylformamide, or acetonitrile is used as reaction solvent, as well as a metal carbonate salt (e.g.,sodium carbonate,potassium carbonate,

calcium carbonate, cesium carbonate, and the like) may be used as a base. A reaction temperature and a reaction time are not specifically limited, but the reaction may be carried out for a period of time from 0.5 to 12 hours at temperatures from-20˚C to a temperature at which a solvent used is refluxed.

1)-2

[Formula 19]

1)-2

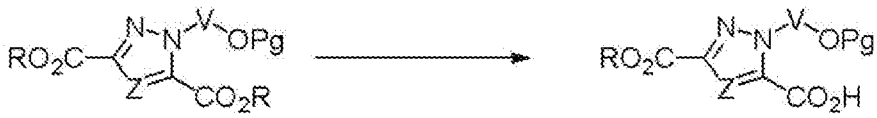

A2 A3

wherein each symbol is defined the same as above.
The above step is of hydrolyzing a compound represented by formula (A2) in the presence of a base to synthesize a compound represented by formula (A3).

A base described in 1)-1 above can be used as a base. Preferably, a base is metal hydroxide (e.g., sodium hydroxide, potassium hydroxide, lithium hydroxide, barium hydroxide, and the like). A solvent described in 1) -1 above can be used as reaction solvent. Preferably, the reaction maybe carried out in an aqueous solution or alcohol solution of a metal hydroxide. A reaction temperature and a reaction time are not specifically limited, but the reaction may be carried out at temperatures from -20 to 50˚C for a period of time from 0.5 to 36 hours.

1)-3

[Formula 20]

1)-3

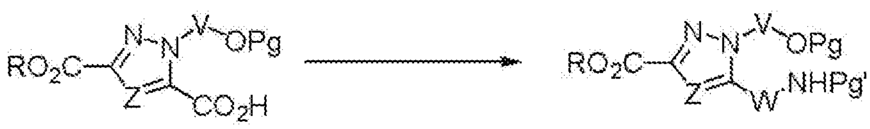

A3 A4

wherein each symbol is defined the same as above, Pg' refers to an amino-protecting group (e.g., t-butoxycarbonyl group, benzyl group, acetyl group, benzoyl group, benzyloxycarbonyl group, and the like).
The above step is of reacting a compound represented by formula (A3) with an azidation reagent or azide compound, followed by pyrolysis to cause a Curtius rearrangement, then treatment with an alcohol which forms a protecting group, and consequently synthesizing a compound represented by formula (A4).

Sodium azide, hydrogen azide, diphenylphosphoryl azide, and the like can be used as azidation reagents or azide compounds. A solvent described in 1)-1 above can be used as solvent. For a preferable example, a reaction in a t-butylalcohol solution produces an amine protected by a t-butoxycarbonyl group. With regard to reaction temperatures, the reaction with an azidation reagent or azide compound is usually carried out at a low temperature (e.g., 0˚C or the like), and pyrolysis is usually carried out under a heating condition (e.g., 100˚C, or the like). A reaction time is not specifically limited, but the reaction may be carried out for a period of time from 0.5 to 12 hours.

1)-4

[Formula 21]

1)-4

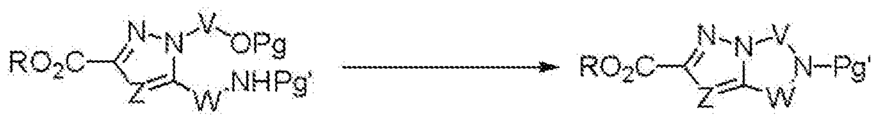

A4 C1

wherein each symbol is defined the same as above.

The above step is of deprotecting a compound represented by formula (A4) from the pg group and then intramolecularly cyclizing the resulting alcohol to synthesize a compound of formula (C1).

A deprotection method is carried out in accordance with a method described in "Protective Groups in Organic Synthesis," Theodora W. Greene (John Wiley & Sons, Inc., New York), second ed. , 1991. For example, when a protecting group is a benzyl or p-methoxybenzyl group, a catalytic reduction reaction may be carried out in the presence of hydrogen.

A method of an intramolecularly cyclization may be carried out under a condition of Mitsunobu reaction. As phosphine reagents, for example, triphenylphosphine ($PPh_3$), tri(*n*-butyl)phosphine ($n$-$Bu_3P$), and the like can be used. As azodicarboxylic acid esters and amides, diethyl azodicarboxylate (DEAD), diisopropyl azodicarboxylate (DIAD), 1,1'-(azodicarbonyl)dipiperidine (ADDP), and the like can be used. A reaction temperature and a reaction time are not specifically limited, but the reaction may be carried out at temperatures from -20 to 50˚C for a period of time from 0.5 to 12 hours. With regard to a method of an intramolecular cyclization, an OH group may be converted to halogen (e.g., Cl, Br, I, and the like), or a leaving group described in 1)-1 above (which indicates, e.g., OTf, OMs, and the like) to carry out an intramolecular cyclization in the presence of a base. In the conversion of a OH group to a halogen, after the reaction with methanesulfonyl chloride to make a OMs group, the OMS group is substituted with $Cl^-$ that generates in the reaction system and consequently the chlorination can be attained. A reaction with carbon tetrabromide, bromine, N-chlorosuccinimide (NCS), or N-bromosuccinimide (NBS) in the presence of triphenylphosphine ($PPh_3$) may be carried out. A solvent described in 1)-1 above can be used as solvent. Preferably, a halogenated hydrocarbon (e.g., dichloromethane, chloroform, 1,2-dichloroethane, and the like), or an ether (e.g., tetrahydrofuran, diethyl ether, dioxane, 1,2-dimethoxyethane, and the like) may be used to carry out the reaction. A reaction temperature and a reaction time are not specifically limited, but the reaction may be carried out at temperatures from -20 to 50˚C for a period of time from 0.5 to 12 hours. A base described in 1)-1 above can be used as a base in an intramolecular cyclization reaction. Preferably, a metal hydride (e.g., sodium hydride, and the like), or a metal alkoxide (e.g., sodium methoxide, sodium ethoxide, potassium t-butoxide, and the like) may be used to carry out the reaction. A solvent described in 1) -1 above can be used as solvent. N,N-dimethylformamide (DMF), a halogenated hydrocarbon (e.g., dichloromethane, chloroform, 1,2-dichloroethane, and the like), or an ether (e.g., tetrahydrofuran, diethyl ether, dioxane, 1,2-dimethoxyethane, and the like) may be used to carry out the reaction. A reaction temperature and a reaction time are not specifically limited, but the reaction may be carried out at temperatures from -20 to 50˚C for a period of time from 0.5 to 12 hours.

2) Method B: Synthesis method of a compound represented by formula (C1) wherein W is $-(CR^8R^9)_n-$.

2)-1

[Formula 22]

2)-1

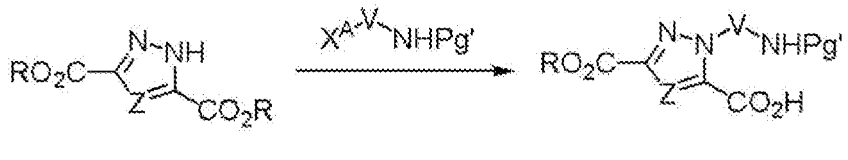


wherein each symbol is defined the same as above; $X^A$ refers to halogen (e.g., Cl, Br, I, and the like) or a leaving group described in 1)-1 above (e.g., OTf, OMs, and the like); and Pg' refers to an amino-protecting group (e.g., t-butoxycarbonyl group, acetyl group, benzoyl group, benzyl group, benzyloxycarbonyl group, and the like). A known compound may be used as a compound represented by formula (A1) and a compound represented by the formula:
[Formula 23]

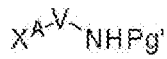

A compound derived from a known compound using a usual method may be also used.
The above step is of reacting a compound represented by formula (A1) with a compound represented by the formula:
[Formula 24]

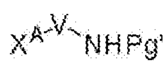

in the presence of a base using a similar method to 1)-1, and further reacting the resulting compound using a similar method to 1) -2 to synthesize a compound represented by formula (B2). This step can be carried out under a reaction condition similar to 1) -1 and 1) -2 above. With regard to the reaction condition at the first step, preferably, N, N-dimethylformamide (DMF), anitrile (e.g., acetonitrile, and the like), or the like may be used as solvent, and the reaction may be carried out at temperatures from -20 to 50˚C for a period of time from 0.5 to 12 hours. With regard to the reaction condition at the second step, preferably, an alcohol (e.g., methanol, ethanol, t-butanol, and the like), water, halogenated hydrocarbon (e.g., dichloromethane, chloroform, 1,2-dichloroethane, and the like), or the like may be used as solvent, and the reaction may be carried out at temperatures from -20 to 50˚C for a period of time from 0.5 to 24 hours.

2)-2

[Formula 25]

2)-2

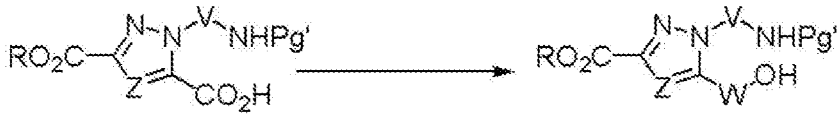

B2 B3

wherein each symbol is defined the same as above.

The above step is of reacting the compound represented by formula (B2) with N,N'-carbonyldiimidazole to convert the carboxyl group of the compound represented by formula (B2) to -C(=O) Im wherein Im is imidazole, then reducing it to synthesize the alcohol represented by formula (B3).

Asa reducing agent,for example,sodiumtetrahydroborate, lithium tetrahydroborate, and the like are used. As solvent, a solvent described in 1)-1 above can be used. Preferably, an ether (e.g., tetrahydrofuran, diethyl ether, dioxane, 1,2-dimethoxyethane, and the like) can be used. A reaction temperature and a reaction time are not specifically limited, but the reaction may be carried out at temperatures from -20 to 50˚C for a period of time from 0.5 to 12 hours.

2)-3

[Formula 26]

2)-3

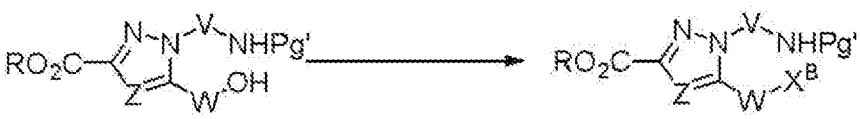

B3 B4

wherein each symbol is defined the same as above, $X^B$ is halogen (e.g., Cl, Br, I, and the like).

The above step is of halogenating the compound represented by formula (B3) to synthesize the compound of formula (B4).

For example, it is possible to react the OH group of the compound represented by formula (B3) with methanesulfonyl chloride to covert it to a OMs group, followed by chlorination by substitution with $Cl^-$ that generates in the reaction system.

With regard to a reaction of halogenating an alcohol, an alcohol may be reacted in the presence of triphenylphosphine ($PPh_3$) with carbon tetrabromide, bromine, N-chlorosuccinimide (NCS), N-bromosuccinimide (NBS). As solvent, a solvent described in 1)-1 above can be used. Preferably, a halogenated hydrocarbon (e.g., dichloromethane, chloroform, 1,2-dichloroethane, or the like), an ether (e.g., tetrahydrofuran, diethyl ether, dioxane, 1,2-dimethoxyethane, and the like) may be used to carry out the reaction. A reaction temperature and a reaction time are not specifically limited, but the reaction may be carried out at temperatures from -20 to 50˚C for a period of time from 0.5 to 12 hours.

2)-4

[Formula 27]

2)-4

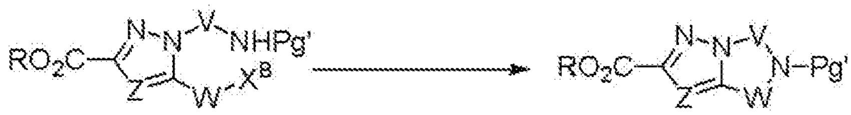

B4 C1

wherein each symbol is defined the same as above, and $X^B$ is halogen (e.g., Cl, Br, I, and the like), or leaving group described in 1)-1 above (e.g., OTf, OMs, and the like).
The above step is of intramolecularly cyclizing the compound represented by formula (B4) in the presence of a base to synthesize the compound of formula (C1).

As a base, a base described in 1)-1 above can be used. Preferably, a metal hydride (e.g., sodium hydride, and the like), or a metal alkoxide (e.g., sodium methoxide, sodium ethoxide, potassium t-butoxide, and the like) may be used to react the reaction. As solvent, a solvent described in 1)-1 above can be used. Preferably, N,N-dimethylformamide (DMF), a halogenated hydrocarbon (e.g., dichloromethane, chloroform, 1,2-dichloroethane, and the like), or an ether (e.g., tetrahydrofuran, diethyl ether, dioxane, 1,2-dimethoxyethane, and the like) may be used to carry out the reaction. A reaction temperature and a reaction time are not specifically limited, but the reaction may be carried out at temperatures from -20 to 50˚C for a period of time from 0.5 to 12 hours.

3) Method C: A method for synthesizing the compound represented by the formula (C4)

3)-1

[Formula 28]

3)-1

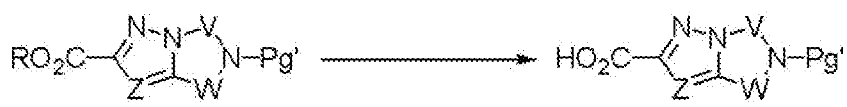

C1 C2

wherein each symbol is defined the same as above, and Pg' is an amino-protecting group (e.g., t-butoxycarbonyl group, benzyl group, acetyl group, benzoyl group, benzyloxycarbonyl group, and the like). A known compound may be used as a compound represented by formula (C1), and a compound derived from a known compound using a usual method may be also used.
The above step is of hydrolyzing the compound represented by the formula (C1) in the presence of a base using the same method as 1)-2 to synthesize the compound represented by formula (C2).

3)-2

[Formula 29]

3)-2

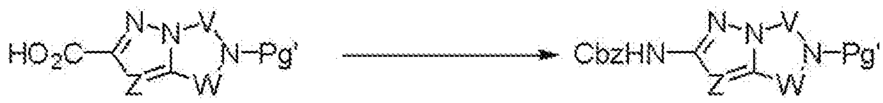

C2 C3

wherein each symbol is defined the same as above, and Cbz is a benzyloxycarbonyl group.
The above step is of reacting the compound represented by formula (C2) with an azidation reagent or an azide compound using the same method as 1)-3, followed by pyrolysis to cause a Curtius rearrangement, then further treatment with benzylalcohol, and consequently synthesizing the compound represented by formula (C3).
As solvent, a solvent described in 1)-1 above can be used. Preferably, the reaction may be carried out in a benzylalcohol solution.

3)-3

[Formula 30]

3)-3

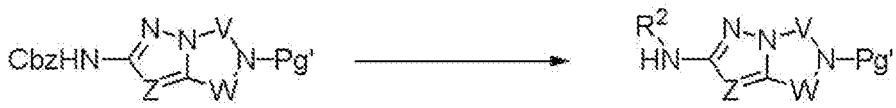

C3 C4

wherein each symbol is defined the same as above, and Cbz is a benzyloxycarbonyl group.
The above step is of deprotecting the compound represented by formula (C3) from the benzyloxycarbonyl group to synthesize the compound represented by formula (C4). The deprotection is carried out using a method described in "Protective Groups in Organic Synthesis," Theodora W. Greene (John Wiley & Sons, Inc., New York), 2nd ed., 1991. When $R^2$ is hydrogen, the compound represented by formula (C3) may be deprotected from the Cbz group under a condition described above. When $R^2$ is substituted or unsubstituted alkyl, $R^2$ can be introduced by reductive alkylation. When $R^2$ is substituted or unsubstituted acyl, $R^2$ can be introduced, for example, by a reaction with a carboxylic acid halide. Furthermore, after $R^2$ is introduced to the compound represented by formula (C3), the Cbz may be removed by deprotection.

4) Method D: Method for synthesizing the compound represented by formula (D1)

[Formula 31]

4)

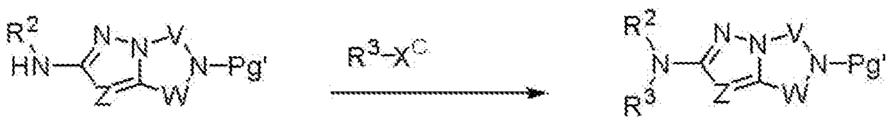


C4 D1

wherein each symbol is defined the same as above, and $X^C$ is halogen (e.g., Cl, Br, I, and the like), or a leaving group described in 1)-1 above (e.g., OTf, OMs, and the like). A known compound maybe used as a compound represented by formula ($R^3$-$X^C$), and a compound derived from a known compound using a usual method may be also used.
The above step is of reacting the compound represented by formula (C4) with the compound represented by formula ($R^3$-$X^C$) in the presence of a palladium catalyst, a phosphine ligand, and a base to synthesize the compound represented by formula (D1).

As solvent, a solvent described in 1)-1 above can be used. Preferably, an ether (e.g., tetrahydrofuran, diethyl ether, dioxane, 1,2-dimethoxyethane, and the like) may be used to carry out the reaction.

As a base, a base described in 1)-1 above can be used. Preferably, metal carbonate salts (e.g., sodium carbonate, potassium carbonate, calcium carbonate, cesium carbonate, and the like) may be used to carry out the reaction.

As a palladium catalyst, the following can be used: tetrakistriphenylphosphine palladium ($Pd(PPh_3)_4$), palladium chloride ($PdCl_2$), tris(dibenzylideneacetone)bispalladium ($Pd_2(dba)_3$), bis(dibenzylideneacetone)palladium ($Pd(dba)_2$), palladium acetate ($Pd(OAc)_2$), [1,1'-bis(diphenylphosphino)ferrocene]-dichloropalladiu m(II)-dichloromethane complex ($PdCl_2(dppf)$), bis(triphenylphosphine)palladium chloride ($PdCl_2(PPh_3)_2$), and the like.

As a phosphine ligand, the following can be used: triphenylphosphine ($PPh_3$), 2,2'-bis(diphenylphosphino)-1,1'-binaphthyl (BINAP), 9,9-dimethyl-4,5-bis(diphenylphosphino)xanthene (Xantphos), 2-dicyclohexylphosphino-2',6'-dimethoxybiphenyl (S-Phos), 2-dicyclohexylphosphino-2',4',6'-triisopropylbiphenyl (X-Phos), 1,1'-bis(diphenylphosphino)ferrocene (DPPF), tri(t-butyl)phosphine(t-Bu3P), tri-o-tolylphosphine, and the like.

A reaction temperature and a reaction time are not specifically limited, but the reaction may be carried out for a period of time from 0.5 to 24 hours at temperatures from 20˚C to a temperature at which a solvent used is refluxed.

Furthermore, as another method for synthesizing the compound represented by formula (D1), the compound represented by formula (D1) can be synthesized through a reductive amination of the compound represented by formula (C4).

4') Method D' : In the case where $R^3$ is a group represented by the formula: -C(=O)-$R^{14}$:

[Formula 32]

4')

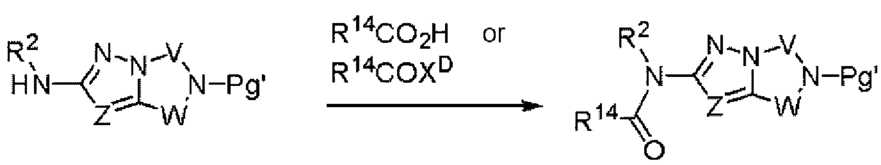


C4 D1'

wherein each symbol is defined the same as above, and $X^D$ is halogen (e.g., Cl, Br, I, and the like). A known compound can be used as a compound represented by $R^{14}CO_2H$ and $R^{14}COX^D$, and a compound derived from a known compound using a usual method may be also used.
The above step is of reacting the compound represented by formula (C4) with a compound represented by $R^{14}CO_2H$ in the presence of a condensing agent and a base to synthesize the compound represented by formula (D1').

As a condensing agent, the following can be used: dicyclohexylcarbodiimide (DCC), benzotriazol-1-yloxy-tris (dimethylamino)phosphonium hexafluorophosphate (BOP), benzotriazol-1-yloxy-tripyrrolidinylphosphonium hexafluorophosphate (PyBOP), PyBroP, O-(7-azabenzotriazol-1-yl)-N,N,N',N'-tetramethyluronium hexafluorophosphate (HATU), diethyl cyanophosphonate, 1-ethyl-3-(3-dimethylaminopropyl)carbodiimide hydrochloride (WSC), 4-(4,6-dimethoxy-1,3,5-triazin-2-yl)-4-methylmorpholini um hydrochloride (DMT-MM), and the like. Furthermore, these reagents can be used in combination with, for example, N-hydroxysuccinimide (HOSu),N-hydroxybenzotriazole (HOBt), N-hydroxy-7-azabenzotriazole (HOAt), and the like.

As solvent, a solvent described in 1)-1 above can be used. Preferably, an ether (e.g., tetrahydrofuran, diethyl ether, dioxane, 1,2-dimethoxyethane, and the like), and dimethylformamide may be used to carry out the reaction.

As a base, a base described in 1)-1 above can be used. Preferably, an organic amine (e.g., triethylamine, N-methylmorpholine, and the like) may be used to carry out the reaction.

A reaction temperature and a reaction time are not specifically limited, but the reaction may be carried out for a period of time from 0.5 to 24 hours at temperatures from-20˚C to a temperature at which a solvent used is refluxed.

Furthermore, the compound represented by formula (C4) can be reacted with $R^{14}COX^D$ in the presence of a base to synthesize the compound represented by formula (D1'). As solvent and a base, a solvent and a base described in 1)-1 above can be used.

4") Method D": In the case where $R^3$ is a group represented by the formula: $-C(=O)-NR^{15}R^{16}$:

[Formula 33]

4")

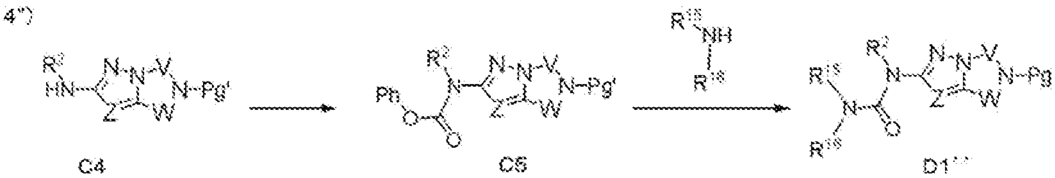

C4 C5 D1"

wherein each symbol is defined the same as above, and Ph is a phenyl group.

The above step is of reacting the compound represented by formula (C4) with phenyl chloroformate to form the compound represented by formula (C5), followed by reaction with a compound represented by ($R^{15}R^{16}NH$), and consequently synthesizing the compound represented by formula (D1").

In the reaction at the first step, a solvent and a base described in 1)-1 above can be used. Preferably, an ether (e.g., tetrahydrofuran, diethyl ether, dioxane, 1,2-dimethoxyethane, and the like) may be used as solvent to carry out the reaction, as well as an organic amine (e.g., triethylamine, and the like) may be used as abase. A reaction temperature and a reaction time are not specifically limited, but the reaction may be used at temperatures from -20 to 50˚C for a period of time from 0.5 to 12 hours. In place of phenyl chloroformate, p-nitrophenyl chloroformate can be used.

In the reaction at the second step, a solvent described in 1)-1 above can be used. Preferably, dimethylsulfoxide may be used to carry out the reaction. A reaction temperature and a reaction time are not specifically limited, but the reaction may be carried out at temperatures from -20 to 50˚C for a period of time from 0.5 to 12 hours.

Furthermore, in another method for synthesizing the compound represented by formula (D1"), it maybe synthesized by reacting the compound represented by formula (C4) with a corresponding carbamate or isocyanate.

5) Method E: Synthesis method of Compound I

[Formula 34]

5)

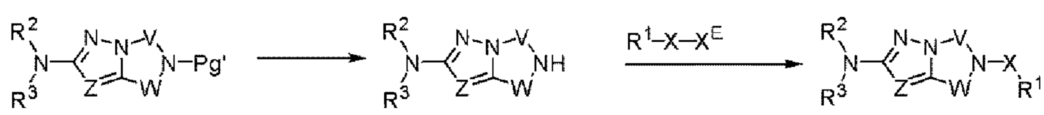


D1 D2 Compound I

wherein each symbol is defined the same as above. $X^E$ is halogen (e.g., Cl, Br, I, and the like) or leaving group described in 1)-1 above (e.g., OTf, OMs, and the like), and Pg' is an amino-protecting group (e.g., t-butoxycarbonyl

(Boc) group, benzyl group, acetyl group, benzoyl group, benzyloxycarbonyl group, and the like). Known compounds may be used as a compound represented by formula (D1) and a compound represented by $R^1$-X-$X^E$, and a compound derived from a known compound using a usual method may be also used.
The above step is of deprotecting the compound represented by formula (D1) from the Pg' group, followed by reaction with the above-described $R^1$-X-$X^E$ in the presence of a base to synthesize Compound I of the present invention. As a base, a base described in 1)-1 above can be used.

A deprotection is carried out using a method described in "Protective Groups in Organic Synthesis," Theodora W. Greene (John Wiley & Sons, Inc., New York), 2nd ed., 1991. Compound I of the present invention can be synthesized, for example, through a substitution reaction of the compound represented by formula (D2) with the compound represented by formula ($R^1$-X-$X^E$) using N,N-dimethylformamide (DMF) or dimethylsulfoxide (DMSO) as solvent, and a metal carbonate salt (e.g., sodium carbonate, potassium carbonate, calcium carbonate, cesium carbonate, and the like) as a base.

The reaction may be carried out for a period of time from 0.5 to 24 hours at temperatures from-20°C to a temperature at which a solvent used is refluxed.

Moreover, using the same method as method D described above, Compound I of the present invention can be synthesized by reacting the compound represented by formula (D2) with the above-described $R^1$-X-$X^E$ in the presence of a base, a palladium catalyst, and a phosphine ligand.

Compounds IA and IB can be synthesized similarly to Compound I.

In the case where a group represented by formula (G) is the group represented by formula (G1), (G5), or (G6):

6) Synthesis method of Compound I' of which W is a single bond.

[Formula 35]

6)

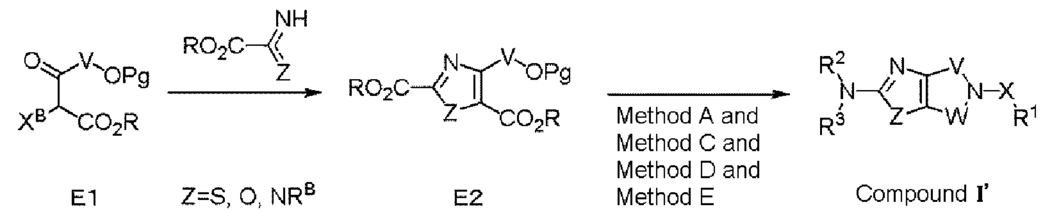


wherein each symbol is defined the same as above; R is C1-C6 alkyl; $X^B$ is halogen (e.g., Cl, Br, I, and the like); Pg is a hydroxy-protecting group (e.g., benzyl group and acetyl group, and the like). Known compounds may be used as a compound represented by formula (E1) and a compound represented by formula:

[Formula 36]

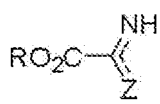

A compound derived from a known compound using a usual method may be also used.
The above step is of reacting the compound represented by formula (E1) using a method described in International Publication No. WO 2006/066174 pamphlet to synthesize a compound of formula (E2). An additional step is of reacting the compound of formula (E2) similarly to the above-described methods (methods A and C and D and/or E) to synthesize Compound I'.

7) Synthesis method of Compound I' of which W is - ($CR^8R^9$)n-.

[Formula 37]

7)

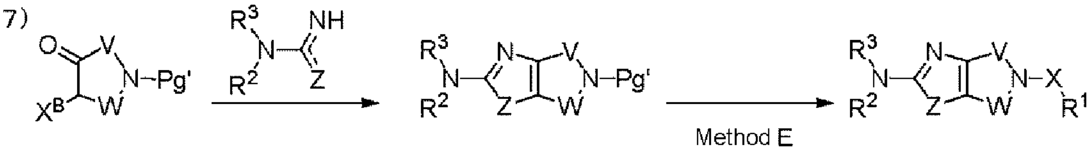


F1 Z=S, O, $NR^B$ F2 Compound I'

wherein each symbol is defined the same as above; $X^B$ is halogen (e.g., Cl, Br, I, and the like) ; and Pg' is an amino-protecting group (e.g., t-butoxycarbonyl (Boc) group, benzyl group, benzyloxycarbonyl group, and the like). Known compounds can be used as a compound represented by formula (F1) and a compound represented by formula:

[Formula 38]

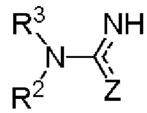

wherein Z = S, O, or $NR^8$
and a compound derived from a known compound using a usual method may be also used.
The above step is of reacting the compound represented by formula (F1) using the same method as 6) to synthesize the compound represented by formula (F2). An additional step is of reacting the compound of formula (F2) using the same method as the above-describedmethod (method E) to synthesize Compound I'.

In the case where a group represented by formula (G) is the group represented by formula (G4):

8) Synthesis method of Compound I"

[Formula 39]

8)

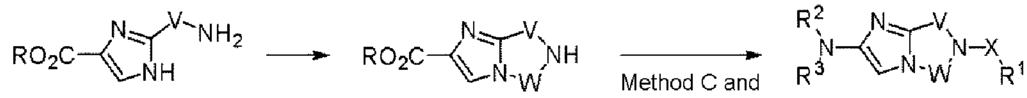


G1 G2 Compound I''

wherein each symbol is defined the same as above, R is C1-C6 alkyl. A known compound may be used as the compound represented by formula (G1), and a compound derived from a known compound using a usual method may be also used.
The compound represented by formula (G1) can reacted with paraformaldehyde to form a cyclic imine, followed by reduction of the imine with a reducing agent (e.g., sodium borohydride, sodium triacetoxyborohydride), consequently synthesizing the compound represented by formula (G2).

As solvent, a solvent described in 1)-1 above can be used.

The reaction may be carried out at temperatures from -50 to 50˚C for a period of time from 0.5 to 24 hours. An additional step is of reacting the compound represented by formula (G2) similarly to the above-described methods (methods C and D and E) to synthesize Compound I".

In the case where a group represented by formula (G) is the group represented by (G1); W is a single bond; and V is $-CR^6=CR^7-$:

9) Synthesis method of Compound I'''

[Formula 40]

9)

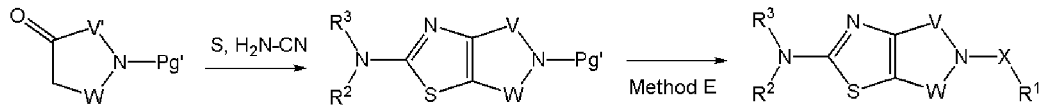


H1 H2 Compound I'''

wherein each symbol is defined the same as above; V' is -$CR^6H$-$CR^7H$-; and Pg' is an amino-protecting group (e.g., t-butoxycarbonyl (Boc) group, benzyl group, benzyloxycarbonyl group, and the like). A known compound can be used as a compound represented by formula (H1), and a compound derived from a known compound using a usual method may be also used.
The above step is of reacting the compound represented by formula (H1) with sulfur and cyanamide to synthesize the compound represented by formula (H2).
As solvent, a solvent described in 1)-1 above can be used. For example, the reaction may be carried out with pyridine.
A reaction temperature and a reaction time are not specifically limited, but the reaction may be carried out for a period of time from 0.5 to 24 hours at temperatures from 20°C to a temperature at which a solvent used is refluxed.
An additional step is of reacting the compound represented by formula (H2) similarly to the above-described method (method E) to synthesize Compound I'''.
In the above general synthesis methods, reaction steps are not limited to the above ones, and the compound of the present invention can be synthesized after changing the order of reactions.
For example, the compound I of the present invention can be synthesized by reacting the compound represented by formula (C1) using method E, method C, and method D in that order.
The compound of the present invention can be protected with a protecting group (s). For example, it can be produced by protecting an appropriate substituent using a method known in the relevant field among, typically, halogen (I, Br, Cl, F, and the like), lower (which, here, typically refers to C1-C6, but is not limited thereto) alkoxy, lower (e.g., C1-C6) alkylthio, lower (e.g., C1-C6) alkylsulfonyloxy, arylsulfonyloxy, and the like). Examples of such protecting groups can include protecting groups, such as ethoxy carbonyl, t-butoxycarbonyl, acetyl, benzyl, and the like, which are described in Protective Groups in Organic Synthesis, written by T. W. Green, John Wiley & Sons Inc. (2nd edition, 1991), or the like. Methods for the introduction and removal of a protecting group are methods commonly used in synthetic organic chemistry (see, for example, methods described in Protective Groups in Organic Synthesis, written by T. W. Greene, John Wiley & Sons Inc. (2nd edition, 1991)) or the like, or can be obtained in accordance therewith. Furthermore, a functional group included in each substituent can be converted by a known method (for example, those described in Comprehensive Organic Transformations, written by R. C. Larock (1989), and the like) in addition to the above production methods. Some of the compounds of the present invention can be used as a synthetic intermediate, leading to a new derivative. Intermediates and target compounds produced in each of the above production methods can be isolated andpurifiedby a purification method commonly used in synthetic organic chemistry, for example, subjecting them to neutralization, filtration, extraction, washing, drying, concentration, recrystallization, any kind of chromatography, or the like. Furthermore, intermediates can be subjected to a next reaction without any purification.

(Medicament)

The compound of the present invention or a pharmaceutically acceptable salt can be administered alone as it is, but it is usually preferable to provide it as a variety of pharmaceutical formulations. Furthermore, those pharmaceutical formulations are used for an animal and a human.
With regard to an administration route, it is preferable to use the most effective route on therapy. It can be peroral administration, or parenteral administration, for example, intrarectal; intraoral; subcutaneous; intramuscular; intravenous; percutaneous such as application; pulmonary with a spray such as powder, aerosol, and the like; or the like.
Administration forms include capsule, tablet, granule, powder, syrup, emulsion, suppository, injection, and the

like. Aliquidpreparation, such as emulsion and syrup, which is suitable for oral administration, can be produced using: water; sugars such as sucrose, sorbite, fructose, and the like; glycols such as polyethylene glycol, propylene glycol, and the like; oils such as sesame oil, olive oil, soybean oil, and the like; antiseptics such as p-hydroxybenzoate esters, and the like; and flavors such as strawberry flavor, peppermint, and the like. Furthermore, a capsule, a tablet, a powder, a granule, and the like can be produced using: an excipient such as lactose, glucose, sucrose, mannite, and the like; a disintegrator such as starch, sodium alginate and the like; a lubricant such as magnesium stearate, talc, and the like; a binder such as polyvinyl alcohol, hydroxypropylcellulose, gelatin, and the like; surfactant such as fatty ester and the like; and a plasticizer such as glycerin and the like.

A formulation suitable for parenteral administration preferably consists of a sterilized-water-based formulation containing an active compound and being isotonic to blood of a recipient. For example, in the case of injection, a solution for an injection is prepared using: a carrier consisting of a salt solution, a glucose solution, or a mixture of salt water and a glucose solution; and the like.

A topical formulation is prepared by dissolving or suspending an active compound in one or more kinds of media, such as mineral oil, petroleum, polyalcohol, and the like, or other bases used for a topical pharmaceutical formulation. A formulation for enteral administration is prepared using a general carrier such as cacao butter, hydrogenated fat, hydrogenated fatty carboxylic acid, and the like, and then provided as a suppository.

In the present invention, to a parenteral agent can be added one or more kinds of auxiliary ingredients selected from glycols, oils, flavors, antiseptics (including antioxidants), excipients, disintegrators, lubricants, binders, surfactants, plasticizer, and the like exemplified in an oral agent.

An effective dose and the frequency of administration of the compound of the present invention or a pharmaceutically acceptable salt are different according to administration form, the age of a patient, weight, characteristics or the severity, and the like of a condition to be treated. Generally, a dose is 0.01 to 1000 mg/person per day, preferably 5 to 500 mg/person per day, and a frequency of administration is preferably once per day or divided administration.

All of the compounds of the present invention are immediately applicable to therapeutic use as a kinase inhibitor for controlling kinase dependent diseases in mammals, particularly, a kinase inhibitor related to phosphatidylinositol-3-kinase.

The compound of the present invention is preferably a compound having an $IC_{50}$ value in a range of 0.1 nM to 10 $\mu$M. A certain compound of the present invention wherein the compound is capable of specifically inhibiting one of four types of Class I phosphatidylinositol-3-kinase (e.g., $\alpha$, $\beta$, $\gamma$, and $\delta$) can be selected. For example, by utilizing a compound selectively inhibiting only y type, merely diseases related to inflammation, such as a lymphocyte and the like can be treated. In the case that a compound is $\alpha$-type selective, the utility as a selective anticancer agent can be found.

Phosphatidylinositol-3-kinase dependent diseases include inflammatory diseases (allergic diseases (allergic dermatitis/allergic rhinitis, and the like), articular rheumatism, anaphylaxis, and the like), arteriosclerosis, vascular/circulatory diseases, cancer/tumors (hyperproliferative malfunction), immune system diseases, cell-proliferative diseases, infectious diseases, and the like initiated/maintained by unusual phosphatidylinositol-3-kinase enzyme activity. Examples thereof include psoriasis, pulmonary fibrosis, glomerulonephritis, cancers, atherosclerosis, and antiangiogenesis (e.g., tumor growth, diabetic retinopathy). Specifically, for example, the pharmaceutical composition of the present invention may be used for the prophylaxis and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernalkeratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The present invention is also related to a system, an apparatus, and a kit for producing the pharmaceutical composition of the present invention. It is understood that, as elements of such a system, an apparatus, and a kit, matters publicly known in the relevant field are available, and those skilled in the art can appropriately design them.

The present invention is also related to a system, an apparatus, and a kit using the compound of the present invention, a pharmaceutically acceptable salt thereof, or a prodrug thereof (such as a hydrate thereof and the like). It is understood that, as elements of such a system, an apparatus, and a kit, matters publicly known in the relevant field are available, and those skilled in the art can appropriately design them.

Wortmannin, which is a classical PI3K inhibitor, has low inhibition selectivity, high toxicity, and the like, and consequently is highly cytotoxic. Thus, by using a usual test to measure cytotoxicity, a PI3K inhibitor (or another class of a kinase inhibitor) that intends to cause an unpreferable side effect due to lack of the selectivity can be identified.

The compound of the present invention is a compound having utility as a medicament. Here, utility as a medicament includes the following points: the compound has good metabolic stability; the induction of a drug-metabolizing enzyme is low; the inhibition of a drug-metabolizing enzyme which metabolizes another drug is also low; the compound has high oral absorbency; the clearance is low; the half-life is sufficiently long to express the efficacy; and the like.

Reference including scientific literature, patents, patent applications, and the like cited herein is incorporated herein by reference in its entirety at the same level as the case where each reference is specifically described.

Hereinafter, Examples describe the constitution of the present invention in more detail, but the present invention is not limited thereto. Regarding reagents and the like used below, unless specified otherwise, those commercially available are used.

EXAMPLES

Hereinafter, the present invention is described in more detail with Examples. However, the technical scope of the present invention is not limited by the Examples and the like.

In Examples, abbreviations described below are used.

DMSO: Dimethylsulfoxide

HPLC: High Performance Liquid Chromatography

Me: methyl

(Method for identifying a compound)

The LC/MS data and NMR spectra of compounds of the present application and intermediates thereof are measured under a condition selected from the four conditions below (Methods A to D), and the retention times and $[M+H]^+$ are shown.

(Method A)

Column: Waters Phenomenex Luna C18 (2) (5 μm, 50 x 4.6 mm) Flow rate: 3 mL/min

UV detection wavelength: 254 nm

Mobile phase: [A] was aqueous 0.1% formic-acid-containing solution, and [B] was 0.1% formic-acid-containing solution in acetonitrile.

From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Method B)

Column: Waters Xbridge C18 (5 μm 50 x 4.6 mm)
Flow rate: 2 mL/min
UV detection wavelength: 254 nm
Mobile phase: [A] was aqueous 10 mmol/L ammonium-carbonate-containing solution, and [B] was acetonitrile.

From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Method C)

Column: Shimadzu Shim-pack XR-ODS (2.2 μm, 50 x 3.0 mm) Flow rate: 1.6 mL/min
UV detection wavelength: 254 nm
Mobile phase: [A] was aqueous 0.1% formic-acid-containing solution, and [B] was 0.1% formic-acid-containing solution in acetonitrile.

From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Method D)

Column: Waters Xbrigde C18 (5μm 4.6×50mm)
Flow rate: 3 mL/min
UV detection wavelength: 254nm
Mobile phase: [A] was aqueous 0.1% formic-acid-containing solution, and [B] was 0.1% formic-acid-containing solution in acetonitrile.

From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Example 1 Synthesis of Compound I-154)

In this Example, as an example of a representative intermediate, Compound I-154 was produced. Hereinafter the scheme thereof is described in detail.

[Formula 41]

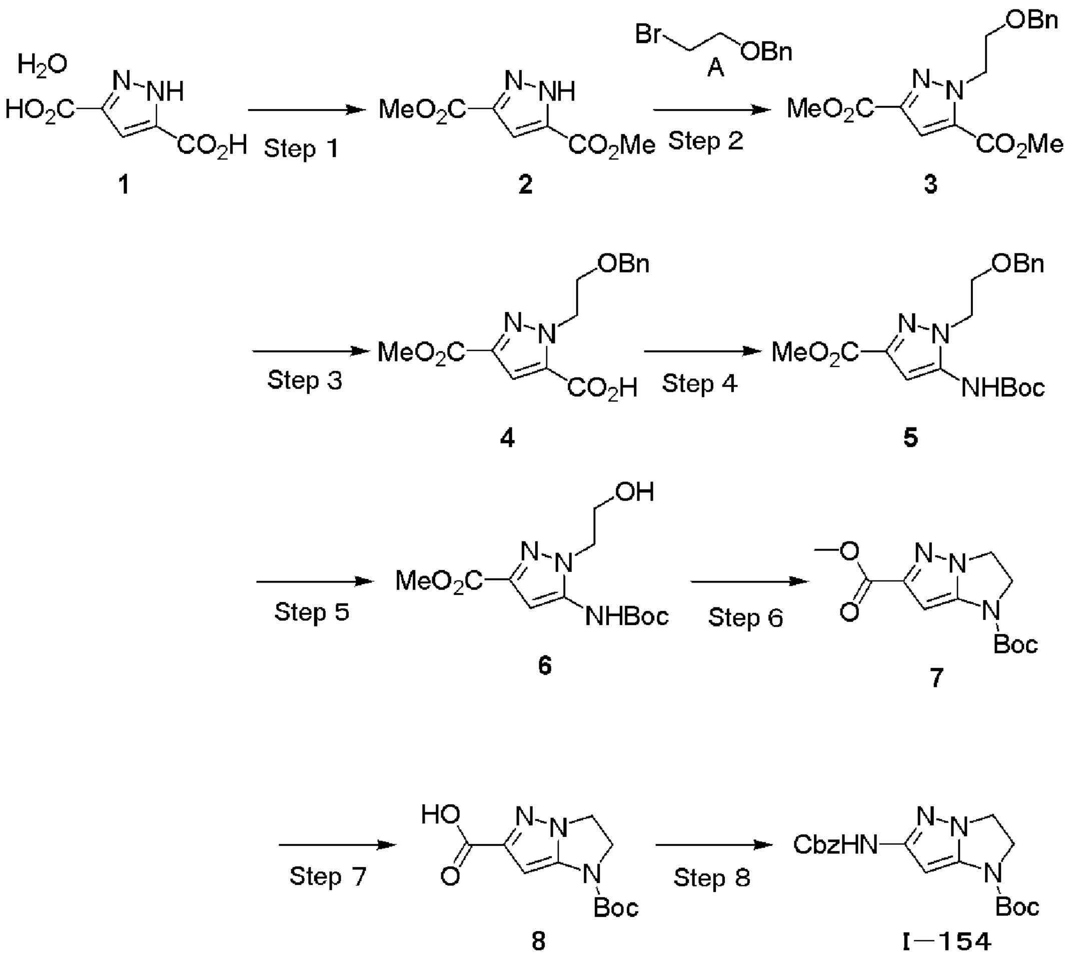


1 2 3 4 5 6 7 8 I−154

Step 1

To a solution of Compound 1 (60 g, 345 mmol) in methanol (600 mL), under nitrogen atmosphere, thionyl chloride (75 mL, 1034 mmol) was added at 0˚C, followed by heating at reflux for 4 hours. The reaction solution was concentrated *in vacuo* to yield crude product 2 (64.3 g).
LC/MS (Method A): 0.93 min, $[M+H]^+$ = 185.

Step 2

To an acetonitrile (250 mL) solution of crude product 2 (24.5 g) obtained from Step 1, benzyl-2-bromoethylether A (27.3 mL, 173 mmol) and potassium carbonate (27.5 g, 199 mmol) were added, under nitrogen atmosphere, followed by heating at reflux for 4 hours and a half. The reaction solution was filtered and then the filtrate was concentrated *in vacuo.* The residue was dissolved in ethyl acetate, washed sequentially with water and saturated brine, then dried with anhydrous magnesium sulfate and concentrated *in vacuo* to yield crude product 3 (54.6 g).
LC/MS (Method A): 1.97 min, $[M+H]^+$ = 319.

Step 3

To a methanol (250 mL) solution of crude product 3 (40.8 g) obtained from Step 2, potassium hydroxide (2.0 mol/L, a methanol solution) (64.1 mL, 128 mmol) was added. The reaction solution was stirred under nitrogen atmosphere at room temperature overnight, and then the reaction solution was concentrated *in vacuo.* The residue was dissolved in water, acidified, and then extracted with ethyl acetate. The extract was dried with anhydrous magnesium sulfate, and then concentrated *in vacuo* to yield crude product 4 (50.8 g). LC/MS (Method A): 1.54 min, $[M+H]^+$ = 305.

Step 4

To a t-butylalcohol (110 mL) solution of crude product 4 (16.1 g) obtained from Step 3, under nitrogen atmosphere, diphenylphosphoryl azide (13.7 mL, 63.4 mmol) and triethylamine (9.53 mL, 68.7 mmol) were added. The solution was then stirred at 100˚C for 4 hours. Water was added to the reaction solution, and then extracted with ethyl acetate. The extract was washed sequentially with water and saturated brine, and then dried with anhydrous magnesium sulfate. After concentrating *in vacuo,* purification by silica gel column chromatography (*n*-hexane: ethyl acetate = 2:1 → 1:1) yielded Compound 5 (13.1 g, 66%).
LC/MS (Method A): 2.13 min, $[M+H]^+$ = 376
$^1$H-NMR ($CDCl_3$) δ: 7.91 (1H, s), 7.35-7.25 (5H, m), 6.82 (1H, s), 4.51 (2H, s), 4.37 (2H, t, J = 4.3 Hz), 3.90 (3H, s), 3.85 (2H, t, J = 4.3 Hz), 1.44 (9H, s).

Step 5

To a solution of Compound 5 (13.1 g, 34.9 mmol) in methanol (150 mL), 20% palladium hydroxide-carbon (50% of water content, 1.3 g) was added. The solution was then stirred under hydrogen atmosphere at room temperature for 6 hours. The reaction solution was filtered, and then the filtrate was concentrated *in vacuo* to yield Compound 6 (10.3 g, 100%) .
LC/MS (Method A): 1.29 min, $[M+H]^+$ = 286
$^1$H-NMR ($CDCl_3$) δ: 7.87 (1H, s), 6.75 (1H, s), 4.28 (2H, s), 4.02 (2H, s), 3.89 (3H, s), 3.02 (1H, br s), 1.50 (9H, s).

Step 6

To a solution of Compound 6 (5.52 g, 19.4 mmol) in tetrahydrofuran (250 mL), under nitrogen atmosphere, tri-*n*-butylphosphine (5.73 mL, 23.2 mmol) and 1,1'-(azodicarbonyl)dipiperidine (5.86 g, 23.2 mmol) were added at 0˚C. The solution was then stirred at room temperature for 2 hours and a half. The reaction solution was filtered, and then the filtrate was concentrated *in vacuo.* Water was added to the residue, and then extracted with ethyl acetate. The extract was washed sequentially with water and saturated brine, and then dried with anhydrous magnesium sulfate. After concentrating *in vacuo,* purification by silica gel column chromatography (*n*-hexane:ethyl acetate = 1:1 → 1:2) yielded Compound 7 (4.59 g, 89%).
LC/MS (Method A): 1.54 min, $[M+H]^+$ = 268
$^1$H-NMR ($CDCl_3$) δ: 6.17 (1H, s), 4.39 (4H, s), 3.91 (3H, s), 1.57 (9H, s).

Step 7

To a solution of Compound 7 (3.51 g, 13.1 mmol) in tetrahydrofuran:methanol (1:1, 50 mL), aqueous 1 mol/L lithium hydroxide solution (26.3 mL, 26.3 mmol) was added. The reaction solution was then stirred at room temperature for one and a half hours. The reaction solution was then concentrated *in vacuo.* The residue was dissolved in water, acidified, and then extracted with ethyl acetate. The extract was dried with anhydrous magnesium sulfate, and then concentrated *in vacuo* to yield Compound 8 (2.50 g, 75%).
LC/MS (Method A): 1.26 min, $[M+H]^+$ = 254
$^1$H-NMR (DMSO-$d_6$) δ: 12.64 (1H, br s), 5.95 (1H, s), 4.34 (4H, s), 1.50 (9H, s).

Step 8

To a suspension of Compound 8 (2.50 g, 9.87 mmol) in toluene (50 mL), under nitrogen atmosphere, benzylalcohol (1.23 mL, 11.9 mmol), diphenylphosphoryl azide (2.55 mL, 11.9 mmol), and triethylamine (1.78mL, 12.8 mmol) were added. The reaction solution was then stirred at 100˚C for 5 hours and a half. Water was added to the reaction solution, and then extracted with ethyl acetate. The extract was concentrated *in vacuo,* and then the residue was washed with ethyl acetate:methanol (1:1) to yield Compound I-154 (2.19 g, 62%).
LC/MS (Method A): 1.98 min, $[M+H]^+$ = 359
$^1$H-NMR (DMSO-$d_6$) δ: 9.99 (1H, br s), 7.40-7.33 (5H, m), 5.83 (1H, br s), 5.12 (2H, s), 4.21 (2H, t, J = 7.2 Hz), 4.12 (2H, t, J = 7.2 Hz), 1.48 (9H, s).

(Example 1-1 Synthesis of Compounds I-155, I-1, and I-2)

In this Example, Compounds I-155, I-1, and I-2 of the present invention were produced from Compound I-154.
[Formula 42]

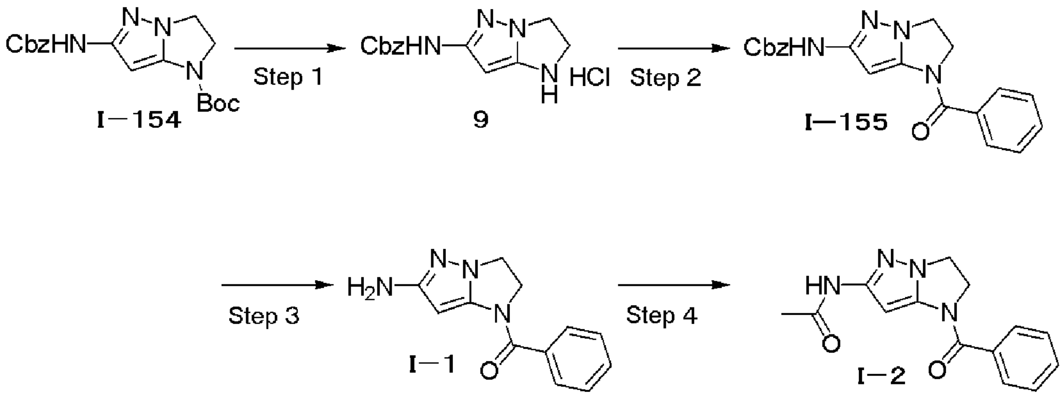


Step 1

To Compound I-154 (203 mg, 0.567 mmol), hydrochloric acid (4 mol/L, a dioxane solution) (2.0 mL, 8.0 mmol) was added at 0˚C. The reaction solution was stirred at room temperature for 2 hours, and then the reaction solution was concentrated *in vacuo* to yield crude product 9 (174 mg). LC/MS (Method A): 1.09 min, $[M+H]^+$ = 259.

Step 2

To a tetrahydrofuran (3.5 mL) solution of crude product 9 (173 mg) obtained from Step 1, under nitrogen atmosphere, triethylamine (244 μL, 1.758 mmol) and benzoyl chloride (88 μL, 0.726 mmol) were added at 0˚C. The solution was then stirred at room temperature for 2 hours. Aqueous saturated sodium bicarbonate solution was added to the reaction solution, and then extracted with ethyl acetate. The extract was washed sequentially with water and saturated brine, and then dried with anhydrous magnesium sulfate. After concentrating *in vacuo,* purification by aminosilica gel column chromatography (*n*-hexane : ethyl acetate = 1:2 → 1:3) yielded Compound I-155 (156 mg, 73%).
LC/MS (Method A): 1.71 min, $[M+H]^+$ = 363
$^1$H-NMR (DMSO-$d_6$) δ: 10.01 (1H, br s), 7.58-7.32 (10H, m), 6.26 (0.5H, br s), 5.03 (2H, br s), 4.73 (0.5H, br s), 4.45 (2H, t, J = 7.3Hz), 4.19 (2H, t, J=7.3Hz).

Step 3

To a solution of Compound I-155 (151 mg, 0.417 mmol) in tetrahydrofuran:methanol (1:1, 10 mL), 20% palladium hydroxide-carbon (50% of water content, 15 mg) was added. The reaction solution was then stirred under hydrogen atmosphere at room temperature overnight. After the reaction solution was filtered, the filtrate was concentrated *in vacuo,* and then purified by silica gel column chromatography (chloroform:methanol = 100:0 → 96:4) to yield Compound I-1 (77.1 mg, 81%).
LC/MS (Method A): 0.68 min, $[M+H]^+$ = 229
$^1$H-NMR (DMSO-$d_6$) δ: 7.57-7.49 (5H, m), 5.41 (0.3H, br s), 4.69 (2H, br s), 4.38 (2H, t, J = 7.5Hz), 4.01 (2H, br s), 3.77 (0.7H, br s).

Step 4

To a suspension of Compound I-1 (28.8 mg, 0.103 mmol) in tetrahydrofuran (1.0 mL), acetic acid (17.8 mg, 0.166 mmol), O-(7-azabenzotriazol-1-yl)-N,N,N',N'-tetramethyluronium hexafluorophosphate (HATU) (44 mg, 0.117 mmol), and N-methylmorpholine (25.7 μL, 0.234 mmol) were added. The reaction solution was stirred under nitrogen atmosphere at room temperature overnight. Aqueous saturated sodium bicarbonate solution was added to the reaction solution, and then extracted with ethyl acetate. The extract was washed with saturated brine, and then dried with anhydrous magnesium sulfate. After concentrating *in vacuo,* purification by silica gel column chromatography (chloroform:methanol =99:1 → 95:5) yielded Compound I-2 (17.8 mg, 96%).
LC/MS (Method A): 0.96 min, $[M+H]^+$ = 271
$^1$H-NMR ($CDCl_3$) δ: 8.05 (1H, s), 7.59-7.55 (3H, m), 7.48 (2H, t, J = 7.1 Hz), 5.31 (1H, br s), 4.57 (2H, t, J = 7.3 Hz), 4.27 (2H, t, J = 7.3 Hz), 2.06 (3H, s).

(Example 1-2 Synthesis of Compounds I-156 and I-5)

[Formula 43]

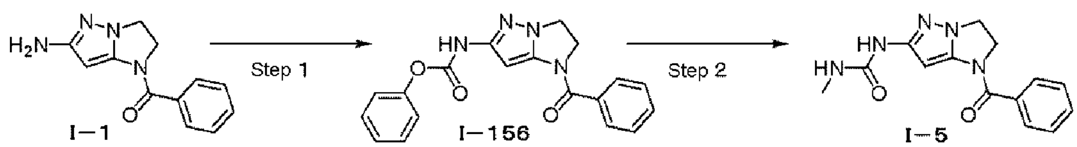


I-1 I-156 I-5

Step 1

To a suspension of Compound I-1 (17.4 mg, 0.076 mmol) in tetrahydrofuran (1.0 mL), under nitrogen atmosphere, triethylamine (16 μL, 0.114 mmol) and phenyl chloroformate (12 μL, 0.091 mmol) were added at 0˚C. The solution was then stirred at room temperature for one and a half hours. Water was added to the reaction solution, and then extracted with ethyl acetate. The extract was washed with saturated brine, dried with anhydrous magnesium sulfate, and then concentrated *in vacuo* to yield crude product I-156 (33.6 mg).
LC/MS (Method A): 1.66 min, $[M+H]^+$ = 349.

Step 2

To a dimethylsulfoxide (900 μL) solution of crude product I-156 (33.6 mg) obtained from Step 1, methylamine (2.0 mol/L solution in tetrahydrofuran) (228 μL, 0.456 mmol) was added. The solution was then stirred under nitrogen atmosphere at room temperature for one and a half hours. Separation and purification by reverse phase HPLC yielded Compound I-5 (12.5 mg, 58%).
LC/MS (Method A): 0.99 min, $[M+H]^+$ = 286
$^1$H-NMR ($CDCl_3$) δ: 7.58-7.36 (7H, m), 4.54 (2H, br s), 4.36 (1H, br s), 4.21 (2H, t, J = 7.8 Hz), 2.85 (3H, d, J = 4.3 Hz).

(Example 1-3 Synthesis of Compound I-4)

[Formula 44]

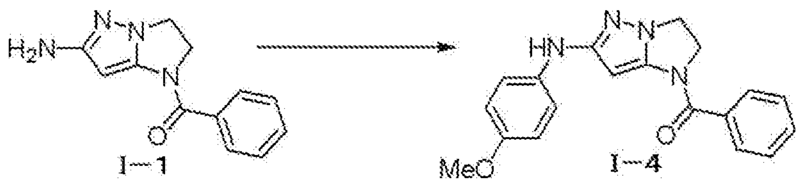

I-1 I-4

To a suspension of Compound I-1 (20.1 mg, 0.088 mmol) in dioxane (1.0 mL), p-bromoanisole (13 μL, 0.106 mmol), 4,5-bis(diphenylphosphino)-9,9-dimethylxanthene (7.6 mg, 0.013 mmol), cesium carbonate (86 mg, 0.264 mmol), and palladium(II) acetate (2.0 mg, 8.81 μmol) were added. The solution was then stirred under nitrogen atmosphere at 100˚C for 8 hours. Water was added to the reaction solution, and then extracted with ethyl acetate. The extract was washed sequentially with water and saturated brine, and then dried with anhydrous magnesium sulfate. After concentrating *in vacuo*, separation and purification by reverse phase HPLC yielded Compound I-4 (10.6 mg, 36%).
LC/MS (Method A): 1.65 min, $[M+H]^+$ = 335
$^1$H-NMR ($CDCl_3$) δ: 7.59-7.44 (6H, m), 7.04 (2H, br s), 6.79 (2H, d, J = 8.3 Hz), 5.66 (1H, br s), 4.53 (2H, br s), 4.25 (2H, t, J = 7.3 Hz), 3.75 (3H, s).

(Example 1-4 Synthesis of Compounds I-157, I-8, and I-11)

[Formula 45]

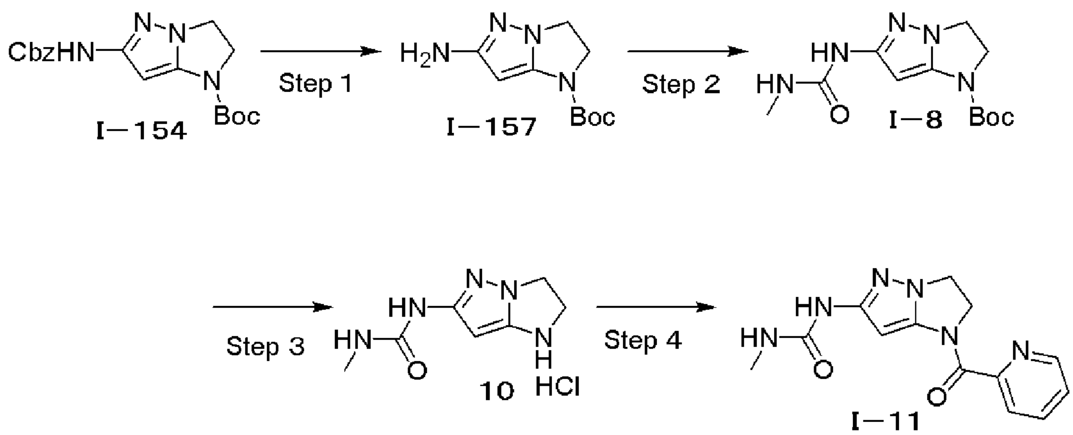


Step 1

To a solution of Compound I-154 (2.42 g, 6.75 mmol) in tetrahydrofuran:methanol (1:1, 110 mL), 20% palladium hydroxide-carbon (50% of water content, 480 mg) was added. The solution was then stirred under hydrogen atmosphere at room temperature overnight. The reaction solution was filtered, and then the filtrate was concentrated *in vacuo* to yield Compound I-157 (1.52 g, 100%).
LC/MS (Method A): 0.79 min, $[M+H]^+$ = 225
$^1$H-NMR ($CDCl_3$) δ: 5.13 (1H, s), 4.23 (2H, t, J = 7.5 Hz), 4.08 (2H, t, J = 7.5 Hz), 1.54 (9H, s).

Step 2

To a solution of Compound I-157 (1.47 g, 6.55 mmol) in methylene chloride (30 mL), under nitrogen atmosphere, pyridine (794 μL, 9.83 mmol) andp-nitrophenyl chloroformate (1. 72 g, 8.52 mmol) were added at 0˚C. The solution was then stirred at room temperature for one and a half hours. To the reaction solution, under nitrogen atmosphere, methylammonium chloride (1.33g,19.7mmol) and triethylamine (4.54 mL, 32.8 mmol) were added at 0˚C. The solution was then stirred at room temperature for 1 hour. Aqueous saturated sodium bicarbonate solution was added to the reaction solution, and then extracted with ethyl acetate. The extract was washed sequentially with aqueous saturated sodium bicarbonate solution, water, and saturated brine then dried with anhydrous magnesium sulfate. After concentrating *in vacuo,* purification by aminosilica gel column chromatography (chloroform) yielded Compound I-8 (1.50 g, 81%).
LC/MS (Method A): 1.25 min, $[M+H]^+$ = 282
$^1$H-NMR ($CDCl_3$) δ: 7.52 (2H, br s), 5.30 (1H, br s), 4.28 (2H, t, J = 7.6 Hz), 4.16 (2H, t, J = 7.6 Hz), 2.90 (3H, d, J = 4.3 Hz), 1.55 (9H, s).

Step 3

To a solution of Compound I-8 (1.50 g, 5.31 mmol) in methylene chloride (45 mL), hydrochloric acid (4 mol/L, a dioxane solution) (13.3 mL, 53.1 mmol) was added. The reaction solution was stirred at room temperature for 5 hours and a half, and then the reaction solution was concentrated *in vacuo* to yield crude product 10 (1.20 g).
LC/MS (Method A): 0.18 min, $[M+H]^+$ = 182.

Step 4

To a dimethylformamide (500 μL) suspension of crude product 10 (18.0 mg, 0.083 mmol) obtained from Step 3, picolinic acid (12.2 mg, 0.099 mmol), O-(7-azabenzotriazol-1-yl)-N,N,N',N'-tetramethyluronium hexafluorophosphate (HATU) (40.9 mg, 0.108 mmol), and N-methylmorpholine (24 μL, 0.215 mmol) were added. The reaction solution was then stirred under nitrogen atmosphere at room temperature overnight. Aqueous saturated sodium bicarbonate solution was added to the reaction solution, and then the precipitated solid was washed with water to yield Compound I-11 (10.8 mg, 45%).

LC/MS (Method A): 0.87 min, $[M+H]^+ = 287$
$^1$H-NMR (DMSO-$d_6$) δ: 8.86 (1H, s), 8.69 (1H, br s), 8.03-7.97 (2H, m), 7.65-7.62 (2H, m), 6.74 (1H, br s), 4.84 (2H, t, J = 7.5 Hz), 4.20 (2H, t, J= 7.5 Hz), 2.68 (3H, d, J = 4.3 Hz).

(Example 1-5 Synthesis of Compounds I-51 and I-52)

[Formula 46]

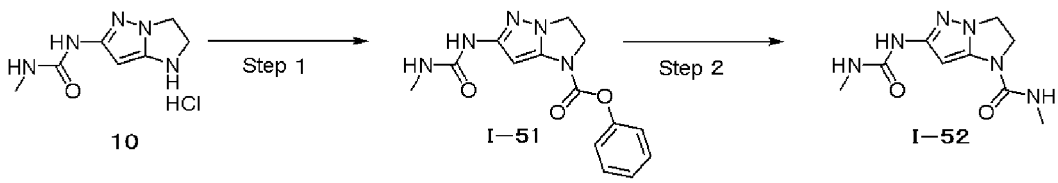


10 I-51 I-52

Step 1

To a tetrahydrofuran (1.0 mL) suspension of crude product 10 (50.7mg) obtained from Step 3 of Example 1-4, undernitrogen atmosphere, triethylamine (97 μL, 0.699 mmol) and phenyl chloroformate (37 μL, 0.303 mmol) were added at 0˚C. The solution was then stirred at room temperature for one and a half hours. Water was added to the reaction solution, and then extracted with ethyl acetate. The extract was washed with saturated brine, and then dried with anhydrous magnesium sulfate. After concentrating *in vacuo,* purification by silica gel column chromatography (chloroform:methanol = 100:0 → 95:5) yielded Compound I-51 (18.1 mg, 26%).
LC/MS (Method A): 1.26 min, $[M+H]^+ = 302$
$^1$H-NMR (DMSO-$d_6$) δ: 8.83 (1H, s), 7.46 (2H, t, J = 7.7 Hz), 7.31-7.25 (3H, m), 6.72-6.64 (1H, m), 5.78 (1H, s), 4.54 (1H, br s), 4.35 (1H, br s), 4.22 (2H, br s), 2.65 (3H, s).

Step 2

To a solution of Compound I-51 (14.5 mg, 0.048 mmol) in dimethylsulfoxide (600 μL), methylamine (2.0 mol/L solution in tetrahydrofuran) (144 μL, 0.289 mmol) was added. The solution was then stirred under nitrogen atmosphere at room temperature overnight. Methylamine (2.0 mol/L solution in tetrahydrofuran) (144 μL, 0.289 mmol) was added to the reaction solution. The reaction solution was then stirred under nitrogen atmosphere at 60˚C for 6 hours. Separation and purification by reverse phase HPLC yielded Compound I-52 (11.0 mg, 96%).
LC/MS (Method A): 0.54 min, $[M+H]^+ = 239$
$^1$H-NMR (DMSO-$d_6$) δ: 8.75 (1H, s), 6.91 (1H, br s), 6.76 (1H, s), 5.65 (1H, s), 4.18 (2H, t, J = 7.1 Hz), 4.10 (2H, t, J = 7.1 Hz), 2.66 (3H, s), 2.65 (3H, s).

(Example 1-6 Synthesis of Compound I-50)

[Formula 47]

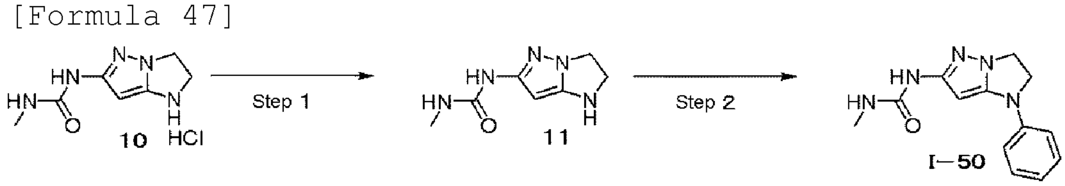


10 11 I-50

Step 1

To a chloroform (1.0 mL) suspension of crude product 10 (147.0 mg) obtained from Step 3 of Example 1-4,

triethylamine (281 μL, 2.026 mmol) was added at 0°C. The solution was then stirred at room temperature for 30 0 minutes. The reaction solution was purified by silica gel column chromatography (chloroform: methanol = 100:0 → 90: 10) to yield Compound 11 (113.5 mg, 93%).
LC/MS (Method A): 0.19 min, $[M+H]^+$ = 182
$^1$H-NMR (DMSO-$d_6$) δ: 8.58 (1H, s), 7.10 (1H, br s), 5.74 (1H, s), 5.01 (1H, s), 3.86 (2H, t, J = 7.5 Hz), 3.70 (2H, t, J = 7.5 Hz), 2.65 (3H, d, J = 4.5 Hz).

Step 2

To a suspension of Compound 11 (18.4 mg, 0.102 mmol) in dioxane (600 μL), bromobenzene (13 μL, 0.122 mmol), 4,5-bis(diphenylphosphino)-9,9-dimethylxanthene (8.8 mg, 0.015 mmol), cesium carbonate (99 mg, 0.305 mmol), and palladium(II) acetate (2.3 mg, 10.2 μmol) were added. The solution was then stirred under nitrogen atmosphere at 100°C for 7 hours. Water was added to the reaction solution, and then extracted with ethyl acetate. The extract was washed sequentially with water and saturated brine, and then dried with anhydrous magnesium sulfate. After concentrating *in vacuo*, separation and purification by reverse phase HPLC yielded Compound I-50 (13.2 mg, 51%).
LC/MS (Method A): 1.27 min, $[M+H]^+$ = 258
$^1$H-NMR (DMSO-$d_6$) δ: 8.81 (1H, s), 7.37 (2H, t, J = 7.3 Hz), 7.01 (2H, d, J = 8.1 Hz), 6.93 (1H, t, J = 7.3 Hz), 6.85 (1H, br s), 5.73 (1H, s), 4.30 (2H, t, J = 7.7 Hz), 4.16 (2H, t, J = 7.7 Hz), 2.68 (3H, d, J = 4.3 Hz).

(Example 1-7 Synthesis of Compound 1-53)

[Formula 48]

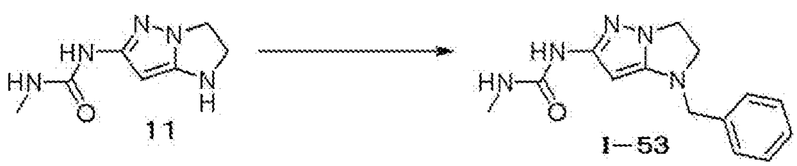

11

I-53

To a solution of Compound 11 (16.8 mg, 0.093 mmol) in dimethylsulfoxide (500 μL), benzyl bromide (14 μL, 0.121 mmol) and potassium carbonate (19.2 mg, 0.139 mmol) were added. The solution was then stirred under nitrogen atmosphere at room temperature for 2 hours and a half. After the reaction solution was filtered, the filtrate was separated andpurifiedby reverse phase HPLC to yield Compound I-53 (9.0 mg, 36%).
LC/MS (Method D): 1.06 min, $[M+H]^+$ = 272
$^1$H-NMR ($CDCm_3$) δ: 7.70 (1H, br s), 7.36-7.32 (5H, m), 7.07 (1H, br s), 4.74 (1H, s), 4.19 (2H, s), 3.96 (2H, t, J = 7.3 Hz), 3.57 (2H, t, J = 7.3 Hz), 2.88 (3H, d, J = 3.3 Hz) .

(Example 1-8 Synthesis of Compound I-6)

[Formula 49]

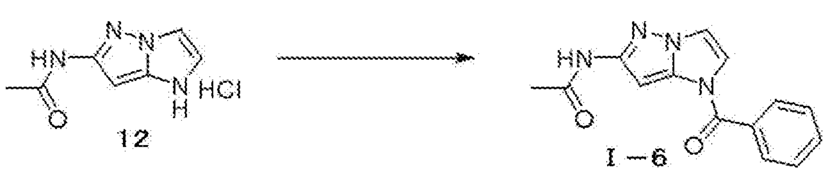

12

I-6

Compound 12 was synthesized using a method described in Synthetic Communications, 29(2), 311-341 (1999). To a suspension of Compound 12 (32.0 mg, 0.160 mmol) in dimethylformamide (500 μL), benzoic acid (23.4 mg, 0.191

mmol) , O-(7-azabenzotriazol-1-yl)-N,N,N',N'-tetramethyluronium hexafluorophosphate (HATU) (79.0 mg, 0.207 mmol), and N-methylmorpholine (46 μL, 0.415 mmol) were added. The reaction solution was then stirred under nitrogen atmosphere at room temperature overnight. Aqueous saturated sodium bicarbonate solution was added to the reaction solution, and then extracted with ethyl acetate. The extract was washed sequentially with water and saturated brine, and then dried with anhydrous magnesium sulfate. After concentrating *in vacuo,* separation and purification by reverse phase HPLC yielded Compound I-6 (20.9 mg, 49%).
LC/MS (Method A): 1.25 min, $[M+H]^+$ = 269
$^1$H-NMR ($CDCl_3$) δ: 8.47 (1H, s), 7.78 (2H, d, J = 7.3 Hz), 7.66 (1H, t, J = 7.5 Hz), 7.56 (2H, t, J = 7.5 Hz), 7.25 (2H, s), 6.41 (1H, s), 2.15 (3H, s).

(Example 1-9 Synthesis of Compound I-7)

[Formula 50]

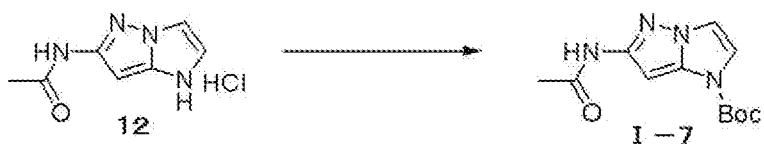

Compound 12 was synthesized using a method described in Synthetic Communications, 29(2), 311-341 (1999). To a suspension of Compound 12 (110.0 mg, 0.548 mmol) in tetrahydrofuran (1.5 mL), triethylamine (99 μL, 0.713 mmol), di-t-butyl dicarbonate (37 μL, 0.303 mmol), and 4-dimethylaminopyridine (6.7 mg, 0.055 mmol) were added. The solution was then stirred under nitrogen atmosphere at 40°C for 2 hours. The reaction solution was concentrated *in vacuo,* and then purified by silica gel column chromatography (n-hexane: ethyl acetate = 1 : 2 → 1 : 5) to yield Compound I-7 (78.5 mg, 54%).
LC/MS (Method A): 1.39 min, $[M+H]^+$ = 265
$^1$H-NMR ($CDCl_3$) δ: 8.51 (1H, br s), 7.16 (2H, s), 6.55 (1H, br s), 2.17 (3H, s), 1.65 (9H, s).

(Example 2-1 Synthesis of Compounds I-158, I-55, I-60, and I-56)

[Formula 51]

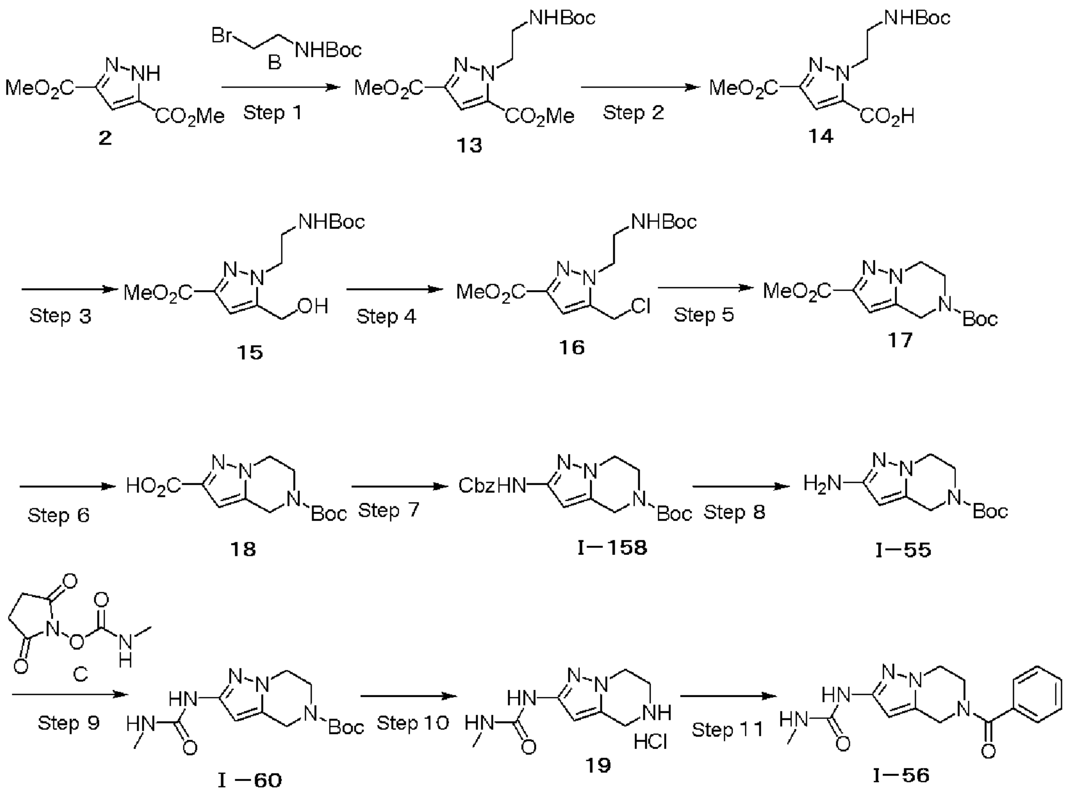


2, 13, 14, 15, 16, 17, 18, I−158, I−55, I −60, 19, I−56

Step 1

To a dimethylformamide (20 mL) solution of crude product 2 (3.74 g) obtained from Step 1 of Example 1, 2-(t-butoxycarbonylamino) ethyl bromide B (5.01 g, 22.3 mmol) and potassium carbonate (3. 65 g, 26.4 mmol) were added. The solution was then stirred under nitrogen atmosphere at 0˚C for one and a half hours. Water (40 mL) was then added to the reaction solution. The precipitated solid was collected, and then washed with water to yield crude product 13 (11.1 g).
LC/MS (Method D): 1.52 min, $[M+H]^+$ = 328.

Step 2

To a solution of crude product 13 (11.1 g) (obtained from Step 1) in methylene chloride:methanol (1:1, 100 mL), potassium hydroxide (2.0 mol/L, a methanol solution) (10.2 mL, 20.3 mmol) was added. The reaction solution was stirred under nitrogen atmosphere at room temperature for 8 hours. Potassium hydroxide (2.0 mol/L, a methanol solution) (2.03 mL, 4.06 mmol) was added to the reaction solution. The reaction solution was then stirred under nitrogen atmosphere at room temperature overnight. The reaction solution was then concentrated *in vacuo.* The residue was dissolved in water, acidified, and then extracted with ethyl acetate. The extract was dried with anhydrous magnesium sulfate, and then concentrated *in vacuo* to yield crude product 14 (6.17 g).
LC/MS (Method D): 1.14 min, $[M+H]^+$ = 314.

Step 3

To a tetrahydrofuran (80 mL) suspension of crude product 14 (6.37 g) obtained from Step 2, under nitrogen atmosphere, N,N'-carbonyldiimidazole (4.29g, 26.4 mmol) was added. The solution was then stirred at 45˚C for 30 minutes. To the reaction solution, aqueous 2 mol/L sodium tetrahydroborate solution (40 mL, 80.0 mmol) was added at 0˚C. The solution was then stirred at room temperature for 30 minutes. Aqueous saturated ammonium chloride solution was added to the reaction solution, and then extracted with ethyl acetate. The extract was washed sequentially with water and saturated brine. The solution was then dried with anhydrous magnesium sulfate. After concentrating *in vacuo,*

purification by silica gel column chromatography (n-hexane:ethyl acetate = 1:2 → 1:4) yielded Compound 15 (3.51 g, 58%).
LC/MS (Method D): 1.10 min, $[M+H]^+$ = 300
$^1$H-NMR ($CDCl_3$) δ: 6.75 (1H, s), 5.03 (1H, br s), 4.70 (2H, s), 4.36 (2H, t, J = 6.2 Hz), 3.91 (3H, s), 3.61 (2H, dd, J = 12.3, 6.2 Hz), 3.57 (1H, br s), 1.38 (9H, s).

Step 4

To a solution of Compound 15 (3.46 g, 11.56 mmol) in methylene chloride (50 mL), under nitrogen atmosphere, triethylamine (2.40 mL, 17.34 mmol) and methanesulfonyl chloride (1.08 mL, 13.87 mmol) were added at 0˚C. The solution was then stirred at room temperature for one and a half hours. Aqueous saturated sodiumbicarbonate solution was added to the reaction solution, and then extracted with ethyl acetate. The extract was washed sequentially with water and saturated brine, dried with anhydrous magnesium sulfate, and then concentrated *in vacuo* to yield crude product 16 (3.70 g).
LC/MS (Method D): 1.57 min, $[M+H]^+$ = 318.

Step 5

To a dimethylformamide (50 mL) solution of crude product 16 (3. 67 g) obtained from Step 4, under nitrogen atmosphere, 60% sodium hydride (555 mg, 13.87 mmol) was added at 0˚C. The solution was then stirred at room temperature for one and a half hours. To the reaction solution, 60% sodium hydride (231 mg, 5.78 mmol) was added at 0˚C. The solution was then stirred at room temperature for 1 hour. Aqueous saturated ammonium chloride solution was added to the reaction solution, and then extracted with ethyl acetate. The extract was washed sequentially with water (two times) and saturated brine, and then dried with anhydrous magnesium sulfate. After concentrating *in vacuo,* purification by silica gel column chromatography (n-hexane:ethyl acetate = 2:1 → 1:1) yielded Compound 17 (2.10 g, 65%).
LC/MS (Method D): 1.46 min, $[M+H]^+$ = 282
$^1$H-NMR ($CDCl_3$) δ: 6.62 (1H, s), 4.68 (2H, s), 4.25 (2H, t, J = 4.5 Hz), 3.92-3.90 (5H, m), 1.50 (9H, s).

Step 6

To a solution of Compound 17 (2.10 g, 7.47 mmol) in tetrahydrofuran:methanol (1:1, 30 mL), aqueous 2 mol/L sodium hydroxide solution (7.47 mL, 14.9 mmol) was added. The solution was then stirred at room temperature for one and a half hours. Water was added to the reaction solution, acidified, and then extracted with ethyl acetate. The extract was dried with anhydrous magnesium sulfate, and then concentrated *in vacuo* to yield Compound 18 (2.02 g, 100%) .
LC/MS (Method D): 1.21 min, $[M+H]^+$ = 268
$^1$H-NMR (DMSO-$d_6$) δ: 12.58 (1H, brs), 6.58 (1H, s), 4.61 (2H, s), 4.16 (2H, t, J = 5.5 Hz), 3.82 (2H, t, J = 5.5 Hz), 1.44 (9H, s).

Step 7

To a suspension of Compound 18 (1.93 g, 7.22 mmol) in toluene (30 mL), under nitrogen atmosphere, benzylalcohol (0.90 mL, 8.67 mmol), diphenylphosphoryl azide (1.87 mL, 8.67 mmol), and triethylamine (1.30 mL, 9. 39 mmol) were added. The solution was then stirred at 100˚C for 4 hours. Water was added to the reaction solution and then extracted with ethyl acetate. The extract was washed sequentially with water and saturated brine. The solution was dried with anhydrous magnesium sulfate and then concentrated *in vacuo.* The residue was washed with methanol to yield Compound I-158 (1.70 g, 63%).
LC/MS (Method D): 1.89 min, $[M+H]^+$ = 373
$^1$H-NMR (DMSO-$d_6$) δ: 9.98 (1H, s), 7.39-7.32 (5H, m), 6.17 (1H, s), 5.12 (2H, s), 4.54 (2H, s), 3.93 (2H, t, J = 5.0 Hz), 3.78 (2H, t, J = 5.0 Hz), 1.43 (9H, s).

Step 8

To a solution of Compound I-158 (508.7 mg, 1.366 mmol) in tetrahydrofuran:methanol (1:1, 7.0 mL), 20% palladium hydroxide-carbon (50% of water content, 100 mg) was added. The solution was then stirred under hydrogen atmosphere at room temperature for 4 hours. The reaction solution was filtered, and then the filtrate was concentrated *in vacuo* to yield Compound I-55 (1.52 g, 100%).
LC/MS (Method D): 0.92 min, $[M+H]^+$ = 239

$^1$H-NMR ($CDCl_3$) δ: 5.41 (1H, s), 4.53 (2H, s), 3.95 (2H, t, J = 5.3 Hz), 3.84 (2H, t, J = 5.3 Hz), 1.48 (9H, s).

Step 9

To a solution of Compound I-55 (278.2 mg, 1.168 mmol) in acetonitrile (4.0 mL), diisopropylethylamine (306 μL, 1.751 mmol) and 2,5-dioxopyrrolidin-1-yl methylcarbamate C (241.0 g, 1.401 mmol) was added. The solution was then stirred under nitrogen atmosphere at 60 ˚C for 5 hours. To the reaction solution, diisopropylethylamine (204 μL, 1.168 mmol) and 2,5-dioxopyrrolidin-1-ylmethylcarbamate C (161.0 g, 0.934 mmol) were added. The solution was then stirred under nitrogen atmosphere at 60˚C for 5 hours. To the reaction solution, diisopropylethylamine (102 μL, 0.584 mmol) and 2,5-dioxopyrrolidin-1-ylmethylcarbamate C (100.0 g, 0.584 mmol) were added. The solution was then stirred under nitrogen atmosphere at 60˚C for 5 hours. Aqueous saturated sodium bicarbonate solution was added to the reaction solution, and then extracted with ethyl acetate. The extract was washed sequentially with water and saturated brine, and then dried with anhydrous magnesium sulfate. After concentrating *in vacuo,* purification by aminosilica gel column chromatography (chloroform) yielded Compound I-60 (331.8 mg, 96%) .
LC/MS (Method D): 1.20 min, $[M+H]^+$ = 296
$^1$H-NMR ($CDCl_3$) δ: 7.56 (1H, br s), 7.23 (1H, br s), 5.60 (1H, s), 4.57 (2H, s), 4.02 (2H, t, J = 5.2 Hz), 3.87 (2H, t, J = 5.2 Hz), 2.91 (3H, d, J = 4.3 Hz), 1.49 (9H, s).

Step 10

To a solution of Compound I-60 (295.6 mg, 1.00 mmol) in methylene chloride (3.0 mL), hydrochloric acid (4 mol/L, a dioxane solution) (2.50 mL, 10.0 mmol) was added. The reaction solution was stirred at room temperature for 2 hours, and then the reaction solution was concentrated *in vacuo* to yield crude product 19 (271.9 mg).
LC/MS (Method D): 0.20 min, $[M+H]^+$ = 196.

Step 11

To a dimethylformamide (500 μL) solution of crude product 19 (25.0 mg) obtained from Step 10, benzoic acid (15.8 mg, 0.129 mmol), O-(7-azabenzotriazol-1-yl)-N,N,N',N'-tetramethyluronium hexafluorophosphate (53. 3 mg, 0.140 mmol), and triethylamine (39 μL, 0.281 mmol) were added. The solution was then stirred under nitrogen atmosphere at room temperature for 4 hours. After the reaction solution was filtered, the filtrate was separated andpurifiedby reverse phase HPLC to yield Compound I-56 (20.2 mg, 64%).
LC/MS (Method D): 1.71 min, $[M+H]^+$ = 363
$^1$H-NMR ($CDCl_3$) δ: 7.53-7.44 (7H, m), 5.58 (1H, br s), 4.75 (2H, br s), 4.10-4.02 (4H, m), 2.91 (3H, d, J = 4.5 Hz).

(Example 2-2 Synthesis of Compound I-58)

[Formula 52]

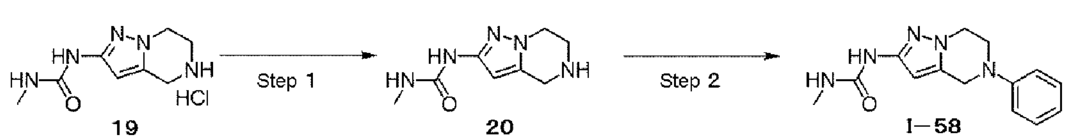


Step 1

To a chloroform (400 μL) suspension of crude product 19 (39.7 mg) obtained from Step 10 of Example 2-1, triethylamine (71 μL, 0.514 mmol) was added at 0˚C. The solution was then stirred at room temperature for 15 minutes. The reaction solution was purified by silica gel column chromatography (chloroform:methanol = 100:0 → 96:4) to yield Compound 20 (19.8 mg, 61%).
LC/MS (Method D): 0.18 min, $[M+H]^+$ = 196
$^1$H-NMR (DMSO-$d_6$) δ: 8.65 (1H, s), 6.77 (1H, br s), 5.75 (1H, s), 3.79-3.77 (4H, m), 3.05 (2H, t, J = 5.2 Hz), 2.65 (3H, d, J = 4.5 Hz), 2.54 (1H, s).

Step 2

To a suspension of Compound 20 (19.2 mg, 0.098 mmol) in dioxane (500 μL), bromobenzene (12 μL, 0.118 mmol), 4,5-bis(diphenylphosphino)-9,9-dimethylxanthene (8.5 mg, 0.015 mmol), cesium carbonate (96 mg, 0.295 mmol), and palladium(II) acetate (2.2 mg, 9.83 μmol) were added. The solution was then stirred under nitrogen atmosphere at 100˚C for 9 hours. Water was added to the reaction solution, and then extracted with ethyl acetate. The extract was washed sequentially with water and saturated brine, and then dried with anhydrous magnesium sulfate. After concentrating *in vacuo,* purification by silica gel column chromatography (chloroform:methanol = 100:0 → 96:4) yielded Compound I-58 (10.7 mg, 40%).
LC/MS (Method D): 1.24 min, $[M+H]^+$ = 272
$^1$H-NMR ($CDCl_3$) δ: 7.60 (1H, br s), 7.43 (1H, s), 7.31 (2H, t, J = 7.1 Hz), 6.98-6.92 (3H, m), 5.65 (1H, s), 4.35 (2H, s), 4.13 (2H, t, J = 5.2 Hz), 3.72 (2H, t, J = 5.2 Hz), 2.91 (3H, d, J = 3.8 Hz).

(Example 2-3 Synthesis of Compound I-63)

[Formula 53]

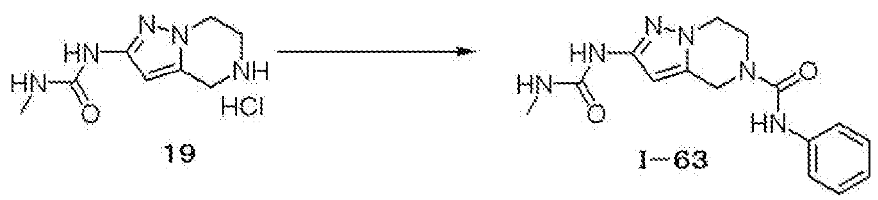

To a tetrahydrofuran (500 μL) suspension of crude product 19 (25.0 mg) obtained from Step 10 of Example 2-1, triethylamine (18 μL, 0.133 mmol) andphenylisocyanate (13 μL, 0.116 mmol) were added. The solution was then stirred under nitrogen atmosphere at room temperature for 2 hours. Water was added to the reaction solution, and then extracted with ethyl acetate. The extract was washed sequentially with water and saturated brine, and then dried with anhydrous magnesium sulfate. After concentrating *in vacuo,* purification by silica gel column chromatography (chloroform:methanol = 100:0 → 95:5) yielded Compound I-63 (15.7 mg, 56%).
LC/MS (Method D): 0.95 min, $[M+H]^+$ = 315
$^1$H-NMR (DMSO-$d_6$) δ: 8.76 (1H, s), 8.73 (1H, s), 7.46 (2H, d, J = 7.8 Hz), 7.25 (2H, t, J = 7. Hz), 6.96 (1H, t, J = 7.2 Hz), 6.64 (1H, br s), 5.96 (1H, s), 4.66 (2H, s), 3.98 (2H, d, J = 4.8 Hz), 3.93 (2H, d, J = 4.8 Hz), 2.66 (3H, d, J = 4.3 Hz).

(Example 3-1 Synthesis of Compounds I-66 and I-77)

[Formula 54]

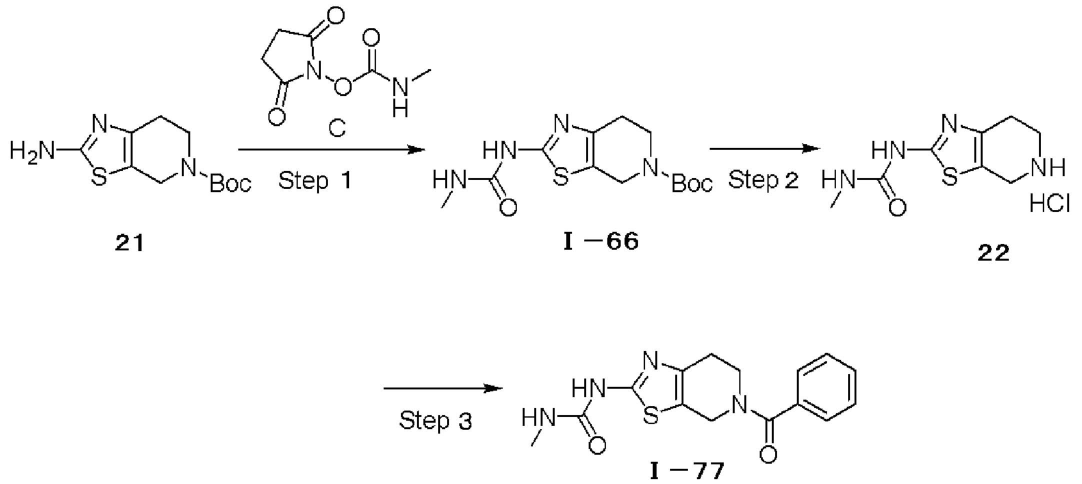


21 I-66 22 I-77

Step 1

Compound 21 was synthesized using a method described in Heterocycles, 63(7), 1555 (2004). To a solution of Compound 21 (97.0 mg, 0.380 mmol) in dimethylformamide (1.0 mL), diisopropylethylamine (199 μL, 1.140 mmol) and 2,5-dioxopyrrolidin-1-ylmethylcarbamate C (131.0mg, 0.706 mmol) were added. The reaction solution was then stirred under nitrogen atmosphere at 80˚C for 2 hours and a half. Aqueous saturated sodium bicarbonate solution was added to the reaction solution, and then extracted with ethyl acetate. The extract was washed sequentially with water (two times) and saturated brine, and then dried with anhydrous magnesium sulfate. After concentrating *in vacuo,* purification by aminosilica gel column chromatography (chloroform:methanol = 100:0 → 95:5) yielded Compound I-66 (110.2 mg, 93%).
LC/MS (Method D): 1.36 min, $[M+H]^+$ = 313
$^1$H-NMR (DMSO-$d_6$) δ: 10.34 (1H, s), 6.41 (1H, br s), 4.42 (2H, s), 3.61 (2H, t, J = 5.3 Hz), 2.67 (3H, d, J = 4.5 Hz), 2.56 (2H, t, J = 5.3 Hz), 1.42 (9H, s).

Step 2

To a solution of Compound I-66 (105.0 mg, 0.336 mmol) in methylene chloride (2.0 mL), hydrochloric acid (4 mol/L, a dioxane solution) (840 μL, 3.36 mmol) was added. The reaction solution was stirred at room temperature for 2 hours, and then the reaction solution was concentrated *in vacuo* to yield crude product 22 (91.1 mg).
LC/MS (Method D): 0.18 min, $[M+H]^+$ = 213.

Step 3

To a dimethylformamide (500 μL) solution of crude product 22 (20.0 mg) obtained from Step 2, benzoic acid (11.8 mg, 0.096 mmol), O-(7-azabenzotriazol-1-yl)-N,N,N',N'-tetramethyluronium hexafluorophosphate (HATU) (39.7 mg, 0.105 mmol) and triethylamine (29 μL, 0.209 mmol) were added. The solution was then stirred under nitrogen atmosphere at room temperature for 4 hours. The reaction solution was filtered, and then the filtrate was separated and purified by reverse phase HPLC to yield Compound I-77 (22.2 mg, 87%).
LC/MS (Method D): 1.04 min, $[M+H]^+$ = 317
$^1$H-NMR ($CDCl_3$) δ: 7.49-7.44 (7H, m), 4.81 (1.2H, br s), 4.54 (0.8H, br s), 4.07 (0.8H, br s), 3.71 (1.2H, br s), 2.92 (3H, d, J = 2.5 Hz), 2.85-2.77 (2H, m).

(Example 3-2 Synthesis of Compound I-106)

[Formula 55]

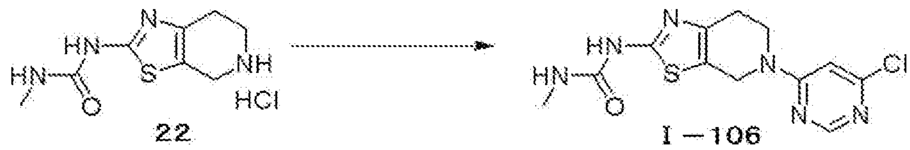

22 I-106

To an acetonitrile (400 μL) suspension of crude product 22 (23.6 mg) obtained from Step 2 of Example 3-1, diisopropylethylamine (50 μL, 0.285 mmol), 4, 6-dichloropyrimidine (14.1 g, 0.095 mmol) was added. The solution was then stirred under nitrogen atmosphere at 60°C for 5 hours. Water was then added to the reaction solution. The precipitated solid was collected, and then washed with water to yield Compound I-106 (22.5 mg, 73%).
LC/MS (Method D): 1.09 min, $[M+H]^+ = 325$
$^1$H-NMR (DMSO-$d_6$) δ: 10.38 (1H, s), 8.39 (1H, s), 7.09 (1H, s), 6.42 (1H, s), 4.74 (2H, s), 4.01 (2H, s), 2.68-2.66 (5H, m) .

(Example 3-3 Synthesis of Compound I-116)

[Formula 56]

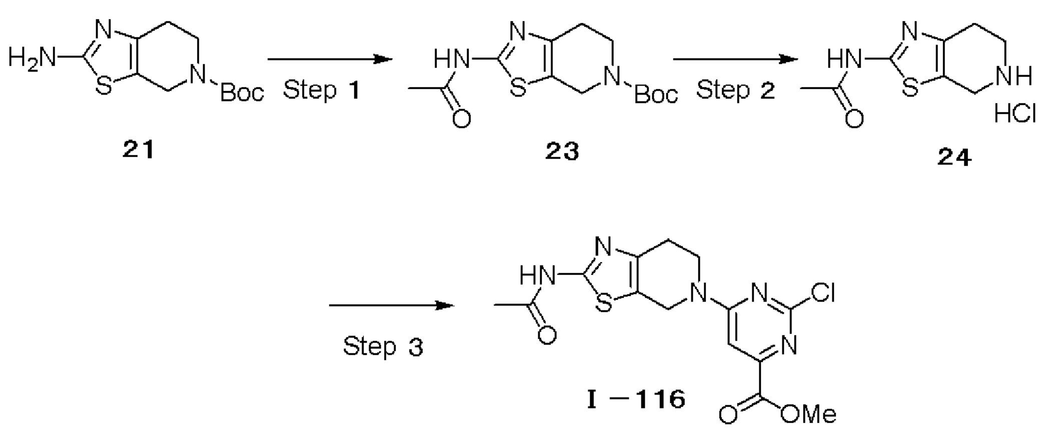


21 23 24 I-116

Step 1

Compound 21 was synthesized using a method described in Heterocycles, 63(7), 1555 (2004). To a suspension of Compound 21 (2.01 g, 7.87 mmol) in pyridine (10 mL), acetic anhydride (967 μL, 10.2 mmol) was added. The solution was then stirred under nitrogen atmosphere at room temperature overnight. To the reaction solution, acetic anhydride (372 μL, 3.94 mmol) was added. The reaction solution was then stirred at 40°C for 3 hours. Water was added to the reaction solution, and then extracted with ethyl acetate. The extract was washed sequentially with water and saturated brine, and then dried with anhydrous magnesium sulfate. After concentrating *in vacuo,* purification by silica gel column chromatography (*n*-hexane:ethyl acetate = 1:1 → 1:3) yielded Compound 23 (2.02 g, 86%).
LC/MS (Method D): 1.43 min, $[M+H]^+ = 298$
$^1$H-NMR ($CDCl_3$) δ: 10.81 (1H, br s), 4.57 (2H, s), 3.74 (2H, s), 2.73 (2H, s), 2.24 (3H, s), 1.49 (9H, s).

Step 2

To a solution of Compound 23 (1.91 g, 6.42 mmol) in methylene chloride (10 mL), hydrochloric acid (4 mol/L, a dioxane solution) (8.03 mL, 32.1 mmol) was added. The solution was then stirred at room temperature for one and a half hours. The precipitated solid was collected to yield crude product 24 (1.95 g).
LC/MS (Method D): 0.23 min, $[M+H]^+ = 198$.

Step 3

To an acetonitrile (1.0 mL) suspension of crude product 24 (42.3 mg) obtained from Step 2, diisopropylethylamine (95 μL, 0.543 mmol), methyl-2,6-dichloropyrimidine-4-carboxylate (37.5g, 0.181 mmol) was added. The solutionwas then stirred under nitrogen atmosphere at 60 °C for one and a half hours. Water was then added to the reaction solution. The precipitated solid was collected, and then washed with water to yield Compound I-116 (45.6 mg, 69%) .
LC/MS (Method D): 1.25 min, $[M+H]^+$ = 368
$^1$H-NMR (DMSO-$d_6$) δ: 12.03 (1H, s), 7.45 (1H, br s), 4.84 (2H, br s), 4.05 (2H, br s), 3.88 (3H, s), 2.76 (2H, s), 2.13 (3H, s).

(Example 4-1 Synthesis of Compound I-415)

[Formula 57]

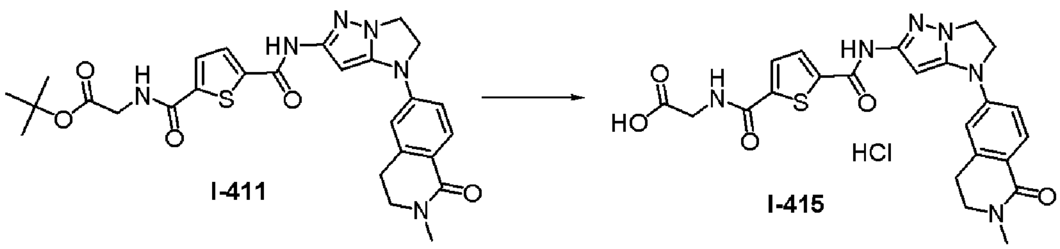

I-411

I-415

To Compound I-411 (16.1 mg, 29 μmol), hydrochloric acid (4 mol/L, a dioxane solution) (0.8 mL) was added. The solution was then stirred at room temperature for 20 minutes . The reaction solution was allowed to stand at room temperature overnight, and then concentrated *in vacuo.* The residue was washed with diisopropyl ether to yield Compound I-415 (15.4 mg, 99%) .
LC-MS (Method C): 1.33 min, [M+] = 495.1
$^1$H-NMR (DMSO-$d_6$) δ: 11.12 (1H, s), 9.06 (1H, s), 8.06 (1H, s), 7.86 (1H, d, J = 8.08 Hz), 7.80 (1H, s), 7.00 (1H, d, J = 8.08 Hz), 6.88 (1H, s), 6.44 (1H, s), 4.48-4.39 (2H, m), 4.38-4.28 (2H, m), 3.97-3.48 (4H, m), 3.04-2.97 (5H, m).

(Example 4-2 Synthesis of Compound I-555)

[Formula 58]

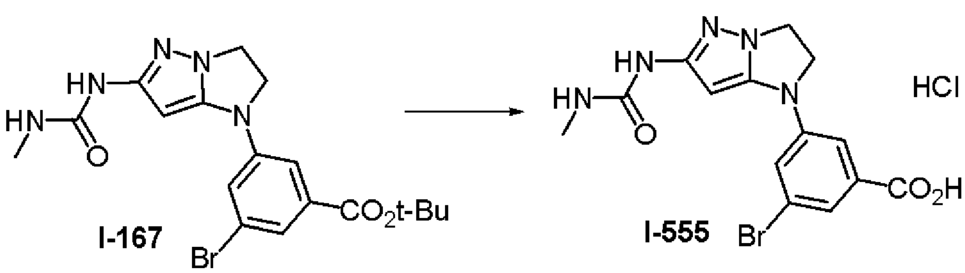

I-167

I-555

Compound I-167 (293 mg, 0.672mmol) was dissolved in dichloromethane (5 mL) . Trifluoroacetic acid (5.17 mL) was added thereto. The solution was then stirred at room temperature for 30 minutes. The reaction solution was concentrated *in vacuo,* and then hydrochloric acid (4 mol/L, a dioxane solution) (4.20 mL) was added to the residue. The resulting solution was then diluted with dichloromethane. The precipitated solid was filtered, and then washed with dichloromethane to yield Compound I-555 (236 mg, 84%).
LC-MS (Method D): 1.26 min, [M+] = 380, 382
$^1$H-NMR (DMSO-$d_6$) δ: 9.32 (1H, s), 7.61-7.52 (2H, m), 7.37 (1H, s), 7.16-6.56 (1H, m), 5.87 (1H, s), 4.46-4.38 (2H, m),

4.24-4.16 (2H, m), 2.68 (3H, s).

(Example 5 Synthesis of Compound I-262)

[Formula 59]

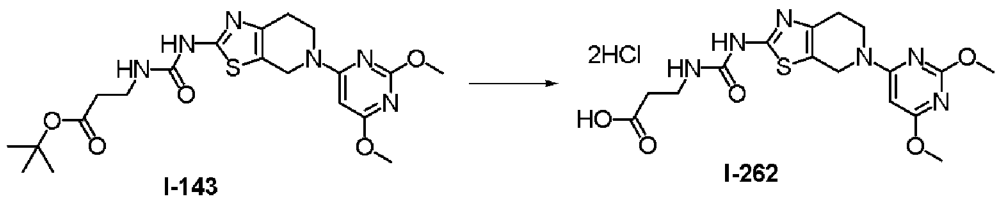

I-143 I-262

To a solution of Compound I-143 (790 mg, 1.70 mmol) in methylene chloride (15 mL), hydrochloric acid (4 mol/L, a dioxane solution) (8.5 mL, 34.0 mmol) was added. The reaction solution was then stirred at room temperature for one and a half hours. The solvent was evaporated *in vacuo* to yield Compound I-262 (830 mg).
LC-MS (Method D): 1.13 min, [M+] = 409
$^1$H-NMR (DMSO-$d_6$) δ: 7.22 (1H, br s), 6.50 (3H, br s), 5.99 (1H, s), 5.76 (1H, s), 4.74 (2H, s), 3.97 (2H, br s), 3.87 (6H, s), 3.33 (2H, br s), 2.69 (2H, br s), 2.44 (2H, br s) .

(Example 6 Synthesis of Compound I-471)

[Formula 60]

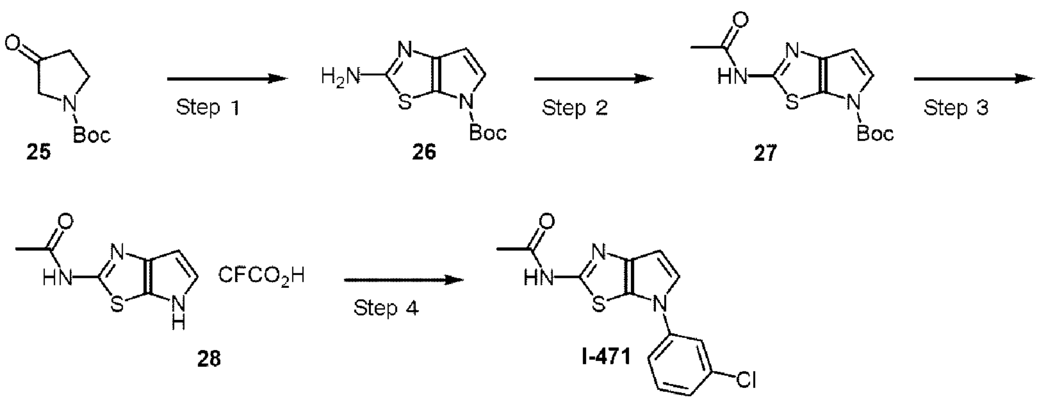


25 26 27 28 I-471

Step 1

To a solution of Compound 25 (5 g, 27 mmol) in pyridine (50 mL), under nitrogen atmosphere, sulfur (1.73 g, 54 mmol) and cyanamide (2.27 g, 54 mmol) were added. The solution was then stirred at 100°C for 4 hours. Aqueous saturated sodium bicarbonate solution was added to the reaction solution, and then extracted with ethyl acetate. The extract was washed sequentially with aqueous saturated sodium bicarbonate solution and saturated brine, and then dried with anhydrous magnesium sulfate. After concentrating *in vacuo,* purification by silica gel column chromatography (hexane:ethyl acetate = 70:30 → 33:67) yielded Compound 26 (2.2 g, 34%) .
$^1$H-NMR ($CDCl_3$) δ: 6.44 (1.0H, d, J = 3.53 Hz), 4.75 (2.0H, s), 1.63 (9.0H, s).

Step 2

To a solution of Compound 26 (2 g, 8.36 mmol) in dichloromethane (30 mL), under nitrogen atmosphere, pyridine (1.01 mL, 12.54 mmol) and acetic anhydride (0.95 mL, 10.03 mmol) were added at 0˚C. The solution was then stirred at room temperature overnight. To the reaction solution, 2 mol/L hydrochloric acid was added, and then extracted with ethyl acetate. The extract was washed sequentially with aqueous saturated sodium bicarbonate solution and saturated brine, and then dried with anhydrous magnesium sulfate. After concentrating *in vacuo,* purification by silica gel column chromatography (hexane:ethyl acetate = 100:0 → 50:50) yielded Compound 27 (1.49 g, 63%).
$^1$H-NMR (DMSO-$d_6$) δ: 7.41 (1.0H, d, J = 3.53 Hz), 6.64 (1.0H, d, J = 3.53 Hz), 2.15 (3.0H, s), 1.60 (9.0H, s).

Step 3

To a solution of Compound 27 (500 mg, 1.78 mmol) in dichloromethane (5 mL), under nitrogen atmosphere, trifluoroacetic acid (2.5 mL) was added. The solution was then stirred at room temperature overnight. The reaction solution was then concentrated *in vacuo.* Ethyl acetate and hexane were added to the residue, and then the precipitated solid was filtered to yield crude product 28 (452 mg, 86%) . $^1$H-NMR (DMSO-$d_6$) δ: 11.82 (1.0H, br s), 11.06 (1.0H, br s), 6.97 (1.0H, t, J = 2.60 Hz), 6.28 (1.0H, dd, J = 2.60, 1.60 Hz), 2.11 (3.0H, s).

Step 4

To an N-methylpiperidone (3 mL) solution of crude product 28 (300 mg, 1.02 mmol) obtained from Step 3, under nitrogen atmosphere, 1-chloro-3-fluorobenzene (266 μL, 2.48 mmol) and sodium t-butoxide (525 mg, 5.46 mmol) were added. The solution was then stirred at 120˚C for 2 hours. Water was added to the reaction solution, and then extracted with ethyl acetate. The extract was washed sequentially with water and saturated brine, and then dried with anhydrous magnesium sulfate. After concentrating *in vacuo,* purification by silica gel column chromatography (n-hexane: ethyl acetate = 100:0 → 33:67) yielded Compound I-471 (22 mg, 7%).
$^1$H-NMR ($CDCl_3$) δ: 10.77 (1.0H, s), 7.53 (1.0H, q, J = 1.29 Hz), 7.42-7.45 (2.0H, m), 7.28 (1.0H, d, J= 3.27 Hz), 6.63 (1.0H, d, J = 3.27 Hz), 2.36 (3.0H, s).

(Example 7 Synthesis of Compounds I-3, I-9, I-10, I-12 to 49, I-54, I-57, I-59, I-61, I-62, I-64, I-65, I-67 to 76, I-78 to 105, I-107 to 115, I-117 to 153, I-159 to 261, I-263 to 414, I-416 to 470, and I-472 to 554)

The above-described compounds were synthesized similarly to Examples above. Identification data are shown below.

(Results)

The following tables show values of the physical properties (retention time, mass spectrum, and measurement condition) for Compound Nos. I-1 to 554.

[Table 1-1]

| Compound No. | Structural Formula | LC/MS Retention Time (min) | MS[M+H]+ | LC Method |
|---|---|---|---|---|
| I-1 | 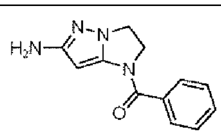 | 0.68 | 228.9 | A |
| I-2 | 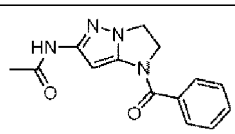 | 0.96 | 270.9 | A |

(continued)

| Compound No. | Structural Formula | LC/MS Retention Time (min) | MS[M+H]+ | LC Method |
|---|---|---|---|---|
| I-3 | 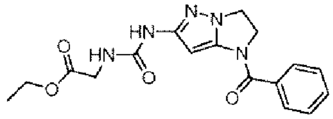 | 1.3 | 358 | A |
| I-4 | 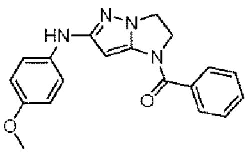 | 1.55 | 335 | A |
| I-5 | 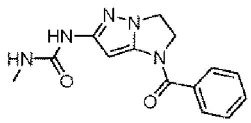 | 0.99 | 285.9 | A |
| I-6 | 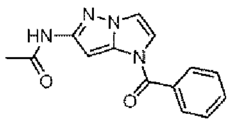 | 1.25 | 268.9 | A |
| I-7 | 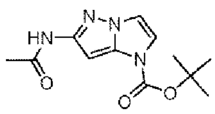 | 1.39 | 265 | A |
| I-8 | 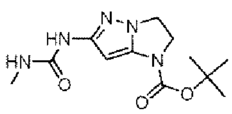 | 1.25 | 282 | A |

[Table 1-2]

| Compound No. | Structural Formula | LC/MS Retention Time (min) | MS[M+H]+ | LC Method |
|---|---|---|---|---|
| I-9 | 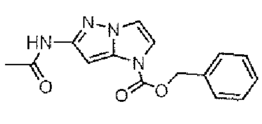 | 1.48 | 298.9 | A |
| I-10 | 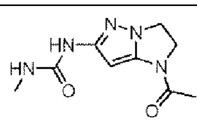 | 0.45 | 224 | A |
| I-11 | 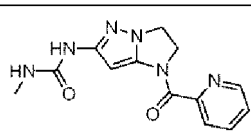 | 0.87 | 286.9 | A |
| I-12 | 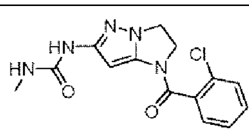 | 1.15 | 320.04 | A |

(continued)

| Compound No. | Structural Formula | LC/MS Retention Time (min) | MS[M+H]+ | LC Method |
|---|---|---|---|---|
| I-13 | 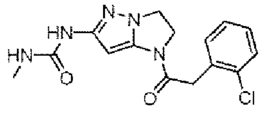 | 1.31 | 334.04 | A |
| I-14 | 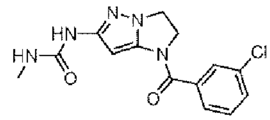 | 1.28 | 320.02 | A |
| I-15 | 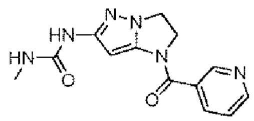 | 0.75 | 287.1 | A |
| I-16 | 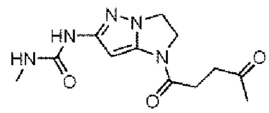 | 0.78 | 280.18 | A |
| I-17 | 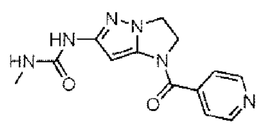 | 0.59 | 287.15 | A |

[Table 1-3]

| Compound No. | Structural Formula | LC/MS Retention Time (min) | MS[M+H]+ Method | LC |
|---|---|---|---|---|
| I-18 | 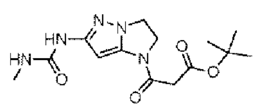 | 1.17 | 324.15 | A |
| I-19 | 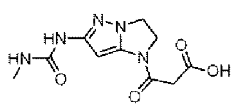 | 0.67 | 268.16 | A |
| I-20 | 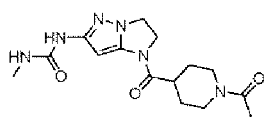 | 0.79 | 335.15 | A |
| I-21 | 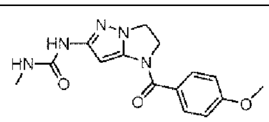 | 1.12 | 316.14 | A |
| I-22 | 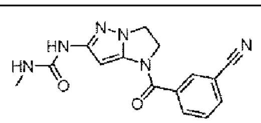 | 1 | 311.1 | A |
| I-23 | 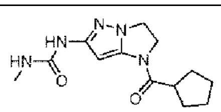 | 1.15 | 278.17 | A |

(continued)

| Compound No. | Structural Formula | LC/MS Retention Time (min) | MS[M+H]+ Method | LC |
|---|---|---|---|---|
| I-24 | 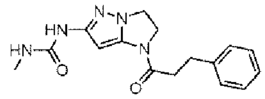 | 1.33 | 314.15 | A |
| I-25 | 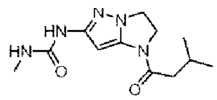 | 1.1 | 266.24 | A |
| I-26 | 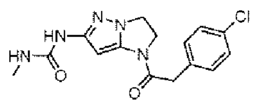 | 1.39 | 334.07 | A |

[Table 1-4]

| Compound No. | Structural Formula | LC/MS Retention Time (min) | MS[M+H]+ | LC Method |
|---|---|---|---|---|
| I-27 | 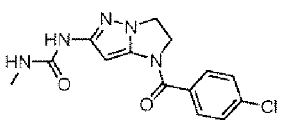 | 1.29 | 320.06 | A |
| I-28 | 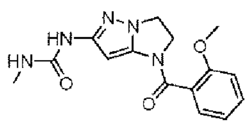 | 1.06 | 316.14 | A |
| I-29 | 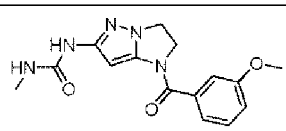 | 1.13 | 316.14 | A |
| I-30 | 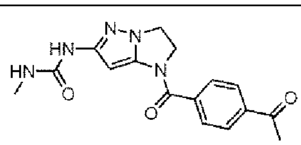 | 1.03 | 328.14 | A |
| I-31 | 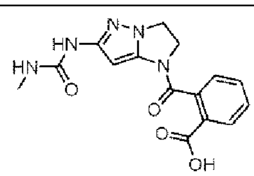 | 0.82 | 330.13 | A |
| I-32 | 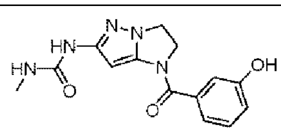 | 0.91 | 302.15 | A |
| I-33 | 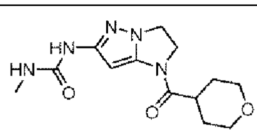 | 0.82 | 294.16 | A |

(continued)

| Compound No. | Structural Formula | LC/MS Retention Time (min) | MS[M+H]+ | LC Method |
|---|---|---|---|---|
| I-34 | 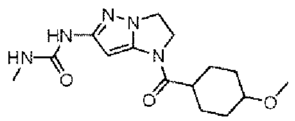 | 1.04 | 322.2 | A |

[Table 1-5]

| Compound No. | Structural Formula | LC/MS Retention Time (min) | MS[M+H]+ | LC Method |
|---|---|---|---|---|
| I-35 | 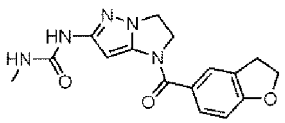 | 1.1 | 328.18 | A |
| I-36 | 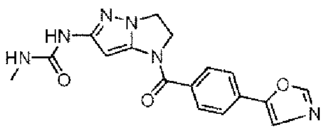 | 1.06 | 353.18 | A |
| I-37 | 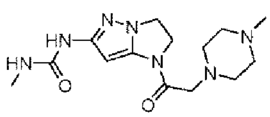 | 0.57 | 322.13 | B |
| I-38 | 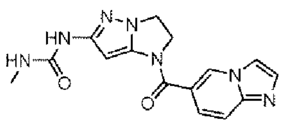 | 0.66 | 326.05 | B |
| I-39 | 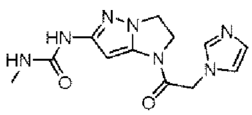 | 0.56 | 290.08 | B |
| I-40 | 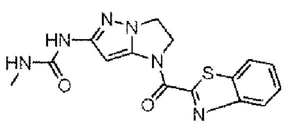 | 1.52 | 343.14 | A |
| I-41 | 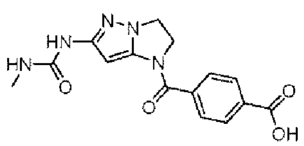 | 0.89 | 330.14 | A |
| I-42 | 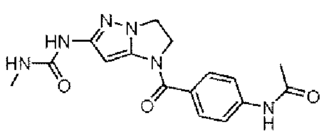 | 0.83 | 343.18 | A |

[Table 1-6]

| Compound No. | Structural Formula | LC/MS Retention Time (min) | MS[M+H]+ | LC Method |
|---|---|---|---|---|
| I-43 | 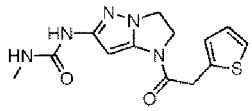 | 1.11 | 306.18 | A |
| I-44 | 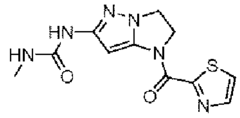 | 1.03 | 293.16 | A |
| I-45 | 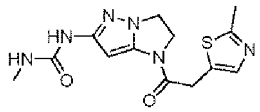 | 0.81 | 321.16 | A |
| I-46 | 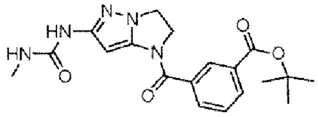 | 1.6 | 386.23 | A |
| I-47 | 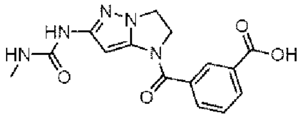 | 0.91 | 330.14 | A |
| I-48 | 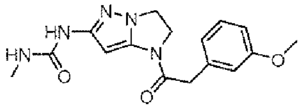 | 1.21 | 330.2 | A |
| I-49 | 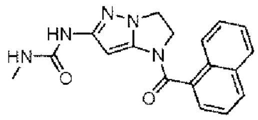 | 1.33 | 336.2 | A |
| I-50 | 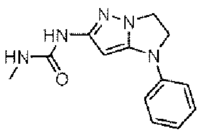 | 1.27 | 258 | A |
| I-51 | 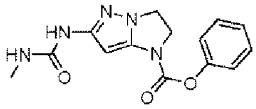 | 1.26 | 301.9 | A |

[Table 1-7]

| Compound No. | Structural Formula | LC/MS Retention Time (min) | MS[M+H]+ | LC Method |
|---|---|---|---|---|
| I-52 | 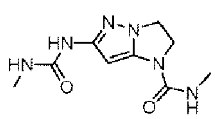 | 0.54 | 238.9 | A |

(continued)

| Compound No. | Structural Formula | LC/MS Retention Time (min) | MS[M+H]+ | LC Method |
|---|---|---|---|---|
| I-53 | 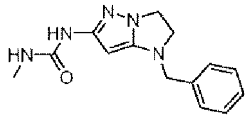 | 1.06 | 271.9 | D |
| I-54 | 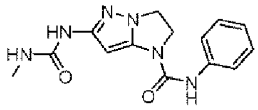 | 1.05 | 300.9 | D |
| I-55 | 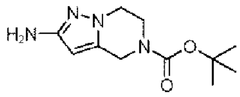 | 0.92 | 239 | D |
| I-56 | 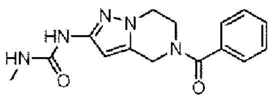 | 0.88 | 300 | D |
| I-57 | 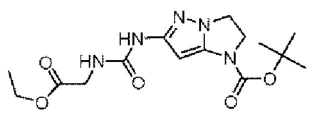 | 1.38 | 354 | D |
| I-58 | 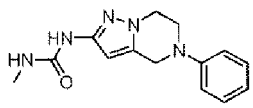 | 1.24 | 272 | D |
| I-59 | 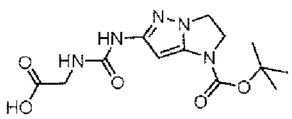 | 1.07 | 325.9 | D |
| I-60 | 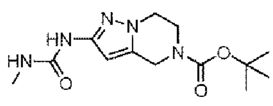 | 1.2 | 296 | D |

[Table 1-8]

| Compound No. | Structural Formula | LC/MS Retention Time (min) | MS[M+H]+ | LC Method |
|---|---|---|---|---|
| I-61 | 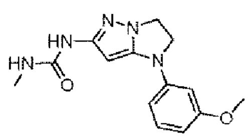 | 1.23 | 288 | D |
| I-62 | 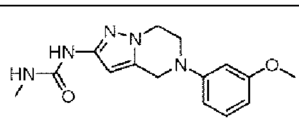 | 1.25 | 302 | D |

(continued)

| Compound No. | Structural Formula | LC/MS Retention Time (min) | MS[M+H]+ | LC Method |
|---|---|---|---|---|
| I-63 | 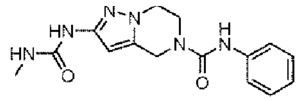 | 0.95 | 315 | D |
| I-64 | 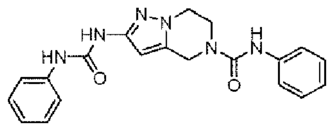 | 1.48 | 377 | D |
| I-65 | 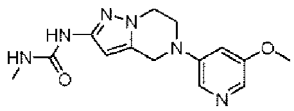 | 0.83 | 303 | B |
| I-66 | 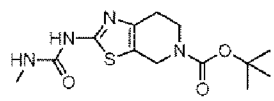 | 1.36 | 313 | D |
| I-67 | 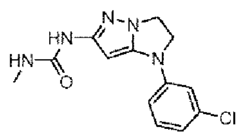 | 1.44 | 291.94 | D |
| I-68 | 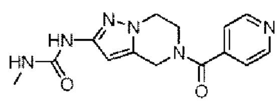 | 0.52 | 301.36 | A |
| I-69 | 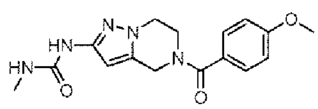 | 1.10 | 330.38 | A |

[Table 1-9]

| Compound No. | Structural Formula | LC/MS Retention Time (min) | MS[M+H]+ Method | LC |
|---|---|---|---|---|
| I-70 | 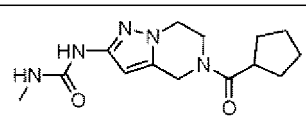 | 1.13 | 292.43 | A |
| I-71 | 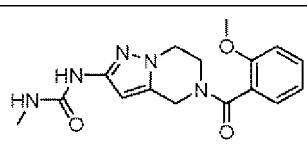 | 1.07 | 330.45 | A |
| I-72 | 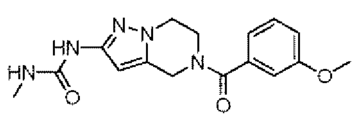 | 1.12 | 330.14 | A |

(continued)

| Compound No. | Structural Formula | LC/MS Retention Time (min) | MS[M+H]+ Method | LC |
|---|---|---|---|---|
| I-73 | 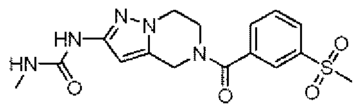 | 0.89 | 378.17 | A |
| I-74 | 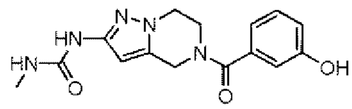 | 0.89 | 316.15 | A |
| I-75 | 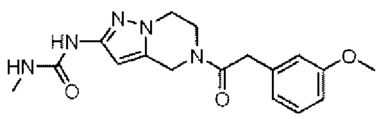 | 1.17 | 344.2 | A |
| I-76 | 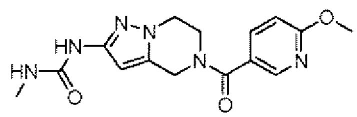 | 0.97 | 331.15 | A |
| I-77 | 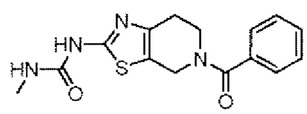 | 1.04 | 317 | D |
| I-78 | 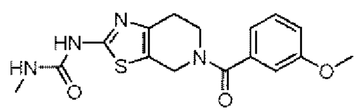 | 1.11 | 347 | D |

[Table 1-10]

| Compound No. | Structural Formula | LC/MS Retention Time (min) | MS[M+H]+ | LC Method |
|---|---|---|---|---|
| I-79 | 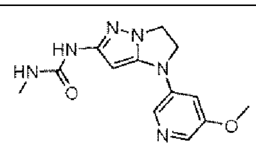 | 0.50 | 289 | D |
| I-80 | 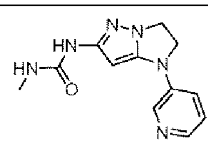 | 0.70 | 259 | B |
| I-81 | 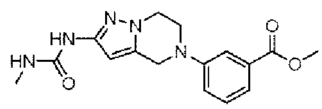 | 1.30 | 330 | D |
| I-82 | 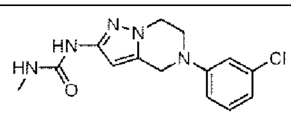 | 1.52 | 306 | D |

(continued)

| Compound No. | Structural Formula | LC/MS Retention Time (min) | MS[M+H]+ | LC Method |
|---|---|---|---|---|
| I-83 | 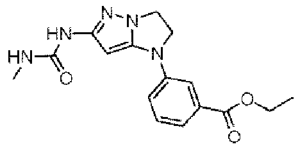 | 1.46 | 330.02 | D |
| I-84 | 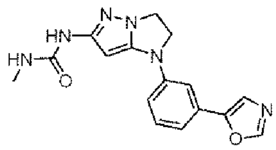 | 1.17 | 325 | D |
| I-85 | 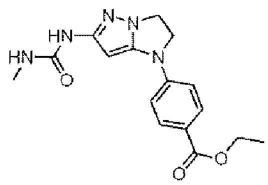 | 1.37 | 330.02 | D |
| I-86 | 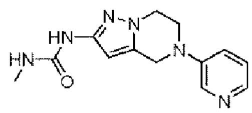 | 0.63 | 273 | B |

[Table 1-11]

| Compound No. | Structural Formula | LC/MS Retention Time (min) | MS[M+H]+ | LC Method |
|---|---|---|---|---|
| I-87 | 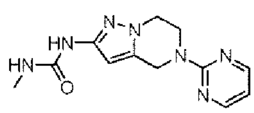 | 1.01 | 274 | A |
| I-88 | 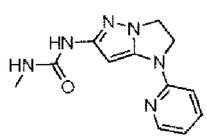 | 0.63 | 259.1 | B |
| I-89 | 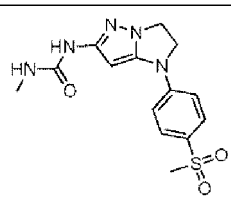 | 0.9 | 336.01 | D |
| I-90 | 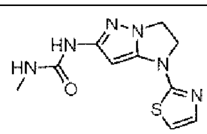 | 0.78 | 264.95 | D |

(continued)

| Compound No. | Structural Formula | LC/MS Retention Time (min) | MS[M+H]+ | LC Method |
|---|---|---|---|---|
| I-91 | 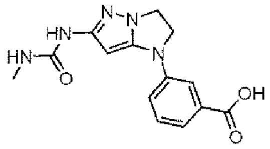 | 1.02 | 302.15 | C |
| I-92 | 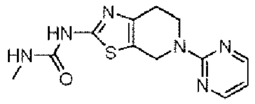 | 1.1 | 291 | A |
| I-93 | 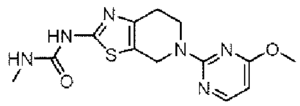 | 0.91 | 321 | A |
| I-94 | 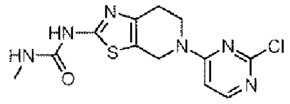 | 1.21 | 325 | A |

[Table 1-12]

| Compound No. | Structural Formula | LC/MS Retention Time (min) | MS[M+H]+ | LC Method |
|---|---|---|---|---|
| I-95 | 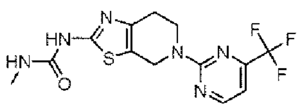 | 1.78 | 359 | A |
| I-96 | 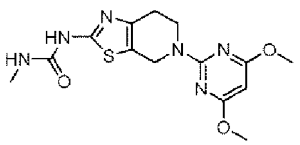 | 1.68 | 351 | A |
| I-97 | 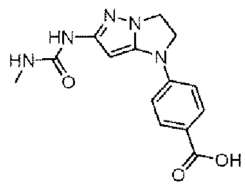 | 1.05 | 302 | C |
| I-98 | 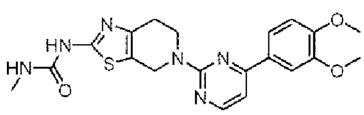 | 1.57 | 427 | A |
| I-99 | 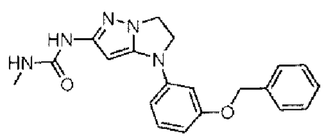 | 1.98 | 364.1 | C |

(continued)

| Compound No. | Structural Formula | LC/MS Retention Time (min) | MS[M+H]+ | LC Method |
| --- | --- | --- | --- | --- |
| I-100 | 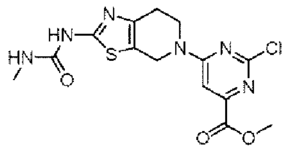 | 1.18 | 383 | D |
| I-101 | 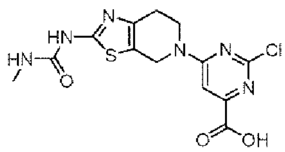 | 0.94 | 369 | D |

[Table 1-13]

| Compound No. | Structural Formula | LC/MS Retention Time (min) | MS[M+H]+ | LC Method |
| --- | --- | --- | --- | --- |
| I-102 | 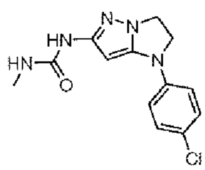 | 1.44 | 292.01 | D |
| I-103 | 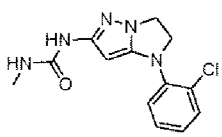 | 1.32 | 291.99 | D |
| I-104 | 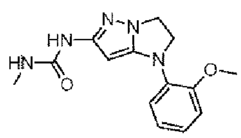 | 1.13 | 288.04 | D |
| I-105 | 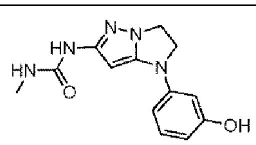 | 0.92 | 274.02 | D |
| I-106 | 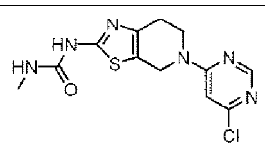 | 1.09 | 325 | D |
| I-107 | 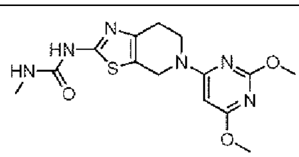 | 1.17 | 351 | D |

(continued)

| Compound No. | Structural Formula | LC/MS Retention Time (min) | MS[M+H]+ | LC Method |
|---|---|---|---|---|
| I-108 | 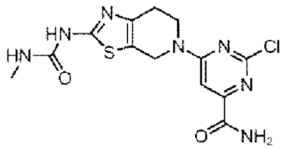 | 1.08 | 368 | D |

[Table 1-14]

| Compound No. | Structural Formula | LC/MS Retention Time (min) | MS[M+H]+ | LC Method |
|---|---|---|---|---|
| I-109 | 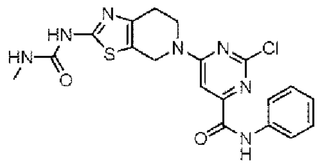 | 1.82 | 444 | D |
| I-110 | 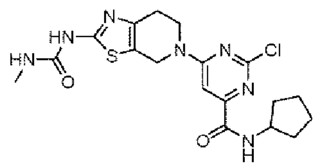 | 1.74 | 436 | D |
| I-111 | 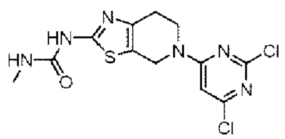 | 1.44 | 359 | D |
| I-112 | 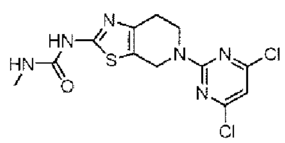 | 1.84 | 359 | D |
| I-113 | 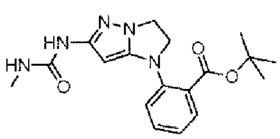 | 1.5 | 358.06 | D |
| I-114 | 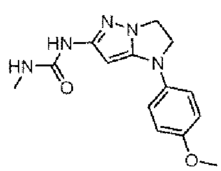 | 1.43 | 288 | C |
| I-115 | 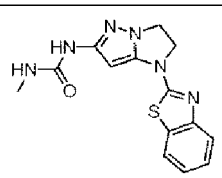 | 1.28 | 315.17 | D |

[Table 1-15]

| Compound No. | Structural Formula | LC/MS Retention Time (min) | MS[M+H]+ | LC Method |
|---|---|---|---|---|
| I-116 | 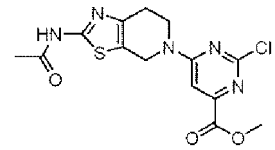 | 1.25 | 368 | D |
| I-117 | 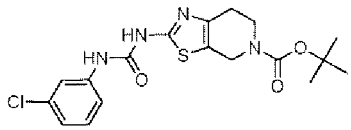 | 2.2 | 409.13 | D |
| I-118 | 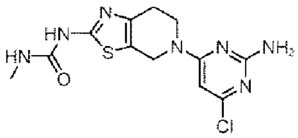 | 0.91 | 340 | D |
| I-119 | 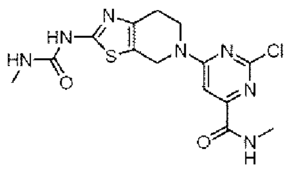 | 1-23 | 382 | D |
| I-120 | 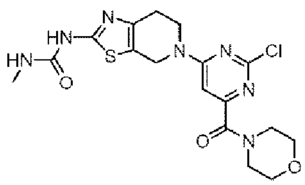 | 1.08 | 438 | D |
| I-121 | 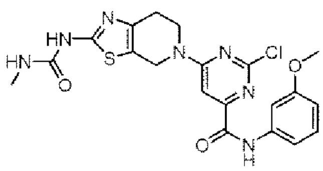 | 1.82 | 474 | D |
| I-122 | 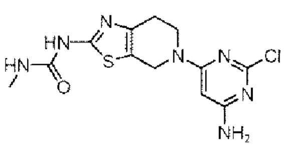 | 1.15 | 340 | D |

[Table 1-16]

| Compound No. | Structural Formula | LC/MS Retention Time (min) | MS[M+H]+ | LC Method |
|---|---|---|---|---|
| I-123 | 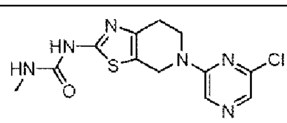 | 1.32 | 325 | D |

(continued)

| Compound No. | Structural Formula | LC/MS Retention Time (min) | MS[M+H]+ | LC Method |
|---|---|---|---|---|
| I-124 | 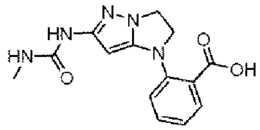 | 1.09 | 302.2 | C |
| I-125 | 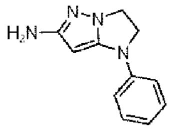 | 0.79 | 201.14 | C |
| I-126 | 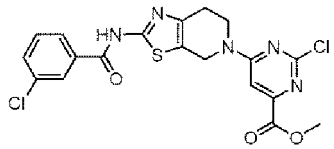 | 2.01 | 464.09 | D |
| I-127 | 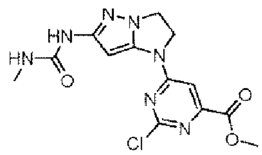 | 1.03 | 352.15 | D |
| I-128 | 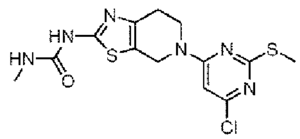 | 1.57 | 371 | D |
| I-129 | 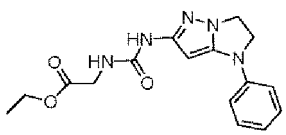 | 1.73 | 330.25 | C |
| I-130 | 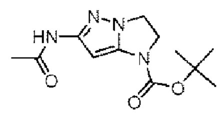 | 1.1 | 267.17 | D |

[Table 1-17]

| Compound No. | Structural Formula | LC/MS Retention Time (min) | MS[M+H]+ | LC Method |
|---|---|---|---|---|
| I - 131 | 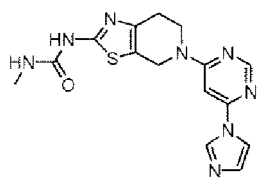 | 0.71 | 357 | D |

(continued)

| Compound No. | Structural Formula | LC/MS Retention Time (min) | MS[M+H]+ | LC Method |
|---|---|---|---|---|
| I - 132 | 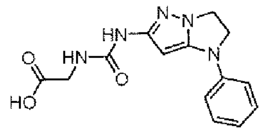 | 1.37 | 302 | C |
| I - 133 | 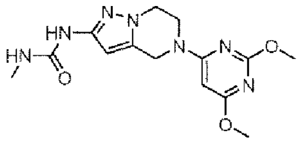 | 1.03 | 334 | D |
| I - 134 | 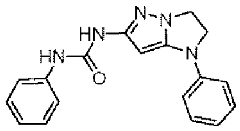 | 2.1 | 320.05 | C |

[Table 1 - 18]

| Compound No. | Structural Formula | LC/MS Retention Time (min) | MS[M+H]+ | LC Method |
|---|---|---|---|---|
| I-135 | 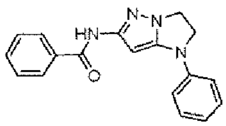 | 2 | 305.05 | C |
| I-136 | 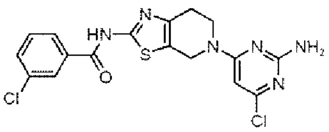 | 1.79 | 421.05 | D |
| I-137 | 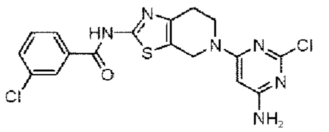 | 2.02 | 421.05 | D |
| I-138 | 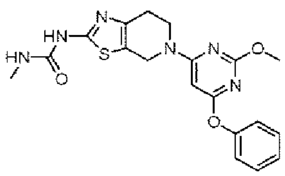 | 1.62 | 413.1 | D |
| I-139 | 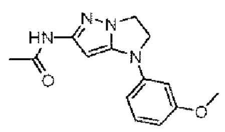 | 1.21 | 273.15 | D |
| I-140 | 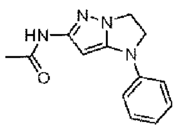 | 1.21 | 243.17 | D |

(continued)

| Compound No. | Structural Formula | LC/MS Retention Time (min) | MS[M+H]+ | LC Method |
|---|---|---|---|---|
| I-141 | 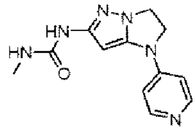 | 0.63 | 259.1 | B |
| I-142 | 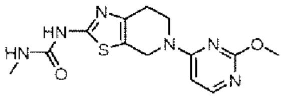 | 0.57 | 321.1 | D |

[Table 1-19]

| Compound No. | Structural Formula | LC/MS Retention Time (min) | MS[M+H]+ | LC Method |
|---|---|---|---|---|
| I-143 | 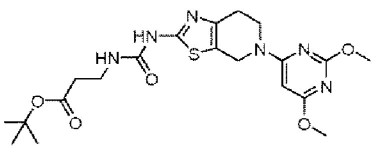 | 1.7 | 465.2 | D |
| I-144 | 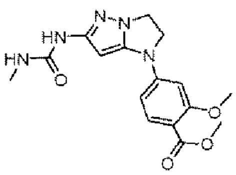 | 1.12 | 346.16 | D |
| I-145 | 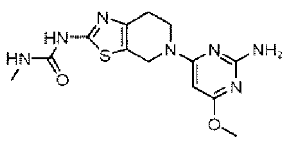 | 0.63 | 336.1 | D |
| I-146 | 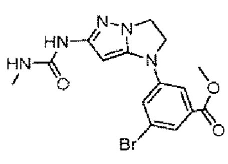 | 1.56 | 396.06 | D |
| I-147 | 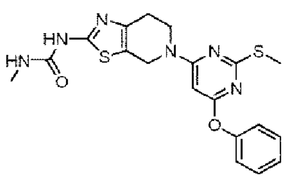 | 1.9 | 429.1 | D |
| I-148 | 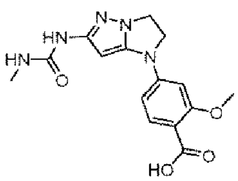 | 1.15 | 332.2 | C |

(continued)

| Compound No. | Structural Formula | LC/MS Retention Time (min) | MS[M+H]+ | LC Method |
|---|---|---|---|---|
| I-149 | 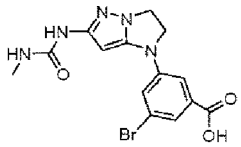 | 1.51 | 382.05 | C |

[Table 1-20]

| Compound No. | Structural Formula | LC/MS Retention Time (min) | MS[M+H]+ | LC Method |
|---|---|---|---|---|
| I-150 | 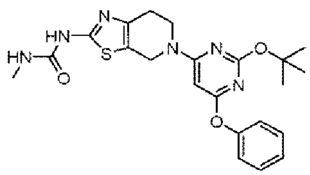 | 2 | 455.2 | D |
| I-151 | 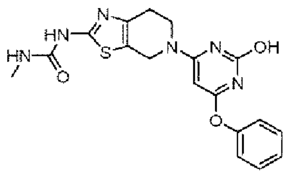 | 0.97 | 399.1 | D |
| I-152 | 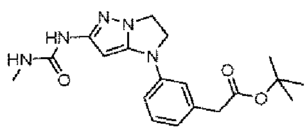 | 1.68 | 372.24 | D |
| I-153 | 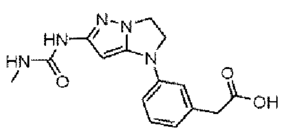 | 0.99 | 316.18 | D |

[Table 1-21]

| Compound No. | Structural Formula | LC/MS Retention Time (min) | MS[M+H]+ | LC Method |
|---|---|---|---|---|
| I-154 | 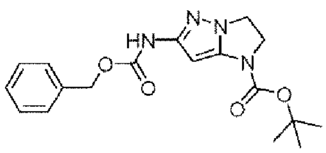 | 1.98 | 359 | A |
| I-155 | 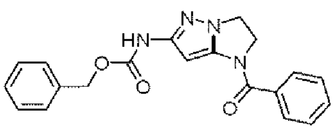 | 1.71 | 363 | A |

(continued)

| Compound No. | Structural Formula | LC/MS Retention Time (min) | MS[M+H]+ | LC Method |
|---|---|---|---|---|
| I-155 | 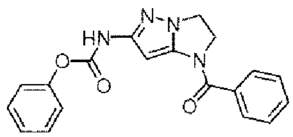 | 1.66 | 349 | A |
| I-157 | 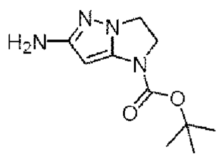 | 0.79 | 225 | A |
| I-158 | 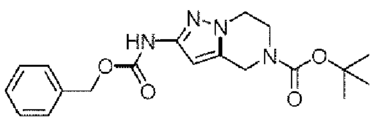 | 1.89 | 373 | D |

[Table 1-22]

| Compound No. | Structural Formula | LC/MS Retention Time (min) | MS[M+H]+ | LC Method |
|---|---|---|---|---|
| I-159 | 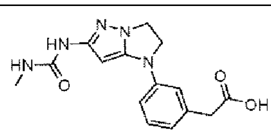 | 1.24 | 316.05 | C |
| I-160 | 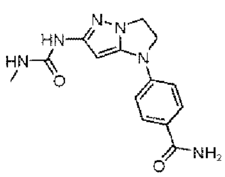 | 0.95 | 301.1 | C |
| I-161 | 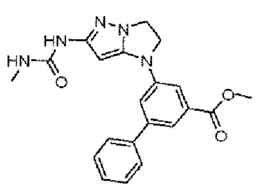 | 2.07 | 392.05 | C |
| I-162 | 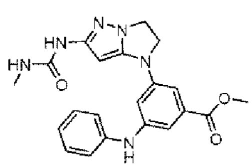 | 1.95 | 407.05 | C |
| I-163 | 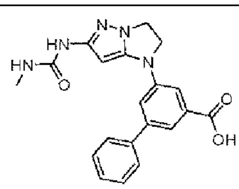 | 1.73 | 378 | C |

(continued)

| Compound No. | Structural Formula | LC/MS Retention Time (min) | MS[M+H]+ | LC Method |
|---|---|---|---|---|
| I-164 | 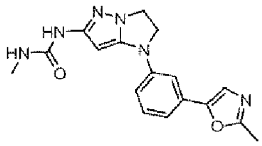 | 1.57 | 339.15 | C |
| I-165 | 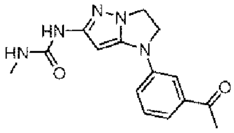 | 1.39 | 300 | C |

[Table 1-23]

| Compound No. | Structural Formula | LC/MS Retention Time (min) | MS[M+H]+ | LC Method |
|---|---|---|---|---|
| I-166 | 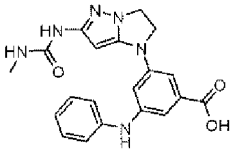 | 1.64 | 393.05 | C |
| I-167 | 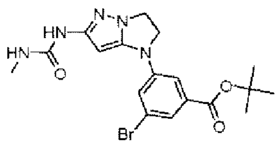 | 2.35 | 436 | C |
| I-168 | 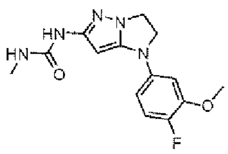 | 1.52 | 306.15 | C |
| I-169 | 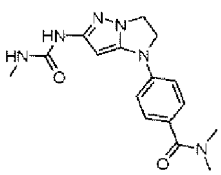 | 1.17 | 329 | C |
| I-170 | 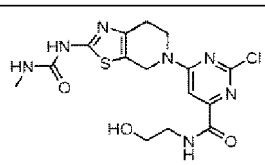 | 1.06 | 412 | D |
| I-171 | 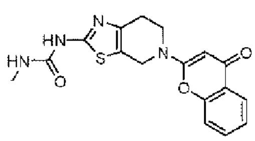 | 1.09 | 357 | D |

(continued)

| Compound No. | Structural Formula | LC/MS Retention Time (min) | MS[M+H]+ | LC Method |
|---|---|---|---|---|
| I-172 | 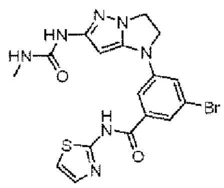 | 1.5 | 462.01 | D |

[Table 1-24]

| Compound No. | Structural Formula | LC/MS Retention Time (min) | MS[M+H]+ | LC Method |
|---|---|---|---|---|
| I-173 | 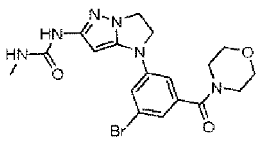 | 1.19 | 449.09 | D |
| I-174 | 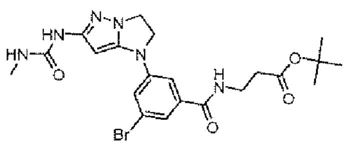 | 1.61 | 507.13 | D |
| I-175 | 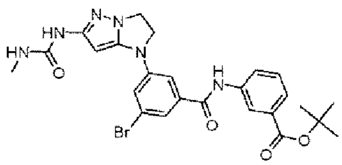 | 2.07 | 555.13 | D |
| I-176 | 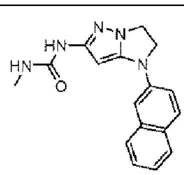 | 1.52 | 308 | D |
| I-177 | 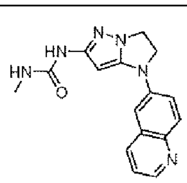 | 0.57 | 309 | D |
| I-178 | 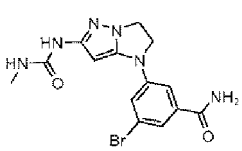 | 1.5 | 379.9 | D |
| I-179 | 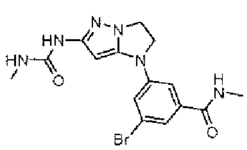 | 1.13 | 393.09 | D |

[Table 1-25]

| Compound No. | Structural Formula | LC/MS Retention Time (min) | MS[M+H]+ | LC Method |
|---|---|---|---|---|
| I-180 | 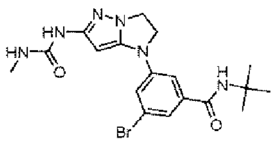 | 1.59 | 435.1 | D |
| I-181 | 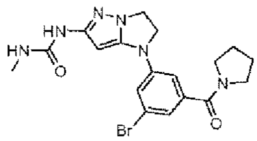 | 1.33 | 433.08 | D |
| I-182 | 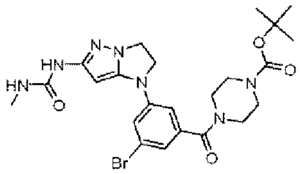 | 1.67 | 548.15 | D |
| I-183 | 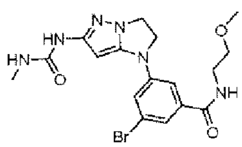 | 1.2 | 437.1 | D |
| I-184 | 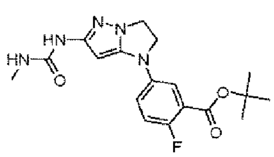 | 2 | 376.05 | C |
| I-185 | 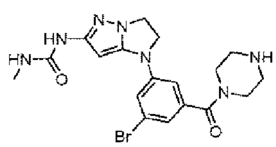 | 0.78 | 448.11 | D |
| I-186 | 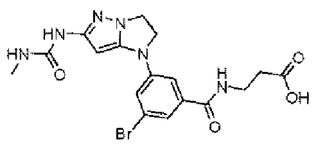 | 1.09 | 451.04 | D |

[Table 1-26]

| Compound No. | Structural Formula | LC/MS Retention Time (min) | MS[M+H]+ | LC Method |
|---|---|---|---|---|
| I-187 | 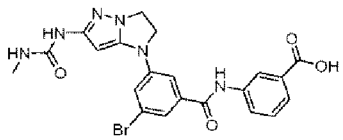 | 1.43 | 499.09 | D |
| I-188 | 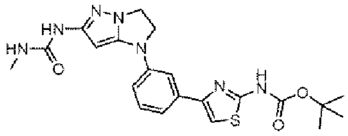 | 1.81 | 456 | D |
| I-189 | 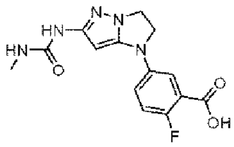 | 1.19 | 320 | C |
| I-190 | 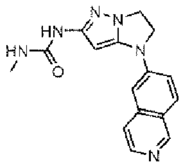 | 0.54 | 309 | D |
| I-191 | 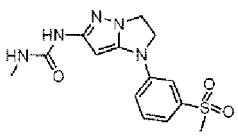 | 1.2 | 336.15 | C |
| I-192 | 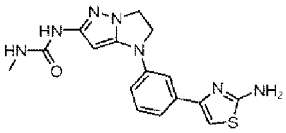 | 0.87 | 356 | D |
| I-193 | 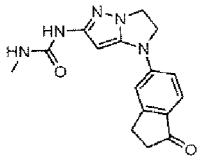 | 1.26 | 312.05 | C |

[Table 1-27]

| Compound No. | Structural Formula | LC/MS Retention Time (min) | MS[M+H]+ | LC Method |
|---|---|---|---|---|
| I-194 | 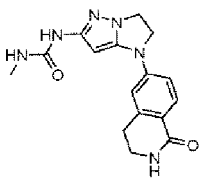 | 1.07 | 327.05 | C |

(continued)

| Compound No. | Structural Formula | LC/MS Retention Time (min) | MS[M+H]+ | LC Method |
|---|---|---|---|---|
| I-195 | 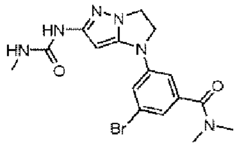 | 1.19 | 407 | D |
| I-196 | 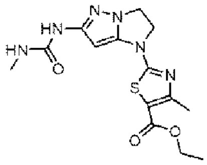 | 1.64 | 351.25 | C |
| I-197 | 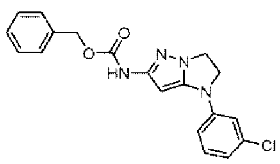 | 2.12 | 369 | D |
| I-198 | 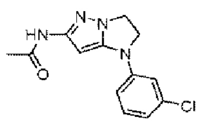 | 1.41 | 277 | D |
| I-199 | 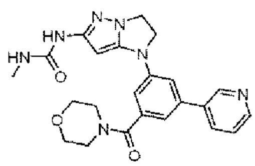 | 0.98 | 448.15 | C |
| I-200 | 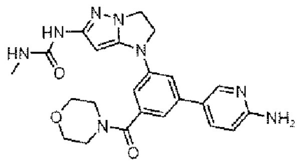 | 0.88 | 463.2 | C |

[Table 1-28]

| Compound No. | Structural Formula | LC/MS Retention Time (min) | MS[M+H]+ | LC Method |
|---|---|---|---|---|
| I-201 | 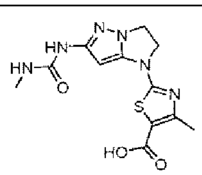 | 1.12 | 323.15 | C |
| I-202 | 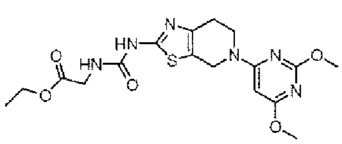 | 1.42 | 423 | D |

(continued)

| Compound No. | Structural Formula | LC/MS Retention Time (min) | MS[M+H]+ | LC Method |
|---|---|---|---|---|
| I-203 | 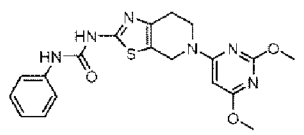 | 1.77 | 413 | D |
| I-204 | 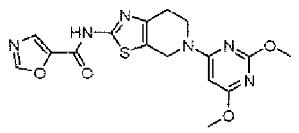 | 1.36 | 389.11 | D |
| I-205 | 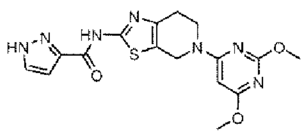 | 1.40 | 388.11 | D |
| I-206 | 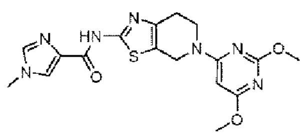 | 1.27 | 402.09 | D |
| I-207 | 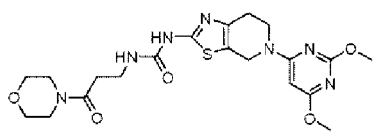 | 1.17 | 478 | D |

[Table 1-29]

| Compound No. | Structural Formula | LC/MS Retention Time (min) | MS[M+H]+ | LC Method |
|---|---|---|---|---|
| I-208 | 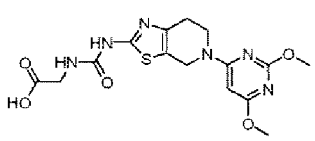 | 1.08 | 395 | D |
| I-209 | 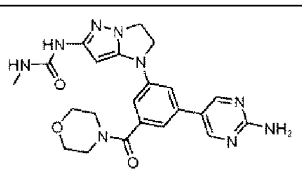 | 1.08 | 464.20 | C |
| I-210 | 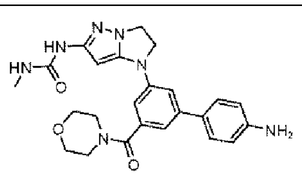 | 1.14 | 462.20 | C |

(continued)

| Compound No. | Structural Formula | LC/MS Retention Time (min) | MS[M+H]+ | LC Method |
|---|---|---|---|---|
| I-211 | 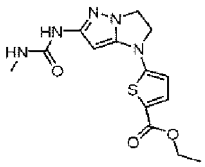 | 1.63 | 336.00 | C |
| I-212 | 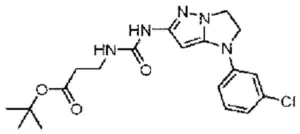 | 1.93 | 406 | D |
| I-213 | 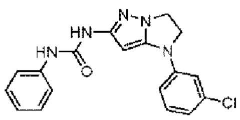 | 1.99 | 354 | D |
| I-214 | 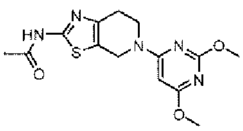 | 1.23 | 336 | D |

[Table 1-30]

| Compound No. | Structural Formula | LC/MS Retention Time (min) | MS[M+H]+ | LC Method |
|---|---|---|---|---|
| I-215 | 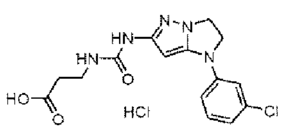 | 1.36 | 350 | D |
| I-216 | 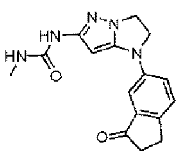 | 1.37 | 312.05 | C |
| I-217 | 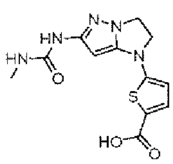 | 1.13 | 308.15 | C |
| I-218 | 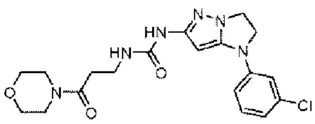 | 1.38 | 419 | D |

(continued)

| Compound No. | Structural Formula | LC/MS Retention Time (min) | MS[M+H]+ | LC Method |
|---|---|---|---|---|
| I-219 | 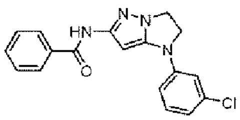 | 1.9 | 339 | D |
| I-220 | 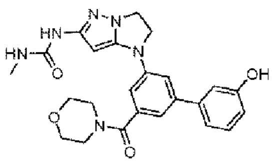 | 1.44 | 463.15 | C |
| I-221 | 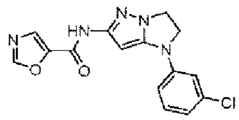 | 1.51 | 330 | D |

[Table 1-31]

| Compound No. | Structural Formula | LC/MS Retention Time (min) | MS[M+H]+ | LC Method |
|---|---|---|---|---|
| I-222 | 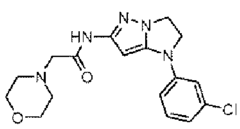 | 1.08 | 362 | D |
| I-223 | 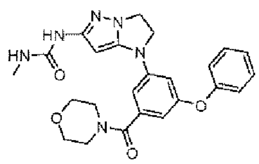 | 1.72 | 463.15 | C |
| I-224 | 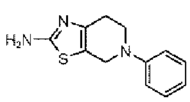 | 0.82 | 232 | D |
| I-225 | 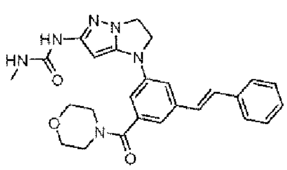 | 1.9 | 473.2 | C |
| I-226 | 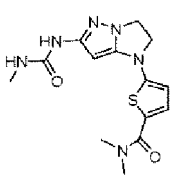 | 1.22 | 335.05 | C |

(continued)

| Compound No. | Structural Formula | LC/MS Retention Time (min) | MS[M+H]+ | LC Method |
|---|---|---|---|---|
| I-227 | 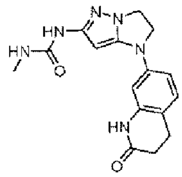 | 1.24 | 327.05 | C |
| I-228 | 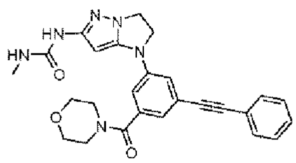 | 1.64 | 471.14 | D |

[Table 1-32]

| Compound No. | Structural Formula | LC/MS Retention Time (min) | MS[M+H]+ | LC method |
|---|---|---|---|---|
| I-229 | 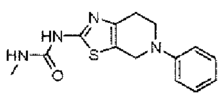 | 1.26 | 289 | D |
| I-230 | 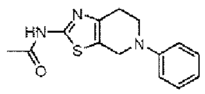 | 1.39 | 274 | D |
| I-231 | 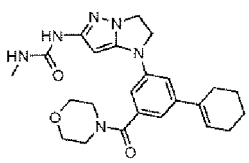 | 1.87 | 451.15 | C |
| I-232 | 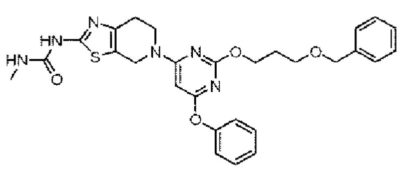 | 2.15 | 547 | D |
| I-233 | 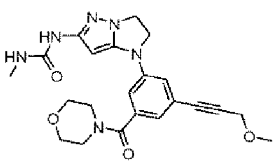 | 1.43 | 439.1 | C |
| I-234 | 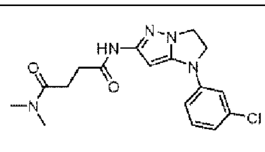 | 1.55 | 362 | A |

(continued)

| Compound No. | Structural Formula | LC/MS Retention Time (min) | MS[M+H]+ | LC method |
|---|---|---|---|---|
| I-235 | 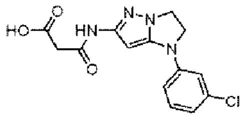 | 1.46 | 321 | A |
| I-236 | 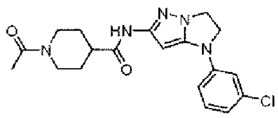 | 1.51 | 388 | A |

[Table 1-33]

| Compound No. | Structural Formula | LC/MS Retention Time (min) | MS[M+H]+ | LC Method |
|---|---|---|---|---|
| I-237 | 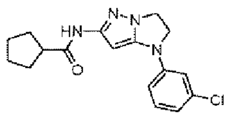 | 2.07 | 331 | A |
| I-238 | 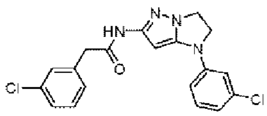 | 2.23 | 387 | A |
| I-239 | 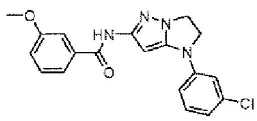 | 2.11 | 369 | A |
| I-240 | 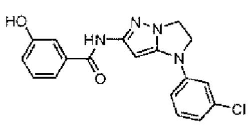 | 1.78 | 355 | A |
| I-241 | 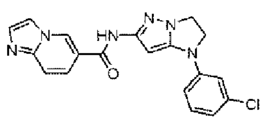 | 1.11 | 379 | A |
| I-242 | 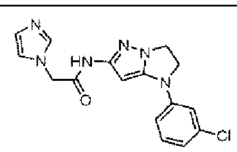 | 1.02 | 343 | A |
| I-243 | 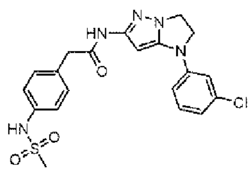 | 1.71 | 446 | A |

(continued)

| Compound No. | Structural Formula | LC/MS Retention Time (min) | MS[M+H]+ | LC Method |
|---|---|---|---|---|
| I-244 | 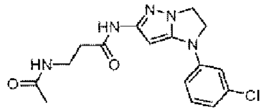 | 1.39 | 348 | A |

[Table 1-34]

| Compound No. | Structural Formula | LC/MS Retention Time (min) | MS[M+H]+ | LC Method |
|---|---|---|---|---|
| I-245 | 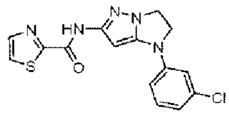 | 1.74 | 346 | A |
| I-246 | 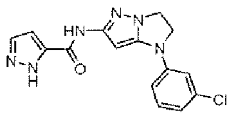 | 1.63 | 329 | A |
| I-247 | 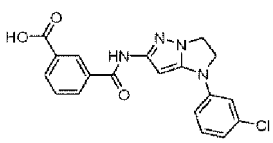 | 1.79 | 383 | A |
| I-248 | 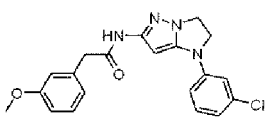 | 2.03 | 383 | A |
| I-249 | 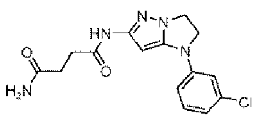 | 1.34 | 334 | A |
| I-250 | 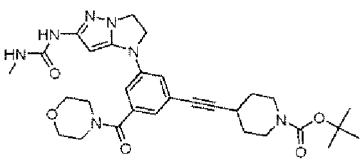 | 2.04 | 578.25 | C |
| I-251 | 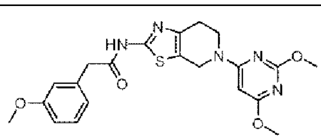 | 1.77 | 442 | D |

[Table 1-35]

| Compound No. | Structural Formula | LC/MS Retention Time (min) | MS[M+H]+ | LC Method |
|---|---|---|---|---|
| I-252 | 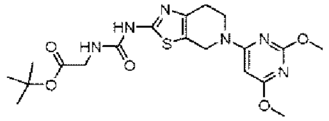 | 1.69 | 451 | D |
| I-253 | 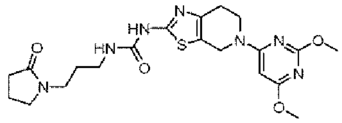 | 1.22 | 462 | D |
| I-254 | 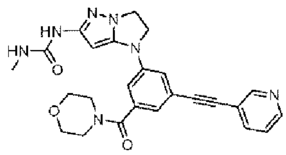 | 1.39 | 472.4 | C |
| I-255 | 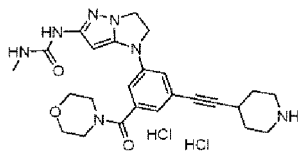 | 0.94 | 478.15 | C |
| I-256 | 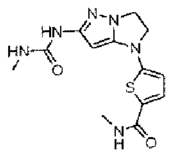 | 1.07 | 321.05 | C |
| I-257 | 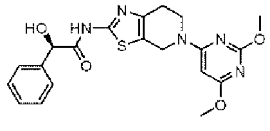 | 1.97 | 428.00 | C |
| I-258 | 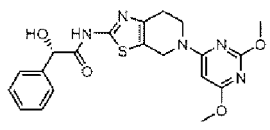 | 1.95 | 428.00 | C |

[Table 1-36]

| Compound No. | Structural Formula | LC/MS Retention Time (min) | MS[M+H]+ | LC Method |
|---|---|---|---|---|
| I-259 | 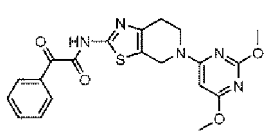 | 2.29 | 426.15 | C |
| I-260 | 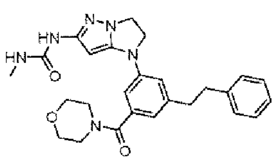 | 1.86 | 475.15 | C |

(continued)

| Compound No. | Structural Formula | LC/MS Retention Time (min) | MS[M+H]+ | LC Method |
|---|---|---|---|---|
| I-261 | 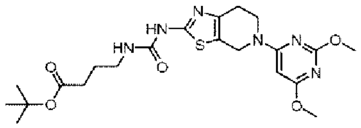 | 1.78 | 479 | D |
| I-262 | 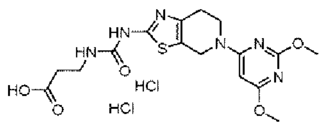 | 1.13 | 409 | D |
| I-263 | 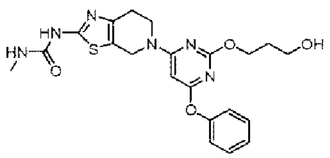 | 1.44 | 457 | D |
| I-264 | 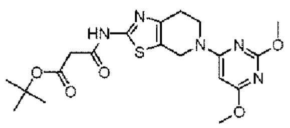 | 2.22 | 436.10 | C |
| I-265 | 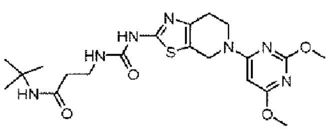 | 1.41 | 464 | D |

[Table 1-37]

| Compound No. | Structural Formula | LC/MS Retention Time (min) | MS[M+H]+ | LC Method |
|---|---|---|---|---|
| I-266 | 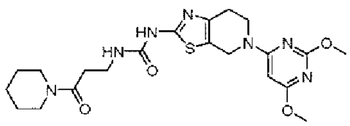 | 1.44 | 476 | D |
| I-267 | 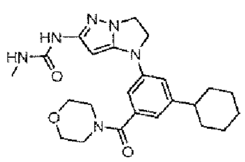 | 1.95 | 453.2 | C |
| I-268 | 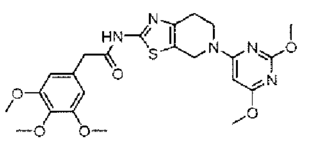 | 1.98 | 502.05 | C |

(continued)

| Compound No. | Structural Formula | LC/MS Retention Time (min) | MS[M+H]+ | LC Method |
|---|---|---|---|---|
| I-269 | 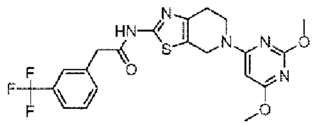 | 2.41 | 480.10 | C |
| I-270 | 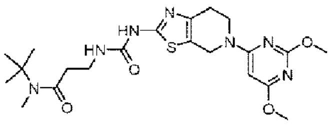 | 1.73 | 478 | A |
| I-271 | 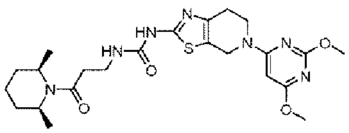 | 1.8 | 504 | A |
| I-272 | 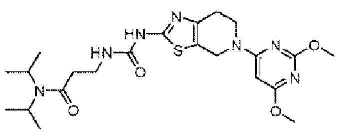 | 1.81 | 492 | A |

[Table 1-38]

| Compound No. | Structural Formula | LC/MS Retention Time (min) | MS[M+H]+ | LC Method |
|---|---|---|---|---|
| I-273 | 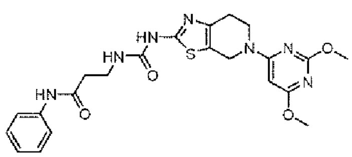 | 1.62 | 484 | A |
| I-274 | 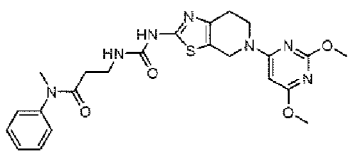 | 1.67 | 498 | A |
| I-275 | 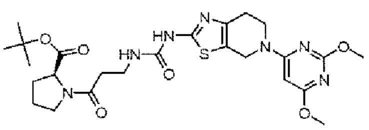 | 1.8 | 562 | A |
| I-276 | 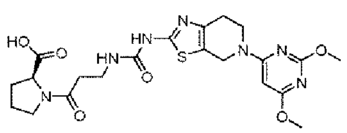 | 1.27 | 506 | A |

(continued)

| Compound No. | Structural Formula | LC/MS Retention Time (min) | MS[M+H]+ | LC Method |
|---|---|---|---|---|
| I-277 | 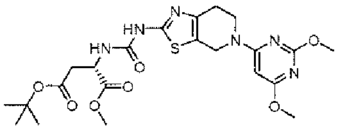 | 1.81 | 523 | D |
| I-278 | 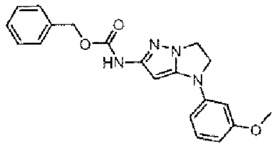 | 2.25 | 365.1 | C |
| I-279 | 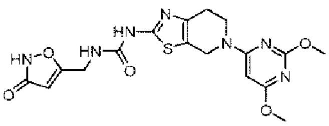 | 1.21 | 434 | D |

[Table 1-39]

| Compound No. | Structural Formula | LC/MS Retention Time (min) | MS[M+H]+ | LC Method |
|---|---|---|---|---|
| I-280 | 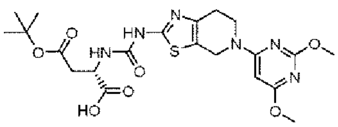 | 1.57 | 509 | D |
| I-281 | 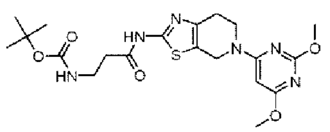 | 2.02 | 465.15 | C |
| I-282 | 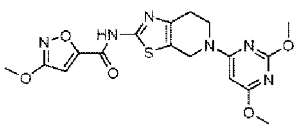 | 1.97 | 419.05 | C |
| I-283 | 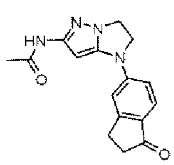 | 1.22 | 297 | C |
| I-284 | 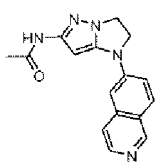 | 0.71 | 294 | C |

(continued)

| Compound No. | Structural Formula | LC/MS Retention Time (min) | MS[M+H]+ | LC Method |
|---|---|---|---|---|
| I-285 | 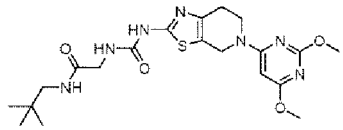 | 1.52 | 464 | D |
| I-286 | 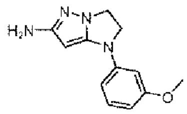 | 1 | 231 | C |

[Table 1-40]

| Compound No. | Structural Formula | LC/MS Retention Time (min) | MS[M+H]+ | LC Method |
|---|---|---|---|---|
| I-287 | 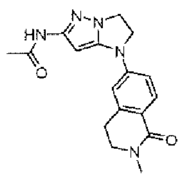 | 1.18 | 326.05 | C |
| I-288 | 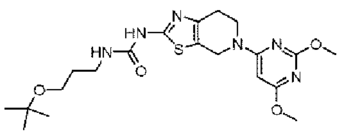 | 1.7 | 451 | D |
| I-289 | 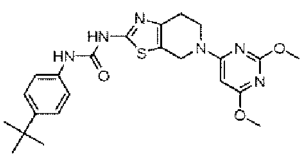 | 2.28 | 469 | D |
| I-290 | 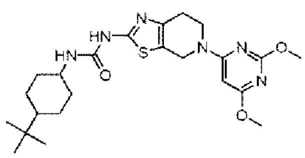 | 2.42 | 475 | D |
| I-291 | 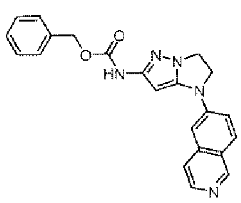 | 1.26 | 386.1 | C |
| I-292 | 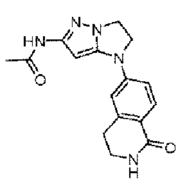 | 1.03 | 312.05 | C |

(continued)

| Compound No. | Structural Formula | LC/MS Retention Time (min) | MS[M+H]+ | LC Method |
|---|---|---|---|---|
| I-293 | 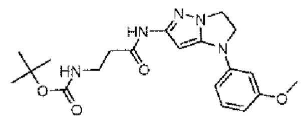 | 1.96 | 402.35 | C |

[Table 1-41]

| Compound No. | Structural Formula | LC/MS Retention Time (min) | MS[M+H]+ | LC Method |
|---|---|---|---|---|
| I-294 | 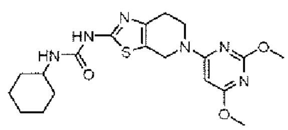 | 1.82 | 419 | D |
| I-295 | 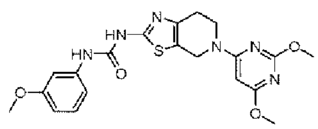 | 1.79 | 443 | D |
| I-296 | 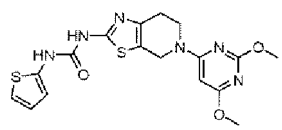 | 1.69 | 419 | D |
| I-297 | 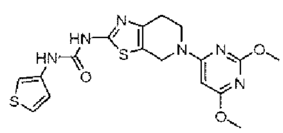 | 1.72 | 419 | D |
| I-298 | 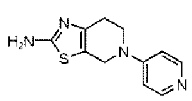 | 0.18 | 233 | D |
| I-299 | 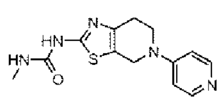 | 0.47 | 290 | D |
| I-300 | 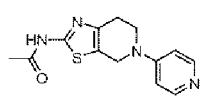 | 0.46 | 275 | D |

[Table 1-42]

| Compound No. | Structural Formula | LC/MS Retention Time (min) | MS[M+H]+ | LC Method |
|---|---|---|---|---|
| I - 301 | 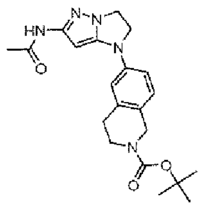 | 1.99 | 398.15 | C |
| I - 302 | 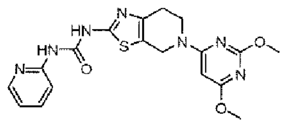 | 1.47 | 414 | D |
| I - 303 | 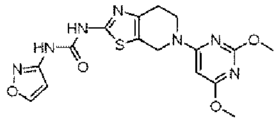 | 1.45 | 404 | D |
| I - 304 | 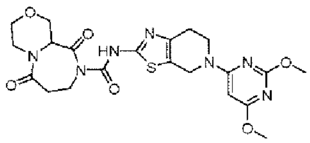 | 1.26 | 504 | D |
| I - 305 | 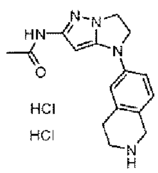 HCl HCl | 0.6 | 298 | C |
| I - 306 | 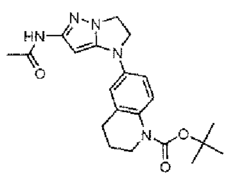 | 2.08 | 398.1 | C |
| I - 307 | 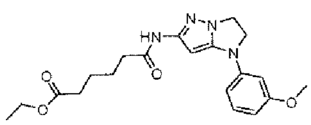 | 2.02 | 387.1 | C |

[Table 1-43]

| Compound No. | Structural Formula | LC/MS Retention Time (min) | MS[M+H]+ | LC Method |
|---|---|---|---|---|
| I-308 | 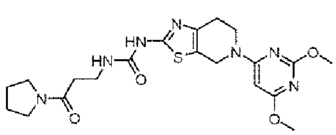 | 1.44 | 462 | A |

(continued)

| Compound No. | Structural Formula | LC/MS Retention Time (min) | MS[M+H]+ | LC Method |
|---|---|---|---|---|
| I-309 | 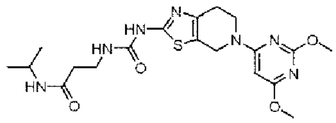 | 1.37 | 450 | A |
| I-310 | 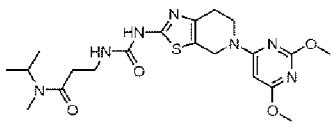 | 1.55 | 464 | A |
| I-311 | 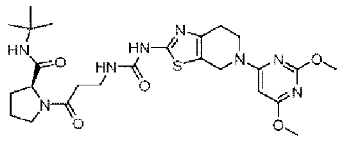 | 1.54 | 561 | A |
| I-312 | 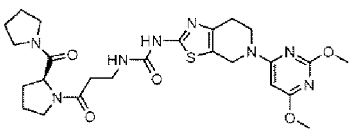 | 1.38 | 559 | A |
| I-313 | 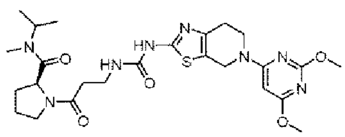 | 1.47 | 561 | A |
| I-314 | 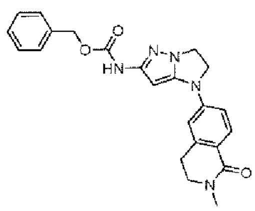 | 1.88 | 418.1 | C |

[Table 1-44]

| Compound No. | Structural Formula | LC/MS Retention Time (min) | MS[M+H]+ | LC Method |
|---|---|---|---|---|
| I-315 | 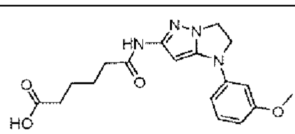 | 1.61 | 359.1 | C |
| I-316 | 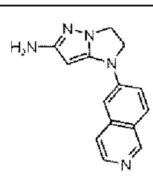 | 0.37 | 252.2 | C |

(continued)

| Compound No. | Structural Formula | LC/MS Retention Time (min) | MS[M+H]+ | LC Method |
|---|---|---|---|---|
| I-317 | 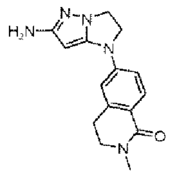 | 0.62 | 284 | D |
| I-318 | 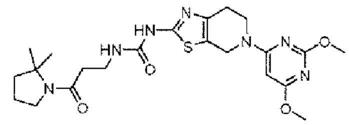 | 1.57 | 490 | D |
| I-319 | 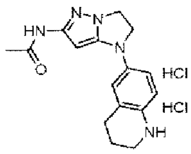 | 0.65 | 297.9 | C |
| I-320 | 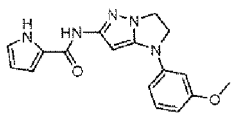 | 1.82 | 324 | C |
| I-321 | 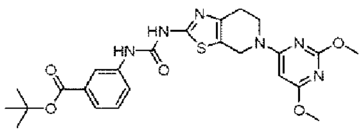 | 2.25 | 513 | D |

[Table 1-45]

| Compound No. | Structural Formula | LC/MS Retention Time (min) | MS[M+H]+ | LC Method |
|---|---|---|---|---|
| I-322 | 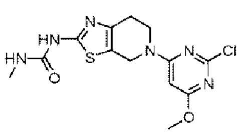 | 1.80 | 355.00 | D |
| I-323 | 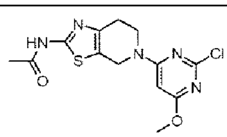 | 1.90 | 339.93 | D |
| I-324 | 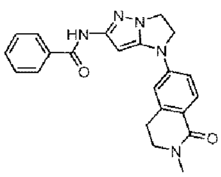 | 1.64 | 388.1 | C |

(continued)

| Compound No. | Structural Formula | LC/MS Retention Time (min) | MS[M+H]+ | LC Method |
|---|---|---|---|---|
| I-325 | 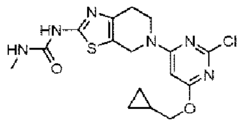 | 2.12 | 394.96 | D |
| I-326 | 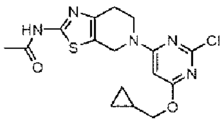 | 2.20 | 379.97 | D |
| I-327 | 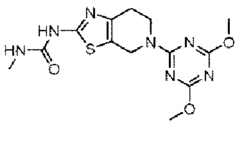 | 1.21 | 352.00 | D |

[Table 1-46]

| Compound No. | Structural Formula | LC/MS Retention Time (min) | MS[M+H]+ | LC Method |
|---|---|---|---|---|
| I-328 | 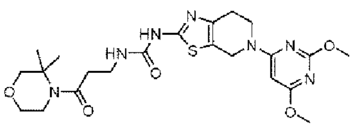 | 1.39 | 506 | D |
| I-329 | 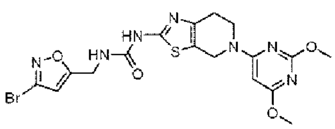 | 1.59 | 496 | D |
| I-330 | 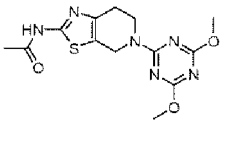 | 1.26 | 337.01 | D |
| I-331 | 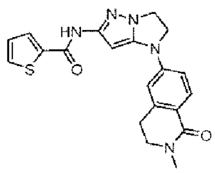 | 1.59 | 394.05 | C |
| I-332 | 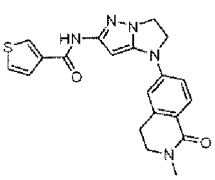 | 1.57 | 394.05 | C |

(continued)

| Compound No. | Structural Formula | LC/MS Retention Time (min) | MS[M+H]+ | LC Method |
|---|---|---|---|---|
| I-333 | 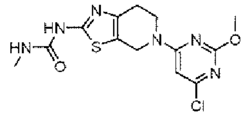 | 1.37 | 354.98 | D |
| I-334 | 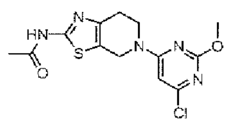 | 1.42 | 339.93 | D |

[Table 1-47]

| Compound No. | Structural Formula | LC/MS Retention Time (min) | MS[M+H]+ | LC Method |
|---|---|---|---|---|
| I-335 | 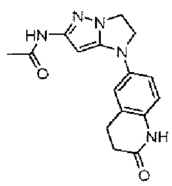 | 1.05 | 312.00 | C |
| I-336 | 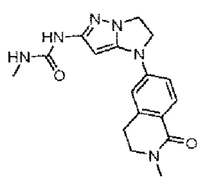 | 1.22 | 341.10 | C |
| I-337 | 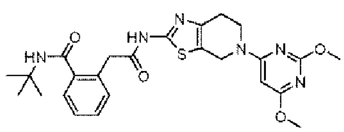 | 2.01 | 511 | D |
| I-338 | 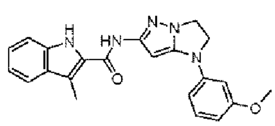 | 2.32 | 388.10 | C |
| I-339 | 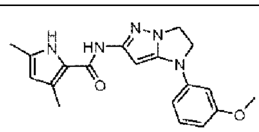 | 2.05 | 352.40 | C |
| I-340 | 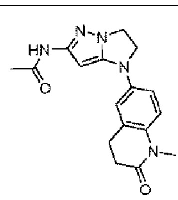 | 7.23 | 326.10 | C |

(continued)

| Compound No. | Structural Formula | LC/MS Retention Time (min) | MS[M+H]+ | LC Method |
|---|---|---|---|---|
| I-341 | 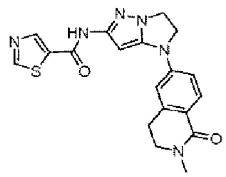 | 1.34 | 395.05 | C |

[Table 1-48]

| Compound No. | Structural Formula | LC/MS Retention Time (min) | MS[M+H]+ | LC Method |
|---|---|---|---|---|
| I-342 | 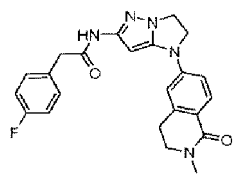 | 1.65 | 420.10 | C |
| I-343 | 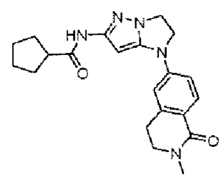 | 1.60 | 380.10 | C |
| I-344 | 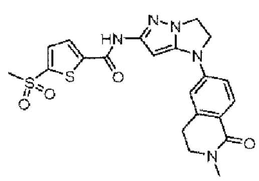 | 1.26 | 471.96 | D |
| I-345 | 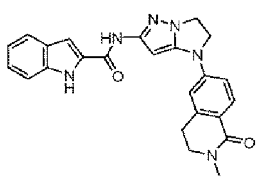 | 1.54 | 427.05 | D |
| I-346 | 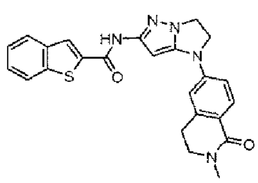 | 1.68 | 443.99 | D |
| I-347 | 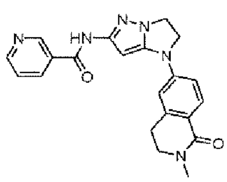 | 1.02 | 389.04 | D |

(continued)

| Compound No. | Structural Formula | LC/MS Retention Time (min) | MS[M+H]+ | LC Method |
|---|---|---|---|---|
| I-348 | 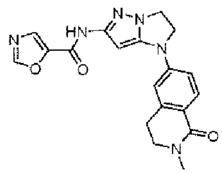 | 1.00 | 379.06 | D |

[Table 1-49]

| Compound No. | Structural Formula | LC/MS Retention Time (min) | MS[M+H]+ | LC Method |
|---|---|---|---|---|
| I-349 | 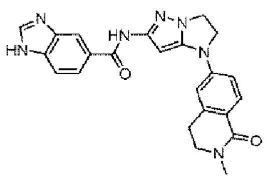 | 0.84 | 428.02 | D |
| I-350 | 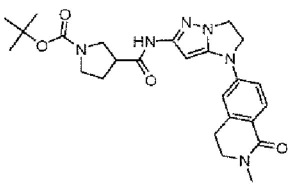 | 1.49 | 481.11 | D |
| I-351 | 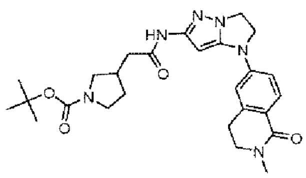 | 1.50 | 495.13 | D |
| I-352 | 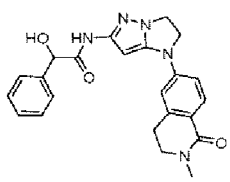 | 1.25 | 418.05 | D |
| I-353 | 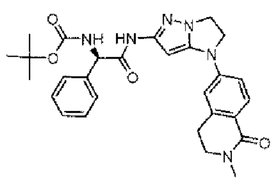 | 1.66 | 517.11 | D |
| I-354 | 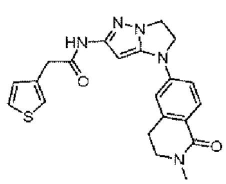 | 1.34 | 408.01 | D |

(continued)

| Compound No. | Structural Formula | LC/MS Retention Time (min) | MS[M+H]+ | LC Method |
|---|---|---|---|---|
| I-355 | 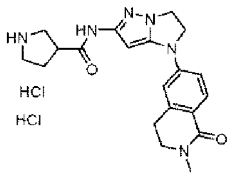 | 0.86 | 381.10 | C |

[Table 1-50]

| Compound No. | Structural Formula | LC/MS Retention Time (min) | MS[M+H]+ | LC Method |
|---|---|---|---|---|
| I-356 | 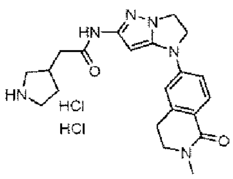 | 0.73 | 395.11 | D |
| I-357 | 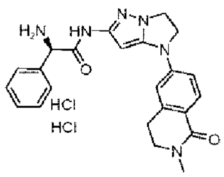 | 1.03 | 417.15 | C |
| I-358 | 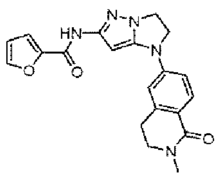 | 1.44 | 378.1 | C |
| I-359 | 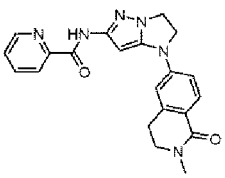 | 1.61 | 389.05 | C |
| I-360 | 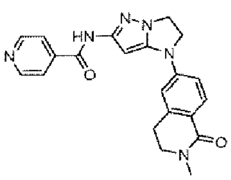 | 1.18 | 389.10 | C |
| I-361 | 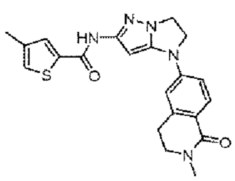 | 1.75 | 408.10 | C |

(continued)

| Compound No. | Structural Formula | LC/MS Retention Time (min) | MS[M+H]+ | LC Method |
|---|---|---|---|---|
| I-362 | 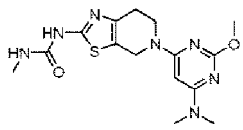 | 0.84 | 364.00 | D |

[Table 1-51]

| Compound No. | Structural Formula | LC/MS Retention Time (min) | MS[M+H]+ | LC Method |
|---|---|---|---|---|
| I-363 | 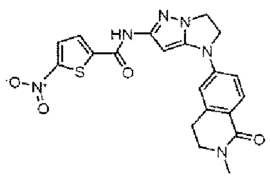 | 1.80 | 439.05 | C |
| I-364 | 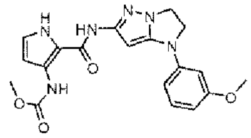 | 1.97 | 397.30 | C |
| I-365 | 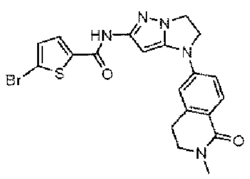 | 1.92 | 472.15 | C |
| I-366 | 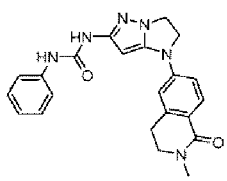 | 1.75 | 403.1 | C |
| I-367 | 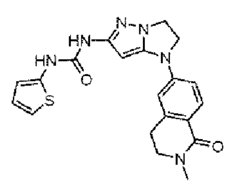 | 1.68 | 409.05 | C |
| I-368 | 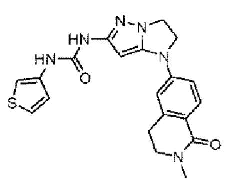 | 1.68 | 409.1 | C |
| I-369 | 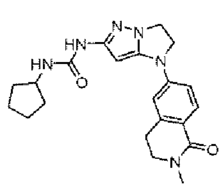 | 1.68 | 395.15 | C |

[Table 1-52]

| Compound No | Structural Formula | LC/MS Retention Time (min) | MS[M+H]+ | LC Method |
|---|---|---|---|---|
| I-370 | 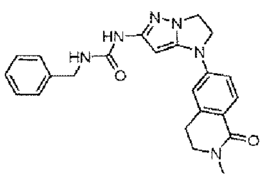 | 1.71 | 417.1 | C |
| I-371 | 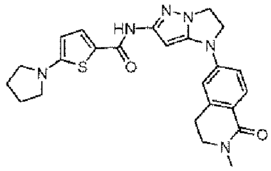 | 0.9 | 463.09 | D |
| I-372 | 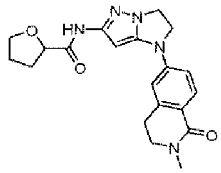 | 1.1 | 382.26 | D |
| I-373 | 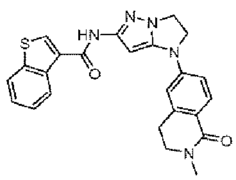 | 1.64 | 444.21 | D |
| I-374 | 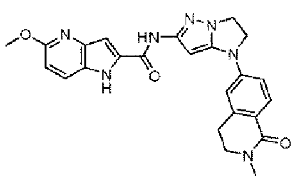 | 1.28 | 458.24 | D |
| I-375 | 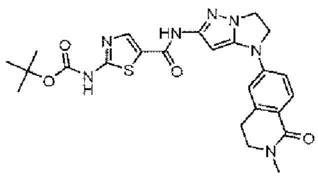 | 1.51 | 510.22 | D |
| I-376 | 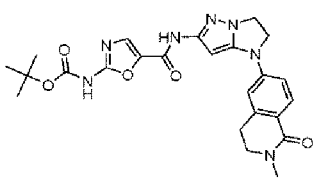 | 1.33 | 494.23 | D |

[Table 1-53]

| Compound No. | Structural Formula | LC/MS Retention Time (min) | MS[M+H]+ | LC Method |
|---|---|---|---|---|
| I-377 | 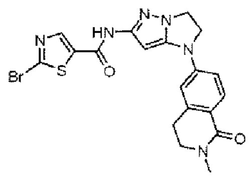 | 1.42 | 473.09 | D |
| I-378 | 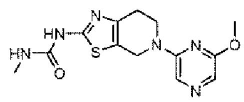 | 1.18 | 321.16 | D |
| I-379 | 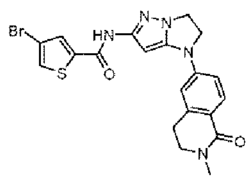 | 1.88 | 471.95 | C |
| I-380 | 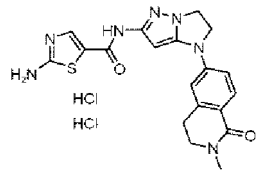 HCl HCl | 1.16 | 410.25 | C |
| I-381 | 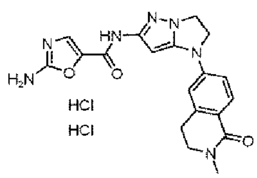 HCl HCl | 1.14 | 394.05 | C |
| I-382 | 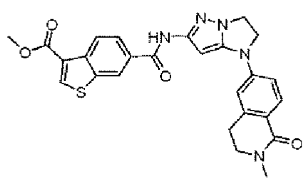 | 1.62 | 502.13 | D |
| I-383 | 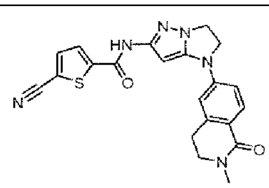 | 1.41 | 419.15 | D |

[Table 1-54]

| Compound No. | Structural Formula | LC/MS Retention Time (min) | MS[M+H]+ | LC Method |
|---|---|---|---|---|
| I-384 | 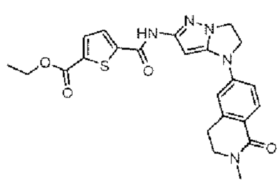 | 1.58 | 466.13 | D |

(continued)

| Compound No. | Structural Formula | LC/MS Retention Time (min) | MS[M+H]+ | LC Method |
|---|---|---|---|---|
| I-385 | 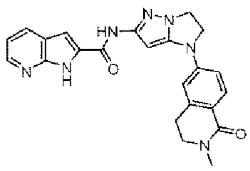 | 1.16 | 428.17 | D |
| I-386 | 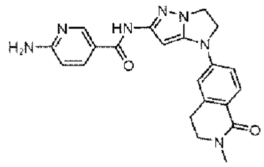 | 0.95 | 404.2 | C |
| I-387 | 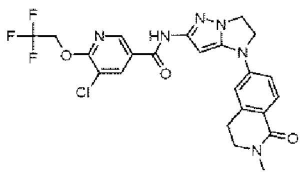 | 1.83 | 521.12 | D |
| I-388 | 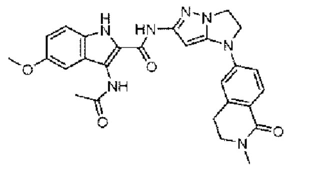 | 1.32 | 514.25 | C |
| I-389 | 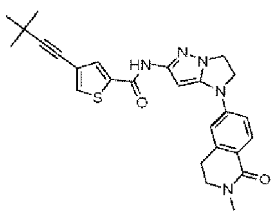 | 2.33 | 474.2 | C |
| I-390 | 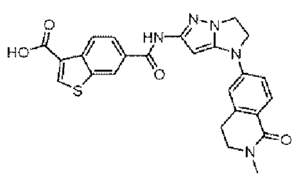 | 1.63 | 488.2 | C |

[Table 1-55]

| Compound No | Structural Formula | LC/MS Retention Time (min) | MS[M+H]+ | LC Method |
|---|---|---|---|---|
| I-391 | 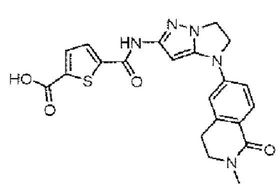 | 1.44 | 438.15 | C |
| I-392 | 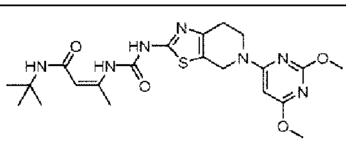 | 1.91 | 476 | D |

(continued)

| Compound No | Structural Formula | LC/MS Retention Time (min) | MS[M+H]+ | LC Method |
|---|---|---|---|---|
| I-393 | 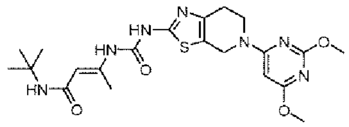 | 1.71 | 476 | D |
| I-394 | 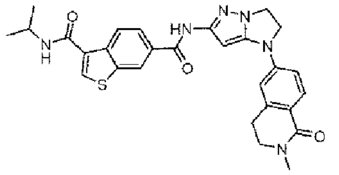 | 1.76 | 529.25 | C |
| I-395 | 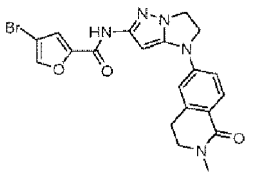 | 1.73 | 456.05 | C |
| I-396 | 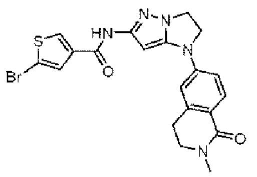 | 1.88 | 472.05 | C |
| I-397 | 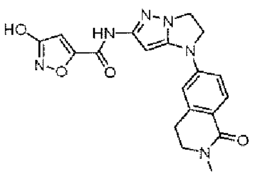 | 1.3 | 395.15 | C |

[Table 1-56]

| Compound No. | Structural Formula | LC/MS Retention Time (min) | MS[M+H]$^+$ | LC Method |
|---|---|---|---|---|
| I-398 | 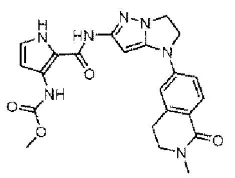 | 1.6 | 450.2 | C |
| I-399 | 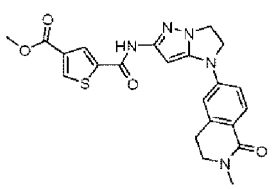 | 1.7 | 452.15 | C |
| I-400 | 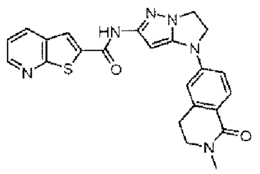 | 1.64 | 455.15 | C |

(continued)

| Compound No. | Structural Formula | LC/MS Retention Time (min) | MS[M+H]+ | LC Method |
|---|---|---|---|---|
| I-401 | 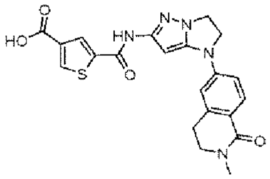 | 1.46 | 438.15 | C |
| I-402 | 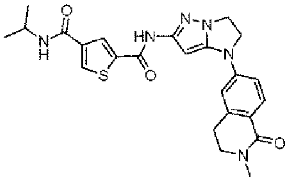 | 1.6 | 479.2 | C |
| I-403 | 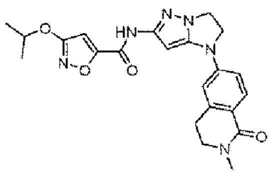 | 1.82 | 437.2 | C |
| I-404 | 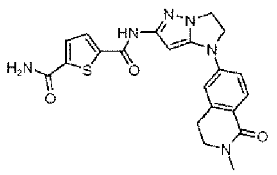 | 1.3 | 437.15 | C |

[Table 1-57]

| Compound No. | Structural Formula | LC/MS Retention Time (min) | MS[M+H]+ | LC Method |
|---|---|---|---|---|
| I-405 | 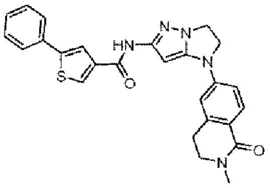 | 2.12 | 470.2 | C |
| I-406 | 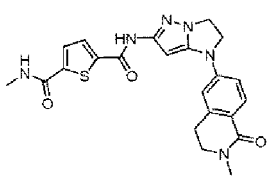 | 1.38 | 451.15 | C |
| I-407 | 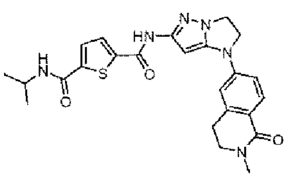 | 1.62 | 479.2 | C |

(continued)

| Compound No. | Structural Formula | LC/MS Retention Time (min) | MS[M+H]+ | LC Method |
|---|---|---|---|---|
| I-408 | 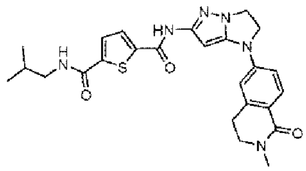 | 1.76 | 493.2 | C |
| I-409 | 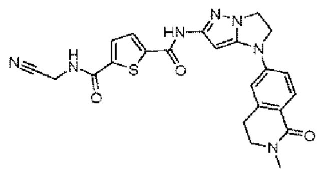 | 1.45 | 476.15 | C |
| I-410 | 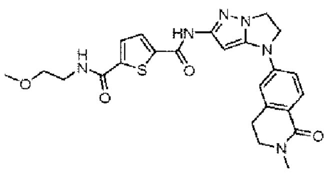 | 1.46 | 495.2 | C |
| I-411 | 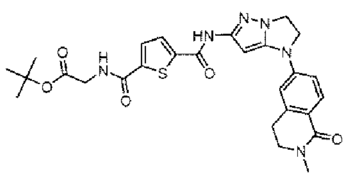 | 1.82 | 551.2 | C |

[Table 1-58]

| Compound No. | Structural Formula | LC/MS Retention Time (min) | MS[M+H]+ | LC Method |
|---|---|---|---|---|
| I-412 | 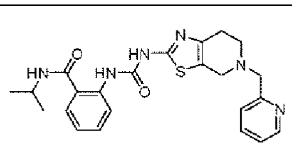 | 1.01 | 451 | D |
| I-413 | 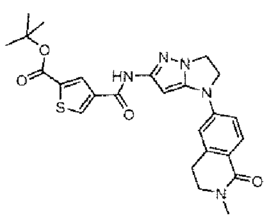 | 2.12 | 494.2 | C |
| I-414 | 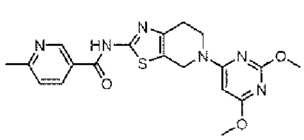 | 1.63 | 413 | D |
| I-415 | 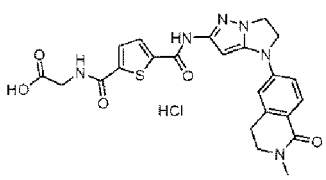 HCl | 1.33 | 495.1 | C |

(continued)

| Compound No. | Structural Formula | LC/MS Retention Time (min) | MS[M+H]+ | LC Method |
|---|---|---|---|---|
| I-416 | 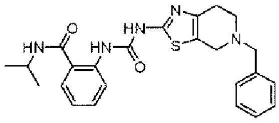 | 1.16 | 450 | D |
| I-417 | 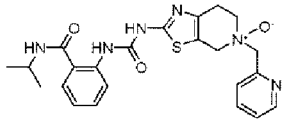 | 1.04 | 467 | D |
| I-418 | 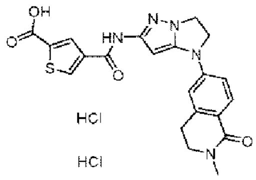 HCl HCl | 1.42 | 438.25 | C |

[Table 1-59]

| Compound No. | Structural Formula | LC/MS Retention Time (min) | MS[M+H]+ | LC Method |
|---|---|---|---|---|
| I-419 | 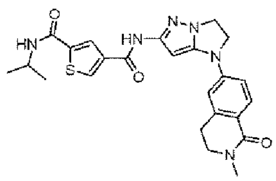 | 1.81 | 419.15 | C |
| I-420 | 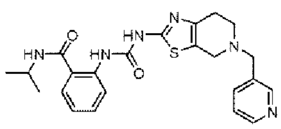 | 0.88 | 451 | D |
| I-421 | 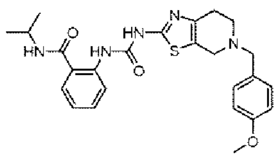 | 1.2 | 480 | D |
| I-422 | 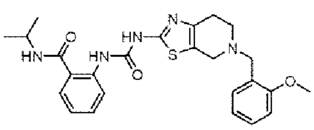 | 1.22 | 480 | D |
| I-423 | 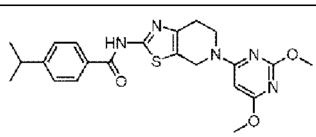 | 2.59 | 440 | D |

(continued)

| Compound No. | Structural Formula | LC/MS Retention Time (min) | MS[M+H]+ | LC Method |
|---|---|---|---|---|
| I-424 | 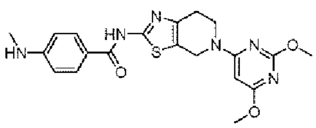 | 2.04 | 327 | D |
| I-425 | 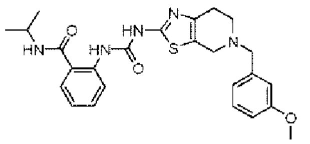 | 1.23 | 480 | D |

[Table 1-60]

| Compound No. | Structural Formula | LC/MS Retention Time (min) | MS[M+H]+ | LC Method |
|---|---|---|---|---|
| I-426 | 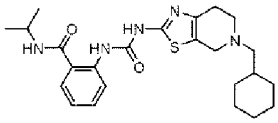 | 1.27 | 456 | D |
| I-427 | 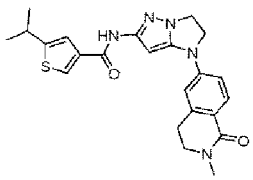 | 2.04 | 436.15 | C |
| I-428 | 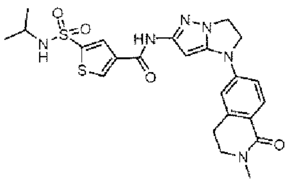 | 1.71 | 515.15 | C |
| I-429 | 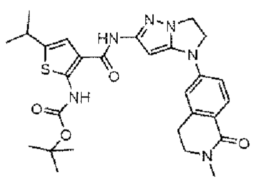 | 2.7 | 551.3 | C |
| I-430 | 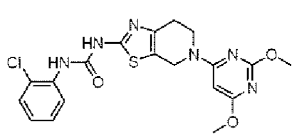 | 2.35 | 447 | D |
| I-431 | 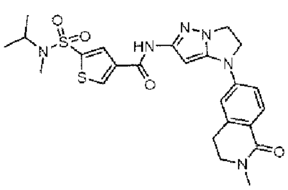 | 1.9 | 529.4 | C |

(continued)

| Compound No. | Structural Formula | LC/MS Retention Time (min) | MS[M+H]+ | LC Method |
|---|---|---|---|---|
| I-432 | 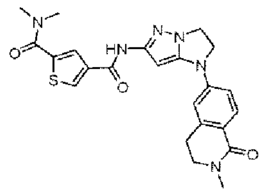 | 1.45 | 465.15 | C |

[Table 1-61]

| Compound No. | Structural Formula | LC/MS Retention Time (min) | MS[M+H]+ | LC Method |
|---|---|---|---|---|
| I-433 | 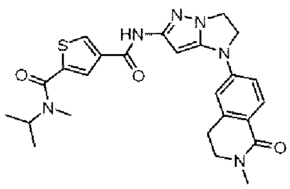 | 1.72 | 493 | C |
| I-434 | 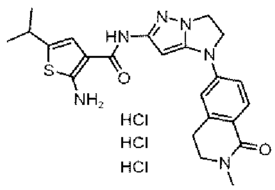 | 2.00 | 451 | C |
| I-435 | 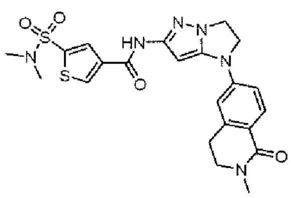 | 1.41 | 501 | D |
| I-436 | 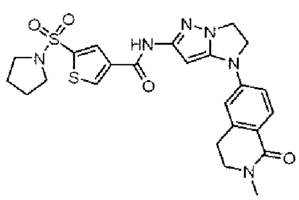 | 1.54 | 527 | D |
| I-437 | 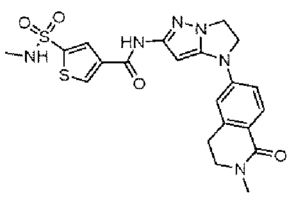 | 1.23 | 487 | D |
| I-438 | 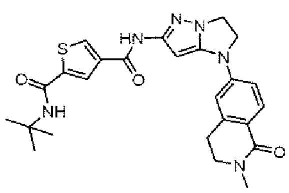 | 1.51 | 493 | D |

[Table 1-62]

| Compound No. | Structural Formula | LC/MS Retention Time (min) | MS[M+H]+ | LC Method |
|---|---|---|---|---|
| I-439 | 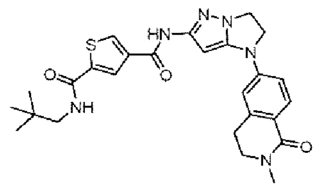 | 1.86 | 507 | C |
| I-440 | 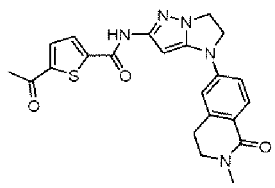 | 1.60 | 436 | C |
| I-441 | 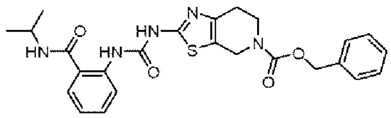 | 2.04 | 494 | D |
| I-442 | 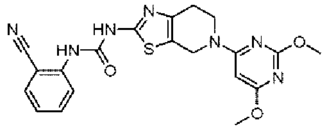 | 2.30 | 438 | C |
| I-443 | 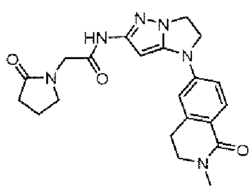 | 1.20 | 409 | C |
| I-444 | 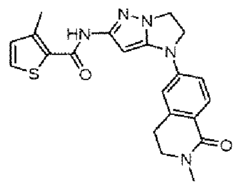 | 1.70 | 408 | C |
| I-445 | 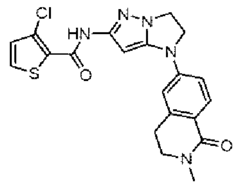 | 1.81 | 428 | C |

[Table 1-63]

| Compound No. | Structural Formula | LC/MS Retention Time (min) | MS[M+H]+ | LC Method |
|---|---|---|---|---|
| I-446 | 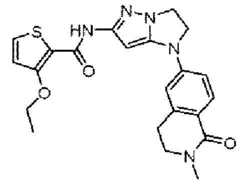 | 1.84 | 438 | C |
| I-447 | 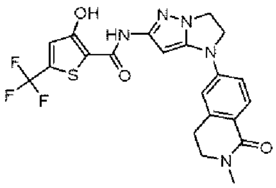 | 1.95 | 478 | C |
| I-448 | 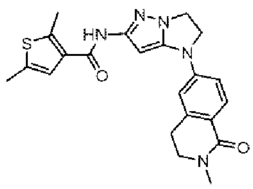 | 1.88 | 422 | C |
| I-449 | 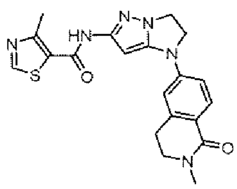 | 1.38 | 409 | C |
| I-450 | 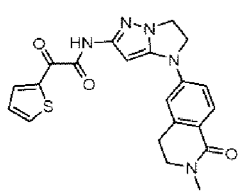 | 1.78 | 422 | C |

[Table 1-64]

| Compound No. | Structural Formula | LC/MS Retention Time (min) | MS[M+H]+ | LC Method |
|---|---|---|---|---|
| I-451 | 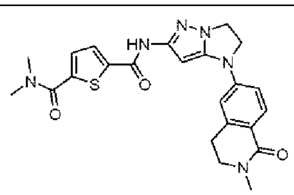 | 1.46 | 465.1 | C |
| I-452 | 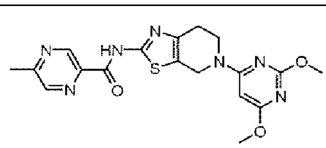 | 1.95 | 414 | C |

(continued)

| Compound No. | Structural Formula | LC/MS Retention Time (min) | MS[M+H]+ | LC Method |
|---|---|---|---|---|
| I-453 | 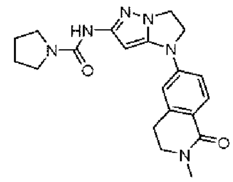 | 1.33 | 381 | C |
| I-454 | 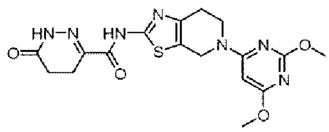 | 1.63 | 418 | C |
| I-455 | 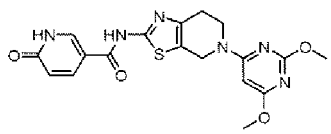 | 1.52 | 415 | C |
| I-456 | 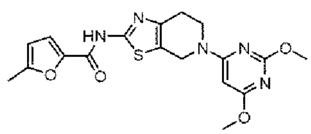 | 2.04 | 402 | C |
| I-457 | 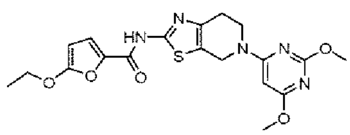 | 2.12 | 432 | C |

[Table 1-65]

| Compound No. | Structural Formula | LC/MS Retention Time (min) | MS[M+H]+ | LC Method |
|---|---|---|---|---|
| I-468 | 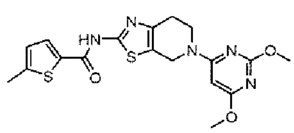 | 2.23 | 418 | C |
| I-459 | 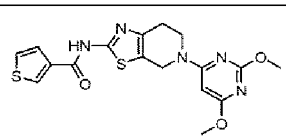 | 2.05 | 404 | C |
| I-460 | 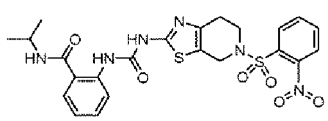 | 1.89 | 545 | D |
| I-461 | 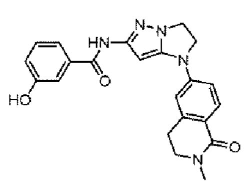 | 1.43 | 404 | C |

(continued)

| Compound No. | Structural Formula | LC/MS Retention Time (min) | MS[M+H]$^+$ | LC Method |
|---|---|---|---|---|
| I-462 | 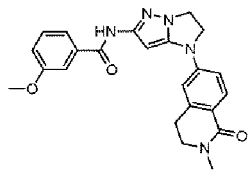 | 1.69 | 418 | C |
| I-463 | 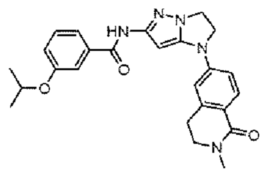 | 1.95 | 446 | C |
| I-464 | 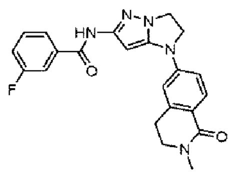 | 1.72 | 406 | C |
| I-465 | 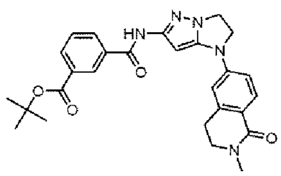 | 2.12 | 488 | C |

[Table 1-66]

| Compound No. | Structural Formula | LC/MS Retention Time (min) | MS[M+H]$^+$ | LC Method |
|---|---|---|---|---|
| I-466 | 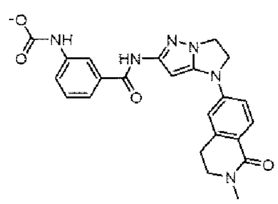 | 1.96 | 503 | C |
| I-467 | 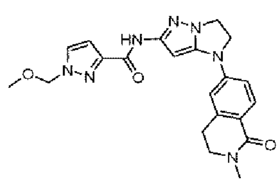 | 1.43 | 422 | C |
| I-408 | 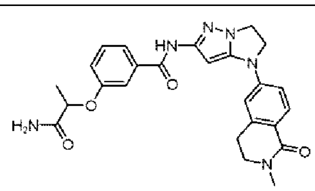 | 1.43 | 475 | C |

(continued)

| Compound No. | Structural Formula | LC/MS Retention Time (min) | MS[M+H]+ | LC Method |
|---|---|---|---|---|
| I-469 | 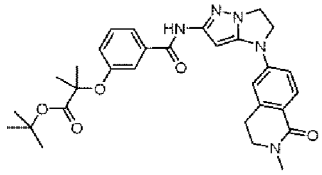 | 2.24 | 546 | C |
| I-470 | 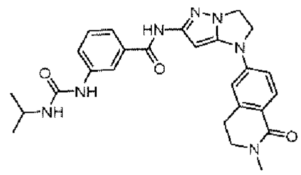 | 1.60 | 488 | C |
| I-471 | 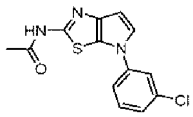 | 2.13 | 292 | C |
| I-472 | 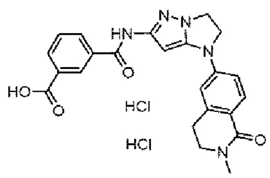 | 1.46 | 432 | C |

[Table 1-67]

| Compound No. | Structural Formula | LC/MS Retention Time (min) | MS[M+H]+ | LC Method |
|---|---|---|---|---|
| I-473 | 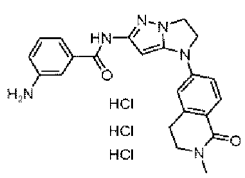 | 1.25 | 403 | C |
| I-474 | 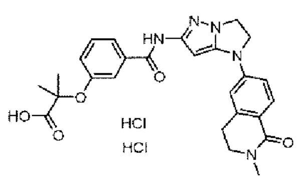 | 1.65 | 490 | C |
| I-475 | 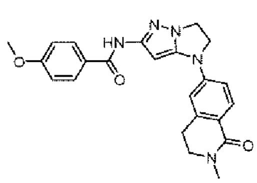 | 1.66 | 418 | C |
| I-476 | 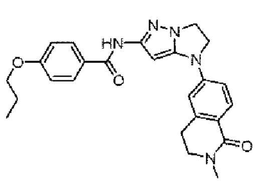 | 1.99 | 446 | C |

(continued)

| Compound No. | Structural Formula | LC/MS Retention Time (min) | MS[M+H]+ | LC Method |
|---|---|---|---|---|
| I-477 | 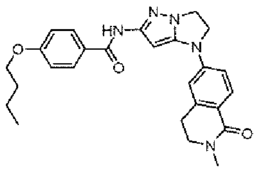 | 2.16 | 460 | C |
| I-478 | 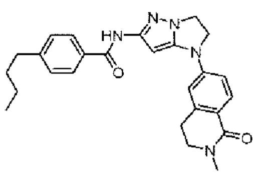 | 2.25 | 444 | C |
| I-479 | 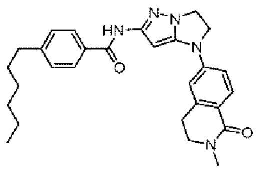 | 2.57 | 472 | C |

[Table 1-68]

| Compound No. | Structural Formula | LC/MS Retention Time (min) | MS[M+H]+ | LC Method |
|---|---|---|---|---|
| I-480 | 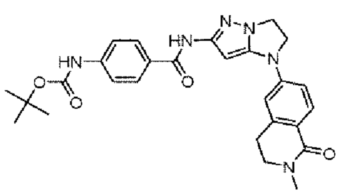 | 1.93 | 503 | C |
| I-481 | 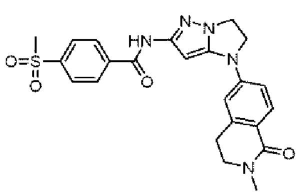 | 1.45 | 466 | C |
| I-482 | 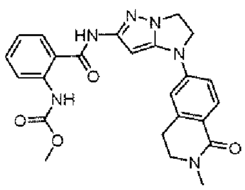 | 1.80 | 461 | C |
| I-483 | 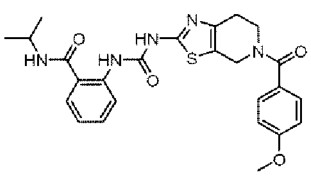 | 1.69 | 494 | D |

(continued)

| Compound No. | Structural Formula | LC/MS Retention Time (min) | MS[M+H]+ | LC Method |
|---|---|---|---|---|
| I-484 | 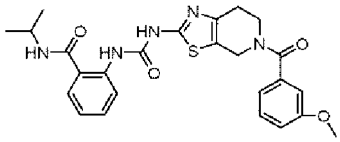 | 1.74 | 494 | D |

[Table 1-69]

| Compound No. | Structural Formula | LC/MS Retention Time (min) | MS[M+H]+ Method | LC Method |
|---|---|---|---|---|
| I-485 | 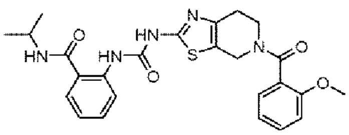 | 1.70 | 494 | D |
| I-485 | 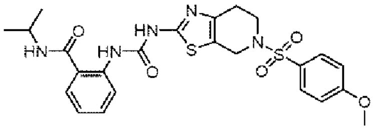 | 1.88 | 530 | D |
| I-487 | 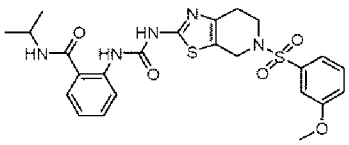 | 1.92 | 530 | D |
| I-488 | 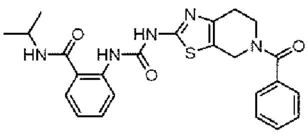 | 1.70 | 464 | D |
| I-489 | 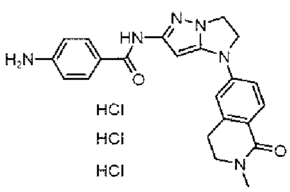 | 1.33 | 403 | C |
| I-490 | 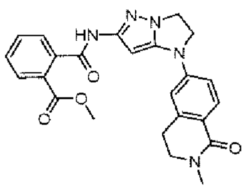 | 1.53 | 446 | C |
| I-491 | 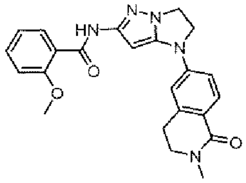 | 1.78 | 418 | C |

[Table 1-70]

| Compound No. | Structural Formula | LC/MS Retention Time (min) | MS[M+H]+ | LC Method |
|---|---|---|---|---|
| I-492 | 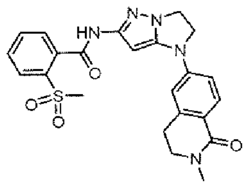 | 1.43 | 466 | C |
| I-493 | 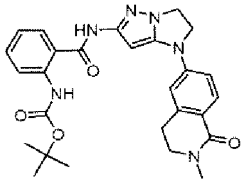 | 2.21 | 503 | C |
| I-494 | 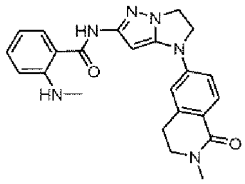 | 1.82 | 417 | C |
| I-495 | 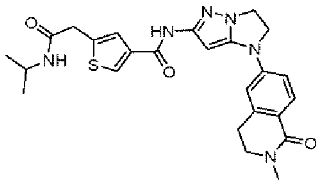 | 1.53 | 493 | C |
| I-496 | 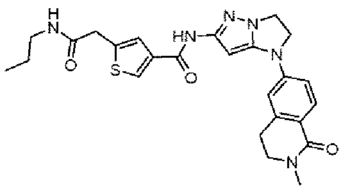 | 1.54 | 493 | C |
| I-497 | 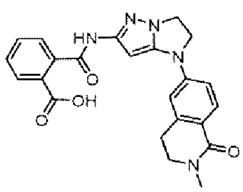 | 1.35 | 432 | C |

[Table 1-71]

| Compound No. | Structural Formula | LC/MS Retention Time (min) | MS[M+H]+ | LC Method |
|---|---|---|---|---|
| I-498 | 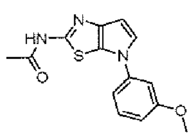 | 1.96 | 288 | C |

(continued)

| Compound No. | Structural Formula | LC/MS Retention Time (min) | MS[M+H]+ | LC Method |
|---|---|---|---|---|
| I-499 | 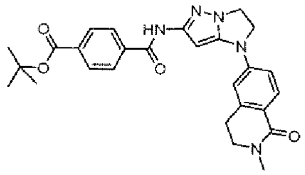 | 2.15 | 488 | C |
| I-500 | 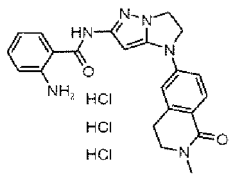 | 1.55 | 403 | C |
| I-501 | 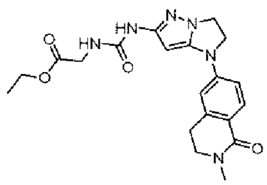 | 1.42 | 413 | C |
| I-502 | 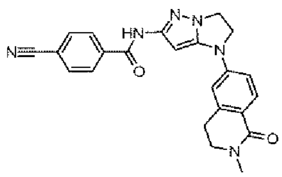 | 1.63 | 413 | C |
| I-503 | 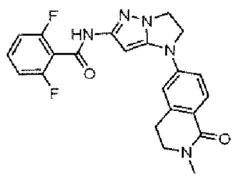 | 1.53 | 424 | C |
| I-504 | 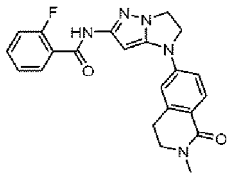 | 1.68 | 406 | C |

[Table 1-72]

| Compound No. | Structural Formula | LC/MS Retention Time (min) | MS[M+H]+ | LC Method |
|---|---|---|---|---|
| I-505 | 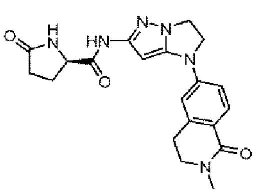 | 1.11 | 395 | C |

(continued)

| Compound No. | Structural Formula | LC/MS Retention Time (min) | MS[M+H]+ | LC Method |
|---|---|---|---|---|
| I-506 | 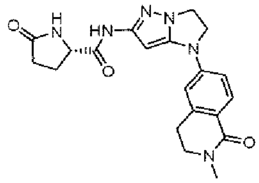 | 1.11 | 395 | C |
| I-507 | 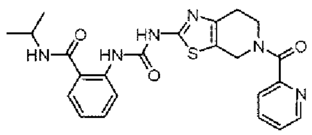 | 1.44 | 465 | D |
| I-508 | 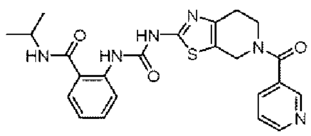 | 1.29 | 465 | D |
| I-509 | 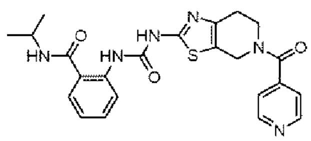 | 1.22 | 465 | D |
| I-510 | 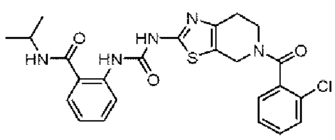 | 1.79 | 499 | D |
| I-511 | 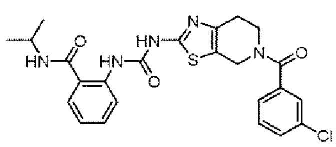 | 1.85 | 499 | D |

[Table 1-73]

| Compound No. | Structural Formula | LC/MS Retention Time (min) | MS[M+H]+ | LC Method |
|---|---|---|---|---|
| 1-512 | 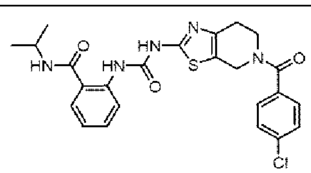 | 1.86 | 499 | D |
| 1-513 | 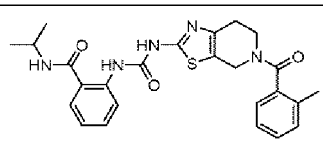 | 1.75 | 478 | D |
| 1-514 | 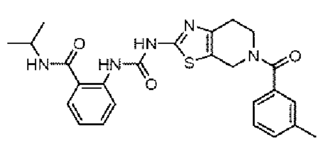 | 1.80 | 478 | D |

(continued)

| Compound No. | Structural Formula | LC/MS Retention Time (min) | MS[M+H]+ | LC Method |
|---|---|---|---|---|
| 1-515 | 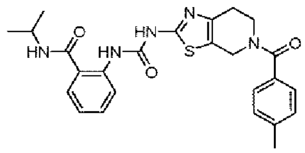 | 1.83 | 478 | D |
| I-516 | 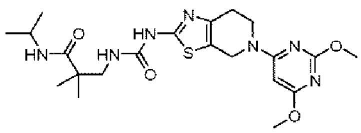 | 1.82 | 478 | C |
| 1-517 | 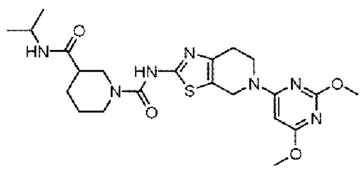 | 1.78 | 490 | C |
| 1-518 | 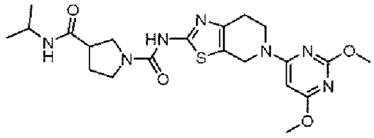 | 1.62 | 476 | C |
| 1-519 | 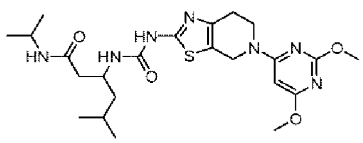 | 2.02 | 506 | C |

[Table 1-74]

| Compound No. | Structural Formula | LC/MS Retention Time (min) | MS[M+H]+ | LC Method |
|---|---|---|---|---|
| I-520 | 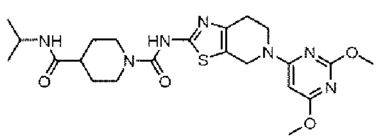 | 1.67 | 490 | C |
| I-521 | 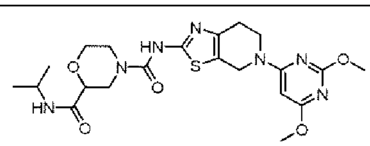 | 1.75 | 492 | C |
| I-522 | 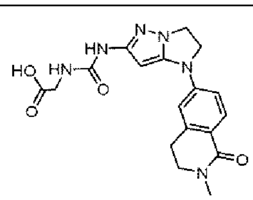 | 1.14 | 385 | C |

(continued)

| Compound No. | Structural Formula | LC/MS Retention Time (min) | MS[M+H]+ | LC Method |
|---|---|---|---|---|
| I-523 | 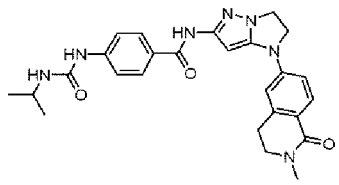 | 1.58 | 488 | C |
| I-524 | 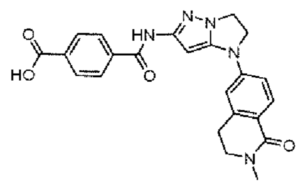 | 1.43 | 432 | C |
| I-525 | 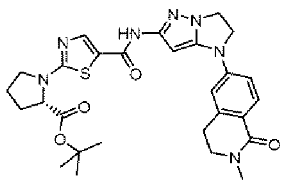 | 2.00 | 564 | C |
| I-526 | 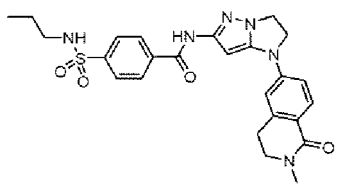 | 1.70 | 509 | C |

[Table 1-75]

| Compound No. | Structural Formula | LC/MS Retention Time (min) | MS[M+H]+ | LC Method |
|---|---|---|---|---|
| I-527 | 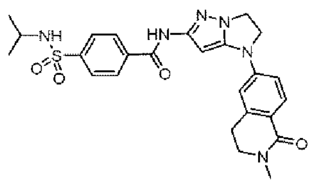 | 1.69 | 509 | C |
| I-528 | 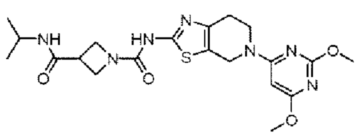 | 1.56 | 462 | C |
| I-529 | 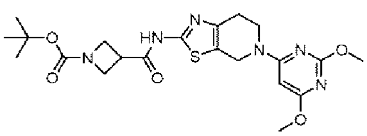 | 2.14 | 477 | C |

(continued)

| Compound No. | Structural Formula | LC/MS Retention Time (min) | MS[M+H]+ | LC Method |
|---|---|---|---|---|
| I-530 | 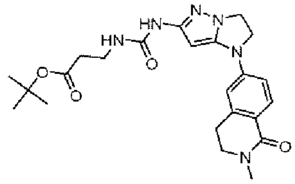 | 1.75 | 455 | C |
| I-531 | 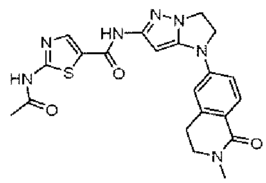 | 1.37 | 452 | C |
| I-532 | 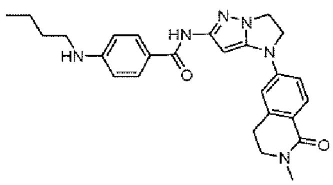 | 2.00 | 449 | C |
| I-533 | 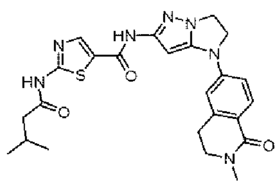 | 1.74 | 494 | C |

[Table 1-76]

| Compound No. | Structural Formula | LC/MS Retention Time (min) | MS[M+H]+ | LC Method |
|---|---|---|---|---|
| I-534 | 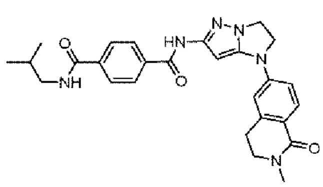 | 1.69 | 487 | C |
| I-535 | 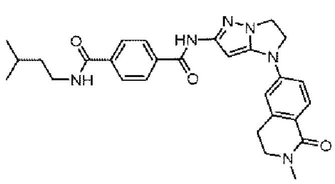 | 1.84 | 501 | C |
| I-536 | 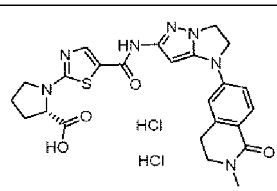 | 1.43 | 508 | C |

(continued)

| Compound No. | Structural Formula | LC/MS Retention Time (min) | MS[M+H]+ | LC Method |
|---|---|---|---|---|
| I-537 | 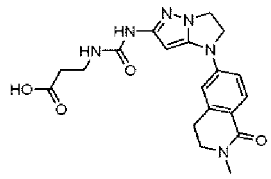 | 1.17 | 399 | C |
| I-538 | 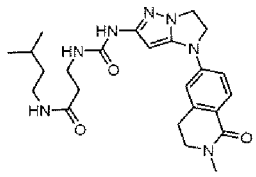 | 1.54 | 468 | C |
| I-539 | 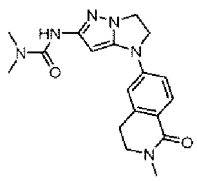 | 1.21 | 355 | C |
| I-540 | 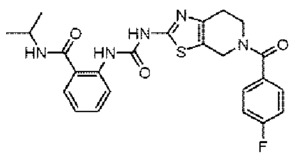 | 1.75 | 482 | D |

[Table 1-77]

| Compound No. | Structural Formula | LC/MS Retention Time (min) | MS[M+H]+ | LC Method |
|---|---|---|---|---|
| I-541 | 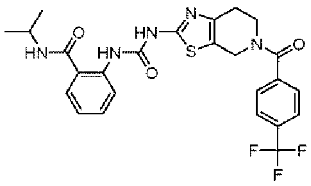 | 1.95 | 532 | D |
| I-542 | 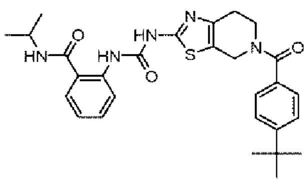 | 2.15 | 520 | D |
| I-543 | 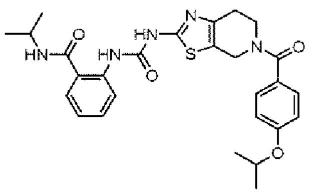 | 1.96 | 522 | D |

(continued)

| Compound No. | Structural Formula | LC/MS Retention Time (min) | MS[M+H]+ | LC Method |
|---|---|---|---|---|
| I-544 | 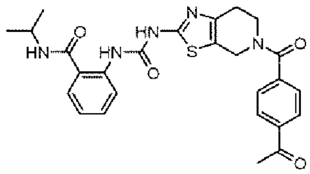 | 1.63 | 506 | D |
| I-545 | 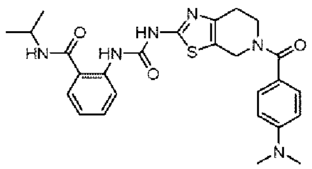 | 1.75 | 507 | D |
| I-546 | 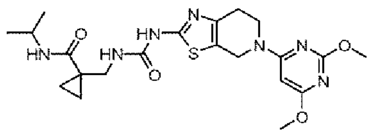 | 1.84 | 476 | C |

[Table 1-78]

| Compound No. | Structural Formula | LC/MS Retention Time (min) | MS[M+H]+ | LC Method |
|---|---|---|---|---|
| I-547 | 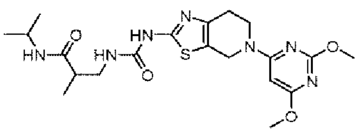 | 1.66 | 464 | C |
| I-548 | 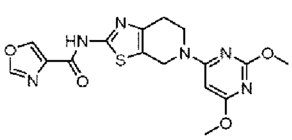 | 1.74 | 389 | C |
| I-543 | 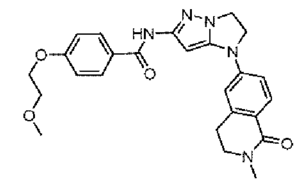 | 1.64 | 462 | C |
| I-550 | 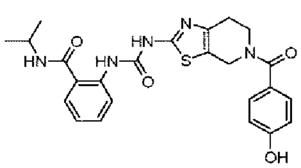 | 1.44 | 480 | D |
| I-551 | 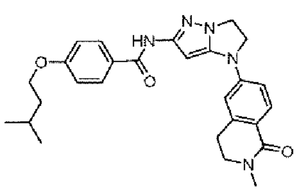 | 2.30 | 474 | C |

(continued)

| Compound No. | Structural Formula | LC/MS Retention Time (min) | MS[M+H]+ | LC Method |
|---|---|---|---|---|
| I-552 | 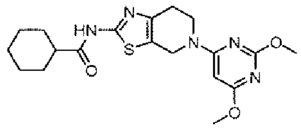 | 2.28 | 404 | C |
| I-553 | 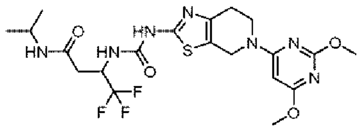 | 1.88 | 518 | C |
| I-554 | 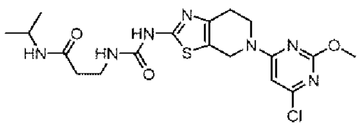 | 1.72 | 454,456 | C |

(Example 8: Biological activity: Measurement of PI3Kγ inhibitory activity)

For each compound synthesized in the above Examples, PI3Kγ inhibitory activity was measured.

(Method)

The PI3Kγ inhibitory activity of a compound was evaluated using PI3-kinase HTRF™ assay (Millipore) according to the following procedure:

To each well of a testing plate, 5 μL of a compound solution containing 10% DMSO (200 μmol/L or 40 μmol/L as the concentration of the compound), 5 μL of a substrate solution (40 μmol/L phosphatidylinositol (4,5)-bisphosphate, 20 mmol/L $MgCl_2$, 10mmol/L DTT), and 5 μL of a enzyme solution (80 μg/mL PI-3 kinase γ, 10 mmol/L $MgCl_2$, 5 mmol/L DTT) were added, and then allowed to stand for 10 minutes.

Then, 5 μL of a reaction solution (40 μmol/L ATP, 10 mmol/L $MgCl_2$, 5 mmol/L DTT) was added thereto. After reacting for 30 minutes at room temperature, 5μL of a solution containing EDTA and biotinylated phosphatidylinositol (3,4,5)-triphosphate were added to quench the reaction.

5 μL of a detection reagent containing a europium-labeled anti-GST antibody, the GST-tagged PH domain, and allophycocyanin-labeled streptavidin was added thereto. After 18 hours, HTRF (excitation wavelength: 330nm, measured wavelengths: 620nm and 665nm) was measured.

HTRF ratio was defined as the value obtained by dividing the amount of fluorescence obtained at the measured wavelength 665nm by the amount of fluorescence obtained at 620 nm. The HTRF ratio in the absence of a compound was defined as 100% activity and the HTRF ratio in the absence of PI-3 kinase γ was defined as 0% activity to calculate an inhibition ratio which was defined as the PI3Kγ inhibitory activity of a compound at 50 μmol/L or 10 μmol/L.

(Results)

Results are shown in the tables below.

[Table 2-1]

| Compound No. | Inhibition Ratio % (50μM) | Compound No. | Inhibition Ratio % (50μM) | Compound No. | Inhibition Ratio % (50μM) | Compound No. | Inhibition Ratio % (50μM) |
|---|---|---|---|---|---|---|---|
| I-2 | ≧95 | I-81 | 75 | I-108 | ≧95 | I-135 | ≧95 |
| I-4 | ≧95 | I-83 | ≧95 | I-110 | ≧95 | 1-137 | 87 |
| I-5 | 89 | I-84 | ≧95 | I-111 | ≧95 | I-138 | ≧95 |
| I-6 | 90 | I-85 | ≧95 | I-114 | 83 | I-139 | ≧95 |

(continued)

| Compound No. | Inhibition Ratio % (50μM) | Compound No. | Inhibition Ratio % (50μM) | Compound No. | Inhibition Ratio % (50μM) | Compound No. | Inhibition Ratio % (50μM) |
|---|---|---|---|---|---|---|---|
| I-7 | ≧95 | I-86 | 90 | I-115 | ≧95 | I-140 | ≧95 |
| I-8 | ≧95 | I-88 | 85 | I-116 | ≧95 | I-141 | ≧95 |
| I-35 | 77 | I-89 | ≧95 | I-117 | 76 | I-142 | ≧95 |
| I-40 | 73 | I-91 | ≧95 | I-118 | ≧95 | I-143 | ≧95 |
| I-50 | ≧95 | I-93 | 92 | I-119 | ≧95 | I-144 | ≧95 |
| I-51 | 87 | I-94 | ≧95 | I-122 | 76 | I-145 | ≧95 |
| I-57 | ≧95 | I-95 | 93 | I-123 | ≧95 | I-146 | ≧95 |
| I-59 | 94 | I-97 | ≧95 | I-126 | ≧95 | I-147 | ≧95 |
| I-61 | ≧95 | I-100 | ≧95 | I-127 | 79 | I-148 | ≧95 |
| I-65 | ≧95 | I-101 | ≧95 | I-128 | ≧95 | I-149 | ≧95 |
| I-66 | 92 | I-102 | ≧95 | I-129 | 81 | I-150 | ≧95 |
| I-67 | ≧95 | I-105 | ≧95 | I-130 | ≧95 | I-151 | 80 |
| I-79 | ≧95 | I-106 | ≧95 | I-132 | ≧95 | I-152 | ≧95 |
| I-80 | ≧95 | I-107 | ≧95 | I-133 | ≧95 | I-153 | 74 |

[Table 2-2]

| Compound No. | inhibition Ratio % (50 μM) | Inhibition Ratio % (10 μM) | Compound No. | inhibition Ratio % (50 μM) | Inhibition Ratio % (10 μM) |
|---|---|---|---|---|---|
| I-160 | ≧95 | | I-191 | ≧95 | |
| I-161 | ≧95 | | I-192 | ≧95 | |
| I-162 | ≧95 | | I-193 | ≧95 | |
| I-163 | ≧95 | | I-194 | 95 | |
| I-164 | ≧95 | | I-195 | 93 | |
| I-165 | ≧95 | | I-196 | 79 | |
| I-166 | ≧95 | | I-198 | 94 | |
| I-167 | 92 | | I-199 | 89 | |
| I-168 | ≧95 | | I-200 | ≧95 | |
| I-169 | ≧95 | | I-201 | ≧95 | |
| I-170 | ≧95 | | I-202 | ≧95 | |
| I-173 | ≧95 | | I-203 | 95 | |
| I-174 | ≧95 | | I-204 | 88 | |
| I-175 | 94 | | I-207 | ≧95 | |
| I-176 | ≧95 | | I-208 | 93 | |
| I-177 | ≧95 | | I-209 | ≧95 | |
| I-178 | ≧95 | | I-210 | ≧95 | |
| I-179 | ≧95 | | I-211 | 89 | |

(continued)

| Compound No. | inhibition Ratio % (50 μM) | Inhibition Ratio % (10 μM) | Compound No. | inhibition Ratio % (50 μM) | Inhibition Ratio % (10 μM) |
|---|---|---|---|---|---|
| I-180 | ≧95 | | I-212 | 80 | |
| I-181 | ≧95 | | I-214 | ≧95 | |
| I-182 | 79 | | I-215 | ≧95 | |
| I-183 | ≧95 | | I-216 | ≧95 | |
| I-184 | ≧95 | | I-217 | ≧95 | |
| I-185 | ≧95 | | I-218 | ≧95 | |
| I-186 | 89 | | I-219 | ≧95 | |
| I-187 | 94 | | I-220 | ≧95 | |
| I-188 | 91 | | I-221 | ≧95 | |
| I-189 | ≧95 | | I-223 | 91 | |
| I-190 | ≧95 | | I-225 | 93 | |

[Table 2-3]

| Compound No. | Inhibition Ratio % (50 μM) | Inhibition Ratio % (10 μM) | Compound No. | Inhibition Ratio % (50 μM) | Inhibition Ratio % (10 μM) |
|---|---|---|---|---|---|
| I-226 | ≧95 | | I-259 | 89 | |
| I-227 | ≧95 | | I-260 | 93 | |
| I-228 | 85 | | I-261 | | 90 |
| I-230 | 81 | | I-262 | | ≧95 |
| I-231 | ≧95 | | I-263 | | ≧95 |
| I-232 | 76 | | I-264 | | 89 |
| I-233 | 84 | | I-265 | | ≧95 |
| I-234 | ≧95 | | I-266 | | ≧95 |
| I-235 | 89 | | I-287 | ≧95 | |
| I-236 | 88 | | I-268 | | ≧95 |
| I-237 | 84 | | I-269 | | ≧95 |
| I-239 | 92 | | I-270 | | ≧95 |
| I-240 | 91 | | I-271 | | ≧95 |
| I-241 | 84 | | I-272 | | ≧95 |
| I-242 | ≧95 | | I-273 | | ≧95 |
| I-243 | 81 | | I-274 | | ≧95 |
| I-244 | 87 | | I-275 | | ≧95 |
| I-245 | 76 | | I-276 | | ≧95 |
| I-246 | 90 | | I-277 | | ≧95 |
| I-247 | 95 | | I-279 | | ≧95 |
| I-249 | ≧95 | | I-280 | | ≧95 |

(continued)

| Compound No. | Inhibition Ratio % (50 μM) | Inhibition Ratio % (10 μM) | Compound No. | Inhibition Ratio % (50 μM) | Inhibition Ratio % (10 μM) |
|---|---|---|---|---|---|
| I-260 | ≧95 | | I-281 | | ≧95 |
| I-251 | | 88 | I-282 | | 86 |
| I-252 | | 88 | I-283 | | ≧95 |
| I-253 | | 82 | I-284 | | ≧95 |
| I-254 | 81 | | I-285 | | ≧95 |
| I-256 | | 78 | I-286 | 77 | |
| I-257 | 83 | | I-287 | | ≧95 |
| I-258 | 92 | | I-288 | | ≧95 |

[Table 2-4]

| Compound No. | Inhibition Ratio % (50μM) | Inhibition Ratio % (10μM) | Compound No. | inhibition Ratio % (50μM) | Inhibition Ratio % (10μM) |
|---|---|---|---|---|---|
| 1-289 | | 90 | I-324 | | 93 |
| 1-290 | 88 | | 1-325 | 81 | |
| 1-291 | | ≧95 | 1-327 | | 84 |
| 1-292 | | ≧ 95 | 1-328 | | 87 |
| 1-293 | | 86 | 1-329 | | 89 |
| 1-294 | | 84 | I-330 | | 83 |
| I-295 | | 93 | 1-331 | | 84 |
| I-296 | | 88 | 1-332 | | ≧95 |
| I-297 | | 91 | 1-333 | | 88 |
| 1-301 | ≧95 | | 1-334 | | 88 |
| I-302 | | 85 | I-335 | | ≧95 |
| 1-303 | | 94 | 1-336 | | 89 |
| I-304 | | 89 | 1-337 | | 82 |
| 1-305 | 84 | | I-340 | | ≧95 |
| 1-306 | | ≧ 95 | 1-341 | | ≧95 |
| 1-307 | | 86 | 1-342 | | 92 |
| I-308 | | ≧95 | 1-344 | | 80 |
| 1-309 | | ≧95 | 1-345 | | 80 |
| 1-310 | | ≧95 | 1-347 | | 91 |
| 1-311 | | ≧95 | 1-348 | | ≧95 |
| 1-312 | | ≧95 | 1-349 | | ≧95 |
| 1-313 | | 89 | 1-350 | ≧95 | |
| 1-314 | | 88 | 1-351 | ≧95 | |
| I-315 | | ≧95 | 1-352 | 81 | |

(continued)

| Compound No. | Inhibition Ratio % (50μM) | Inhibition Ratio % (10μM) | Compound No. | inhibition Ratio % (50μM) | Inhibition Ratio % (10μM) |
|---|---|---|---|---|---|
| 1-316 | | 75 | 1-355 | 92 | |
| I-318 | | ≧95 | I-356 | | ≧95 |
| 1-319 | | ≧95 | I-358 | | ≧95 |
| I-320 | | ≧95 | 1-360 | | ≧95 |
| 1-321 | | 95 | 1-361 | | 80 |

[Table 2-5]

| Compound No | Inhibition Ratio % (50μM) | Inhibition Ratio % (10μM) | Compound No. | Inhibition Ratio % (50μM) | Inhibition Ratio % (10μM) |
|---|---|---|---|---|---|
| I-362 | | ≧95 | I-397 | | ≧95 |
| I-364 | | ≧95 | 1-398 | | ≧95 |
| I-365 | 81 | | I-399 | | ≧95 |
| I-367 | | 86 | I-401 | | 93 |
| I-368 | | 95 | I-402 | | 91 |
| I-369 | ≧85 | | I-403 | | ≧95 |
| I-370 | | 84 | I-404 | | 78 |
| I-371 | | ≧95 | I-406 | | ≧98 |
| I-372 | 84 | | I-407 | | ≧95 |
| I-373 | 82 | | I-409 | | ≧95 |
| I-375 | | ≧95 | I-410 | | ≧95 |
| I-376 | | ≧95 | I-411 | | ≧95 |
| I-377 | | ≧95 | I-413 | | 87 |
| I-378 | | ≧95 | I-415 | | ≧95 |
| I-380 | | ≧95 | I-418 | | 93 |
| I-381 | | ≧95 | I-419 | | 83 |
| I-385 | | 91 | I-427 | | 88 |
| I-386 | | ≧95 | I-428 | | 90 |
| I-391 | | ≧95 | I-430 | | 84 |
| I-392 | | ≧95 | I-432 | | ≧95 |
| I-396 | | 76 | | | |

[Table 2-6]

| Compound No. | Inhibition Ratio % (50μM) | Inhibition Ratio % (10μM) | Compound No. | Inhibition Ratio % (50μM) | Inhibition Ratio % (10μM) |
|---|---|---|---|---|---|
| I-433 | | ≧95 | I-473 | | ≧95 |
| I-436 | | ≧95 | I-474 | | ≧95 |

(continued)

| Compound No. | Inhibition Ratio % (50μM) | Inhibition Ratio % (10μM) | Compound No. | Inhibition Ratio % (50μM) | Inhibition Ratio % (10μM) |
|---|---|---|---|---|---|
| I-437 | | ≧95 | I-476 | | 94 |
| I-438 | | 86 | I-477 | | 82 |
| I-439 | | ≧95 | I-480 | | 86 |
| I-440 | | 92 | I-487 | | 92 |
| I-441 | | 88 | I-482 | | ≧95 |
| I-444 | | 84 | I-483 | | ≧95 |
| I-445 | | 89 | I-484 | | ≧95 |
| I-449 | | ≧95 | I-485 | | 79 |
| I-450 | | ≧95 | I-488 | | 88 |
| I-451 | | 90 | I-489 | | ≧95 |
| I-453 | | 76 | I-493 | | 91 |
| I-456 | | 87 | I-494 | | 78 |
| I-457 | | 88 | I-495 | | ≧95 |
| I-458 | | 92 | I-496 | | ≧95 |
| I-459 | | 92 | I-500 | | 81 |
| I-461 | | 95 | I-507 | | 81 |
| I-462 | | ≧95 | I-506 | | 84 |
| I-463 | | ≧95 | I-51 | | 75 |
| I-464 | | ≧95 | I-514 | | 76 |
| I-455 | | ≧95 | I-515 | | 85 |
| I-466 | | 83 | I-522 | | 94 |
| I-467 | | ≧95 | I-524 | | 92 |
| I-468 | | ≧95 | I-525 | | 94 |
| I-469 | | 85 | I-520 | | 90 |
| I-470 | | 79 | I-527 | | 79 |
| I-471 | | 91 | I-528 | | 76 |
| I-472 | | ≧95 | I-529 | | 82 |

[Table 2-7]

| Compound No. | Inhibition Ratio % (50μM) | Inhibition Ratio % (10μM) | Compound No. | Inhibition Ratio % (50μM) | Inhibition Ratio % (10μM) |
|---|---|---|---|---|---|
| I-530 | | 92 | I-539 | | ≧95 |
| I-531 | | 75 | I-546 | | 86 |
| I-532 | | ≧95 | I-547 | | ≧95 |
| I-533 | | 95 | I-549 | | ≧95 |
| I-534 | | 91 | I-550 | | 81 |

(continued)

| Compound No. | Inhibition Ratio % (50μM) | Inhibition Ratio % (10μM) | Compound No. | Inhibition Ratio % (50μM) | Inhibition Ratio % (10μM) |
|---|---|---|---|---|---|
| I-536 | | ≧95 | I-551 | | 84 |
| I-537 | | 87 | I-552 | | 88 |
| I-538 | | 94 | I-554 | | 88 |

(Example 9: Biological activity: Measurement of AKT phosphorylation inhibitory activity)

Cells were used to measure whether or not the inhibitory activity was exhibited.

(Method)

(1) The AKT phosphorylation inhibitory activity of a compound was evaluated according to the following procedure.
(2) Human monocyte-like cell line THP-1 was washed with RPMI-1640 media, incubated in the presence of 5% $CO_2$ at 37˚C for 3 hours, washed with Hank's balanced salt solution (HBSS), adjusted to a cell concentration of 6.6 x $10^6$ /mL, and then used in an experiment.
(3) 30 μL of the cell suspension and 60 μL of each compound solution containing 0.2% DMSO/HBSS were mixed and then preincubated at 37 ˚C for 5 minutes. 30 μL of HBSS containing 4 μg/mL of MCP-1 was added thereto and then incubated for 30 seconds at 37˚C.
(4) 30 μL of a cell lysate (20 mmol/L Tris-HCl (pH 7.5), 150 mmol/L NaCl, 1 mmol/L $Na_2$EDTA, 1 mmol/L EGTA, 1% Triton, 2 . 5 mmol/L sodium pyrophosphate, 1mmol/L β-glycerophosphate, 1 mmol/L $Na_3VO_4$, 1 μg/ml leupeptin, 50nmol/L APMSF) was added thereto to lyse cells.
(5) The amount of AKT phosphorylation in the cell lysate was measured by ELISA method.
(6) Anti-phospho-Akt (Ser473) antibodies (clone 193H12, derived from a rabbit) were immobilized onto the solid phase of a micro well plate. 100 μL of the prepared cell lysate was added to the micro well plate, incubated for 2 hours at 37˚C, and then washed four times with Phosphate Buffered Saline containing 0.05% Tween-20.
(7) An anti-AKT1 antibody (clone 2H10, derived from a mouse) was added thereto, incubated for 1 hour at 37˚C, washed similarly, and then reacted with an HRP-labeled anti-mouse IgG antibody.
(8) After incubating at 37˚C for 30 minutes and then washing similarly, 100 μL of TMB (3,3',5,5''-tetramethylbenzidine) was added thereto, followed by reacting at room temperature for 30 minutes.
(9) 100 μL of 1 mol/L sulfuric acid was added to quench the color reaction and then the absorbance at 450 nm was measured.
(10) A series of diluted cell lysates of a positive control (a sample in the absence of a compound) were used to prepare a calibration curve, the amount of AKT phosphorylation in a sample in the absence of MCP-1 was defined as 0% activity to calculate an inhibition ratio. The measurement was performed for a 1 μmol/L solution of each compound.

(Results)

Compound No. I-177: >99.5%
Compound No. I-190: >99.5%
Compound No. I-198: 97.3%
Compound No. I-202: >99.5%
Compound No. I-214: >99.5%
Compound No. I-265: >99.5%
Compound No. I-266: >99.5%
Compound No. I-284: >99.5%
Compound No. I-287: >99.5%
Compound No. I-308: 98.4%
Compound No. I-309: 92%

Compound No. I-310: >99.5%
Compound No. I-318: >99.5%
Compound No. I-331: 93%
Compound No. I-332: 96%
Compound No. I-341: 97.8%
Compound No. I-347: 80.6%
Compound No. I-348: >99.5%
Compound No. I-358: 95.9%
Compound No. I-378: >99.5%.

(Example 10: Measurement of PI3Kγ inhibitory activity (Ki value))

The PI3Kγ inhibitory activity (Ki value) of a compound was evaluated according to the following procedure:

5 μL of a compound solution containing 10% DMSO and 200 μmol/L of a compound was changed to 5 μL of a compound solution containing 10% DMSO and either 200, 64, 20, 6.4, 2, 0.64, or 0.20 μmol/L of the compound (optionally, this is diluted to a lower concentration). Using a method similar to the method for measuring PI3Kγ inhibitory activity, inhibition ratios were measured in the presence of the compound at 50, 16, 5, 1.6, 0.5, 0.16, and 0.05 μmol/L (optionally, a lower concentration). Then an $IC_{50}$ value was calculated by a logistic approximation method or the linear regression method using two concentrations that across 50% inhibition. Separately, the ATP concentration in the reaction solution (40 μmol/L ATP, 10 mmol/L $MgCl_2$, 5 mmol/L DTT) at the time of the start of a reaction in the absence of the compound was changed to 80, 40, 20, 10, 5, 2.5, 1.25, or 0.625 μmol /L. Then, HTRF ratios were measured by a similar method. The value obtained by subtracting the HTRF ratio at each ATP concentration from the HTRF ratio in the absence of PI-3 kinase γ was defined as the value obtained by multiplying reaction rate (v) at each ATP concentration with a constant. The Michaelis-Menten constant Km was then calculated by the Lineweaver-Burk plot method. The Ki value of a compound was calculated by the following formula.

$$Ki = IC_{50}\ \text{value}/(1 + 10\ \mu M\ (\text{test ATP concentration})/Km\ (\mu M))$$

(Results)

Compound No. I-202: 0.049 μM
Compound No. I-208: 0.022 μM
Compound No. I-262: 0.047 μM
Compound No. I-265: 0.042 μM
Compound No. I-266: 0.036 μM
Compound No. I-277: 0.036 μM
Compound No. I-279: 0.029 μM
Compound No. I-284: 0.040 μM
Compound No. I-292: 0.043 μM
Compound No. I-303: 0.045 μM
Compound No. I-310: 0.043 μM
Compound No. I-318: 0.045 μM
Compound No. I-375: 0.023 μM
Compound No. I-380: 0.032 μM
Compound No. I-398: 0.049 μM
Compound No. I-415: 0.034 μM.

(Example 11: Measurement of PI3Kα inhibitory activity)

The PI3Kα inhibitory activity of a compound was evaluated according to the following procedure:

According to Example 8 above, after 80 μg/mL PI-3 kinase γ of the enzyme solution (80 μg/mL PI-3 kinase γ, 10 mmol/L $MgCl_2$, 5 mmol/L DTT) was changed to 0.8 μg/mL PI-3 kinase α, an inhibition ratio was calculated by a method similar to the method for measuring PI3Kγ inhibitory activity, and then defined as the PI3Kα inhibitory activity

at 50 μmol/L.

(Example 12: Measurement of PI3Kα inhibitory activity (Ki value))

The α inhibitory activity (Ki value) of a compound was evaluated according to the following procedure:
According to Example 10 above, after 80μg/mL PI-3 kinase γ of the enzyme solution (80 μg/mL PI-3 kinase γ, 10 mmol/L $MgCl_2$, 5 mmol/L DTT) was changed to 0.8 μg/mL PI-3 kinase α, a Km value measured with PI3Kα was used to calculate a Ki value for PI3Kα by a method similar to the PI3Kγ inhibitory activity (Ki value).

(Example 13: Measurement of PI3Kβ inhibitory activity)

The PI3Kβ inhibitory activity of a compound was evaluated according to the following procedure.
According to Example 8 above, after 80μg/mL PI-3 kinase γ of the enzyme solution (80 μg/mL PI-3 kinase γ, 10 mmol/L $MgCl_2$, 5 mmol/L DTT) was changed to 60 μg/mL PI-3 kinase β, a method similar to the method for measuring PI3Kγ inhibitory activity was used to calculate an inhibition ratio, which was defined as the P13Kβ inhibitory activity at 50 μmol/L.

(Example 14: Measurement of P13Kβ inhibitory activity (Ki value))

The β inhibitory activity (Ki value) of a compound was evaluated according to the following procedure:
According to Example 10 above, after 80 μg/mL PI-3 kinase γ of the enzyme solution (80 μg/mL PI-3 kinase γ, 10 mmol/L $MgCl_2$, 5 mmol/L DTT) was changed to 60 μg/mL PI-3 kinase β, a Km value measured with PI3Kβ was used to calculate a Ki value for PI3Kβ by a method similar to the PI3Kγ inhibitory activity (Ki value).

(Example 15: Method for calculating the selectivity of PI3Kγ and PI3Kα)

The PI3Kγ/α selectivity of a compound was expressed by the value obtained by dividing the Ki value for PI3Kα by the Ki value for PI3Kγ.

(Example 16: Method for calculating the selectivity of PI3Kγ and PI3Kβ)

The PI3Kγ/β selectivity of a compound was expressed by the value obtained by dividing the Ki value for PI3Kβ by the Ki value for PI3Kγ.
According to Examples 17 to 22 shown below, compounds of the present invention were evaluated.

(Example 17: CYP3A4 fluorescence MBI test)

The CYP3A4 fluorescence MBI test is a test to examine the enhancement of CYP3A4 inhibition of a compound by a metabolic reaction. The test was performed using as an index, a reaction to produce a metabolite 7-hydroxytrifluoromethylcoumarin (HFC) which emits fluorescence, in which CYP3A4 expressed in *E. coli* was used as an enzyme and 7-benzyloxytrifluoromethylcoumarin (BFC) is debenzylated by CYP3A4 enzyme.
The reaction conditions are as follows: substrate: 5.6 μmol/L 7-BFC; pre-reaction time; 0 or 30 minutes; reaction time: 15 minutes; reaction temperature: 25˚C (room temperature); content of CYP3A4 (an enzyme expressed in *E. coli*) : 62.5 pmol/mL at the time of a pre-reaction and 6.25 pmol/mL (when diluted 10 times) at the time of a reaction; the concentrations of a test drug: 0.625, 1.25, 2.5, 5, 10, and 20 μmol/L (6 points).
In a 96-well plate, as a pre-reaction solution, an enzyme solution and a test drug solution were added into K-Pi buffer solution (pH 7.4) in the aforementioned ratio. Then, a portion thereof was transferred to another 96-well plate so as to be diluted ten times with a substrate and a K-Pi buffer solution. A coenzyme NADPH was then added to start a reaction that is an index (without a pre-reaction) . After reacting for a predetermined time, 4/1 of acetonitrile/0.5 mol/L Tris (trishydroxyaminomethane) was added to quench the reaction. To the remaining pre-reaction solution, NADPH was also added to start a pre-reaction (with a pre-reaction) . After pre-reacting for a predetermined time, a portion thereof was transferred to another plate so as to be diluted ten times with a substrate and K-Pi buffer solution, and thereby the reaction that is an index started. After reacting for a predetermined time, 4/1 of acetonitrile/0.5 mol/L Tris (trishydroxyaminomethane) was added to quench the reaction. For each plate in which the index reaction was performed, the fluorescence value of a metabolite 7-HFC was measured with a fluorescent plate reader (Ex = 420nm, Em = 535nm).
The case wherein only DMSO (a solvent which dissolved the drug) was added to a reaction system was defined as a control (100%). When a test drug solution was added, the remaining activity (%) at each concentration was calculated, and then $IC_{50}$ was calculated with a concentration and an inhibition ratio by an inverse estimation using a logistic model.

The case that the difference of $IC_{50}$ values was 5 μM or higher was determined as (+). The case that it was 3 μM or lower was determined as (-).

(Results)

Compound No. I-187: (-)
Compound No. I-190: (-)
Compound No. I-208: (-)
Compound No. I-217: (-)
Compound No. I-242: (-)
Compound No. I-262: (-)
Compound No. I-276: (-)
Compound No. I-279: (-)
Compound No. I-330: (-)
Compound No. I-356: (-)
Compound No. I-397: (-)
Compound No. I-409: (-) .

(Example 18: CYP inhibition test)

A commercially available pooled human liver microsome was used in evaluating the degree of produced-metabolite inhibition exhibited by a test compound. O-de-ethylation of 7-ethoxyresorufin (CYP1A2), methyl-hydroxylation of tolbutamide (CYP2C9), 4'-hydroxylation of mephenytoin (CYP2C19), O-demethylation of dextromethorphan (CYP2D6), and hydroxylation of terfenadine (CYP3A4), which are typical substrate for metabolic reactions of human main CYP5 molecular species (CYP1A2, 2C9, 2C19, 2D6, and 3A4), were adopted as indexes.
The reaction conditions are as follows: substrate: 0.5 μmol/Lof ethoxyresorufin (CYP1A2), 100 Hmol/L of tolbutamide (CYP2C9), 50 μmol/L of S-mephenytoin (CYP2C19), 5 μmol/L of dextromethorphan (CYP2D6), and 1 μmol/L of terfenadine (CYP3A4); reaction time; 15 minutes; reaction temperature: 37 ˚C; enzyme: pooled human liver microsome 0.2 mg protein/mL; test drug concentration: 1, 5, 10, and 20 μmol/L (4 points) .
In a 96-well plate, as a reaction solution, five kinds of substrates, a human liver microsome, and a test drug were added to 50 mmol/L Hepes buffer solution in the aforementioned ratio. A coenzyme NADPH was added to start a metabolic reaction, which is an index. After reacting at 37˚C for 15 minutes, a solution of methanol/acetonitrile (1/1 (v/v)) was added to quench the reaction. After centrifugation at 3000 rpm for 15 minutes, resorufin (CYP1A2 metabolite) in the supernatant was quantitated with a fluorescence multilabel counter, and hydroxylated tolbutamide (CYP2C9 metabolite), 4'-hydroxylated mephenytoin (CYP2C19 metabolite), dextromethorphan (CYP2D6 metabolite), terfenadine in alcohol form (CYP3A4 metabolite) were quantitated with LC/MS/MS.
The case wherein only DMSO (a solvent which dissolved a drug) was added to a reaction system was defined as a control (100%) . When a test drug solution was added, the remaining activity (%) at each concentration was calculated, and then $IC_{50}$ was calculated with a concentration and an inhibition ratio by an inverse estimation using a logistic model.

(Results)

Compound No. I- 89: 5 kinds >20μM
Compound No. I-118: 5 kinds >20μM
Compound No. I-142: 5 kinds >20μM
Compound No. I-185: 5 kinds >20μM
Compound No. I-187: 5 kinds >20μM
Compound No. I-200: 5 kinds >20μM
Compound No. I-208: 5 kinds >20μM
Compound No. I-217: 5 kinds >20μM
Compound No. I-234: 5 kinds >20μM
Compound No. I-276: 5 kinds >20μM
Compound No. I-279: 5 kinds >20μM

Compound No. I-330: 5 kinds >20μM
Compound No. I-340: 5 kinds >20μM
Compound No. I-347: 5 kinds >20μM
Compound No. I-356: 5 kinds >20μM
Compound No. I-358: 5 kinds >20μM
Compound No. I-397: 5 kinds >20μM
Compound No. I-402: 5 kinds >20μM
Compound No. I-407: 5 kinds >20μM
Compound No. I-409: 5 kinds >20μM
Compound No. I-432: 5 kinds >20μM

(Example 19: FAT test)

20 μL of *Salmonella enterica* subsp. *typhimurium* (*SalmonellatyphimuriumTA98* line,TA100 line) cryopreserved was inoculated to 10 mL of liquid nutrient medium (2.5% Oxoid nutrient broth No. 2) and then precultured at 37°C for 10 hours with shaking. Regarding TA98 line, after 9mL of a bacterial suspension was centrifuged (2000 x g, 10 minutes) to remove the culture solution, the bacteria was suspended in 9 mL of Micro F buffer solution ($K_2HPO_4$: 3.5 g/L, $KH_2PO_4$: 1 g/L, $(NH_4)_2SO_4$: 1g/L, tri-sodium citric acid dihydrate: 0.25 g/L, $MgSO_4 \cdot 7H_2O$ : 0.1 g/L), and then added to 110 mL of Exposure media (Micro F buffer solution containing biotin: 8 μg/mL, histidine: 0.2 μg/mL, glucose: 8 mg/mL). Regarding TA 100 line, 120 mL of Exposure media was added to 3.16 mL of the bacterial suspension to prepare a test bacterial suspension. 12 μL of a solution of a test substance in DMSO (diluted eight times in a common ratio of 2 from the maximum dose of 50mg/mL); 12 μL of DMSO as a negative control; 50 μg/mL 4-nitroquinoline-1-oxide solution in DMSO for TA98 line or 0.25 μg/mL 2-(2-furyl)-3-(5-nitro-2-furyl)acrylamidesolution in DMSO as a positive control for TA 100 line in the case of a non-metabolism-activation condition; 12 μL of 40 μg /mL 2-aminoanthracene solution in DMSO for TA98 line or 20 μg/mL of 2-aminoanthracene solution in DMSO for TA100 line in the case of a metabolism-activation condition; were each mixed with 588 μL of the test bacterial suspension (in the case of a metabolism activation condition, a mixed solution of 498 μL of the test bacterial suspension and 90 μL S9 mix) and then cultured at 37°C at 90 minutes with shaking. 460 μL of the bacterial suspension to which a test substance was exposed was mixed with 2300 μL of Indicator media (a Micro F buffer solution containing 8 μg/mL biotin, 0.2 μg/mL histidine, 8 mg/mL glucose, and 37.5 μg/mL bromocresol purple). 50 μL thereof was dispensed to microplate 48 wells/dose and then statically cultured at 37°C for 3 days. In a well containing bacteria which obtained proliferation potency by mutation of the amino acid (histidine) synthetase gene, the change of pH caused the color change from purple to yellow. Thus, in 48 wells per dose, the number of the bacteria-proliferation wells in which the color changed to yellow was counted, compared with a group of negative controls, and then evaluated. When the mutagenicity is negative, the compound is shown as (-). When positive, the compound is shown as (+) .

(Results)

Compound No. I-89: (-)
Compound No. I-142: (-)
Compound No. I-181: (-)
Compound No. I-203: (-)
Compound No. I-266: (-)
Compound No. I-327: (-)
Compound No. I-358: (-)
Compound No. I-403: (-)

(Example 20: Solubility test)

The solubility of a compound was determined under a condition in which 1% DMSO was added. 10 mmol/L compound solution was prepared using DMSO, and then 6 μL of the compound solution was added to 594 μL of artificial intestinal juice in pH6.8 (to 250 mL of 0 .2 mol/Lpotassiumdihydrogenphosphate reagent solution was added 118 mL of 0.2 mol/L NaOH reagent solution and water to provide a final volume of 1000 mL). After standing at 25°C for 16 hours, the mixed solution was filtered with suction. The filtrate was diluted twice with methanol/water (1/1), and then a concentration in the filtration was measured with HPLC or LC/MS/MS by the absolute calibration method.

(Results)

Compound No. I- 61: >50 μM
Compound No. I-118: >50 μM
Compound No. I-142: >50 μM
Compound No. I-177: >50 μM
Compound No. I-185: >50 μM
Compound No. I-187: >50 μM
Compound No. I-198: >50 μM
Compound No. I-200: >50 μM
Compound No. I-208: >50 μM
Compound No. I-217: >50 μM
Compound No. I-230: >50 μM
Compound No. I-262: >50 μM
Compound No. I-276: >50 μM
Compound No. I-287: >50 μM
Compound No. I-309: >50 μM
Compound No. I-318: >50 μM
Compound No. I-319: >50 μM
Compound No. I-330: >50 μM
Compound No. I-340: >50 μM
Compound No. I-341: >50 μM
Compound No. I-347: >50 μM
Compound No. I-348: >50 μM
Compound No. I-356: >50 μM
Compound No. I-358: >50 μM
Compound No. I-378: >50 μM
Compound No. I-397: >50 μM

(Example 21: Metabolic stability test)

After a subject compound was reacted for a certain time using a pooled human liver microsome commercially available, a reacted sample and an unreacted sample are compared to calculate a survival ratio, and then the degree of metabolism in the liver was evaluated.

0.2 mL of a buffer solution (50 mmol/L of Tris-HCl in pH 7.4, 150 mmol/L of potassium chloride, and 10 mmol/L of magnesium chloride) containing a human liver microsome of 0.5 mg protein/mL was reacted in the presence of 1 mmol/L NADPH at 37 ˚C for 0 or 30 minutes (oxidative reaction) . After reacting, 50 μL of the reaction solution was added to 100 μL of a solution of methanol/acetonitrile (1/1 (v/v) , mixed, and then centrifuged at 3000 rpm for 15 minutes. The test compound in the supernatant was quantitated with LC/MS/MS. The amount of the compound at a reaction time of 0 minute was defined as 100% and, based on that, the percentage of the test compound remained after reacting for 30 minutes was calculated.

(Results)

Compound No. I- 89: 93.6%
Compound No. I-118: 98.3%
Compound No. I-142: 97.5%
Compound No. I-185: >99.9%
Compound No. I-187: 98.9%
Compound No. I-190: 90.5%
Compound No. I-200: 98.6%
Compound No. I-208: 87.2%
Compound No. I-214: 98.9%
Compound No. I-217: >99.9%

Compound No. I-230: 87.9%
Compound No. I-234: 98.3%
Compound No. I-242: >99.9%
Compound No. I-262: 92.8%
Compound No. I-276: 90.1%
Compound No. I-279: 99.7%
Compound No. I-287: 94.9%
Compound No. I-303: 87.3%
Compound No. I-308: 92.2%
Compound No. I-309: 93.1%
Compound No. I-330: 99%
Compound No. I-332: 87.2%
Compound No. I-340: 98.3%
Compound No. I-347: 92%
Compound No. I-348: 94.5%
Compound No. I-358: 88.4%
Compound No. I-397: 96.7%
Compound No. I-407: 92.9%
Compound No. I-409: 92.2%
Compound No. I-432: >99.9%

(Example 22: hERG test)

For the purpose of risk evaluation of QT interval extension of electrocardiogram, the activity on delayed rectification $K^+$ current ($I_{Kr}$), which plays an important role in a cardiac ventricle repolarization process, was examined using the HEK293 cell that was made to express human ether-a-go-go related gene (hERG) channel.

Using an automatic patch-clamp system (PatchXpress7000A, Axon Instruments Inc.), by a whole-cell patch-clamp method, after the cell was maintained at a membrane potential of -80 mV, $I_{Kr}$ induced upon applying depolarizing stimulation of +50 mV for 2 seconds and further applying repolarizing stimulation of -50 mV for 2 seconds was recorded. After generated electric current became stable, a test substance dissolved at an intended concentrationin extracellularfluid (137 mmol/L NaCl, 4 mmol/L KCl, 1.8 mmol /L $CaCl_2 \cdot 2H_2O$, 1 mmol/L $MgCl_2 \cdot 6H_2O$, 10 mmol/L glucose, 10 mmol/L HEPES (4-(2-hydroxyethyl)-1-piperazineethanesulfonic acid), pH = 7.4) was applied to the cell for 10 minutes under room temperature condition. From the obtained $I_{Kr}$, the absolute value of the maximum tail current was measured using analysis software (DataXpress ver. 1, Molecular Devices Corporation) and the value of the electric current in a maintained membrane potential as baseline. Moreover, an inhibition ratio for the maximum tail current prior to the application of the test substance was calculated and then compared with a group of media-application (0.1 % dimethylsulfoxide solution) to evaluate influence of the test substance on $I_{Kr}$.

(Results)

Compound No. I-89: <0.5%
Compound No. I-118: 8.4%
Compound No. I-160: -0.4%
Compound No. I-185: 8.3%
Compound No. I-200: 7.3%
Compound No. I-208: 2.7%
Compound No. I-230: 9.1%
Compound No. I-262: 1.6%
Compound No. I-279: 5%
Compound No. I-347: 6%

(Results and discussion)

As described above, the compound of the present invention exhibits excellent PI3K inhibitory activity, particularly, PI3Kγ inhibitory activity. The compound of the present invention also exhibits PI3Kα and PI3Kγ inhibitory activity. Accordingly, the pharmaceutical composition of the present invention may be used for the prophylaxis of and/or as a

therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases,andthelike),pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or used as a therapeutic agent for burn or traumatic inflammation.

(Example 23: Formulation example 1 Tablet)

A tablet consisting of the following constitution is produced by a conventional method.

| | |
|---|---|
| The compound of the present invention | 100 mg |
| Lactose | 60 mg |
| Potato starch | 30 mg |
| Polyvinyl alcohol | 2 mg |
| Magnesium stearate | 1 mg |
| Tar dye | minute amount |

(Example 24: Formulation example 2 Powder)

A powder consisting of the following constitution is produced by a conventional method.

| | |
|---|---|
| The compound of the present invention | 150 mg |
| Lactose | 280 mg |

(Example 25: Formulation example 3 Syrup)

Syrup consisting of the following constitution is produced by a conventional method.

| | |
|---|---|
| The compound of the present invention | 100 mg |
| Refined white sugar | 40 g |
| Ethyl p-hydroxybenzoate | 40 mg |
| Propyl p-hydroxybenzoate | 10 mg |
| Chocolate flavor | 0.1 cc |

Water was added to this to provide a total amount of 100 cc.

The present invention has been exemplified so far by referring to preferable embodiments of the present invention, but it should not be construed that the present invention is restricted by the embodiments of the present invention. It shouldbe understood that the scope of the present invention should be construed only by the claims. It would be understood that those skilled in the art can perform an invention practically equivalent to the present invention, based on the description of the present invention and technical common sense from the specific description of preferable embodiments of the present invention. It would be understood that the patents, patent applications and literature cited herein should be incorporated herein by reference to the present specification in their entire contents, similarly to the case where the contents themselves are described specifically herein.

The present application claims priority to Japanese Patent Application No. 2008-221935, which was filed in Japan, and which are herein incorporated by reference in its entirety.

INDUSTRIAL APPLICABILITY

The present invention provides medicaments for the treatment of phosphatidylinositol-3-kinase dependent diseases, a compound used therefor, a pharmaceutically acceptable salt thereof, or a solvate thereof.

## Claims

**1.** A compound represented by formula (I):

[Formula 1]

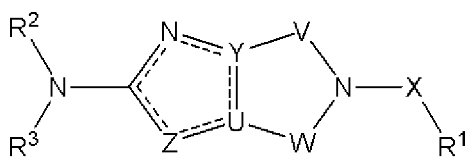

(I)

wherein

V is - $(CR^4R^5)_m$- or $-CR^6=CR^7-$;
W is a single bond, $-(CR^8R^9)_n-$ or -C (=O) -;
X is a single bond, -C(=O)-, $-(CR^{10}R^{11})_p-$, $-(CR^{12}R^{13})_p-C(=O)-$, $-SO_2-$ or -SO-;
a group represented by formula (G):

[Formula 2]

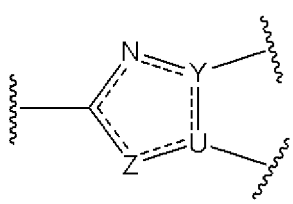

(G)

is selected from the following:

[Formula 3]

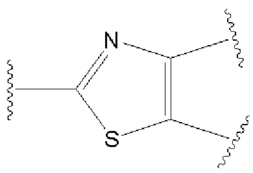

(G1) ,

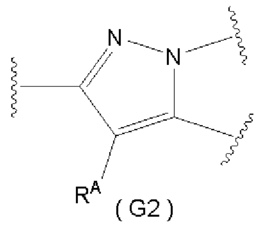

(G2) ,

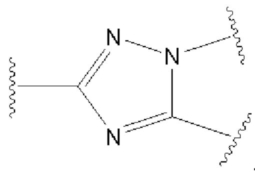

(G3) ,

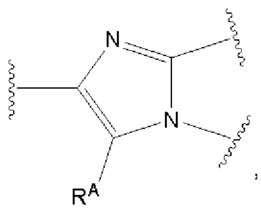

(G4) ,

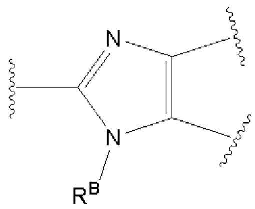

(G5)

or

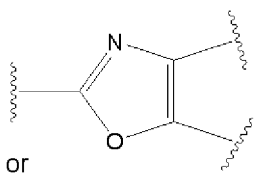

(G6) ,

$R^A$ is hydrogen, halogen, cyano, hydroxy, carboxy, substituted or unsubstituted alkyl, substituted or unsubstituted alkenyl, substituted or unsubstituted alkynyl, substituted or unsubstituted aryl, substituted or unsubstituted heteroaryl, substituted or unsubstituted alkoxy, substituted or unsubstituted aryloxy, substituted or unsubstituted cycloalkyloxy, substituted or unsubstituted heteroaryloxy, substituted or unsubstituted heterocyclyloxy, substituted or unsubstituted acyl, substituted or unsubstituted carbamoyl, substituted or unsubstituted amino, a group represented by the formula: $-SO-R^c$, a group represented by the formula: $-SO_2R^C$, or a group represented by the formula: $-SR^C$;

$R^B$ is hydrogen, substituted or unsubstituted alkyl, substituted or unsubstituted carbamoyl, or substituted or unsubstituted acyl;

$R^C$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^1$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl;

$R^2$ is hydrogen, substituted or unsubstituted alkyl, or substituted or unsubstituted acyl;

$R^3$ is hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-C(=O)-R^{14}$, or a group represented by the formula: $-C(=O)-NR^{15}R^{16}$; or

$R^2$ and $R^3$ may be taken together with the adjacent nitrogen atom to form a substituted or unsubstituted nitrogenated heterocycle;

$R^4$ to $R^{13}$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;

$R^{14}$ are each independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted acyl, or substituted or unsubstituted carbamoyl;
$R^{15}$ and $R^{16}$ are each independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, substituted or unsubstituted amino, or substituted or unsubstituted carbamoyl, or $R^{15}$ and $R^{16}$ may be taken together with the adjacent nitrogen atom to form a substituted or unsubstituted nitrogenated heterocycle;
m, n, and p are each independently an integer from 1 to 3; provided that when X is a single bond, $R^1$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, or substituted or unsubstituted heterocyclyl, and
when a group represented by formula (G) is the group represented by formula (G1),

(i) $R^3$ is a group representedby the formula: -C (=O) -$R^{14}$ wherein $R^{14}$ is defined as above; X is a single bond; $R^1$ is substituted or unsubstituted aryl, substituted or unsubstituted cycloalkyl, substituted or unsubstituted cycloalkenyl, substituted or unsubstituted heteroaryl, or substituted or unsubstituted heterocyclyl; or
(ii) $R^3$ is a group represented by the formula: -C (=O) -$NR^{15}R^{16}$ wherein $R^{15}$ and $R^{16}$ are defined as above; and

the compounds represented by the following formulae are excluded:

[Formula 4]

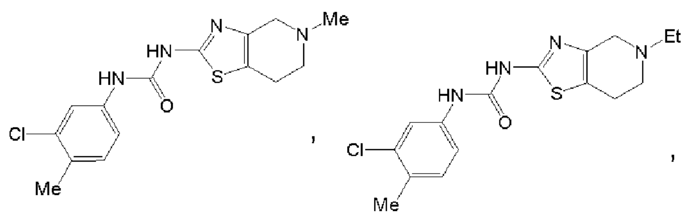

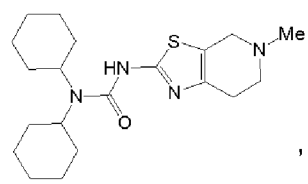
,

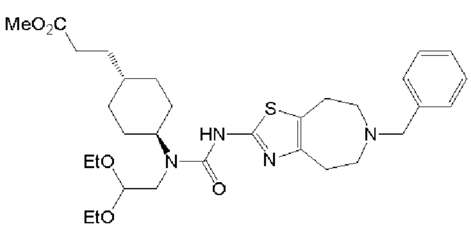
,

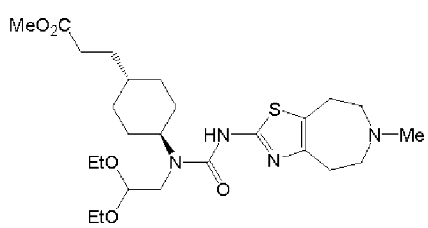
,

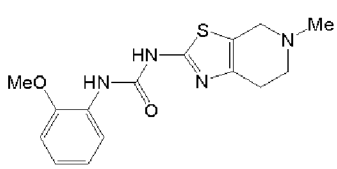
and
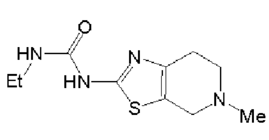
,

or a pharmaceutically acceptable salt thereof, or a solvate thereof.

2. The compound according to claim 1, wherein a group represented by formula (G) is the group represented by formula (G1) or (G2); $R^3$ is substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: -C(=O) -$R^{14}$ wherein $R^{14}$ is defined as in claim 1, or a group represented by the formula: -C(=O)-$NR^{15}R^{16}$ wherein $R^{15}$ and $R^{16}$ are defined as in claim 1, or a pharmaceutically acceptable salt thereof, or

a solvate thereof.

3. The compound according to claim 1 or 2, wherein a group represented by formula (G) is the group represented by formula (G1),
or a pharmaceutically acceptable salt thereof, or a solvate thereof.

4. The compound according to claim 1 or 2, wherein a group represented by formula (G) is the group represented by formula (G2),
or a pharmaceutically acceptable salt thereof, or a solvate thereof.

5. The compound according to claim 4, wherein $R^A$ is hydrogen, or a pharmaceutically acceptable salt thereof, or a solvate thereof.

6. The compound according to claim 3, wherein V is - $(CR^4R^5)m$-wherein $R^4$, $R^5$, and m are defined as in claim 1; and W is $-(CR^8R^9)_n-$ wherein $R^8$, $R^9$, and n are defined as in claim 1, or a pharmaceutically acceptable salt thereof, or a solvate thereof.

7. The compound according to claim 4, wherein V is $-CR^6=CR^7-$wherein $R^6$ and $R^7$ are defined as in claim 1; and W is a single bond,
or a pharmaceutically acceptable salt thereof, or a solvate thereof.

8. The compound according to claim 4, wherein V is - $(CR^4R^5)m$-wherein $R^4$, $R^5$, and m are defined as in claim 1; and W is a single bond or $-(CR^8R^9)_n-$ wherein $R^8$, $R^9$, and n are defined as in claim 1,
or a pharmaceutically acceptable salt thereof, or a solvate thereof.

9. The compound according to claim 6 or 8, wherein $R^4$, $R^5$, $R^8$, and $R^9$ are each hydrogen; m is 2; and n is 1,
or a pharmaceutically acceptable salt thereof, or a solvate thereof.

10. The compound according to claim 7, wherein $R^6$ and $R^7$ are each hydrogen,
or a pharmaceutically acceptable salt thereof, or a solvate thereof.

11. The compound according to claim 1 or 2, wherein X is a single bond or -C(=O)-,
or a pharmaceutically acceptable salt thereof, or a solvate thereof.

12. The compound according to claim 1 or 2, wherein $R^1$ is substituted or unsubstituted alkyl, substituted or unsubstituted aryl, substituted or unsubstituted cycloalkyl, substituted or unsubstituted heteroaryl, substituted or unsubstituted heterocyclyl, substituted or unsubstituted alkoxy,substitutedor unsubstituted aryloxy,orsubstituted or unsubstituted amino,
or a pharmaceutically acceptable salt thereof, or a solvate thereof.

13. The compound according to claim 1 or 2, wherein $R^2$ is hydrogen,
or a pharmaceutically acceptable salt thereof, or a solvate thereof.

14. The compound according to claim 1 or 2, wherein $R^3$ is a group represented by the formula: $-C(=O)-NR^{15}R^{16}$ wherein $R^{15}$ and $R^{16}$ are defined as in claim 1,
or a pharmaceutically acceptable salt thereof, or a solvate thereof.

15. The compound according to claim 14, wherein $R^{15}$ and $R^{16}$ are each independently hydrogen, substituted or unsubstituted alkyl, substituted or unsubstituted aryl, substituted or unsubstituted cycloalkyl, substituted or unsubstituted heteroaryl, or substituted or unsubstituted heterocyclyl; or $R^{15}$ and $R^{16}$ are taken together with the adjacent nitrogen atom to form a substituted or unsubstituted nitrogenated heterocycle,
or a pharmaceutically acceptable salt thereof, or a solvate thereof.

16. The compound according to claim 1 or 2, wherein $R^3$ is a group represented by the formula: $-C(=O)-R^{14}$ wherein $R^{14}$ is defined as in claim 1,
or a pharmaceutically acceptable salt thereof, or a solvate thereof.

17. The compound according to claim 16, wherein $R^{14}$ is substituted or unsubstituted alkyl, substituted or unsubstituted

aryl, substituted or unsubstituted heteroaryl, substituted or unsubstituted heterocyclyl, substituted or unsubstituted cycloalkyl, substituted or unsubstituted alkoxy, or substituted or unsubstituted acyl,
or a pharmaceutically acceptable salt thereof, or a solvate thereof.

**18.** A pharmaceutical composition containing the compound according to any of claims 1 to 17, or a pharmaceutically acceptable salt thereof, or a solvate thereof.

**19.** The pharmaceutical composition according to claim 18, which is a phosphatidylinositol-3-kinase inhibitor.

**20.** A method for the therapy and/or prophylaxis of inflammation, **characterized by** administering the compound, or a pharmaceutically acceptable salt thereof, or a solvate thereof according to any of claims 1 to 17.

**21.** Use of the compound, or a pharmaceutically acceptable salt thereof, or a solvate thereof according to any of claims 1 to 17 for producing a therapeutic agent and/or a prophylactic agent for inflammation.

**22.** The compound, or a pharmaceutically acceptable salt thereof, or a solvate thereof according to any of claims 1 to 17 for the therapy and/or prophylaxis of inflammation.

**INTERNATIONAL SEARCH REPORT**

International application No.
PCT/JP2009/064808

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2005/021550 A1 (Sumitomo Pharmaceuticals Co., Ltd.), 10 March, 2005 (10.03.05), Page 175, referential examples 1, 2 & EP 1659123 A1 & US 2007/0082908 A1 & JP 2005-513519 A | 1,4,7,8, 10-13,22 |
| X | WO 2004/058266 A1 (MERCK & CO., INC.), 15 July, 2004 (15.07.04), Example 5 & EP 1583534 A1 & US 2006/0052382 A1 & JP 2006-513265 A | 1,11-13,16, 17,22 |
| X | JP 48-086885 A (Dr. Karl Thomae GmbH), 15 November, 1973 (15.11.73), Examples & GB 1412377 A & DE 2206385 A1 | 1,11-13,18, 22 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 September, 2009 (16.09.09) | 06 October, 2009 (06.10.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/064808

C (Continuation). DOCUMENTS CONSIDERED TO BE RELEVANT

| Category* | Citation of document, with indication, where appropriate, of the relevant passages | Relevant to claim No. |
|---|---|---|
| X | WO 2008/086047 A1 (BOEHRINGER INGELHEIM INTERNATIONAL GMBH), 17 July, 2008 (17.07.08), Claims; pages 33, 122, 163 to 166 (Family: none) | 1,11-13, 16-18,22 |
| X | JP 47-004875 A (Dr. Karl Thomae GmbH), 10 March, 1972 (10.03.72), Examples 56 to 64 & US 3804849 A & DE 2127267 A1 & GB 1321509 A | 1,11-13,22 |
| A | WO 2007/115931 A1 (BOEHRINGER INGELHEIM INTERNATIONAL GMBH), 18 October, 2007 (18.10.07), Full text & EP 2007382 A1 & US 2007/0238730 A1 & JP 2009-532415 A | 1-19,21,22 |
| A | WO 2007/115932 A1 (BOEHRINGER INGELHEIM INTERNATIONAL GMBH), 18 October, 2007 (18.10.07), Full text & EP 2007772 A1 & US 2007/0238746 A1 & JP 2009-532416 A | 1-19,21,22 |

Form PCT/ISA/210 (continuation of second sheet) (April 2007)

INTERNATIONAL SEARCH REPORT

International application No.
PCT/JP2009/064808

Continuation of A. CLASSIFICATION OF SUBJECT MATTER
(International Patent Classification (IPC))

C07D487/04(2006.01)i, A61K31/4188(2006.01)i, A61K31/422(2006.01)i, A61K31/427(2006.01)i, A61K31/428(2006.01)i, A61K31/429(2006.01)i, A61K31/437(2006.01)i, A61K31/4439(2006.01)i, A61K31/454(2006.01)i, A61K31/497(2006.01)i, A61K31/4985(2006.01)i, A61K31/506(2006.01)i, A61K31/53(2006.01)i, A61K31/5377(2006.01)i, A61P1/00(2006.01)i, A61P1/02(2006.01)i, A61P1/04(2006.01)i, A61P1/06(2006.01)i, A61P1/16(2006.01)i, A61P1/18(2006.01)i, A61P3/04(2006.01)i, A61P3/10(2006.01)i, A61P7/00(2006.01)i, A61P9/00(2006.01)i, A61P9/04(2006.01)i, A61P9/08(2006.01)i, A61P9/10(2006.01)i, A61P9/12(2006.01)i, A61P11/00(2006.01)i, A61P11/02(2006.01)i, A61P11/04(2006.01)i, A61P11/06(2006.01)i, A61P11/08(2006.01)i, A61P11/16(2006.01)i, A61P13/00(2006.01)i, A61P13/02(2006.01)i, A61P13/10(2006.01)i, A61P13/12(2006.01)i, A61P15/00(2006.01)i, A61P15/02(2006.01)i, A61P17/00(2006.01)i, A61P17/02(2006.01)i, A61P17/04(2006.01)i, A61P17/06(2006.01)i, A61P17/18(2006.01)i, A61P19/00(2006.01)i, A61P19/02(2006.01)i, A61P19/08(2006.01)i, A61P21/00(2006.01)i, A61P21/04(2006.01)i, A61P25/00(2006.01)i, A61P27/02(2006.01)i, A61P27/06(2006.01)i, A61P27/16(2006.01)i, A61P29/00(2006.01)i, A61P35/00(2006.01)i, A61P37/02(2006.01)i, A61P37/08(2006.01)i, A61P43/00(2006.01)i, C07D513/04(2006.01)i, C07D519/00(2006.01)i

(According to International Patent Classification (IPC) or to both national classification and IPC)

Continuation of B. FIELDS SEARCHED
Minimum documentation searched (International Patent Classification (IPC))

C07D487/04, A61K31/4188, A61K31/422, A61K31/427, A61K31/428, A61K31/429, A61K31/437, A61K31/4439, A61K31/454, A61K31/497, A61K31/4985, A61K31/506,
A61K31/53, A61K31/5377, A61P1/00, A61P1/02, A61P1/04, A61P1/06, A61P1/16,
A61P1/18, A61P3/04, A61P3/10, A61P7/00, A61P9/00, A61P9/04, A61P9/08, A61P9/10, A61P9/12, A61P11/00, A61P11/02, A61P11/04, A61P11/06, A61P11/08, A61P11/16, A61P13/00, A61P13/02, A61P13/10, A61P13/12, A61P15/00, A61P15/02, A61P17/00, A61P17/02, A61P17/04, A61P17/06, A61P17/18, A61P19/00, A61P19/02, A61P19/08, A61P21/00, A61P21/04, A61P25/00, A61P27/02, A61P27/06, A61P27/16, A61P29/00, A61P35/00, A61P37/02, A61P37/08, A61P43/00, C07D513/04, C07D519/00

Minimum documentation searched (classification system followed by classification symbols)

Form PCT/ISA/210 (extra sheet) (April 2007)

**INTERNATIONAL SEARCH REPORT**

International application No.
PCT/JP2009/064808

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 20
because they relate to subject matter not required to be searched by this Authority, namely:
Claim 20 includes the methods for treatment of the human body or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7145051 A
- JP 7029673 A
- JP 2006040279 A
- JP 2007115929 A
- JP 2007115933 A
- JP 2007115930 A
- JP 2007115931 A
- US 2006053323 A
- US 2005066145 A
- US 9604271 B
- US 9207849 B
- US 4289524 A
- US 2007115932 A
- US 2006040281 A
- WO 2006066174 A
- JP 2008221935 A


### Non-patent literature cited in the description


- **M. P. Wymann et al.** *Biochemical Society Transactions,* 2003, vol. 31, 275-280
- **Rueckle T. et al.** *NATURE REVIEWS DRUG DISCOVERY,* 2006, vol. 5, 903-918
- **Laffargue M. et al.** *Immunity,* 2002, vol. 16, 441-451
- **Hirsch E. et al.** *Science,* 2000, vol. 287, 1049-1053
- **Li Z. et al.** *Science,* 2000, vol. 287, 982-983
- **Sasaki T. et al.** *Science,* 2000, vol. 287, 1040-1046
- **Lupia E. et al.** *Am J Pathol.,* 2004, vol. 165, 2003-2011
- **Barber DF et al.** *Nat Med,* 2005, vol. 11, 933-935
- **Camps.** *Nat Med,* 2005, vol. 11, 936-943
- **Campbell et al.** *Circ Res.,* 2005, vol. 96, 197-206
- **Yadav M. et al.** *J Immunol.,* 2006, vol. 176, 5494-503
- *Japanese Journal of Clinical Immunology,* 2007, vol. 30 (5), 369-374
- **Ui MT et al.** *Trends Biochem. Sci.,* 1995, vol. 20, 303-307
- Labeled Compounds. Isotopes in the Physical and Biomedical Sciences. 1987, vol. 1
- *Synthetic Communications,* 1999, vol. 29 (2), 311-341
- *Heterocycles,* 2004, vol. 63 (7), 1555
- **Theodora W. Greene.** Protective Groups in Organic Synthesis. John Wiley & Sons, Inc, 1991
- **T. W. Green.** Protective Groups in Organic Synthesis. John Wiley & Sons Inc, 1991
- **T. W. Greene.** Protective Groups in Organic Synthesis. John Wiley & Sons Inc, 1991
- **R. C. Larock.** *Comprehensive Organic Transformations,* 1989